# EUROPÄISCHE PATENTSCHRIFT

(11) **EP 0 528 156 B1**
(45) Veröffentlichungstag und Bekanntmachung des Hinweises auf die Patenterteilung: **26.03.1997**
(21) Anmeldenummer: 92111324.7
(22) Anmeldetag: 03.07.1992
(51) Int. Cl.: C07D 307/60, C07D 307/94, C07D 307/68, C07D 409/12, C07D 407/12, C07F 9/655, A01N 43/08

(54) **3-Aryl-4-hydroxy-delta3-dihydrofuranon- und 3-Aryl-4-hydroxy-delta3-dihydrothiophenon-Derivate**
3-Aryl-4-hydroxy-delta3-dihydrofuranone and 3-aryl-4-hydroxy-delta3-dihydrothiophenone derivatives
Dérivés de 3-aryle-4-hydroxy-delta3-dihydrofuranone et 3-aryle-4-hydroxy-delta3-dihydrothiophénone

(30) Priorität: 16.07.1991 DE 4123532; 21.05.1992 DE 4216814
(43) Veröffentlichungstag der Anmeldung: 24.02.1993
(73) Patentinhaber: BAYER AG, 51368 Leverkusen (DE)
(72) Erfinder: Fischer, Reiner, Dr., W-4019 Monheim 2 (DE); Bretschneider, Thomas, Dr., W-5200 Siegburg (DE); Krüger, Bernd-Wieland, Dr., W-5060 Bergisch Gladbach 2 (DE); Bachmann, Jürgen, Dr., W-5090 Leverkusen 1 (DE); Erdelen, Christoph, Dr., W-5653 Leichlingen 1 (DE); Wachendorff-Neumann, Ulrike, Dr., W-4019 Monheim (DE); Santel, Hans-Joachim, Dr., W-5090 Leverkusen 1 (DE); Lürssen, Klaus, Dr., W-5060 Bergisch Gladbach 2 (DE); Schmidt, Robert R., Dr., W-5060 Bergisch Gladbach 2 (DE)

(56) Entgegenhaltungen:
- EP-A- 0 299 694
- EP-A- 0 423 482
- FR-A- 2 054 514
- CHEMICAL ABSTRACTS, vol. 69, no. 23, 2. Dezember 1968, Columbus, Ohio, US; abstract no. 94792j, K. SAKURAI ET AL. 'Antifungal studies on drugs. I. Antifungal activity of five-membered lactone derivatives.' Seite 8861 ;Spalte 2 ;
- JOURNAL OF THE CHEMICAL SOCIETY, PERKIN TRANSACTIONS 1. Nr. 8, 1985, LETCHWORTH GB Seiten 1567 - 1576 A.C. CAMPBELL ET AL. 'Synthesis of (E) and (Z)-Pulvinones'

## Beschreibung

Die vorliegende Erfindung betrifft die Verwendung von teilweise bekannten 3-Aryl-4-hydroxy-Δ³-dihydro-furanon- und 3-Aryl-4-hydroxy-Δ³-dihydrothiophenon-Derivaten, als Insektizide und Akarizide.

Es ist bekannt, daß bestimmte substituierte Δ³-Dihydrofuran-2-on-Derivate herbizide Eigenschaften besitzen (vgl. DE-A 4 014 420). Die Synthese der als Ausgangsverbindungen verwendeten Tetronsäurederivate (wie z.B. 3-(2-Methyl-phenyl)-4-hydroxy-5-(4-fluorphenyl)-Δ³-dihydrofuranon-(2) ist ebenfalls in DE-A 4 014 420 beschrieben. Ähnlich strukturierte Verbindungen ohne Angabe einer insektiziden und/oder akariziden Wirksamkeit sind aus der Publikation Campbell et al. J. Chem. Soc.; Perkin Trans. 1 1985, (8) 1567-76 bekannt.

In der EP-A-0 299 694 werden bestimmte Furan-2(5H)-one mit fungiziden, insektiziden, akariziden und/oder herbiziden Eigenschaften beschrieben. Fungizide 5-Ring-Lactone sind aus Yakugaku Zasshi 1968, 88 (7), 919-24 bekannt.

Weiter sind aus der FR-A 2 054 514 Furan-2(5H)-one bekannt mit insektiziden, nematiziden, akariziden und/oder herbiziden Eigenschaften, die in der 3-Position des Furanonrings jedoch durch Wasserstoff oder einem Alkylrest substituiert sind.

Es wurden nun gefunden, daß die teilweise bekannten 3-Aryl-4-hydroxy-Δ³-dihydrofuranon-und 3-Aryl-4-hydroxy-Δ³-dihydrothiophenon-Derivate der allgemeinen Formel (I) 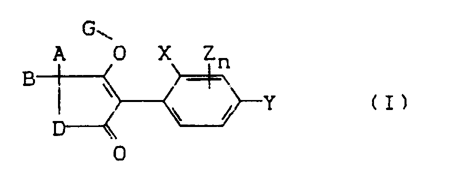 in welcher
- X: für C₁-C₆-Alkyl, Halogen, C₁-C₆-Alkoxy oder C₁-C₃-Halogenalkyl steht,
- Y: für Wasserstoff, C₁-C₆-Alkyl, Halogen; C₁-C₆-Alkoxy, C₁-C₃-Halogenalkyl steht,
- Z: für C₁-C₆-Alkyl, Halogen, C₁-C₆-Alkoxy steht,
- n: für eine Zahl von 0 bis 3 steht,
oder wobei die Reste X und Z gemeinsam mit dem Phenylrest an den sie gebunden sind, den Naphthalinrest der Formel 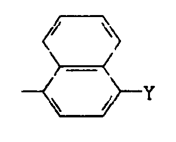 bilden,
in welchem Y die oben angegebene Bedeutung hat,
- A und B: gleich oder verschieden sind und für Wasserstoff oder gegebenenfalls durch Halogen substituiertes geradkettiges oder verzweigtes C₁-C₁₂-Alkyl, C₃-C₈-Alkenyl, C₃-C₈-Alkinyl, C₁-C₁₀-Alkoxy-C₂-C₈-alkyl, C₁-C₈-Polyalkoxy-C₂-C₈-alkyl, C₁-C₁₀-Alkylthio-C₂-C₈-alkyl, Cycloalkyl mit 3 bis 8 Ringatomen, das durch Sauerstoff und/oder Schwefel unterbrochen sein kann oder gegebenenfalls durch Halogen, C₁-C₆-Alkyl, C₁-C₆-Halogenalkyl, C₁-C₆-Alkoxy, C₁-C₆-Halogenalkoxy, Nitro substituiertes Phenyl oder Phenyl-C₁-C₆-alkyl steht,
oder worin
- A und B: gemeinsam mit dem Kohlenstoffatom, an das sie gebunden sind, einen gesättigten oder ungesättigten, gegebenenfalls durch Sauerstoff und/oder Schwefel unterbrochenen und gegebenenfalls durch Halogen, C₁-C₆-Alkyl, C₁-C₆-Alkoxy, C₁-C₄-Halogenalkyl, C₁-C₄-Halogenalkoxy, C₁-C₄-Alkylthio oder gegebenenfalls substituiertes Phenyl substituierten oder gegebenenfalls benzokondensierten 3- bis 8-gliedrigen Ring bilden,
- D: für Sauerstoff oder Schwefel steht,
- G: für Wasserstoff (a) oder für die Gruppen 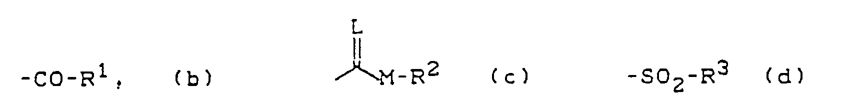 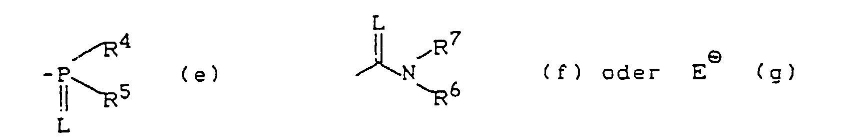 steht,
in welchen
- E^{⊕}: für ein Metallionäquivalent oder ein Ammoniumion steht,
- L und M: für Sauerstoff und/oder Schwefel steht,
- R¹: für gegebenenfalls durch Halogen substituiertes: C₁-C₂₀-Alkyl, C₂-C₂₀-Alkenyl, C₁-C₈-Alkoxy-C₂-C₈-alkyl, C₁-C₈-Alkylthio-C₂-C₈-alkyl, C₁-C₈-Polyalkoxyl-C₂-C₈-alkyl oder Cycloalkyl mit 3-8 Ringatomen, das durch Sauerstoff- und/oder Schwefelatome unterbrochen sein kann, steht,
für gegebenenfalls durch Halogen, Nitro, C₁-C₆-Alkyl, C₁-C₆-Alkoxy, C₁-C₆-Halogenalkyl, C₁-C₆-Halogenalkoxy-substituiertes Phenyl steht;
für gegebenenfalls durch Halogen, C₁-C₆-Alkyl, C₁-C₆-Alkoxy, C₁-C₆-Halogenalkyl, C₁-C₆-Halogenalkoxysubstituiertes Phenyl-C₁-C₆-alkyl steht,
für gegebenenfalls durch Halogen und/oder C₁-C₆-Alkyl substituiertes Pyridyl, Pyrimidyl, Thiazolyl und Pyrazolyl steht,
für gegebenenfalls durch Halogen und/oder C₁-C₆-Alkylsubstituiertes Phenoxy-C₁-C₆-alkyl steht,
- R²: für gegebenenfalls durch Halogen substituiertes C₁-C₂₀-Alkyl, C₂-C₂₀-Alkenyl, C₁-C₈-Alkoxy-C₂-C₈-alkyl, C₁-C₈-Polyalkoxy-C₂-C₈-alkyl steht,
für gegebenenfalls durch Halogen, Nitro, C₁-C₆-Alkyl, C₁-C₆-Alkoxy, C₁-C₆-Halogenalkylsubstituiertes Phenyl oder Benzyl steht,
- R³, R⁴ und R⁵: unabhängig voneinander für gegebenenfalls durch Halogen substituiertes C₁-C₈-Alkyl, C₁-C₈-Alkoxy, C₁-C₈-Alkylamino, Di-(C₁-C₈)-Alkylamino, C₁-C₈-Alkylthio, C₂-C₅-Alkenylthio, C₂-C₅-Alkinylthio, C₃-C₇-Cycloalkylthio, für gegebenenfalls durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl, C₁-C₄-Halogenalkyl substituiertes Phenyl, Phenoxy oder Phenylthio stehen,
- R⁶ und R⁷: unabhängig voneinander für gegebenenfalls durch Halogen substituiertes C₁-C₂₀-Alkyl, C₁-C₂₀-Alkoxy, C₂-C₈-Alkenyl, C₁-C₂₀-Alkoxy-C₁-C₂₀-alkyl, für gegebenenfalls durch Halogen, C₁-C₂₀-Halogenalkyl, C₁-C₂₀-Alkyl oder C₁-C₂₀-Alkoxy substituiertes Phenyl, für gegebenenfalls durch Halogen, C₁-C₂₀-Alkyl, C₁-C₂₀-Halogenalkyl oder C₁-C₂₀-Alkoxy substituiertes Benzyl steht oder zusammen für einen gegebenenfalls durch Sauerstoff unterbrochenen C₂-C₆-Alkylenring stehen,
mit Ausnahme folgender Verbindungen:
3-(2-Chlorphenyl)-4-hydroxy-Δ³-dihydrofuranon-2,
3-(2-Methoxyphenyl)-4-hydroxy-Δ³-dihydrofuranon-2,
3-(2-Fluorphenyl)-4-hydroxy-Δ³-dihydrofuranon-2;
sowie die enantiomerenreinen Formen von Verbindungen der Formel (I) sehr gut zur Bekämpfung von Insekten und Spinnentieren geeignet sind.

Bevorzugt verwendbar sind Verbindungen der Formel (I), in welcher
- X: für C₁-C₄-Alkyl, Halogen, C₁-C₄-Alkoxy oder C₁-C₂-Halogenalkyl steht,
- Y: für Wasserstoff, C₁-C₆-Alkyl, Halogen; C₁-C₄-Alkoxy, C₁-C₂-Halogenalkyl steht,
- Z: für C₁-C₄-Alkyl, Halogen, C₁-C₄-Alkoxy steht,
- n: für eine Zahl von 0 bis 3 steht,
oder wobei die Reste X und Z gemeinsam mit dem Phenylrest an den sie gebunden sind, den Naphthalinrest der Formel 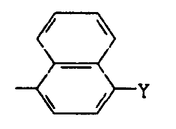 bilden,
in welchem Y die oben angegebene Bedeutung hat,
- A und B: gleich oder verschieden sind und für Wasserstoff, gegebenenfalls durch Halogen substituiertes geradkettiges oder verzweigtes C₁-C₁₀-Alkyl, C₃-C₆-Alkenyl, C₃-C₆-Alkinyl, C₁-C₈-Alkoxy-C₂-C₆-alkyl, C₁-C₆-Polyalkoxy-C₂-C₆-alkyl, C₁-C₈-Alkylthio-C₂-C₆-alkyl, Cycloalkyl mit 3 bis 7 Ringatomen, das durch 1-2 Sauerstoff- und/oder Schwefelatome unterbrochen sein kann oder gegebenenfalls durch Halogen-, C₁-C₄-Alkyl, C₁-C₄-Haloalkyl, C₁-C₄-Alkoxy, Nitro substituiertes Phenyl oder Phenyl-C₁-C₄-alkyl stehen,
oder worin
- A und B: gemeinsam mit dem Kohlenstoffatom, an das sie gebunden sind, einen gesättigten oder ungesättigten, gegebenenfalls durch Sauerstoff und/oder Schwefel unterbrochen und gegebenenfalls durch Halogen, C₁-C₅-Alkyl, C₁-C₅-Alkoxy, C₁-C₃-Halogenalkyl, C₁-C₄-Halogenalkoxy, C₁-C₃-Alkylthio oder gegebenenfalls durch Halogen, Alkyl, Alkoxy substituiertes Phenyl substituierten oder gegebenenfalls benzokondensierten 3- bis 8-gliedrigen Ring bilden,
- D: für Sauerstoff oder Schwefel steht,
- G: für Wasserstoff (a) oder für die Gruppen 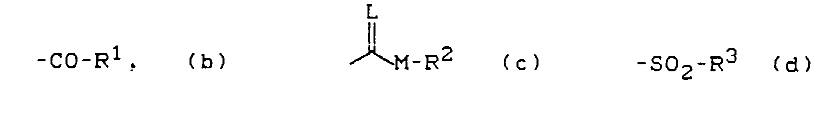 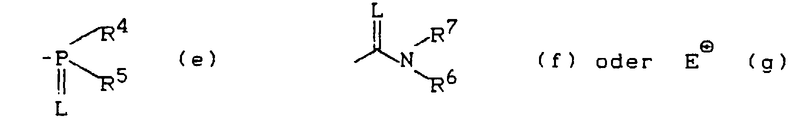 steht,
in welchen
E^{⊕} für ein Metallionäquivalent oder ein Ammoniumion steht L und M jeweils für Sauerstoff und/oder Schwefel steht,
- R¹: für gegebenenfalls durch Halogen substituiertes C₁-C₁₆-Alkyl, C₂-C₁₆-Alkenyl, C₁-C₆-Alkoxy-C₂-C₆-alkyl, C₁-C₁₆-Alkylthio-C₂-C₆-alkyl, C₁,C₆-Polyalkoxy-C₂-C₆-alkyl und Cycloalkyl mit 3-7 Ringatomen, das durch 1-2 Sauerstoff- und/oder Schwefelatome unterbrochen sein kann steht,
für gegebenenfalls durch Halogen, Nitro, C₁-C₄-Alkyl, C₁-C₄-Alkoxy, C₁-C₃-Halogenalkyl, C₁-C₃-Halogenalkoxy substituiertes Phenyl steht,
für gegebenenfalls durch Halogen, C₁-C₄-Alkyl, C₁-C₄-Alkoxy, C₁-C₃-Halogenalkyl, C₁-C₃-Halogenalkoxy substituiertes Phenyl-C₁-C₄-alkyl steht,
für gegebenenfalls durch Halogen- und C₁-C₆-Alkylsubstituiertes Pyridyl, Pyrimidyl, Thiazolyl und Pyrazolyl steht,
für gegebenenfalls durch Halogen und C₁-C₄-Alkylsubstituiertes Phenoxy-C₁-C₅-alkyl steht,
- R²: für gegebenenfalls durch Halogen substituiertes: C₁-C₁₆-Alkyl, C₂-C₁₆-Alkenyl, C₁-C₁₆-Alkox-C₂-C₆-alkyl, C₁-C₆-Polyalkoxy-C₂-C₆-alkyl steht,
für gegebenenfalls durch Halogen, Nitro, C₁-C₄-Alkyl, C₁-C₃-Alkoxy, C₁-C₃-Halogenalkyl substituiertes Phenyl oder Benzyl steht,
- R³, R⁴ und R⁵: unabhängig voneinander für gegebenenfalls durch Halogen substituiertes C₁-C₆-Alkyl, C₁-C₆-Alkoxy, C₁-C₆-Alkylamino, Di-(C₁-C₆)-Alkylamino, C₁-C₆-Alkylthio, C₃-C₄-Alkenylthio, C₂-C₄-Alkinylthio, C₃-C₆-Cycloalkylthio, für gegebenenfalls durch Fluor, Chlor, Brom, Nitro, Cyano, C₁-C₃Alkoxy, C₁-C₃-Halogenalkoxy, C₁-C₃-Alkylthio, C₁-C₃ -Halogenalkylthio, C₁-C₃-Alkyl, C₁-C₃-Halogenalkyl substiutiertes Phenyl, Phenoxy oder Phenylthio stehen,
- R⁶ und R⁷: unabhängig voneinander für gegebenenfalls durch Halogen substituiertes C₁-C₂₀-Alkyl, C₁-C₂₀-Alkoxy, C₂-C₈-Alkenyl, C₁-C₂₀-Alkoxy-C₁-C₂₀-alkyl, für gegebenenfalls durch Halogen, C₁-C₅-Halogenalkyl, C₁-C₅-Alkyl oder C₁-C₅-Alkoxy substituiertes Phenyl, für gegebenenfalls durch Halogen, C₁-C₅-Alkyl, C₁-C₅-Halogenalkyl oder C₁-C₅-Alkoxy substituiertes Benzyl steht,
mit Ausnahme folgender Verbindungen:
3-(2-Chlorphenyl)-4-hydroxy-Δ³-dihydrofuranon-2,
3-(2-Methoxyphenyl)-4-hydroxy-Δ³-dihydrofuranon-2,
3-(2-Fluorphenyl)-4-hydroxy-Δ³-dihydrofuranon-2,
sowie die enantiomerenreinen Formen von Verbindungen der Formel (I).

Besonders bevorzugt verwendbar sind Verbindungen der Formel (I), in welcher
- X: Methyl, Ethyl, Propyl, i-Propyl, Fluor, Chlor, Brom, Methoxy, Ethoxy und Trifluormethyl steht,
- Y: für Wasserstoff, Methyl, Ethyl, Propyl, i-Propyl, Butyl, i-Butyl, tert.-Butyl, Fluor, Chlor, Brom, Methoxy, Ethoxy und Trifluormethyl steht,
- Z: für Methyl, Ethyl, i-Propyl, Butyl, i-Butyl, tert.-Butyl, Fluor, Chlor, Brom, Methoxy und Ethoxy steht,
- n: für eine Zahl von 0 bis 3 steht,
oder wobei die Reste X und Z gemeinsam mit dem Phenylrest an den sie gebunden sind, den Rest der Formel 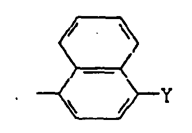 bilden,
in welchem Y die oben angegebene Bedeutung hat,
- A und B: gleich oder verschieden sind und für Wasserstoff, gegebenenfalls durch Halogen substituiertes geradkettiges oder verzweigtes C₁-C₈-Alkyl, C₃-C₄-Alkenyl, C₃-C₄-Alkinyl, C₁-C₆-Alkoxy-C₂-C₄-alkyl, C₁-C₄-Polyalkoxy-C₂-C₄-alkyl, C₁-C₆-Alkylthio-C₂-C₄-alkyl, Cycloalkyl mit 3 bis 6 Ringatomen, das durch 1-2 Sauerstoff- und/oder Schwefelatome unterbrochen sein kann oder gegebenenfalls durch Fluor, Chlor, Brom, Methyl, Ethyl, Propyl, iso-Propyl, Methoxy, Ethoxy, Trifluormethyl, Nitro substituiertes Phenyl oder Phenyl-C₁-C₃-alkyl stehen,
oder worin
- A und B: gemeinsam mit dem Kohlenstoffatom, an das sie gebunden sind, einen gesättigten oder ungesättigten, gegebenenfalls durch Sauerstoff und/oder Schwefel unterbrochenen und gegebenenfalls durch Fluor, Chlor, C₁-C₄-Alkyl, C₁-C₄-Alkoxy, Trifluormethyl, C₁-C₂-Alkylthio oder gegebenenfalls durch Fluor, Chlor, Methyl, Methoxy substituiertes Phenyl substituierten oder gegebenenfalls benzokondensierten 3- bis 8-gliedrigen Ring bilden,
- D: für Sauerstoff oder Schwefel steht,
- G: für Wasserstoff (a) oder für die Gruppen 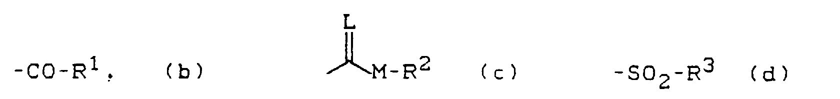 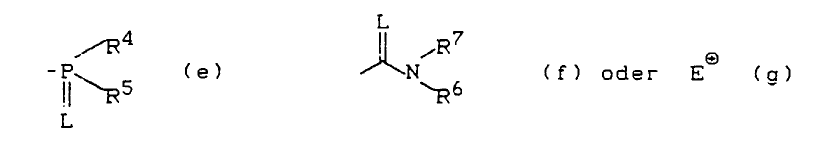 steht,
in welchen
- E^{⊕}: für ein Metallionäquivalent oder ein Ammoniumion steht,
- L und M: für Sauerstoff und/oder Schwefel steht,
- R¹: für gegebenenfalls durch Fluor oder Chlor substituiertes C₁-C₁₄-Alkyl, C₂-C₁₄-Alkenyl, C₁-C₄-Alkoxy-C₂-C₆-alkyl, C₁-C₄-Alkylthio-C₂-C₆-alkyl, C₁-C₄-Polyalkoxy-C₂-C₄-alkyl und Cycloalkyl mit 3-6 Ringatomen, das durch 1-2 Sauerstoff- und/oder Schwefelatome unterbrochen sein kann, steht,
für gegebenenfalls durch Fluor, Chlor, Brom, Methyl, Ethyl, Propyl, i-Propyl, Methoxy, Ethoxy, Trifluormethyl, Trifluormethoxy, Nitro substituiertes Phenyl steht,
für gegebenenfalls durch Fluor, Chlor, Brom, Methyl, Ethyl, Propyl, i-Propyl, Methoxy, Ethoxy, Trifluormethyl, Trifluormethoxy substituiertes Phenyl-C₁-C₃-alkyl steht,
für gegebenenfalls durch Fluor, Chlor, Brom, Methyl, Ethyl substituiertes Pyridyl, Pyrimidyl, Thiazolyl und Pyrazolyl steht,
für gegebenenfalls durch Fluor, Chlor, Methyl, Ethyl-substituiertes Phenoxy-C₁-C₄-alkyl steht,
- R²: für gegebenenfalls durch Fluor oder Chlor substituiertes C₁-C₁₄-Alkyl, C₂-C₁₄-Alkenyl, C₁-C₄-Alkoxy-C₂-C₆-alkyl, C₁-C₄-Polyalkoxy-C₂-C₆-alkyl steht,
oder für gegebenenfalls durch Fluor, Chlor, Nitro, Methyl, Ethyl, Propyl, i-Propyl, Methoxy, Ethoxy, Trifluormethyl substituiertes Phenyl oder Benzyl steht,
- R³, R⁴ und R⁵: unabhängig voneinander für gegebenenfalls
durch Fluor oder Chlor substituiertes C₁-C₄-Alkyl, C₁-C₄-Alkoxy, C₁-C₄-Alkylamino, Di-(C₁-C₄-Alkyl)amino, C₁-C₄-Alkylthio, für gegebenenfalls durch Fluor, Chlor, Brom, Nitro, Cyano, C₁-C₂-Alkoxy, C₁-C₄-Fluoralkoxy, C₁-C₂-Chloralkoxy, C₁-C₂-Alkylthio, C₁-C₂-Fluoralkylthio, C₁-C₂-Chloralkylthio, C₁-C₃-Alkyl substituiertes Phenyl, Phenoxy oder Phenylthio stehen,
- R⁶ und R⁷: unabhängig voneinander für gegebenenfalls durch Fluor, Chlor, Brom substituiertes C₁-C₁₀-Alkyl, C₁-C₁₀-Alkoxy, C₁-C₁₀-Alkoxy-(C₁-C₁₀)alkyl, für gegebenenfalls durch Fluor, Chlor, Brom, C₁-C₂₀-Halogenalkyl, C₁-C₂₀-Alkyl oder C₁-C₄-Alkoxy substituiertes Phenyl, für gegebenenfalls durch Fluor, Chlor, Brom, C₁-C₄-Alkyl, C₁-C₄-Halogenalkyl oder C₁-C₄-Alkoxy substituiertes Benzyl steht,
mit Ausnahme folgender Verbindungen:
3-(2-Chlorphenyl)-4-hydroxy-Δ³-dihydrofuranon-2,
3-(2-Methoxyphenyl)-4-hydroxy-Δ³-dihydrofuranon-2,
3-(2-Fluorphenyl)-4-hydraxy-Δ³-dihydrofuranon-2,
sowie die enantiomerenreinen Formen von Verbindungen der Formel (I).

Neu sind die 3-Aryl-4-hydroxy-Δ³-dihydrofuranon-und 3-Aryl-4-hydroxy-Δ³-dihydrothiophenon-Derivate der allgemeinen Formel (I) 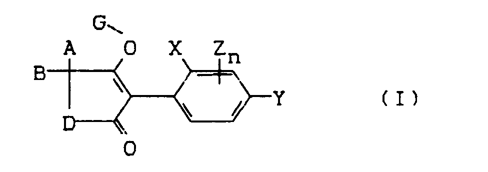 in welcher
- X: für C₁-C₆-Alkyl, Halogen, C₁-C₆-Alkoxy oder C₁-C₃-Halogenalkyl steht,
- Y: für Wasserstoff, C₁-C₆-Alkyl, Halogen, C₁-C₆-Alkoxy, C₁-C₃-Halogenalkyl steht,
- Z: für C₁-C₆-Alkyl, Halogen, C₁-C₆-Alkoxy steht,
- n: für eine Zahl von 0 bis 3 steht,
oder wobei die Reste X und Z gemeinsam mit dem Phenylrest an den sie gebunden sind, den Naphthalinrest der Formel 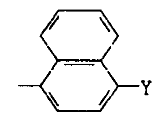 bilden,
in welchem Y die oben angegebene Bedeutung hat,
- A und B: gleich oder verschieden sind und für Wasserstoff oder gegebenenfalls durch Halogen substituiertes geradkettiges oder verzweigtes C₁-C₁₂-Alkyl, C₃-C₈-Alkenyl, C₃-C₈-Alkinyl, C₁-C₁₀-Alkoxy-C₂-C₈-alkyl, C₁-C₈-Polyalkoxy-C₂-C₈-alkyl, C₁-C₁₀-Alkylthio-C₂-C₈-alkyl, Cycloalkyl mit 3 bis 8 Ringatomen, das durch Sauerstoff und/oder Schwefel unterbrochen sein kann oder gegebenenfalls durch Halogen, C₁-C₆-Alkyl, C₁-C₆-Halogenalkyl, C₁-C₆-Alkoxy, C₁-C₆-Halogenalkoxy, Nitro substituiertes Phenyl oder Phenyl-C₁-C₆-alkyl steht,
oder worin
- A und B: gemeinsam mit dem Kohlenstoffatom, an das sie gebunden sind, einen gesättigten oder ungesättigten, gegebenenfalls durch Sauerstoff und/oder Schwefel unterbrochenen und gegebenenfalls durch Halogen, C₁-C₆-Alkyl, C₁-C₆-Alkoxy, C₁-C₄-Halogenalkyl, C₁-C₄-Halogenalkoxy, C₁-C₄-Alkylthio oder gegebenenfalls substituiertes Phenyl substituierten oder gegebenenfalls benzokondensierten 3- bis 8-gliedrigen Ring bilden,
- D: für Sauerstoff oder Schwefel steht,
- G: für Wasserstoff (a) oder für die Gruppen 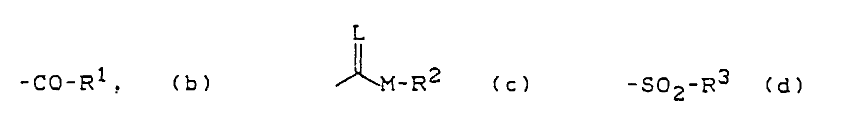 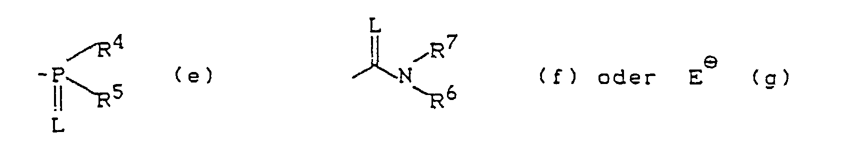 steht,
in welchen
- E^{⊕}: für ein Metallionäquivalent oder ein Ammoniumion steht,
- L und M: für Sauerstoff und/oder Schwefel steht,
- R¹: für gegebenenfalls durch Halogen substituiertes: C₁-C₂₀-Alkyl, C₂-C₂₀-Alkenyl, C₁-C₈-Alkoxy-C₂-C₈-alkyl, C₁-C₈-Alkylthio-C₂-C₈-alkyl, C₁-C₈-Polyalkoxyl-C₂-C₈-alkyl oder Cycloalkyl mit 3-8 Ringatomen, das durch Sauerstoff- und/oder Schwefelatome unterbrochen sein kann, steht,
für gegebenenfalls durch Halogen, Nitro, C₁-C₆-Alkyl, C₁-C₆-Alkoxy, C₁-C₆-Halogenalkyl, C₁-C₆-Halogenalkoxy-substituiertes Phenyl steht;
für gegebenenfalls durch halogen C₁-C₆-Alkyl, C₁-C₆-Alkoxy, C₁-C₆-Halogenalkyl, C₁-C₆-Halogenalkoxy substituiertes Phenyl-C₁-C₆-alkyl steht,
für gegebenenfalls durch Halogen und/oder C₁-C₆-Alkyl substituiertes Pyridyl, Pyrimidyl, Thiazolyl und Pyrazolyl steht,
für gegebenenfalls durch Halogen und/oder C₁-C₆-Alkylsubstituiertes Phenoxy-C₁-C₆-alkyl steht,
- R²: für gegebenenfalls durch Halogen substituiertes C₁-C₂₀-Alkyl, C₂-C₂₀-Alkenyl, C₁-C₈-Alkoxy-C₂-C₈-alkyl, C₁-C₈-Polyalkoxy-C₂-C₈-alkyl steht,
für gegebenenfalls durch Halogen, Nitro, C₁-C₆-Alkyl, C₁-C₆-Alkoxy, C₁-C₆-Halogenalkyl substituiertes Phenyl oder Benzyl steht,
- R³, R⁴ und R⁵: unabhängig voneinander für gegebenenfalls durch Halogen substituiertes C₁-C₈-Alkyl, C₁-C₈-Alkoxy, C₁-C₈-Alkylamino, Di-(C₁-C₈)-Alkylamino, C₁-C₈-Alkylthio, C₂-C₅-Alkenylthio, C₂-C₅-Alkinylthio, C₃-C₇-Cycloalkylthio, für gegebenenfalls durch Halogen, Nitro, Cyano, C₁-C₄-Alkoxy, C₁-C₄-Halogenalkoxy, C₁-C₄-Alkylthiol C₁-C₄-Halogenalkylthio, C₁-C₄-Alkyl, C₁-C₄-Halogenalkyl substituiertes Phenyl, Phenoxy oder Phenylthio stehen,
- R⁶ und R⁷: unabhängig voneinander für gegebenenfalls durch Halogen substituiertes C₁-C₂₀-Alkyl, C₁-C₂₀-Alkoxy, C₂-C₈-Alkenyl, C₁-C₂₀-Alkoxy-C₁-C₂₀-alkyl, für gegebenenfalls durch Halogen, C₁-C₂₀-Halogenalkyl, C₁-C₂₀-Alkyl oder C₁-C₂₀-Alkoxy substituiertes Phenyl, für gegebenenfalls durch Halogen, C₁-C₂₀-Alkyl, C₁-C₂₀-Halogenalkyl oder C₁-C₂₀-Alkoxy substituiertes Benzyl steht oder zusammen für einen gegebenenfalls durch Sauerstoff unterbrochenen C₂-C₆-Alkylenring stehen,
sowie die enantiomerenreinen Formen von Verbindungen der Formel (I),
mit Ausnahme folgender Verbindungen:
3-(2-Chlorphenyl)-4-hydroxy-Δ³-dihydrofuranon-2,
3-(2-Methoxyphenyl)-4-hydroxy-Δ³-dihydrofuranon-2,
3-(2-Fluorphenyl)-4-hydroxy-Δ³-dihydrofuranon-2,
und mit der Maßgabe, daß nicht gleichzeitig X für Halogen oder Halogenmethyl und 1 bis 3 gleichen oder verschiedenen Halogenatomen, Y für Wasserstoff und n für die Zahl 0 steht, wenn D für Sauerstoff und gleichzeitig R¹ für Alkylcarbonyl oder Halogenalkylcarbonyl steht und gleichzeitig A und B unabhängig voneinander für Wasserstoff, Alkyl oder jeweils unsubstituiertes oder einfach oder mehrfach, gleich oder verschieden substituiertes Phenyl oder Phenylalkyl stehen, wobei als Substituenten im Phenylteil ausgewählt sind: Nitro, Halogen, Alkyl, Alkoxy, Halogenalkyl oder Halogenalkoxy.

Unter Einbeziehung der verschiedenen Bedeutungen (a), (b), (c), (d), (e), (f) und (g) der Gruppe G der allgemeinen Formel (I) ergeben sich folgende hauptsächlichen neuen Strukturen (Ia) bis (Ig): 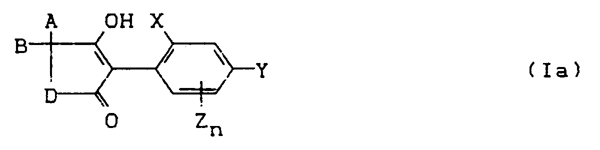 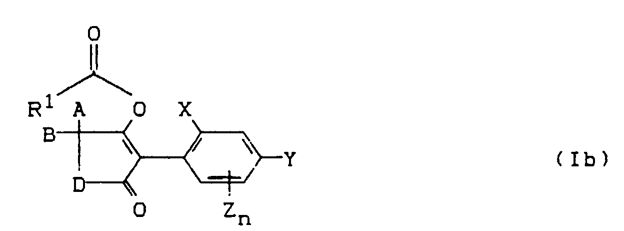 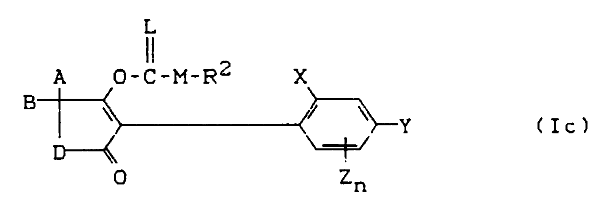 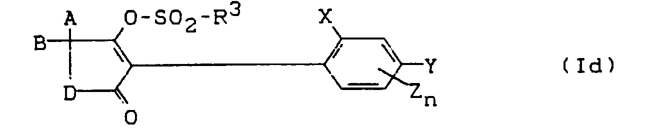 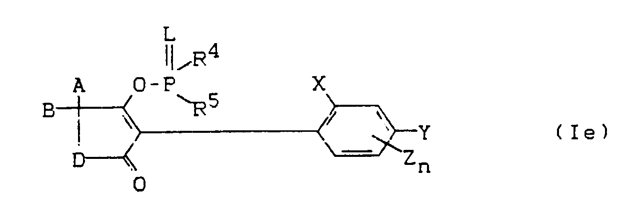 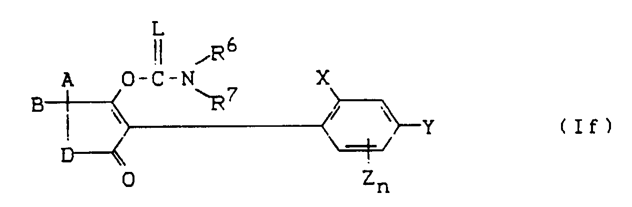 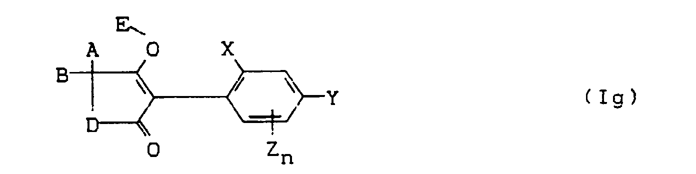 worin
A, B, D, E, L, M, X, Y, Zₙ, R¹, R², R³, R⁴, R⁵, R⁶ und R⁷ die oben für die neuen Verbindungen der Formal (I) angebenenen Bedeutungen besitzen.

Weiterhin wurde gefunden, daß man die neuen 3-Aryl-4-hydroxy-Δ³-dihydrofuranon- und 3-Aryl-4-hydroxy-Δ³-dihydrothiophenon-Derivate der Formel (Ia) 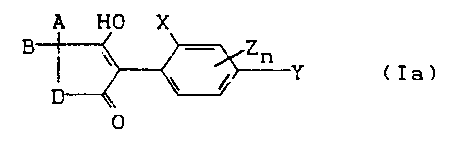 in welcher
A, B, D, X, Y, Z und n die oben angegebene Bedeutung haben,
erhält, wenn man
(A)
   Carbonsäureester der Formel (II) 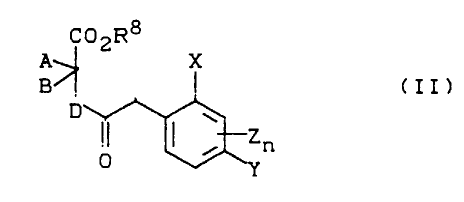 in welcher
   A, B, D, X, Y, Z und n die oben angegebene Bedeutung haben
   und
   - R⁸: für Alkyl steht,
   in Gegenwart eines Verdünnungsmittels und in Gegenwart einer Base intramolekular kondensiert.
(B)
   Außerdem wurde gefunden, daß man die neuen Verbindungen der Formel (Ib) 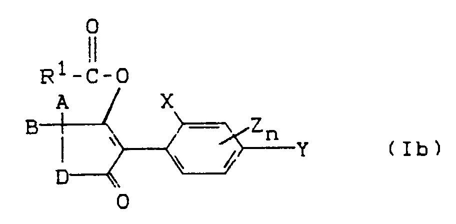 in welcher
   A, B, D, X, Y, Z, R¹ und n die oben angegebene Bedeutung haben,
   erhält, wenn man Verbindungen der Formel (Ia), 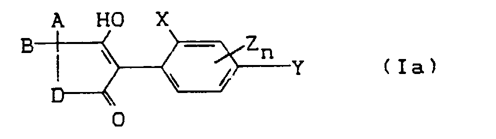 in welcher
   A, B, D, X, Y, Z und n die oben angegebene Bedeutung haben,
   α) mit Säurehalogeniden der allgemeinen Formel (III) 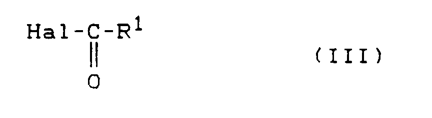 in welcher
      - R¹: die oben angegebene Bedeutung hat
      und
      - Hal: für Halogen, insbesondere Chlor und Brom steht,
      gegebenenfalls in Gegenwart eines Verdünnungsmittels und gegebenenfalls in Gegenwart eines Säurebindemittels umsetzt
      oder
   β) mit Carbonsäureanhydriden der allgemeinen Formel (IV)

      R¹-CO-O-CO-R¹ (IV)

      in welcher
      - R¹: die oben angegebene Bedeutung hat,
      gegebenenfalls in Gegenwart eines Verdünnungsmittels und gegebenenfalls in Gegenwart eines Säurebindemittels,
   umsetzt.
(C)
   Ferner wurde gefunden, daß man die neuen Verbindungen der Formel (Ic) 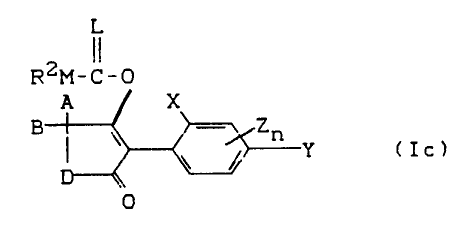 in welcher
   A, B, D, X, Y, Z, R² und n die oben angegebene Bedeutung haben,
   - L: für Sauerstoff
   und
   - M: für Sauerstoff oder Schwefel steht,
   erhält, wenn man Verbindungen der Formel (Ia) 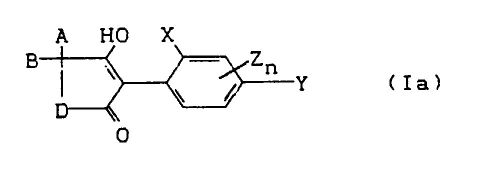 in welcher
   A, B, D, X, Y, Z und n die oben angegebene Bedeutung haben
   mit Chlorameisensäureester oder Chlorameisensäurethiolester der allgemeinen Formel (V)

   R²-M-CO-Cl (V)

   in welcher
   - R² und M: die oben angegebene Bedeutung haben,
   gegebenenfals in Gegenwart eines Verdünnungsmittels und gegebenenfalls in Gegenwart eines Säurebindemittels umsetzt.
D) Ferner wurde gefunden, daß man die neuen Verbindungen der Formel (Ic) 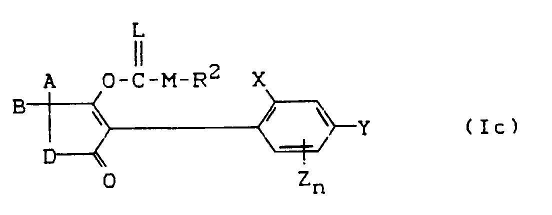 in welcher
   A, B, D, R², X, Y, Z und n die oben angegebene Bedeutung haben,
   - L: für Schwefel
   und
   - M: für Sauerstoff oder Schwefel steht,
   erhält, wenn man Verbindungen der Formel (Ia) 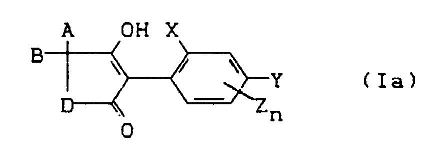 in welcher
   A, B, D, X, Y, Z und n die oben angegebene Bedeutung haben
   α) mit Chlormonothioameisensäureestern oder Chlordithioameisensäureestern der allgemeinen Formel (VI) 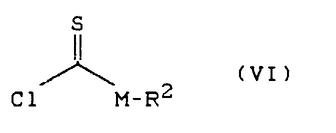 in welcher
      - M und R²: die oben angegebene Bedeutung haben
      gegebenenfalls in Gegenwart eines Verdünnungsmittels und gegebenenfalls in Gegenwart eines Säurebindemittels umsetzt,
      oder
   β) mit Schwefelkohlenstoff und anschließend mit Alkylhalogeniden der allgemeinen Formel (VII)

      R²-Hal (VII)

      in welcher
      - R²: die oben angegebene Bedeutung hat
      und
      - Hal: für Chlor, Brom, Jod
      steht, umsetzt.
E) Außerdem wurde gefunden, daß man die neuen Verbindungen der Formel (Id) 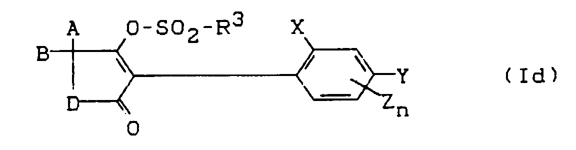 in welcher
   A, B, D, X, Y, Z, R³ und n die oben angegebene Bedeutung haben,
   erhält, wenn man Verbindungen der Formel (Ia) 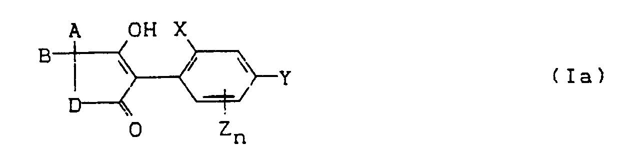 in welcher
   A, B, D, X, Y, Z und n die oben angegebene Bedeutung haben,
   mit Sulfonsäurechloriden der allgemeinen Formel (VIII)

   R³-SO₂-Cl (VIII)

   in welcher
   - R³: die oben angegebene Bedeutung hat
   gegebenenfalls in Gegenwart eines Verdünnungsmittels und gegebenenfalls in Gegenwart eines Säurebindemittels,
   umsetzt.
F) Weiterhin wurde gefunden, daß man die neuen Verbindungen der Formel (Ie) 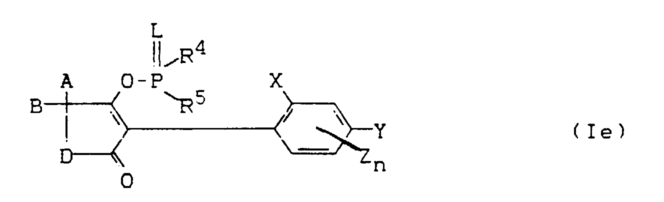 in welcher
   A, B, D, L, X, Y, Z, R⁴, R⁵ und n die oben angegebene Bedeutung haben,
   erhält, wenn man
   Verbindungen der Formel (Ia) 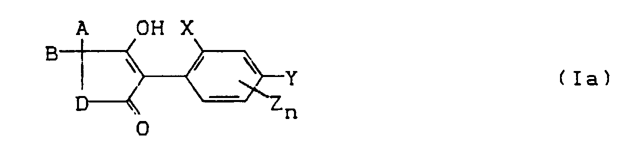 in welcher
   A, B, D, X, Y, Z und n die oben angegebene Bedeutung haben
   mit Phosphorverbindungen der allgemeinen Formel (IX) 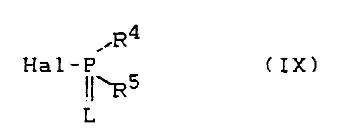 in welcher
   - L, R⁴ und R⁵: die oben angegebene Bedeutung haben
   und
   - Hal: für Halogen, insbesondere Chlor und Brom steht,
   gegebenenfalls in Gegenwart eines Verdünnungsmittels und gegebenenfalls in Gegenwart eines Säurebindemittels umsetzt.
G) Ferner wurde gefunden, daß man die neuen Verbindungen der Formel (If) 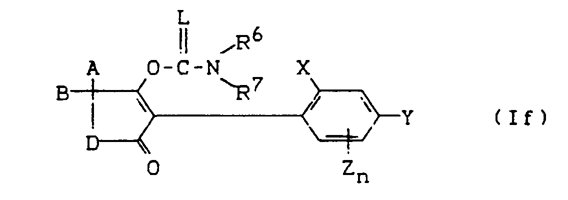 in welcher
   A, B, D, L, X, Y, Z, R⁶, R⁷ und n die oben angegebene Bedeutung haben,
   erhält, wenn man Verbindungen der Formel (Ia), 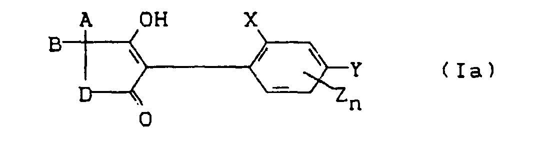 in welcher
   A, B, D, X, Y, Z und n die oben angegebene Bedeutung haben
   α) mit Isocyanaten der allgemeinen Formel (X)

      R⁶-N=C=O (X)

      in welcher
      - R⁶: die oben angegebene Bedeutung hat
      gegebenenfalls in Gegenwart eines Verdünnungsmittels und gegebenenfalls in Gegenwart eines Katalysators umsetzt,
      oder
   β) mit Carbamidsäurechloriden oder Thiocarbamidsäurechloriden der allgemeinen Formel (XI) 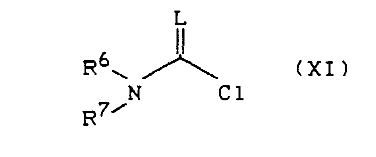 in welcher
      L, R⁶ und R⁷ die oben angegebene Bedeutung haben,
      gegebenenfalls in Gegenwart eines Verdünnungsmittels und gegebenenfalls in Gegenwart eines Säurebindemittels,
   umsetzt.
H) Weiterhin wurde gefunden, daß man die neuen Verbindungen der Formel (Ig) 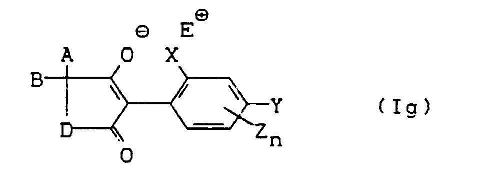 in welcher
   X, Y, Z, A, B, D und n die oben angegebene Bedeutung haben,
   und E^{⊕} für ein Metallionäquivalent oder für ein Ammoniumion steht,
   erhält, wenn man Verbindungen der Formel (Ia) 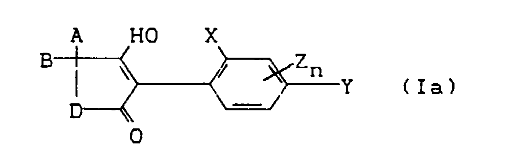 in welcher
   X, Y, Z, A, B, D und n die oben angegebene Bedeutung haben,
   mit Metallhydroxiden oder Aminen der allgemeinen Formeln (XII) und (XIII) 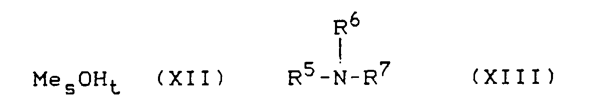 in welchen
   - Me: für ein- oder zweiwertige Metallionen
   - s und t: für die Zahl 1 oder 2 und
   - R⁵, R⁶ und R⁷: unabhängig voneinander für Wasserstoff und Alkyl
   stehen,
   gegebenenfalls in Gegenwart eines Verdünnungsmittels, umsetzt.

Weiterhin wurde gefunden, daß sich die neuen 3-Aryl-4-hydroxy-Δ³-dihydrofuranon- und 3-Aryl-4-hydroxy-Δ³-dihydrothiophenon-Derivate der Formel (I) durch hervorragende herbizide Wirkung auszeichnen.

Bevorzugt sind die neuen Verbindungen der Formel (I), in welcher
- X: für C₁-C₄-Alkyl, Halogen, C₁-C₄-Alkoxy oder C₁-C₂-Halogenalkyl steht,
- Y: für Wasserstoff, C₁-C₆-Alkyl, Halogen, C₁-C₄-Alkoxy, C₁-C₂-Halogenalkyl steht,
- Z: für C₁-C₄-Alkyl, Halogen, C₁-C₄-Alkoxy steht,
- n: für eine Zahl von 0 bis 3 steht,
oder wobei die Reste X und Z gemeinsam mit dem Phenylrest an den sie gebunden sind, den Naphthalinrest der Formel 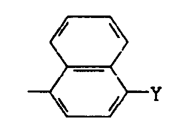 bilden, in welchem Y die oben angegebene Bedeutung hat,
- A und B: gleich oder verschieden sind und für Wasserstoff, gegebenenfalls durch Halogen substituiertes geradkettiges oder verzweigtes C₁-C₁₀-Alkyl, C₃-C₆-Alkenyl, C₃-C₆-Alkinyl, C₁-C₈-Alkoxy-C₂-C₆-alkyl, C₁-C₆-Polyalkoxy-C₂-C₆-alkyl, C₁-C₈-Alkylthio-C₂-C₆-alkyl, Cycloalkyl mit 3 bis 7 Ringatomen, das durch 1-2 Sauerstoff- und/oder Schwefelatome unterbrochen sein kann oder gegebenenfalls durch Halogen, C₁-C₄-Alkyl, C₁-C₄-Haloalkyl-, C₁-C₄-Alkoxy, Nitro substituiertes Phenyl oder Phenyl-C₁-C₄-alkyl stehen,
oder worin
- A und B: gemeinsam mit dem Kohlenstoffatom, an das sie gebunden sind, einen gesättigten oder ungesättigten, gegebenenfalls durch Sauerstoff und/oder Schwefel unterbrochen und gegebenenfalls durch Halogen, C₁-C₅-Alkyl, C₁-C₅-Alkoxy, C₁-C₃-Halogenalkyl, C₁-C₄-Halogenalkoxy, C₁-C₃-Alkylthio oder gegebenenfalls durch Halogen, Alkyl, Alkoxy substituiertes Phenyl substituierten oder gegebenenfalls benzokondensierten 3- bis 8-gliedrigen Ring bilden,
- D: für Sauerstoff oder Schwefel steht,
- G: für Wasserstoff (a) oder für die Gruppen 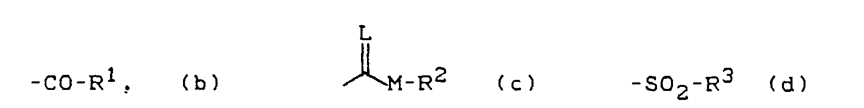 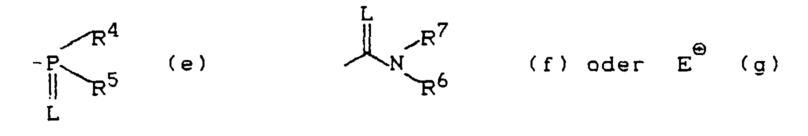 steht,
in welchen
E^{⊕} für ein Metallionäquivalent oder ein Ammoniumion steht L und M jeweils für Sauerstoff und/oder Schwefel steht,
- R¹: für gegebenenfalls durch Halogen substituiertes C₁-C₁₆-Alkyl, C₂-C₁₆-Alkenyl, C₁-C₆-Alkoxy-C₂-C₆-alkyl, C₁-C₁₆-Alkylthio-C₂-C₆-alkyl, C₁.C₆-Polyalkoxy-C₂-C₆-alkyl und Cycloalkyl mit 3-7 Ringatomen, das durch 1-2 Sauerstoff- und/oder Schwefelatome unterbrochen sein kann steht,
für gegebenenfalls durch Halogen, Nitro, C₁-C₄-Alkyl, C₁-C₄-Alkoxy, C₁-C₃-Halogenalkyl-, C₁-C₃-Halogenalkoxy substituiertes Phenyl steht,
für gegebenenfalls durch Halogen, C₁-C₄-Alkyl, C₁-C₄-Alkoxy-, C₁-C₃-Halogenalkyl-, C₁-C₃-Halogenalkoxy substituiertes Phenyl-C₁-C₄-alkyl steht,
für gegebenenfalls durch Halogen- und C₁-C₆-Alkylsubstituiertes Pyridyl, Pyrimidyl, Thiazolyl und Pyrazolyl steht,
für gegebenenfalls durch Halogen und C₁-C₄-Alkylsubstituiertes Phenoxy-C₁-C₅-alkyl steht,
- R²: für gegebenenfalls durch Halogen substituiertes: C₁-C₁₆-Alkyl, C₂-C₁₆-Alkenyl, C₁-C₁₆-Alkox-C₂-C₆-alkyl, C₁-C₆-Polyalkoxy-C₂-C₆-alkyl steht,
für gegebenenfalls durch Halogen, Nitro-, C₁-C₄-Alkyl, C₁-C₃-Alkoxy-, C₁-C₃-Halogenalkyl substituiertes Phenyl oder Benzyl steht,
- R³, R⁴ und R⁵: unabhängig voneinander für gegebenenfalls durch Halogen substituiertes C₁-C₆-Alkyl, C₁-C₆-Alkoxy, C₁-C₆-Alkylamino, Di-(C₁-C₆)-Alkylamino, C₁-C₆-Alkylthio, C₃-C₄-Alkenylthio, C₂-C₄-Alkinylthio, C₃-C₆-Cycloalkylthio, für gegebenenfalls durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl, C₁-C₃-Halogenalkyl substiutiertes Phenyl, Phenoxy oder Phenylthio stehen,
- R⁶ und R⁷: unabhängig voneinander für gegebenenfalls durch Halogen substituiertes C₁-C₂₀-Alkyl, C₁-C₂₀-Alkoxy, C₂-C₈-Alkenyl, C₁-C₂₀-Alkoxy-C₁-C₂₀-alkyl, für gegebenenfalls durch Halogen, C₁-C₅-Halogenalkyl, C₁-C₅-Alkyl oder C₁-C₅-Alkoxy substituiertes Phenyl, für gegebenenfalls durch Halogen, C₁-C₅-Alkyl, C₁-C₅-Halogenalkyl oder C₁-C₅-Alkoxy substituiertes Benzyl steht,
sowie die enantiomerenreinen Formen von Verbindungen der Formel (I),
mit Ausnahme folgender Verbindungen:
3-(2-Chlorphenyl)-4-hydroxy-Δ³-dihydrofuranon-2,
3-(2-Methoxyphenyl)-4-hydroxy-Δ³-dihydrofuranon-2,
3-(2-Fluorphenyl)-4-hydroxy-Δ³-dihydrofuranon-2,
und mit der Maßgabe, daß nicht gleichzeitig X für Halogen oder Halogenmethyl und 1 bis 3 gleichen oder verschiedenen Halogenatomen, Y für Wasserstoff und n für die Zahl 0 steht, wenn D für Sauerstoff und gleichzeitig R¹ für Alkylcarbonyl oder Halogenalkylcarbonyl steht und gleichzeitig A und B unabhängig voneinander für Wasserstoff, Alkyl oder jeweils unsubstituiertes oder einfach oder mehrfach, gleich oder verschieden substituiertes Phenyl oder Phenylalkyl stehen, wobei als Substituenten im Phenylteil ausgewählt sind: Nitro, Halogen, Alkyl, Alkoxy, Halogenalkyl oder Halogenalkoxy.

Ganz besonders bevorzugt sind die neuen Verbindungen der Formel (I), in welcher
- X: Methyl, Ethyl, Propyl, i-Propyl, Fluor, Chlor, Brom, Methoxy, Ethoxy und Trifluormethyl steht,
- Y: für Wasserstoff, Methyl, Ethyl, Propyl, i-Propyl, Butyl, i-Butyl, tert.-Butyl, Fluor, Chlor, Brom, Methoxy, Ethoxy und Trifluormethyl steht,
- Z: für Methyl, Ethyl, i-Propyl, Butyl, i-Butyl, tert.-Butyl, Fluor, Chlor, Brom, Methoxy und Ethoxy steht,
- n: für eine Zahl von 0 bis 3 steht,
oder wobei die Reste X und Z gemeinsam mit dem Phenylrest an den sie gebunden sind, den Rest der Formel 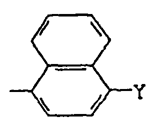 bilden, in welchem Y die oben angegebene Bedeutung hat,
- A und B: gleich oder verschieden sind und für Wasserstoff, gegebenenfalls durch Halogen substituiertes geradkettiges oder verzweigtes C₁-C₈-Alkyl, C₃-C₄-Alkenyl, C₃-C₄-Alkinyl, C₁-C₆-Alkoxy-C₂-C₄-alkyl, C₁-C₄-Polyalkoxy-C₂-C₄-alkyl, C₁-C₆-Alkylthio-C₂-C₄-alkyl, Cycloalkyl mit 3 bis 6 Ringatomen, das durch 1-2 Sauerstoff- und/oder Schwefelatome unterbrochen sein kann oder gegebenenfalls durch Fluor, Chlor, Brom, Methyl, Ethyl, Propyl, iso-Propyl, Methoxy, Ethoxy, Trifluormethyl, Nitro substituiertes Phenyl oder Phenyl-C₁-C₃-alkyl stehen,
oder worin
- A und B: gemeinsam mit dem Kohlenstoffatom, an das sie gebunden sind, einen gesättigten oder ungesättigten, gegebenenfalls durch Sauerstoff und/oder Schwefel unterbrochenen und gegebenenfalls durch Fluor, Chlor, C₁-C₄-Alkyl, C₁-C₄-Alkoxy, Trifluormethyl, C₁-C₂-Alkylthio oder gegebenenfalls durch Fluor, Chlor, Methyl, Methoxy substituiertes Phenyl substituierten oder gegebenenfalls benzokondensierten 3- bis 8-gliedrigen Ring bilden,
- D: für Sauerstoff oder Schwefel steht,
- G: für Wasserstoff (a) oder für die Gruppen 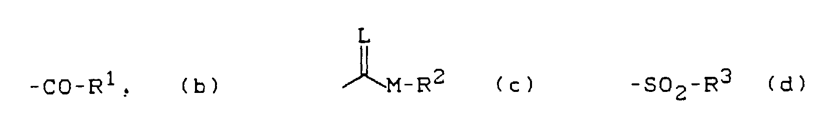 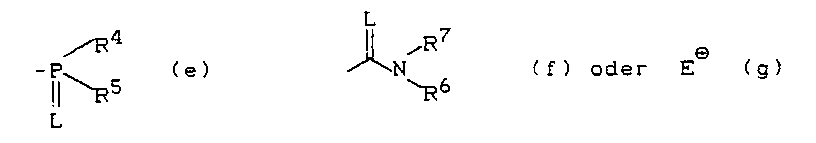 steht,
in welchen
- E^{⊕}: für ein Metallionäquivalent oder ein Ammoniumion steht,
- L und M: für Sauerstoff und/oder Schwefel steht,
- R¹: für gegebenenfalls durch Fluor oder Chlor substituiertes C₁-C₁₄-Alkyl, C₂-C₁₄-Alkenyl, C₁-C₄-Alkoxy-C₂-C₆-alkyl, C₁-C₄-Alkylthio-C₂-C₆-alkyl, C₁-C₄-Polyalkoxy-C₂-C₄-alkyl und Cycloalkyl mit 3-6 Ringatomen, das durch 1-2 Sauerstoff- und/oder Schwefelatome unterbrochen sein kann, steht,
für gegebenenfalls durch Fluor, Chlor, Brom, Methyl, Ethyl, Propyl, i-Propyl, Methoxy, Ethoxy, Trifluormethyl, Trifluormethoxy, Nitro substituiertes Phenyl steht,
für gegebenenfalls durch Fluor, Chlor, Brom, Methyl, Ethyl, Propyl, i-Propyl, Methoxy, Ethoxy, Trifluormethyl, Trifluormethoxy substituiertes Phenyl-C₁-C₃-alkyl steht,
für gegebenenfalls durch Fluor, Chlor, Brom, Methyl, Ethyl-substituiertes Pyridyl, Pyrimidyl, Thiazolyl und Pyrazolyl steht,
für gegebenenfalls durch Fluor, Chlor, Methyl, Ethyl substituiertes Phenoxy-C₁-C₄-alkyl steht,
- R²: für gegebenenfalls durch Fluor oder Chlor substituiertes C₁-C₁₄-Alkyl, C₂-C₁₄-Alkenyl, C₁-C₄-Alkoxy-C₂-C₆-alkyl, C₁-C₄-Polyalkoxy-C₂-C₆-alkyl steht,
oder für gegebenenfalls durch Fluor, Chlor, Nitro, Methyl, Ethyl, Propyl, i-Propyl, Methoxy, Ethoxy, Trifluormethyl substituiertes Phenyl oder Benzyl steht,
- R³, R⁴ und R⁵: unabhängig voneinander für gegebenenfalls
durch Fluor oder Chlor substituiertes C₁-C₄-Alkyl, C₁-C₄-Alkoxy, C₁-C₄-Alkylamino, Di-(C₁-C₄-Alkyl)amino, C₁-C₄-Alkylthio, für gegebenenfalls durch Fluor, Chlor, Brom, Nitro, Cyano, C₁-C₂-Alkoxy, C₁-C₄-Fluoralkoxy, C₁-C₂-Chloralkoxy, C₁-C₂-Alkylthio, C₁-C₂-Fluoralkylthio, C₁-C₂-Chloralkylthio, C₁-C₃-Alkyl substituiertes Phenyl, Phenoxy oder Phenylthio stehen,
- R⁶ und R⁷: unabhängig voneinander für gegebenenfalls
durch Fluor, Chlor, Brom substituiertes C₁-C₁₀-Alkyl, C₁-C₁₀-Alkoxy, C₁-C₁₀-Alkoxy-(C₁-C₁₀)alkyl, für gegebenenfalls durch Fluor, Chlor, Brom, C₁-C₂₀-Halogenalkyl, C₁-C₂₀-Alkyl oder C₁-C₄-Alkoxy substituiertes Phenyl, für gegebenenfalls durch Fluor, Chlor, Brom, C₁-C₄-Alkyl, C₁-C₄-Halogenalkyl oder C₁-C₄-Alkoxy substituiertes Benzyl steht,
sowie die enantionerenreinen Formen von Verbindungen der Formel (I),
mit Ausnahme folgender Verbindungen:
3-(2-Chlorphenyl)-4-hydroxy-Δ³-dihydrofuranon-2,
3-(2-Methoxyphenyl)-4-hydroxy-Δ³-dihydrofuranon-2,
3-(2-Fluorphenyl)-4-hydroxy-Δ³-dihydrofuranon-2,
und mit der Maßgabe, daß nicht gleichzeitig X für Halogen oder Halogenmethyl und 1 bis 3 gleichen oder verschiedenen Halogenatomen, Y für Wasserstoff und n für die Zahl 0 steht, wenn D für Sauerstoff und gleichzeitig R¹ für Alkylcarbonyl oder Halogenalkylcarbonyl steht und gleichzeitig A und B unabhängig voneinander für Wasserstoff, Alkyl oder jeweils unsubstituiertes oder einfach oder mehrfach, gleich oder verschieden substituiertes Phenyl oder Phenylalkyl stehen, wobei als Substituenten im Phenylteil ausgewählt sind: Nitro, Halogen, Alkyl, Alkoxy, Halogenalkyl oder Halogenalkoxy.

Im einzelnen seien außer den bei den Herstellungsbeispielen genannten Verbindungen die folgenden 3-Aryl-4-hydroxy-Δ³-dihydrofuran-Derivate der Formel (Ia) genannt:

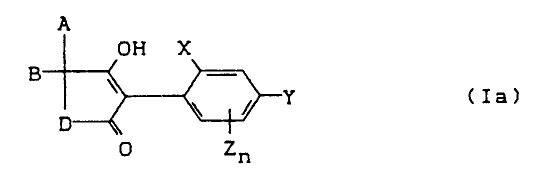

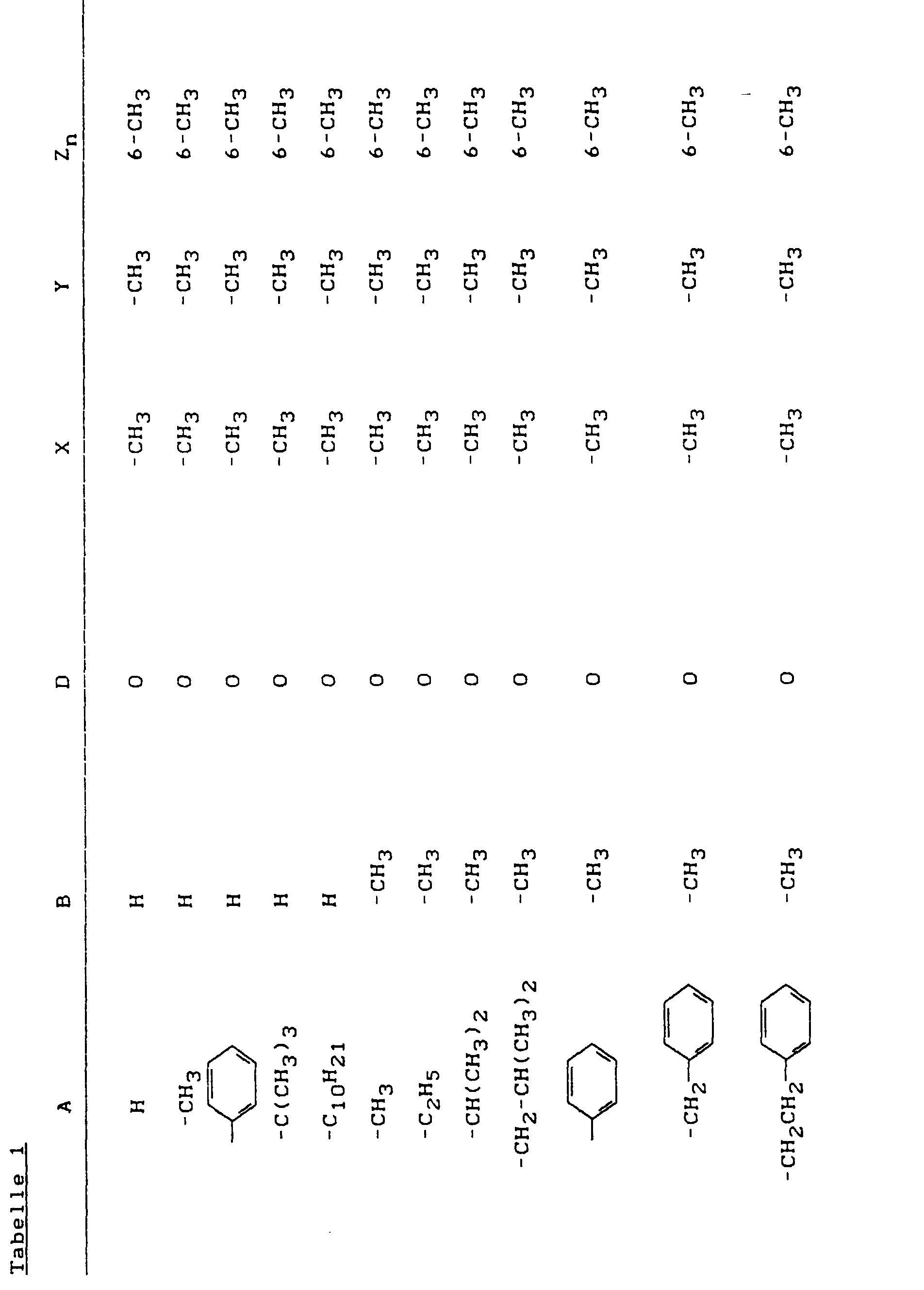

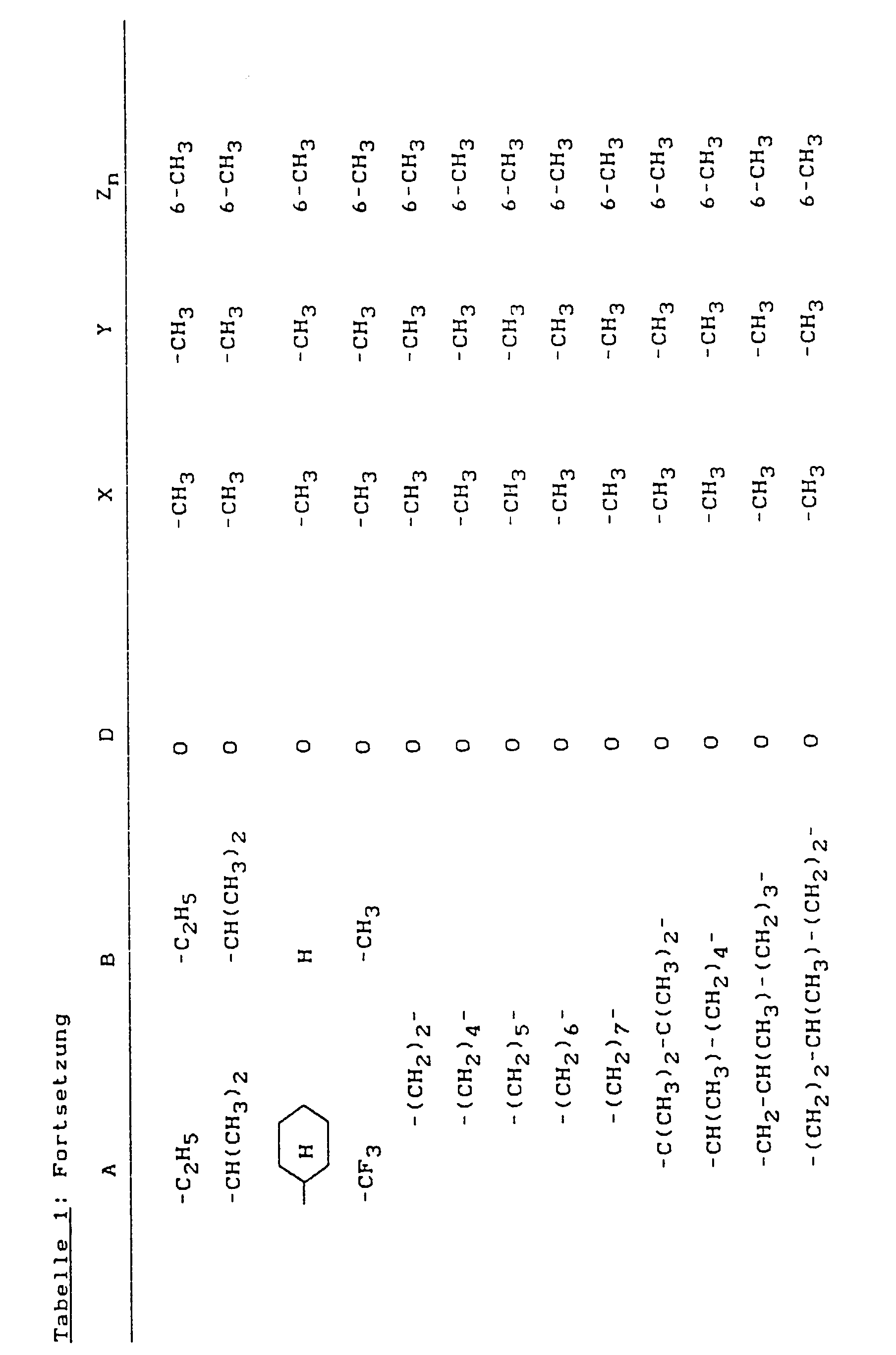

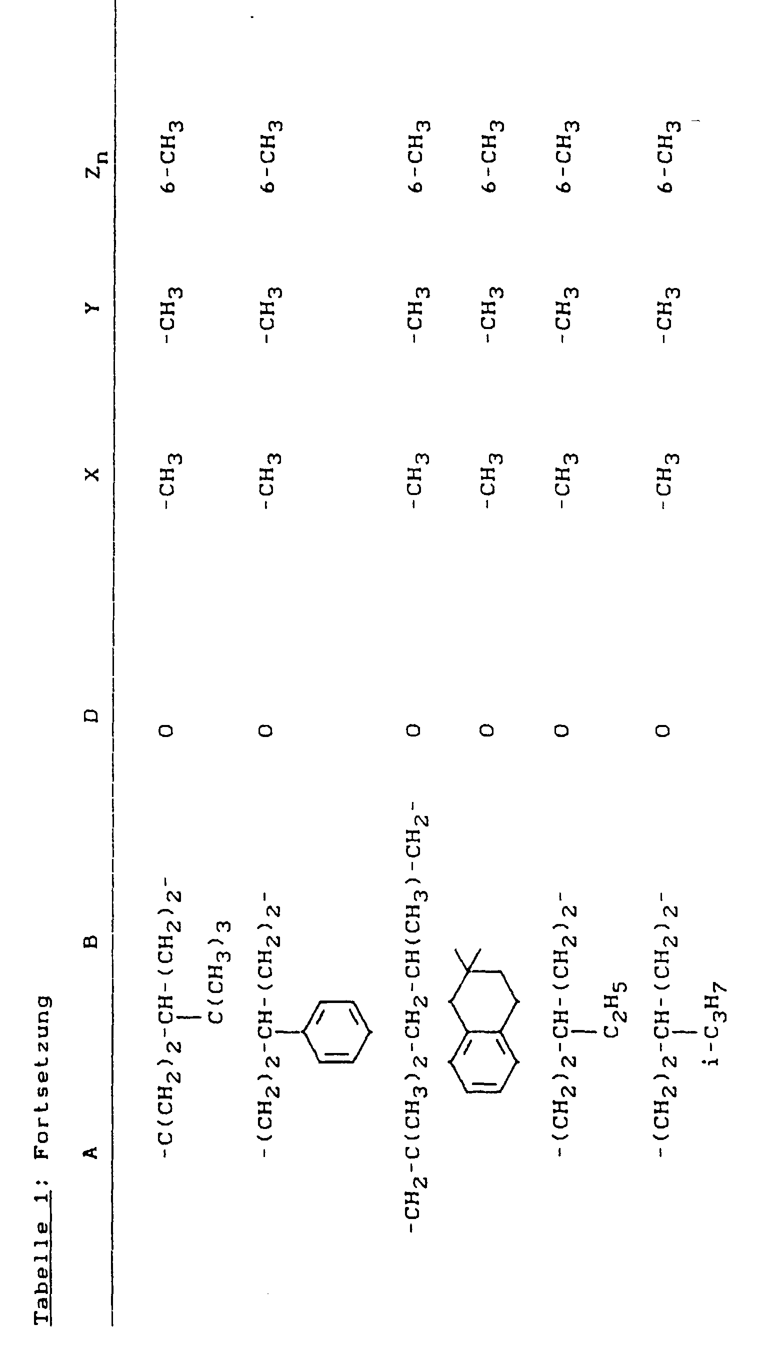

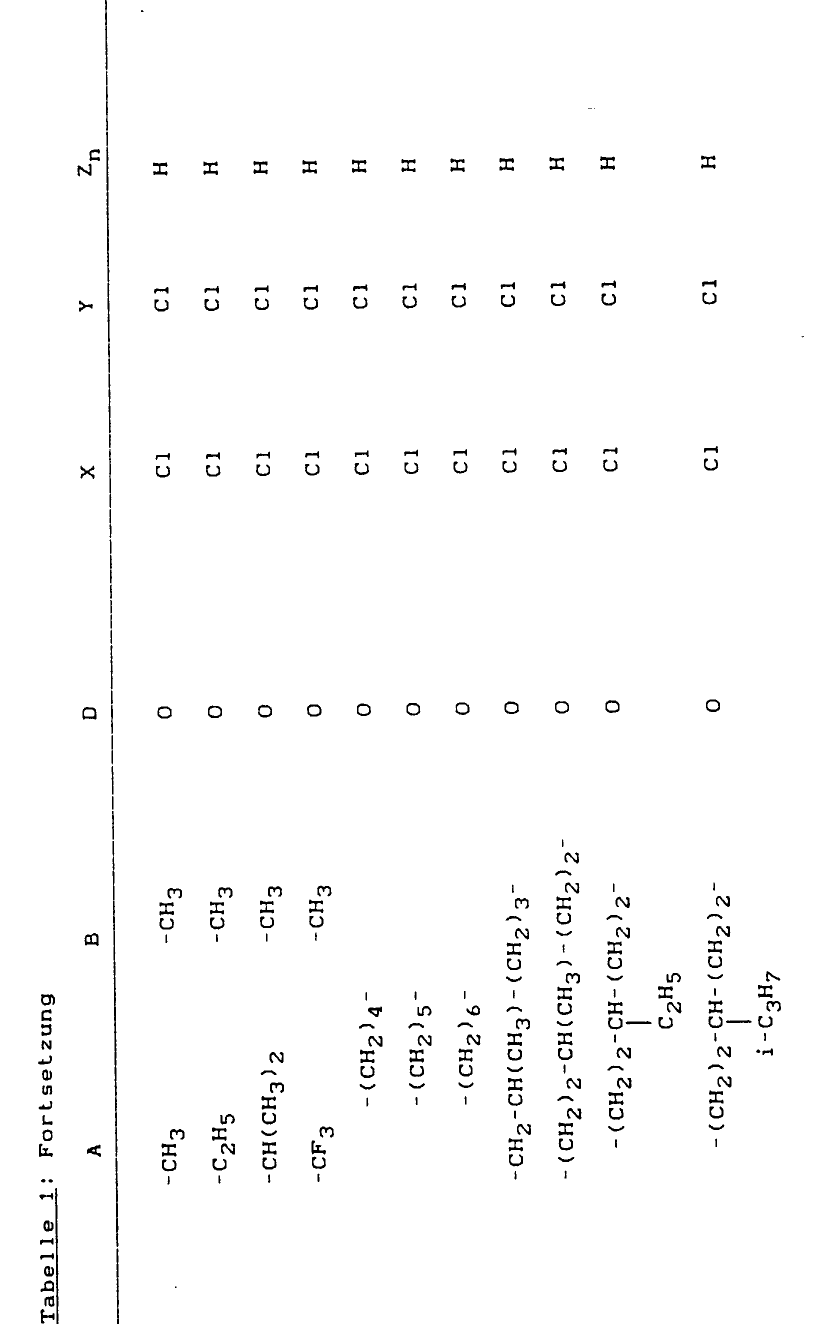

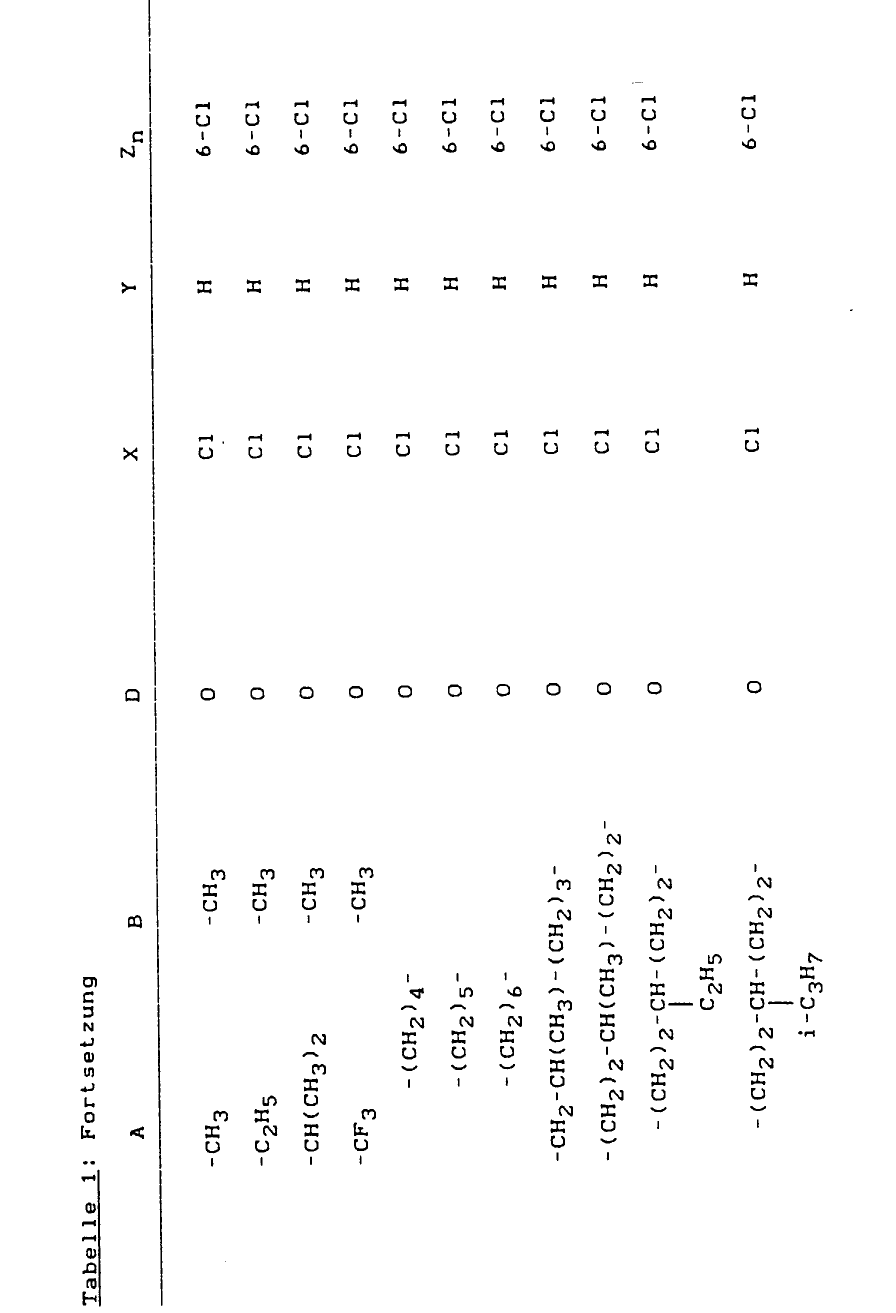

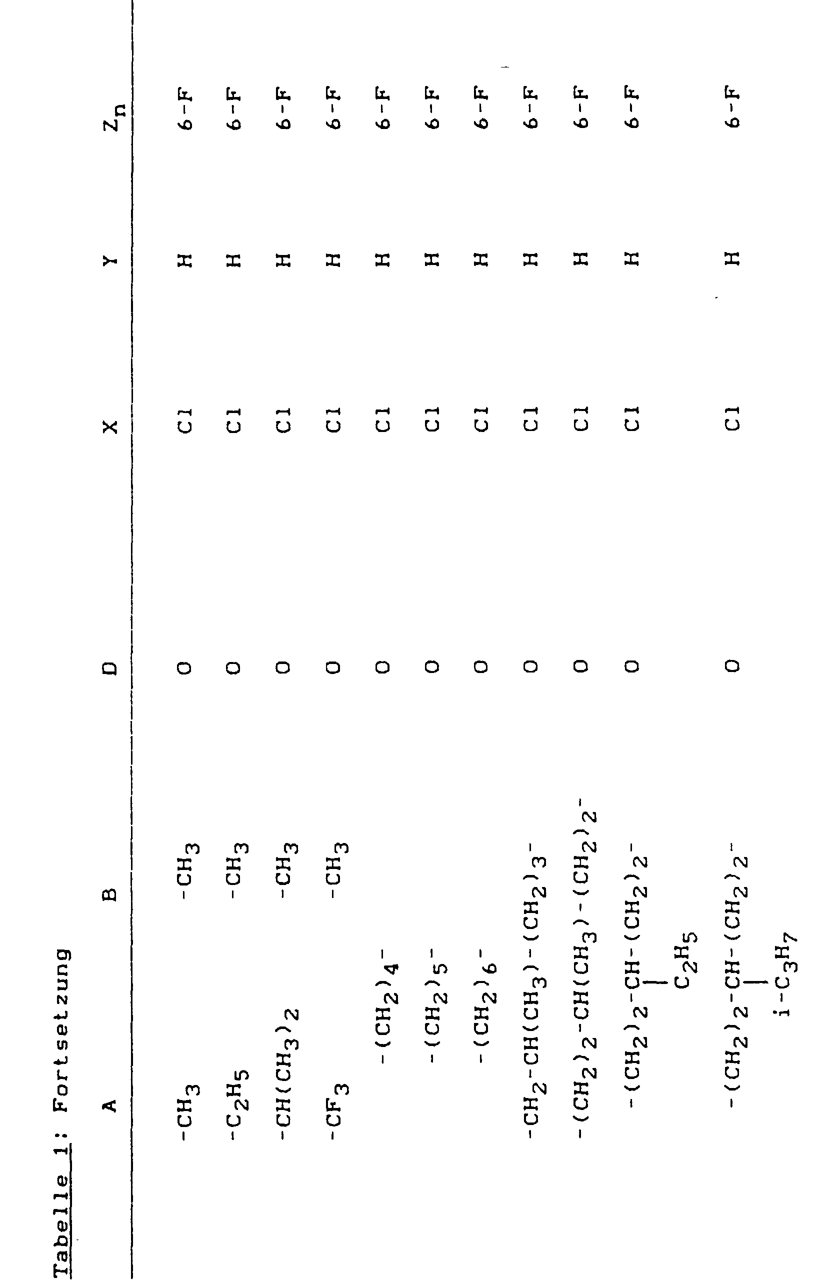

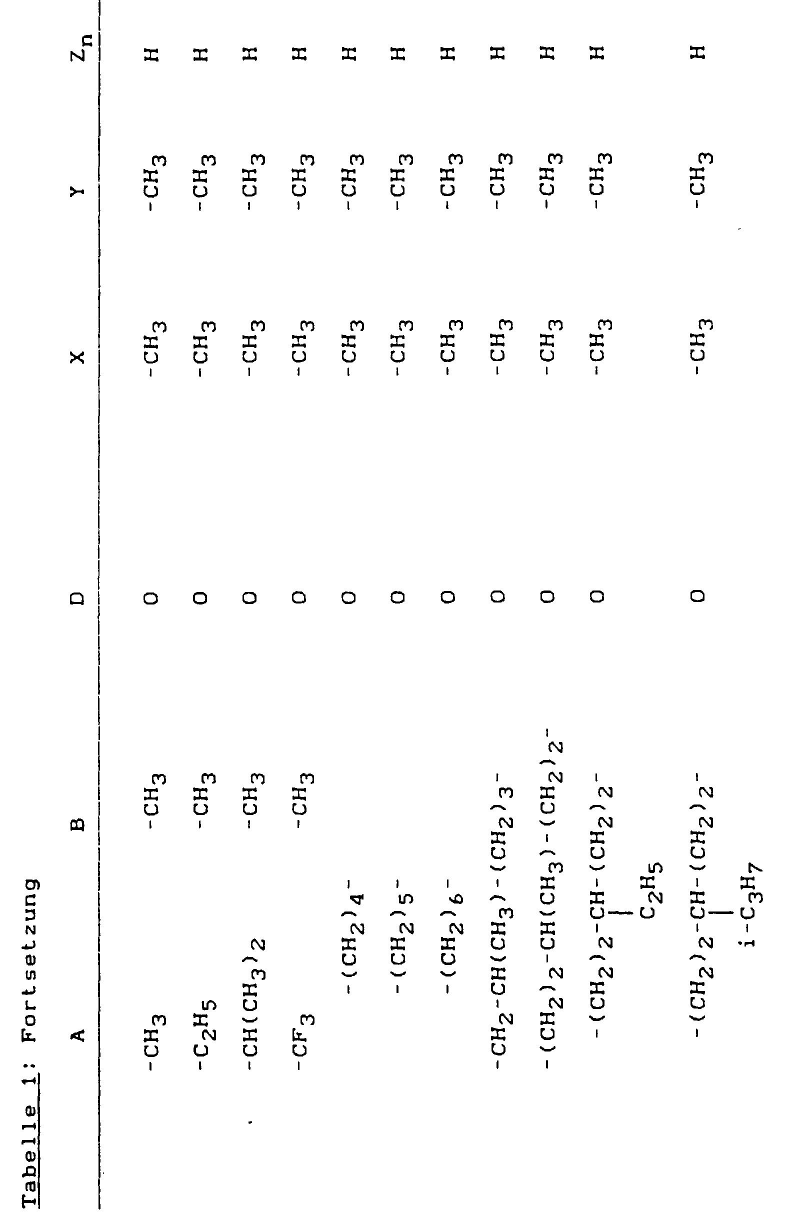

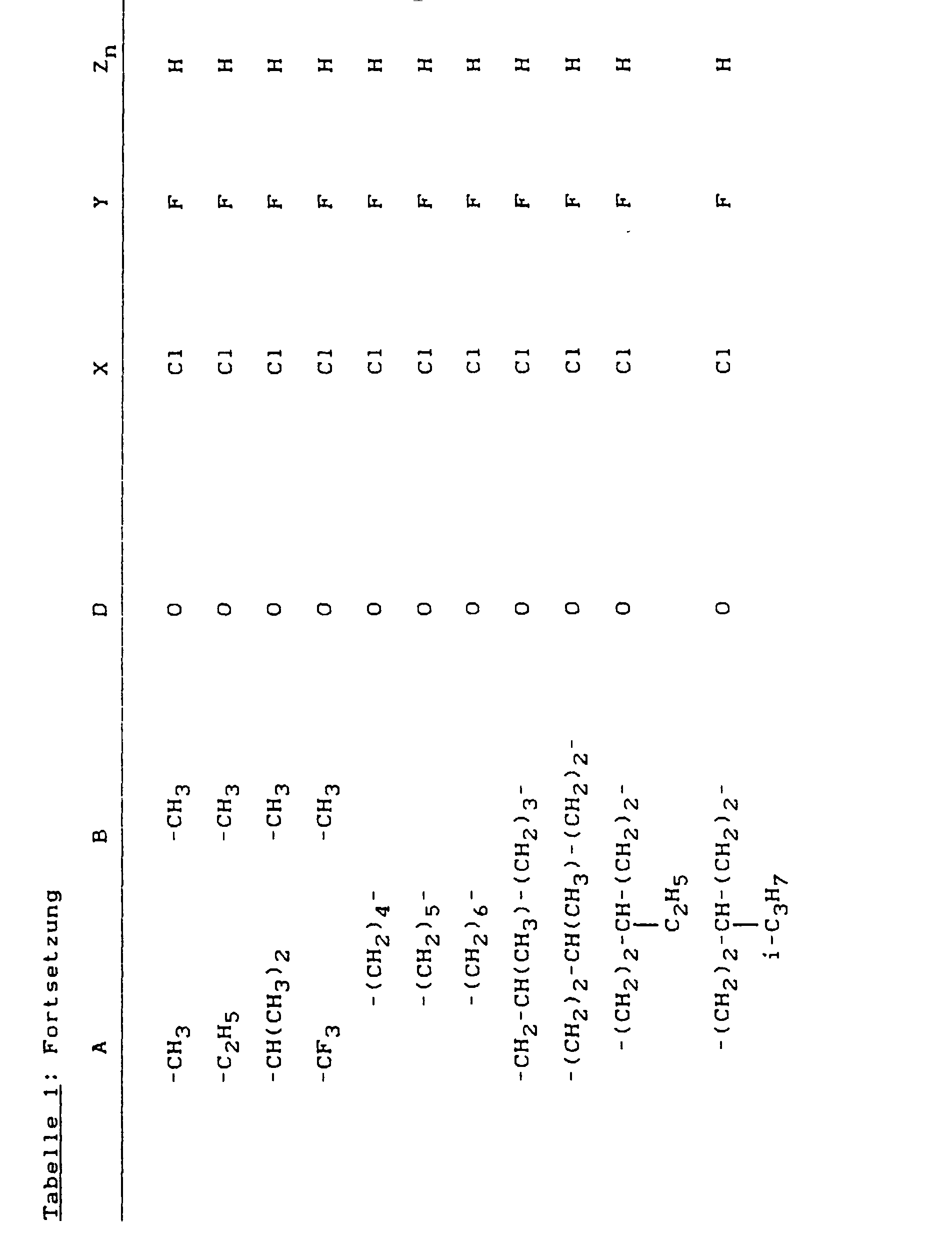

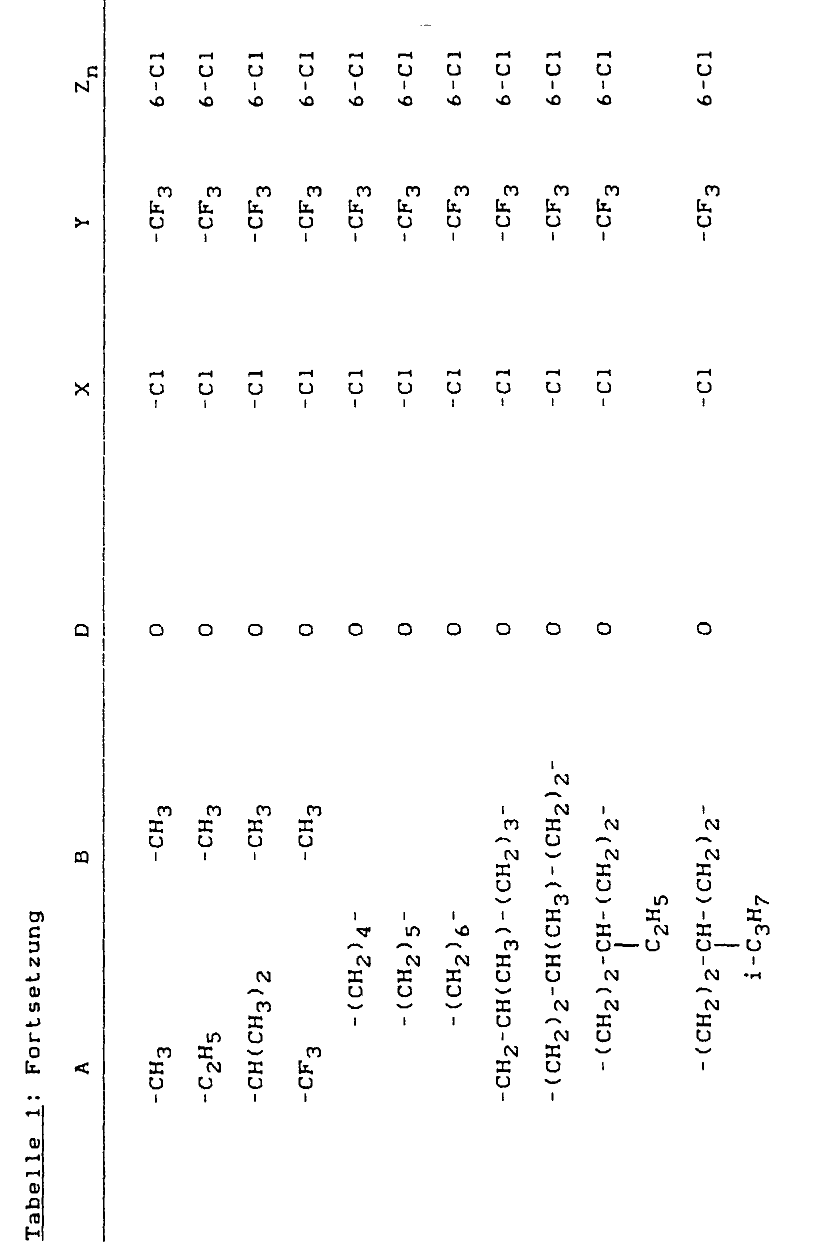

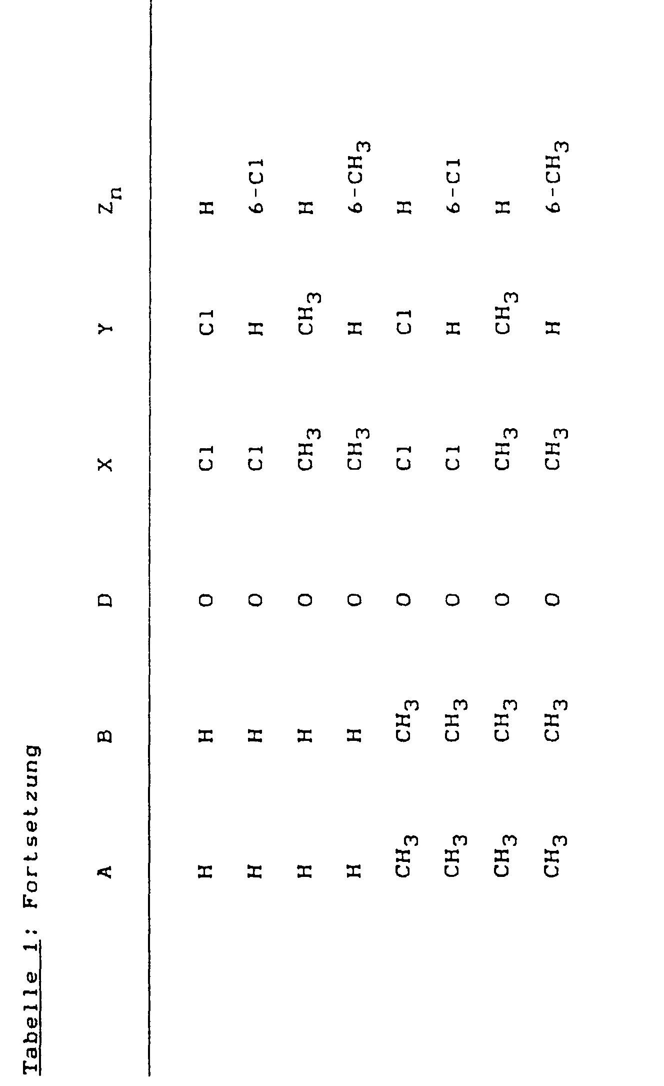

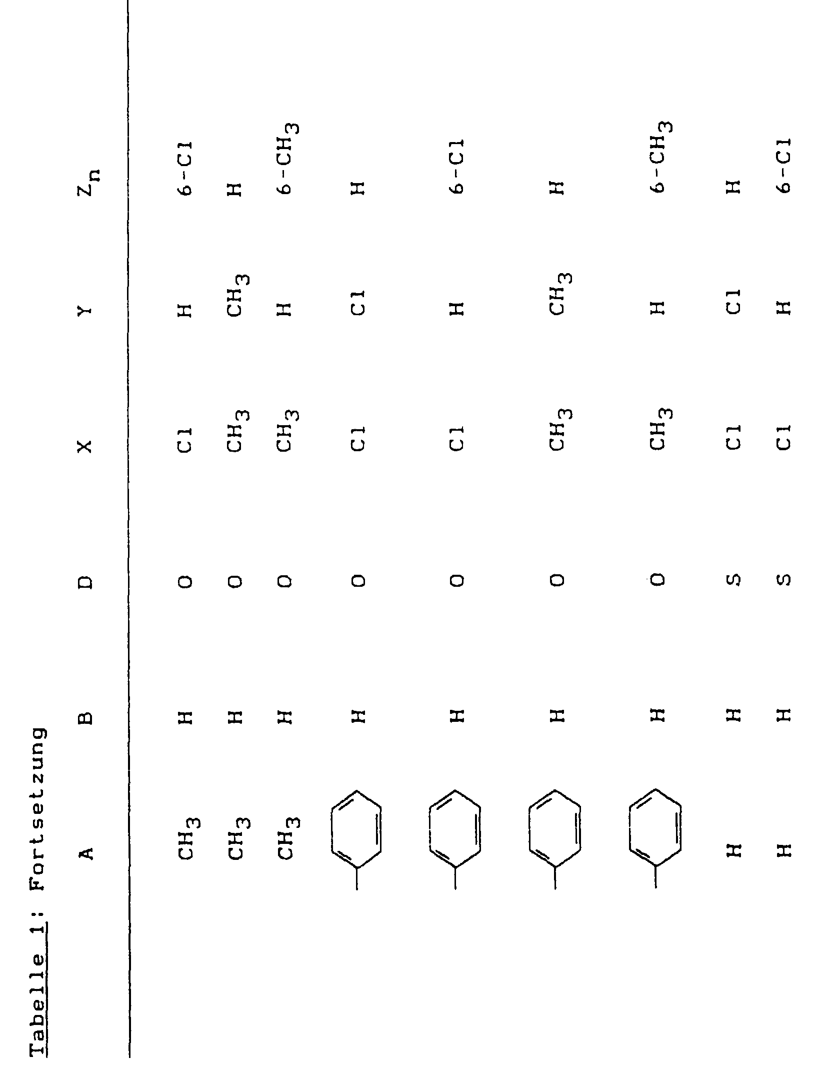

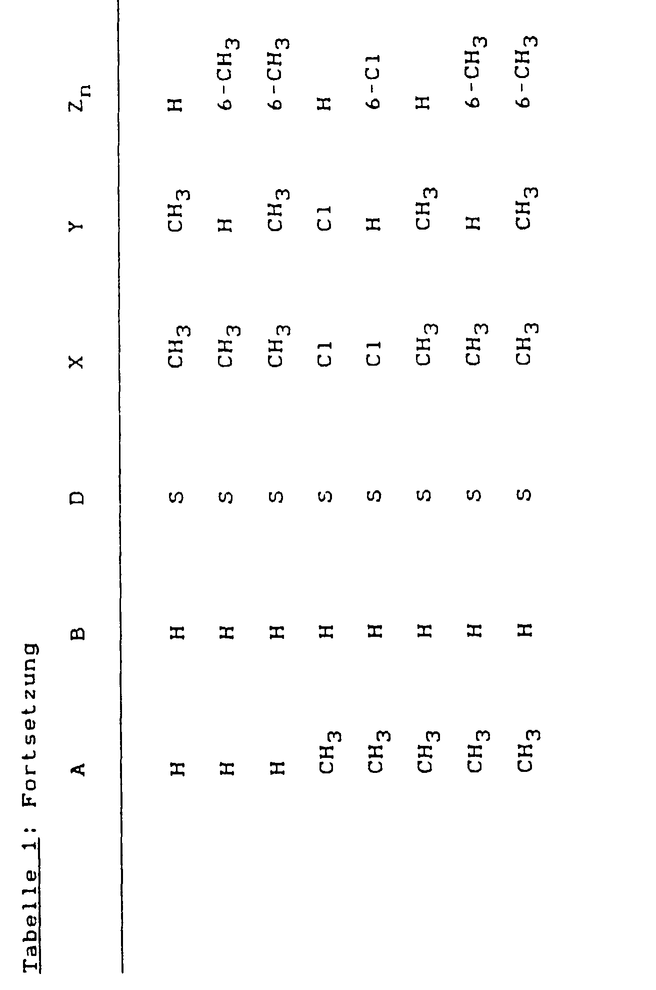

Im einzelnen seien außer den bei den Herstellungsbeispielen genannten Verbindungen die folgenden 3-Aryl-4-hydroxy-Δ³-dihydrofuran-Derivate der Formel (Ib) genannt:

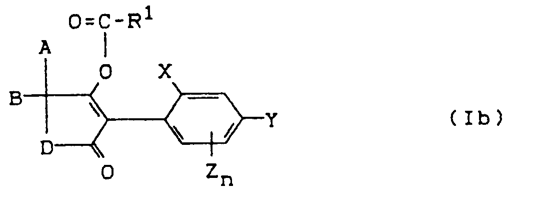

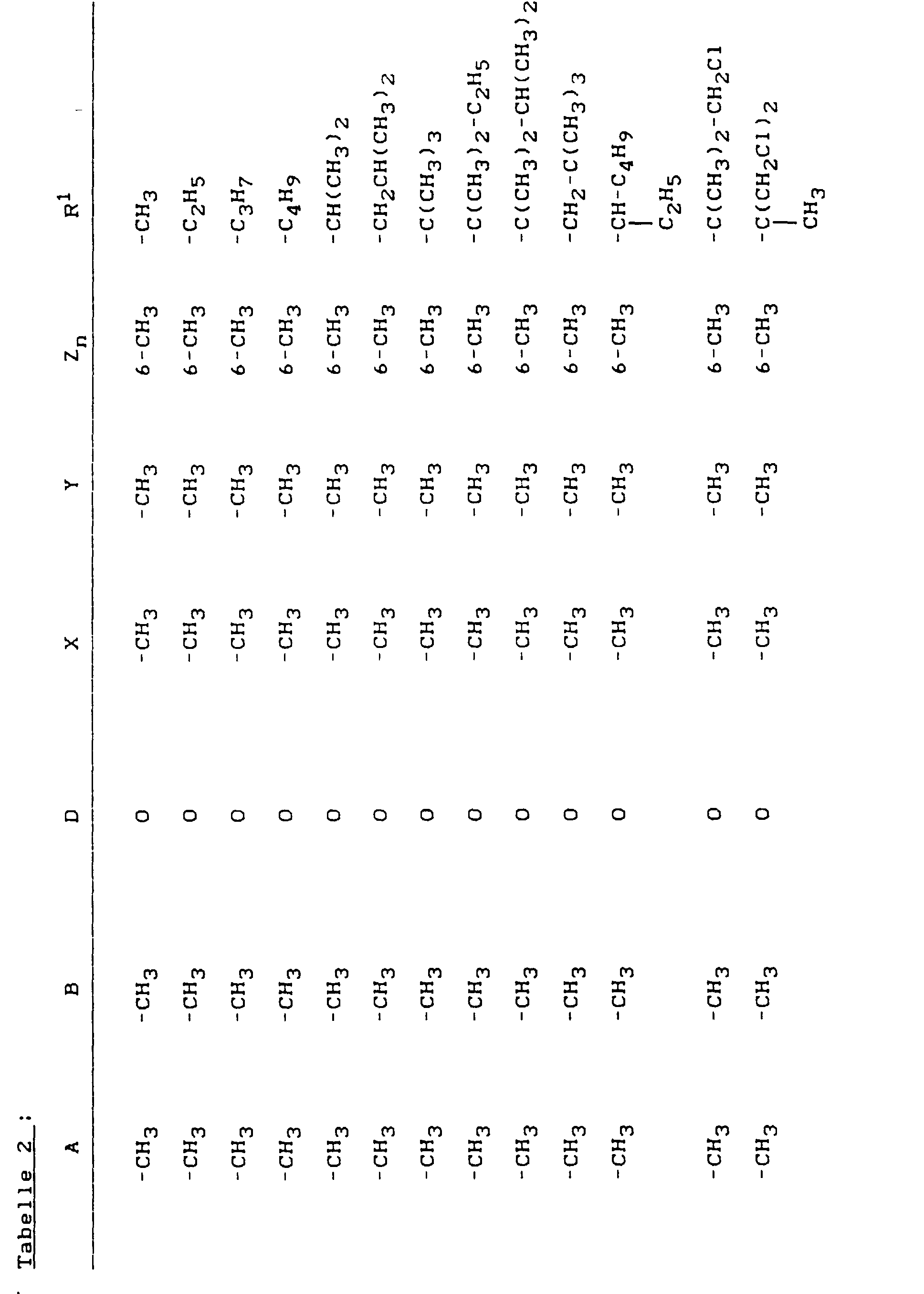

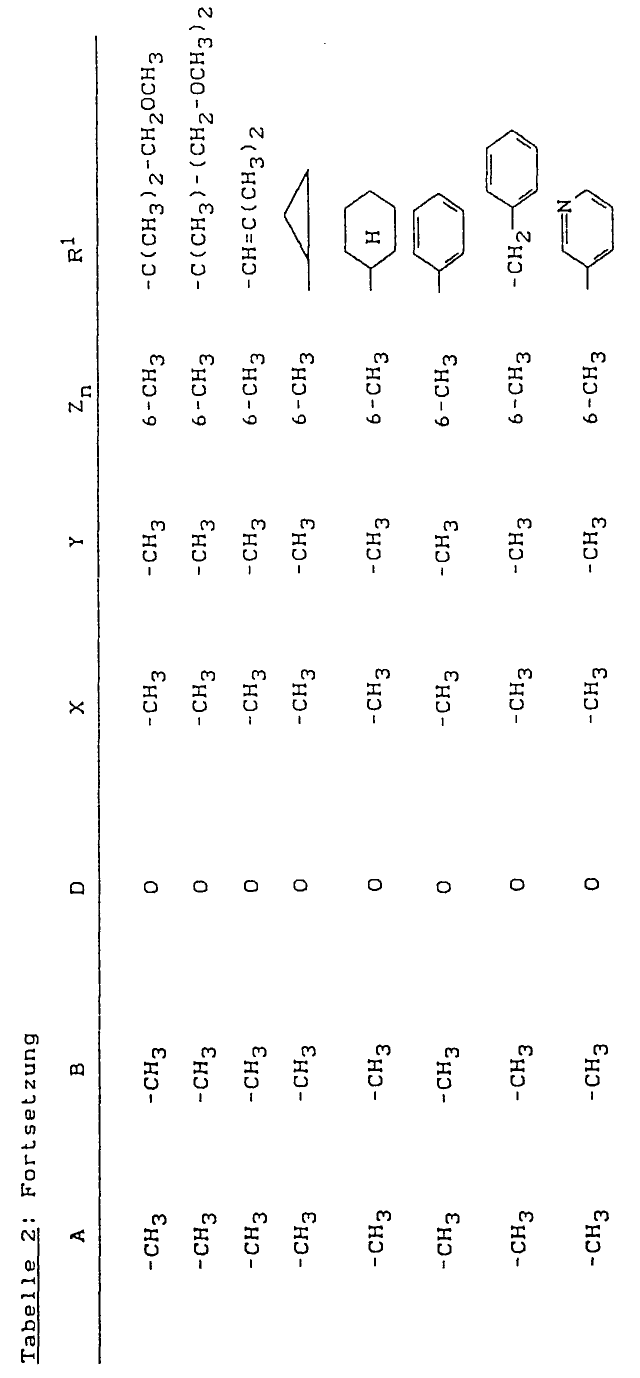

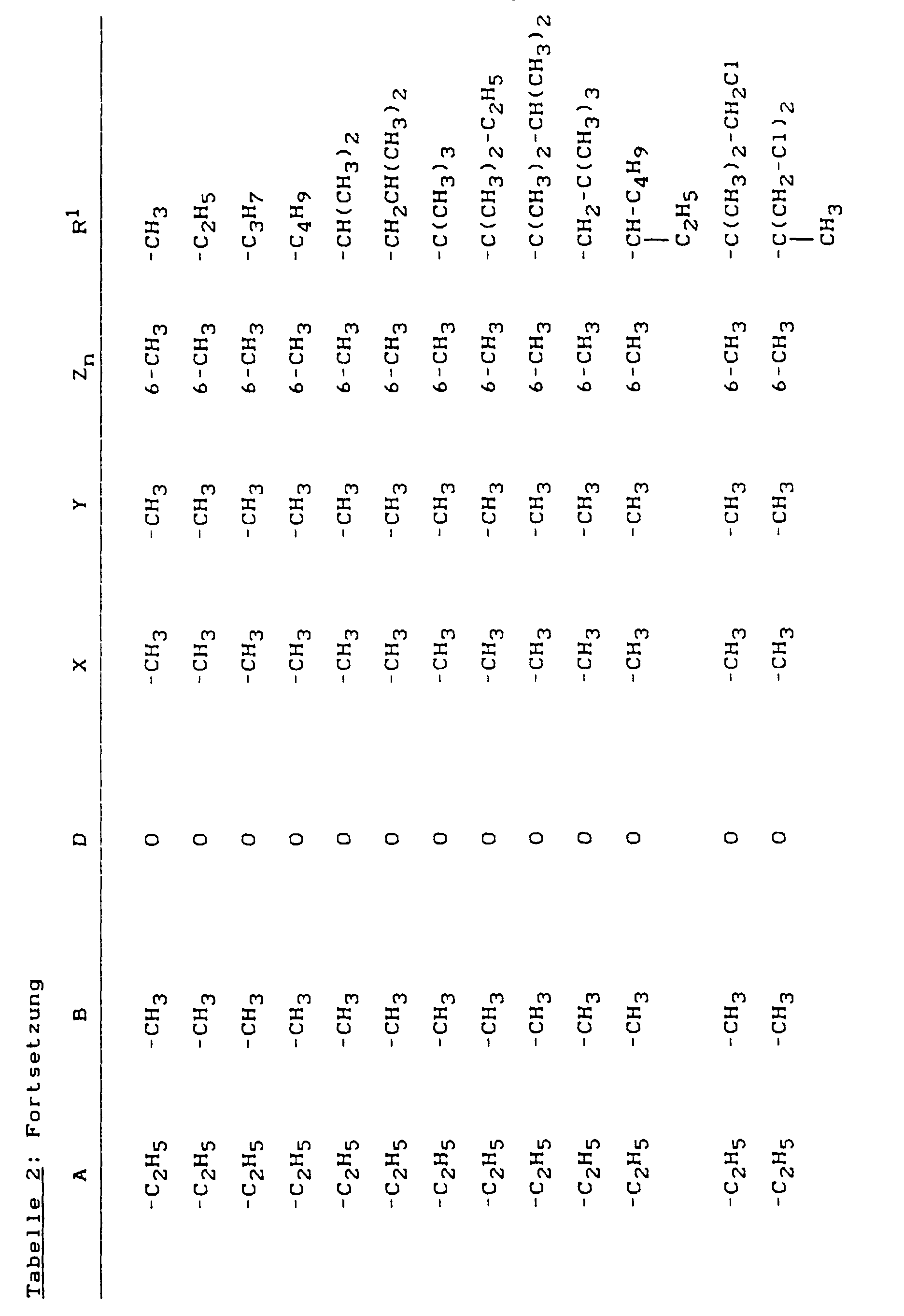

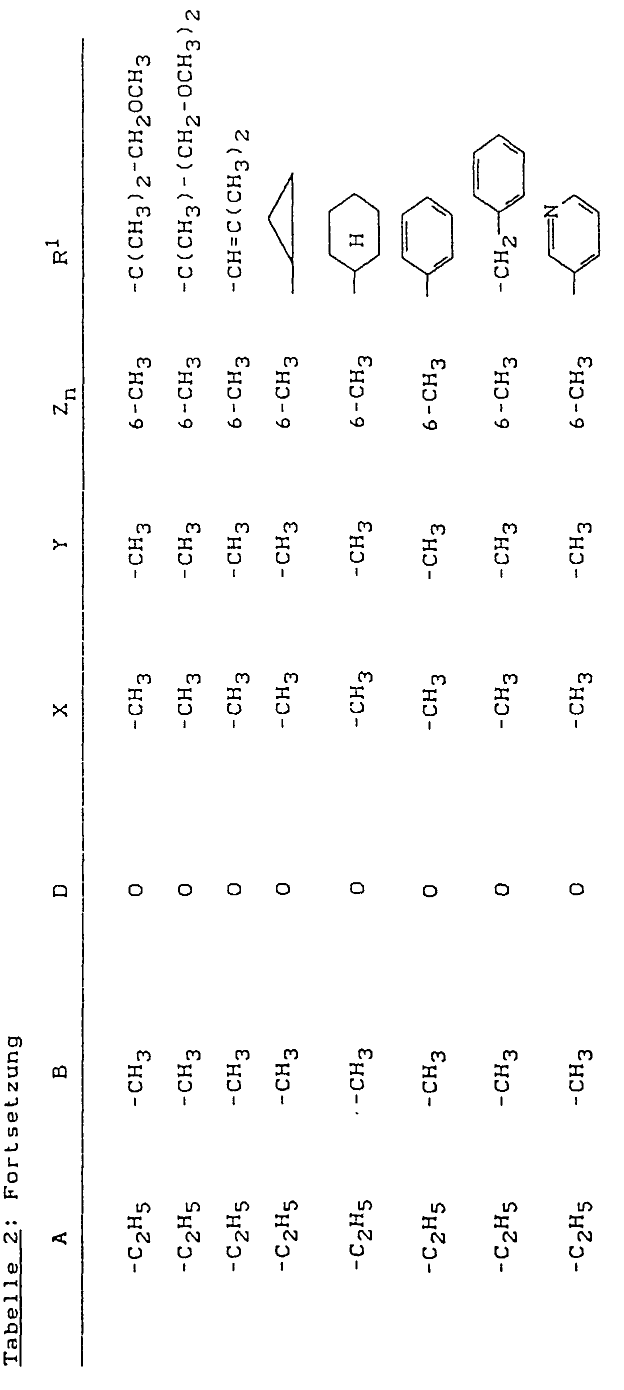

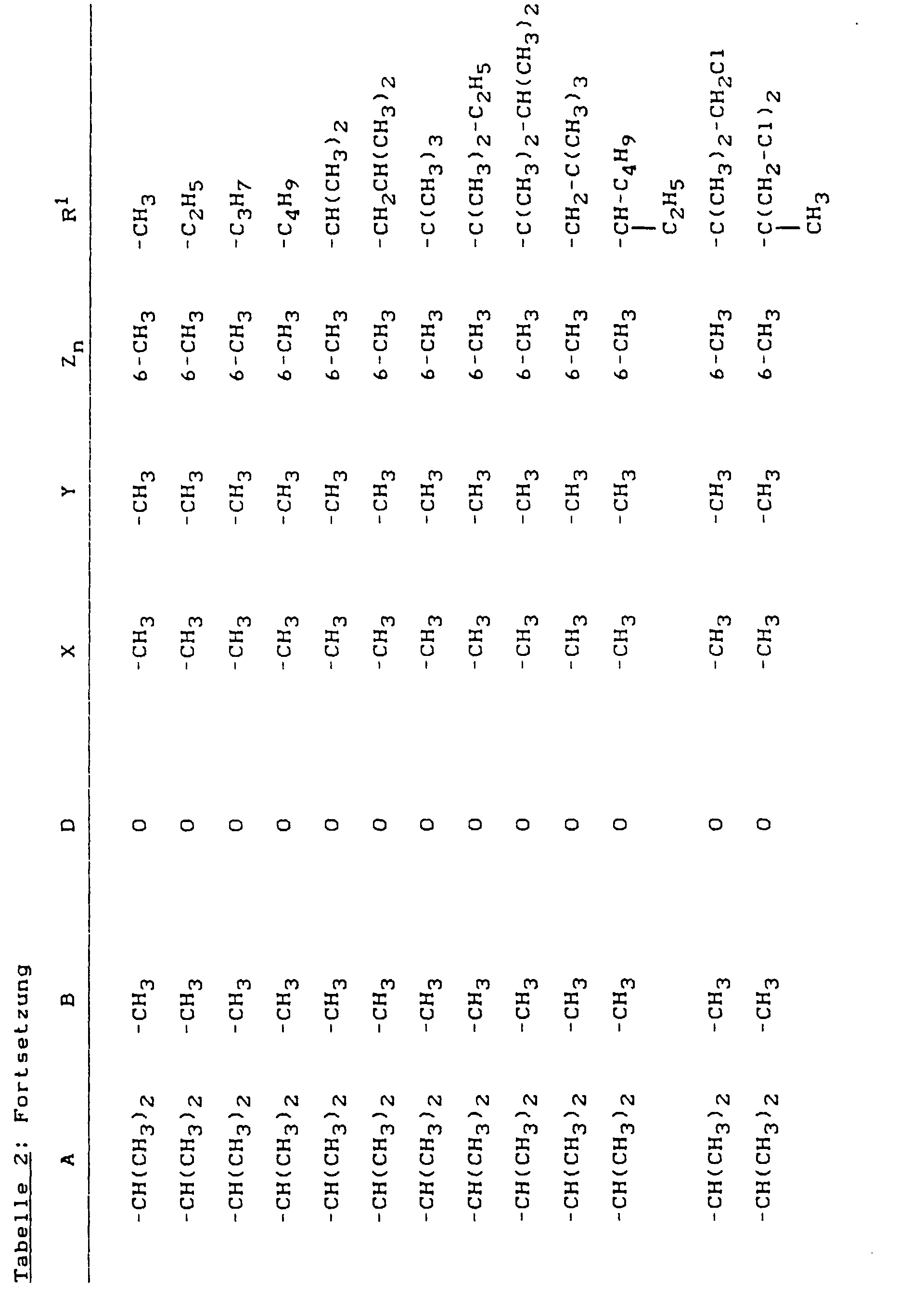

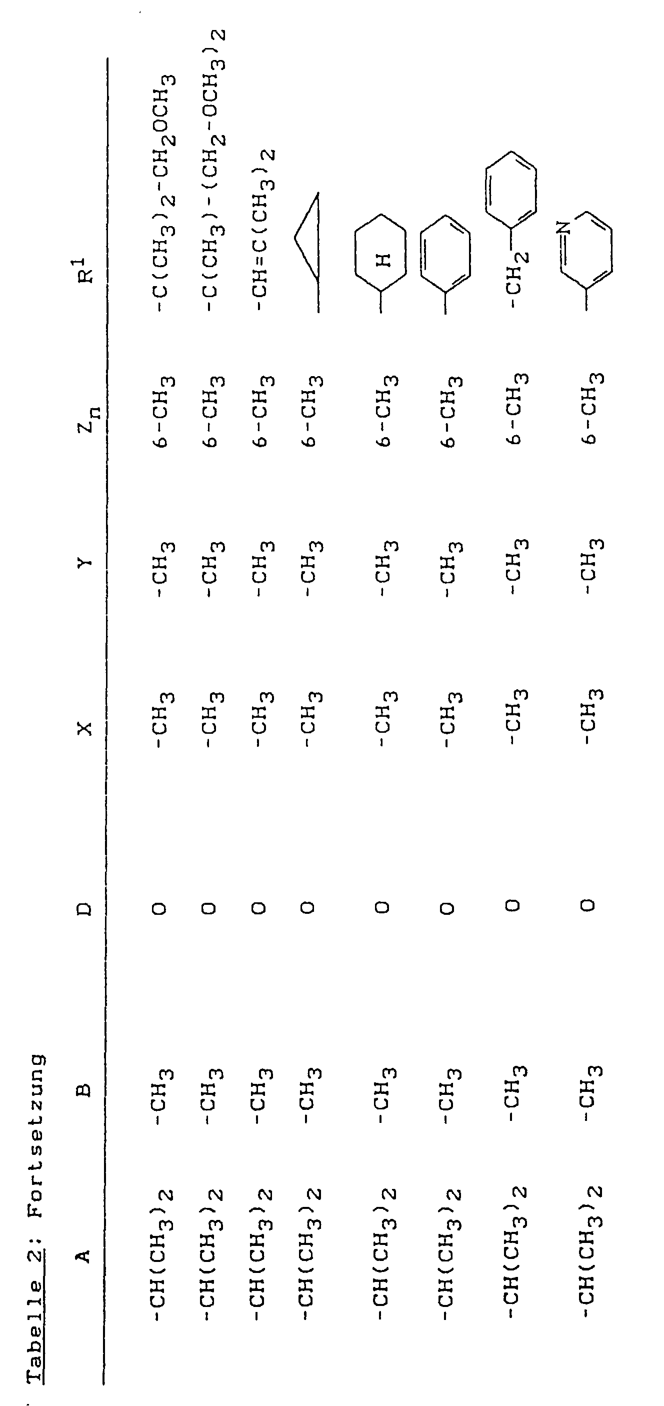

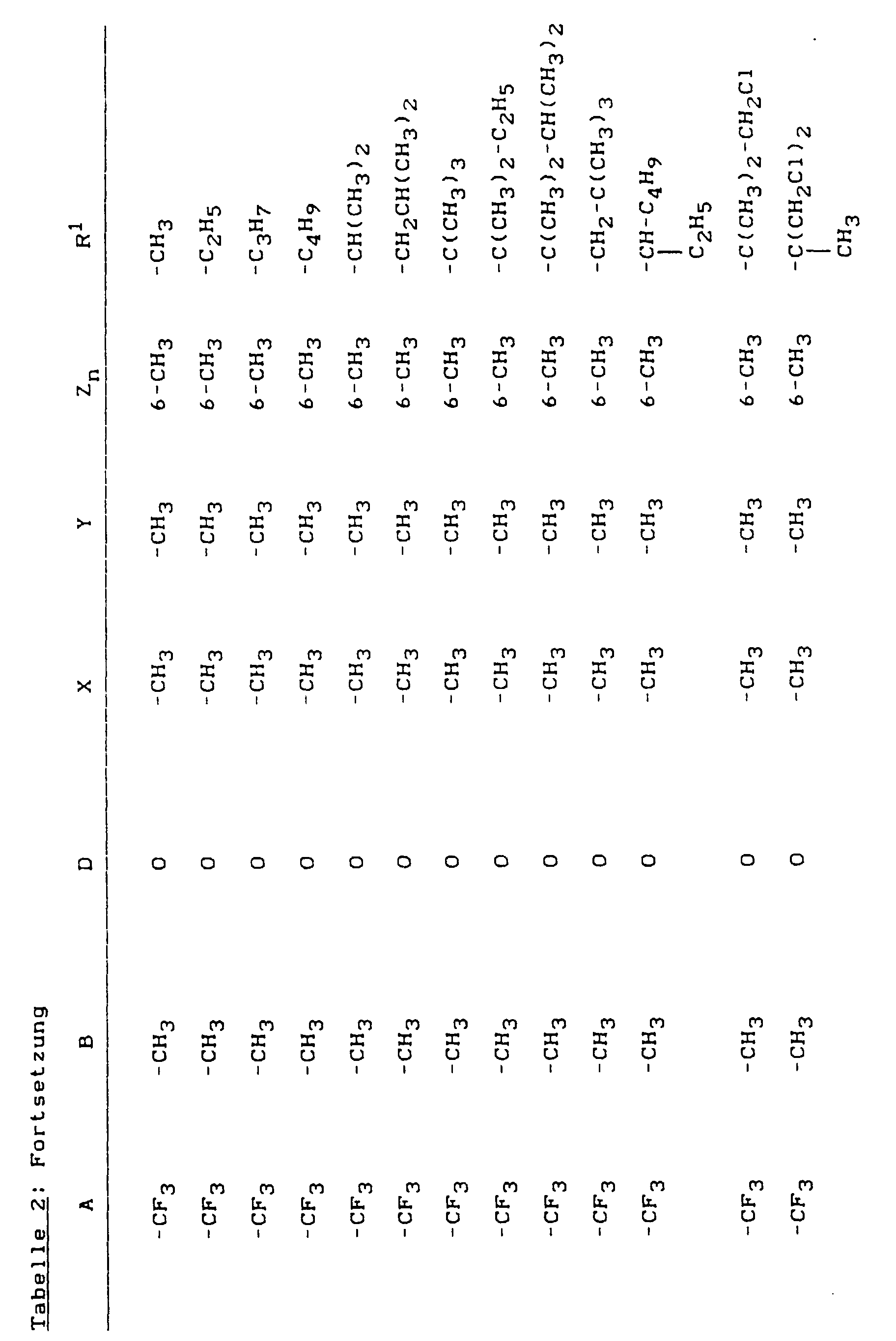

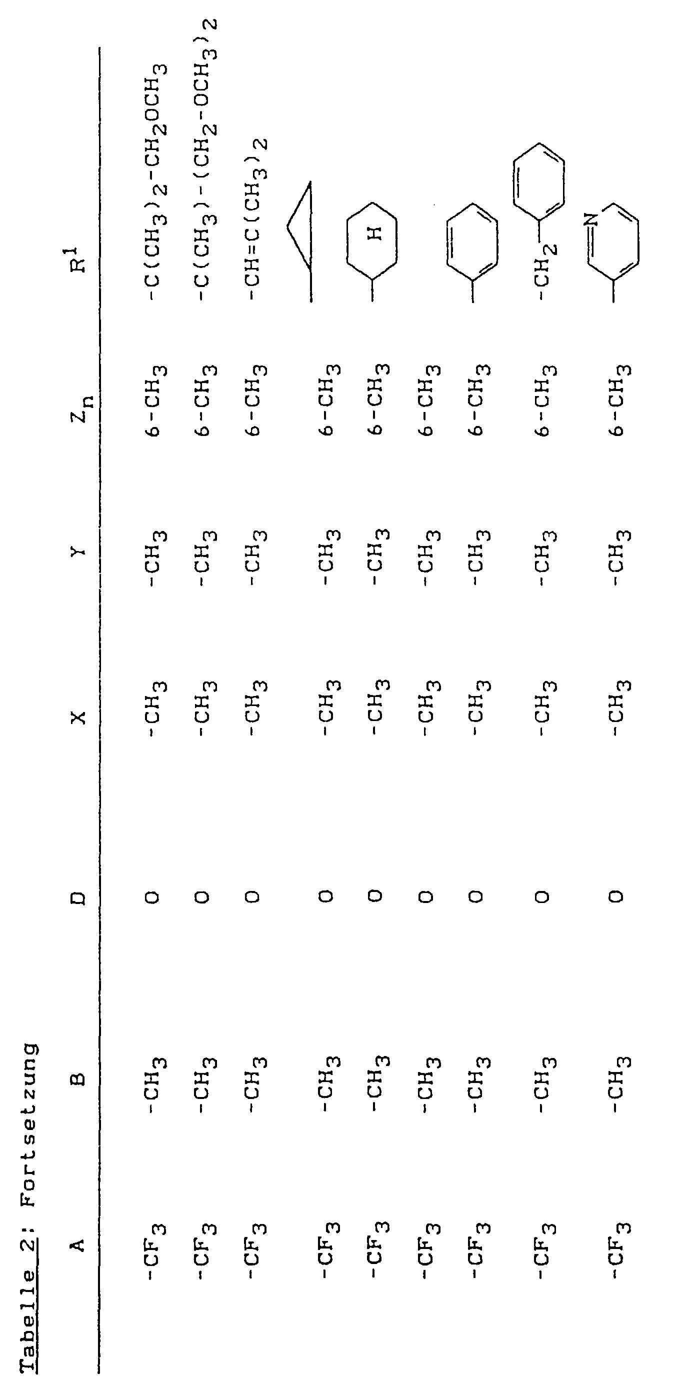

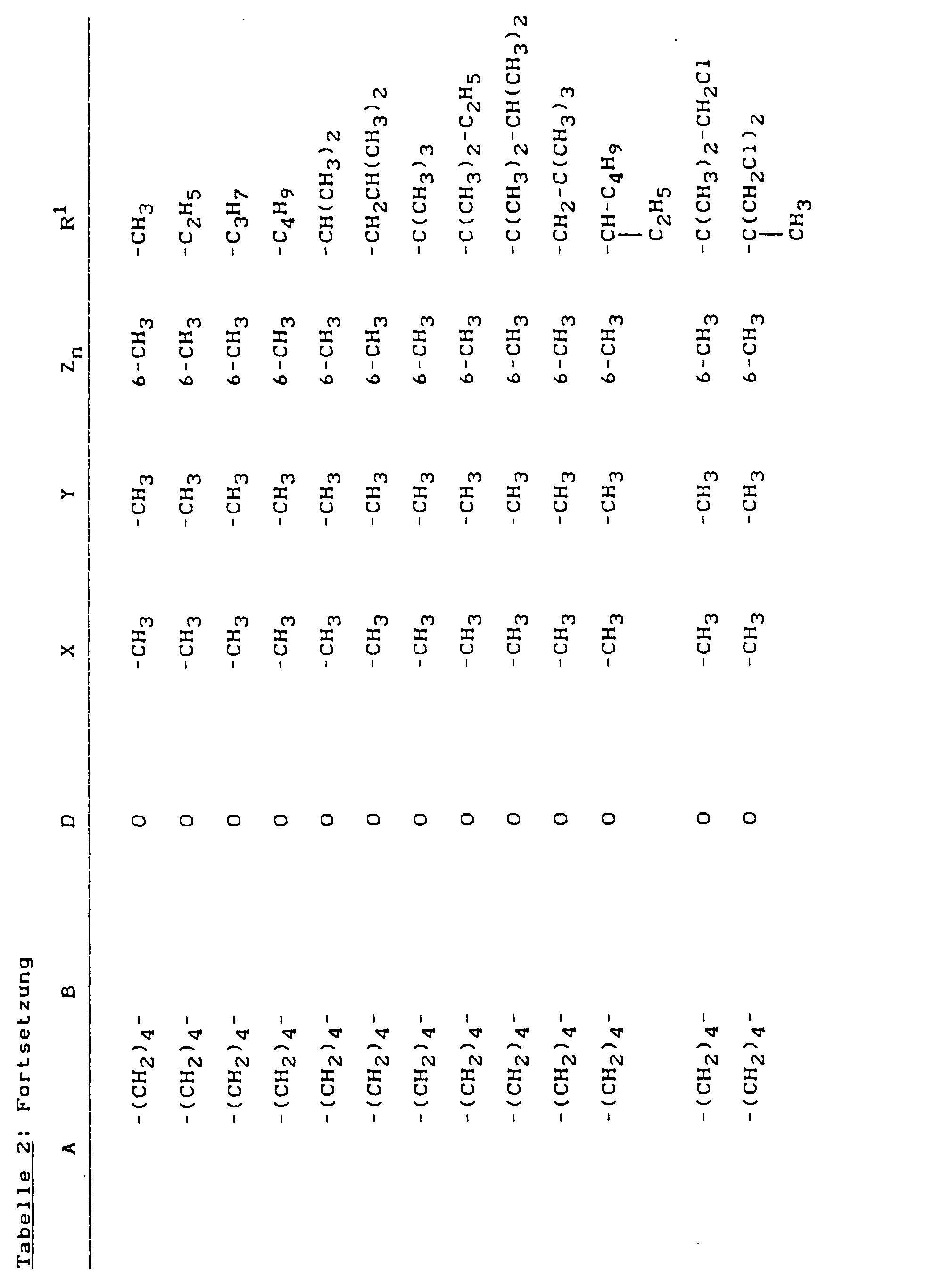

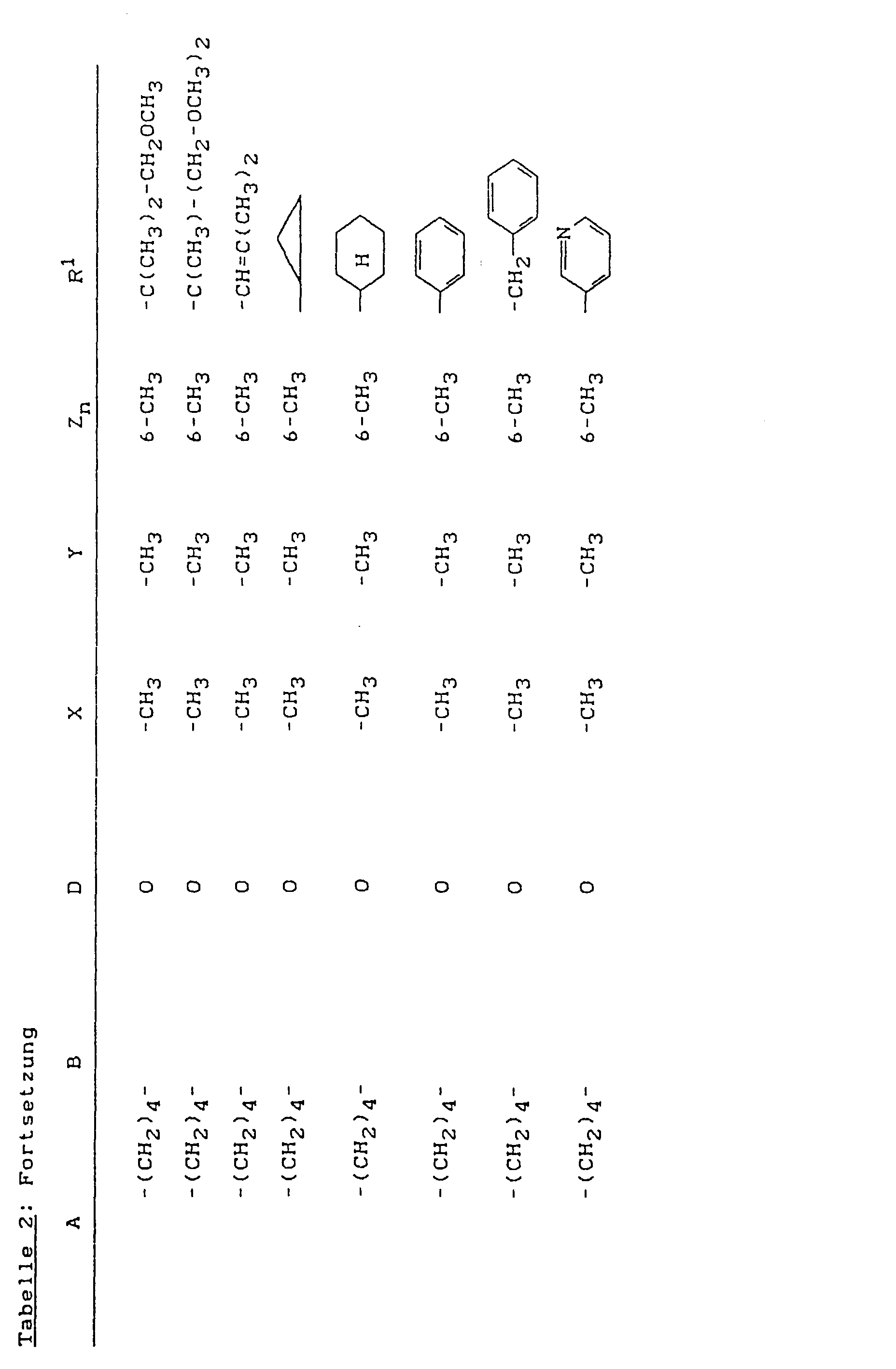

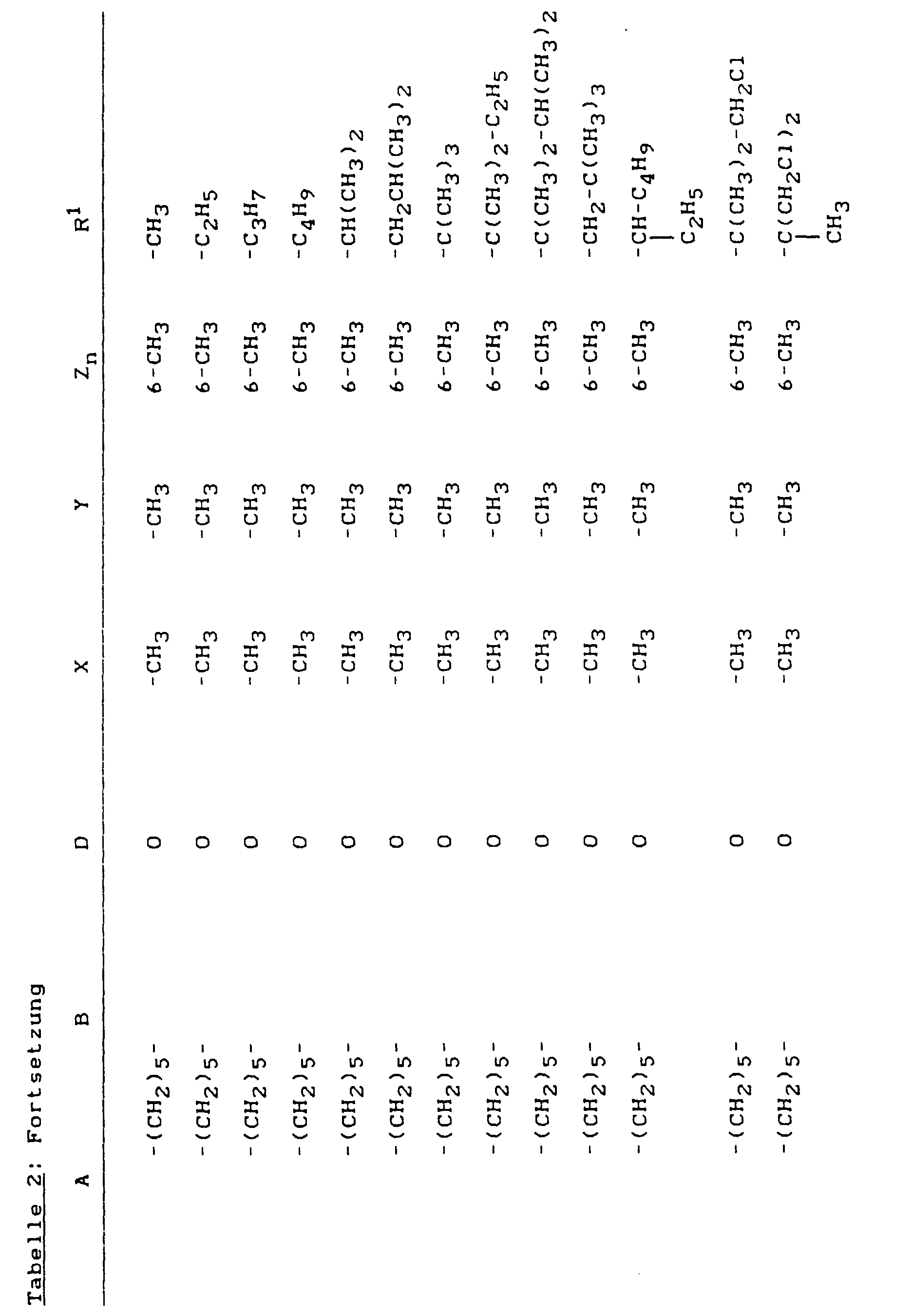

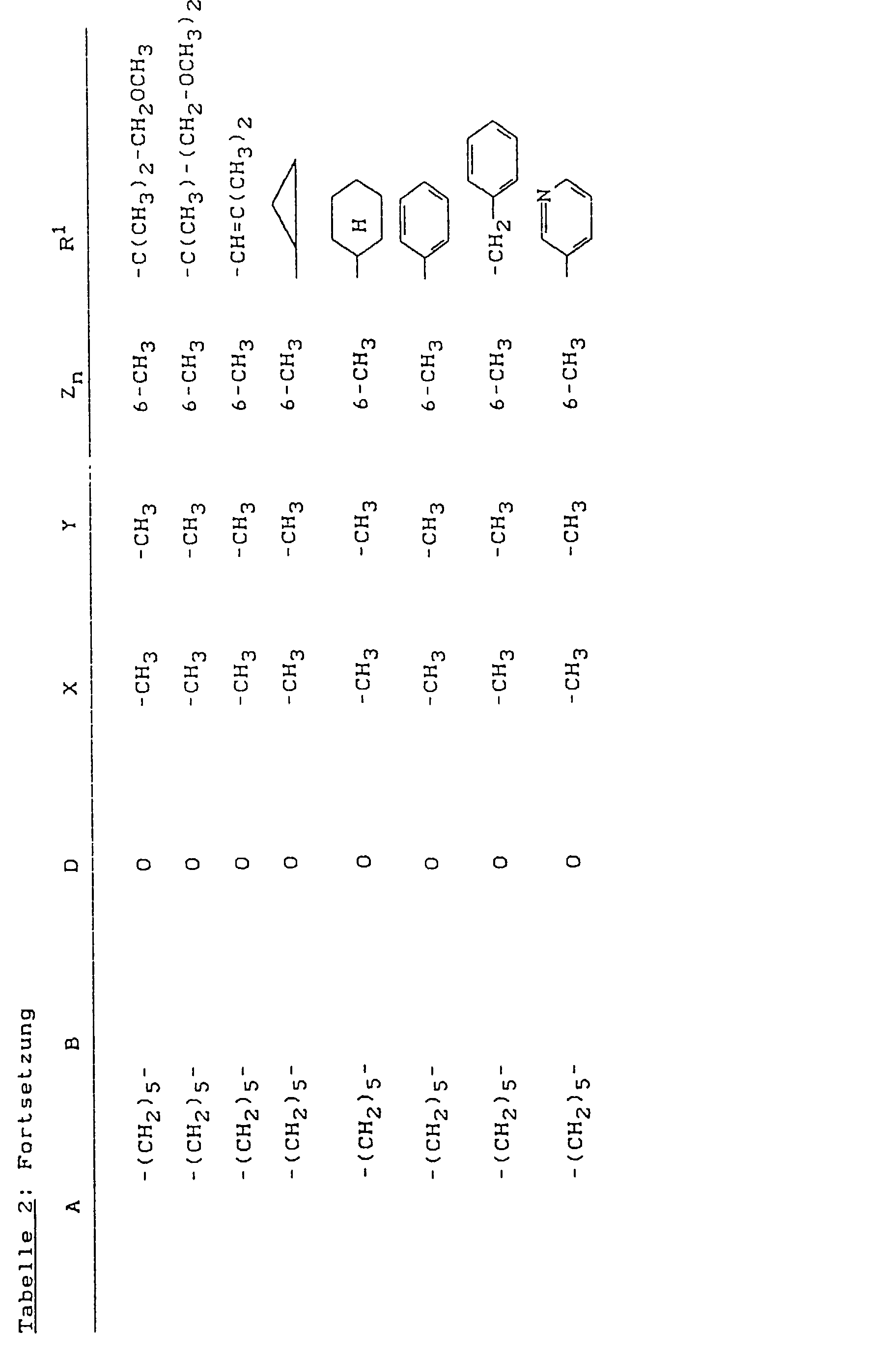

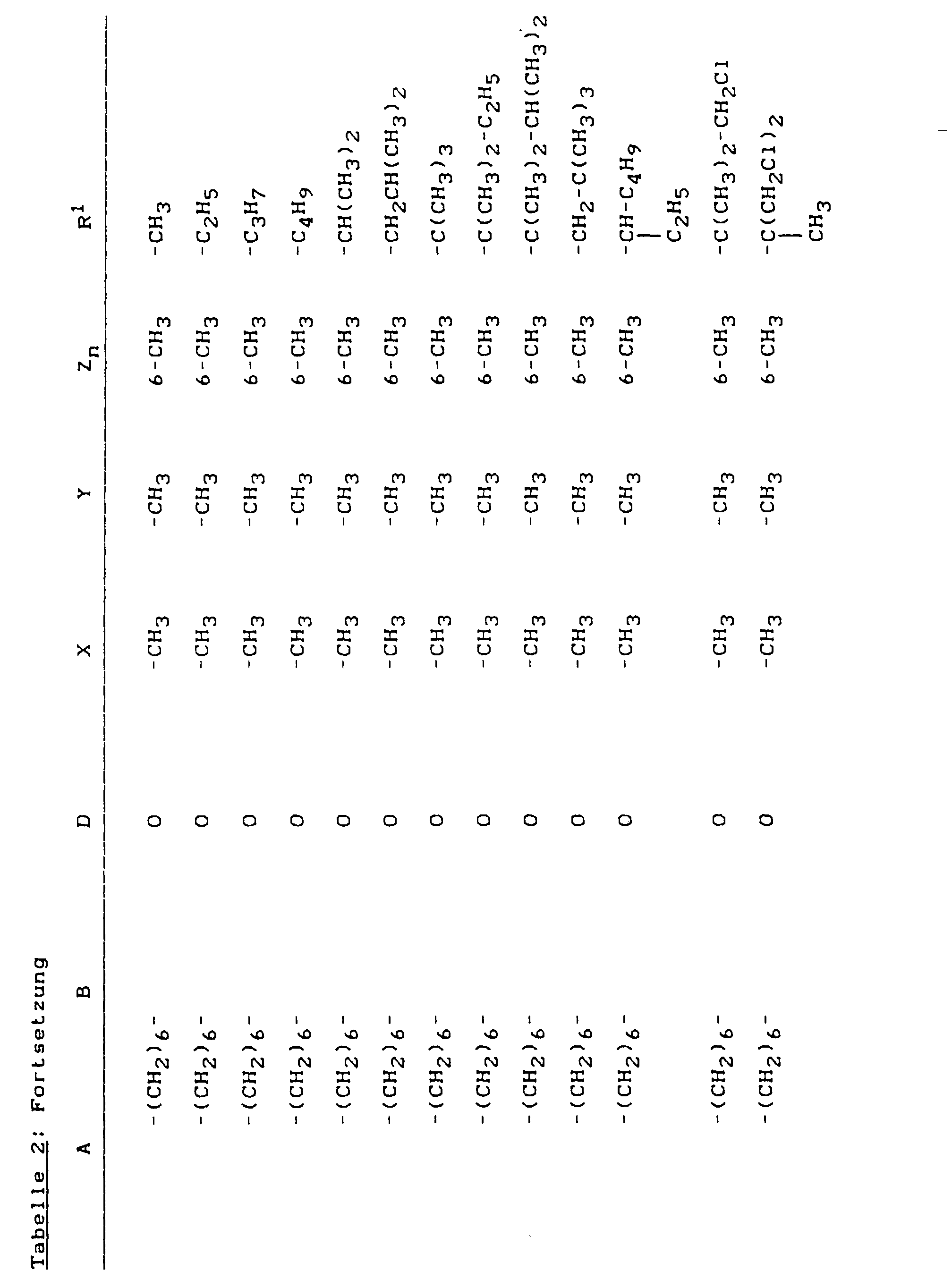

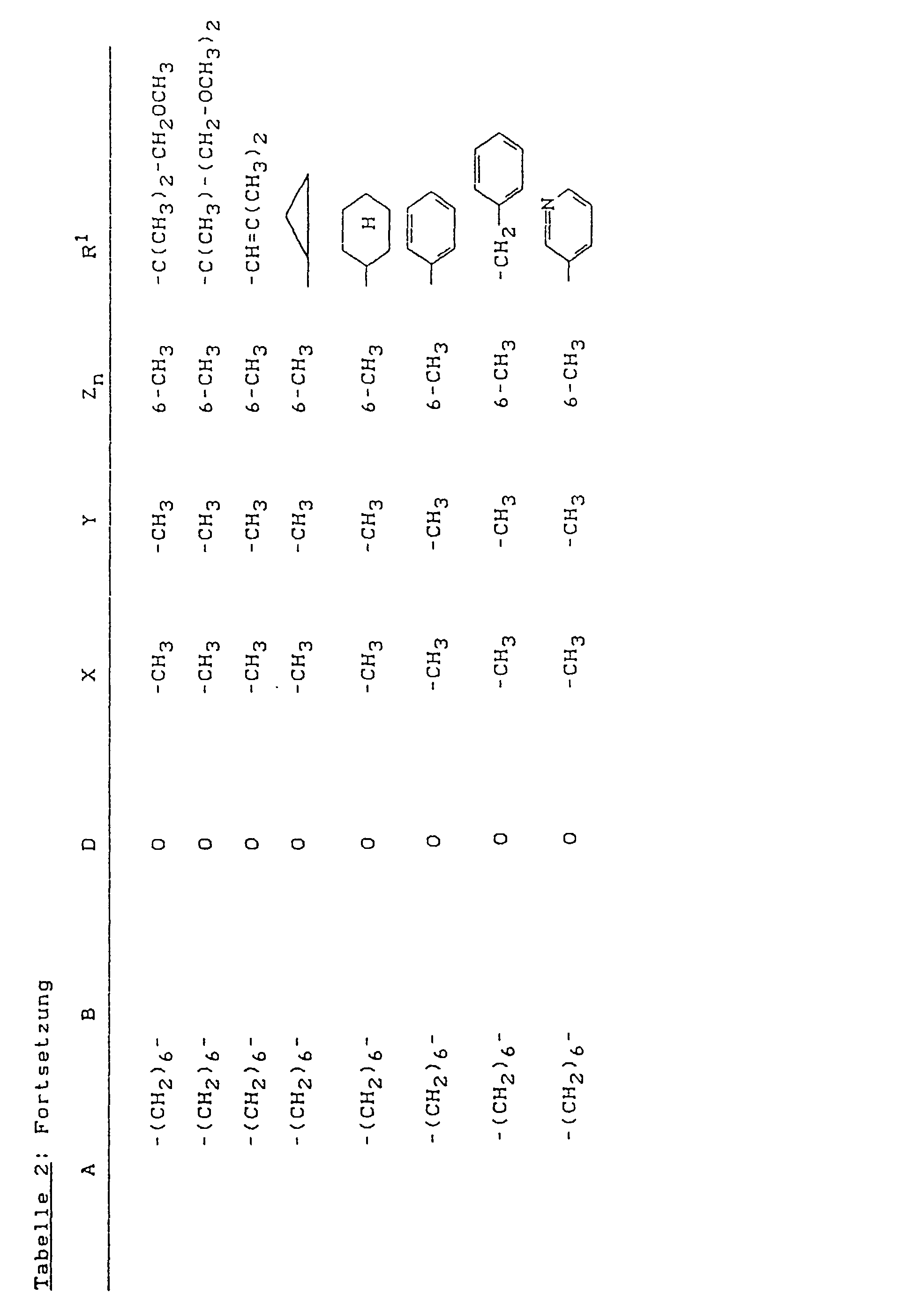

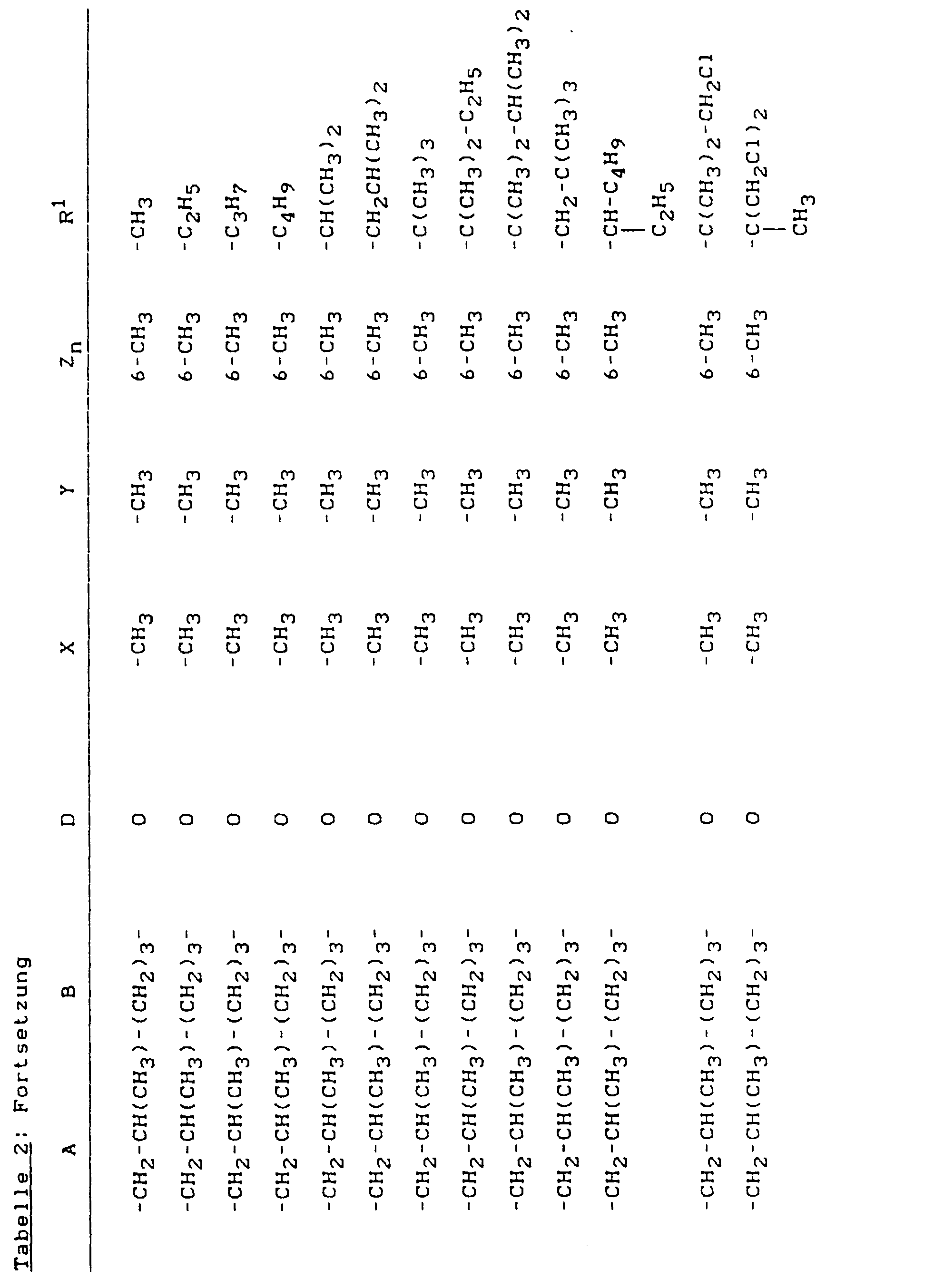

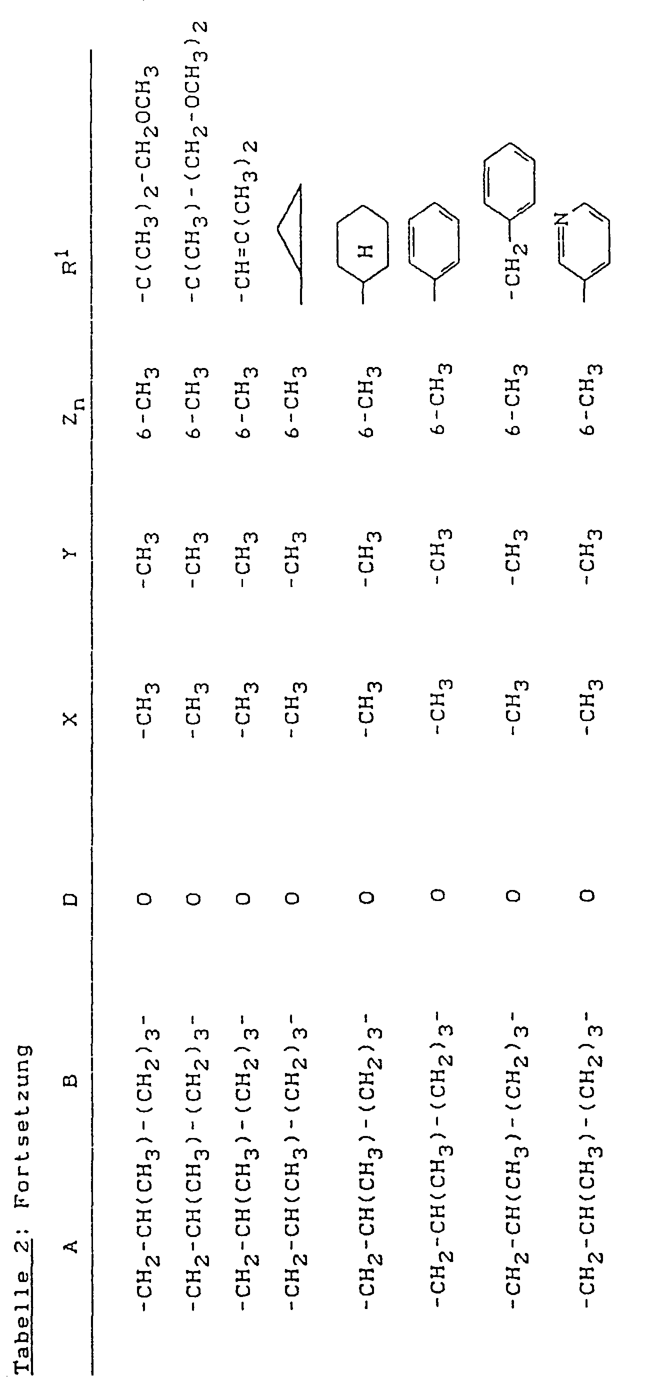

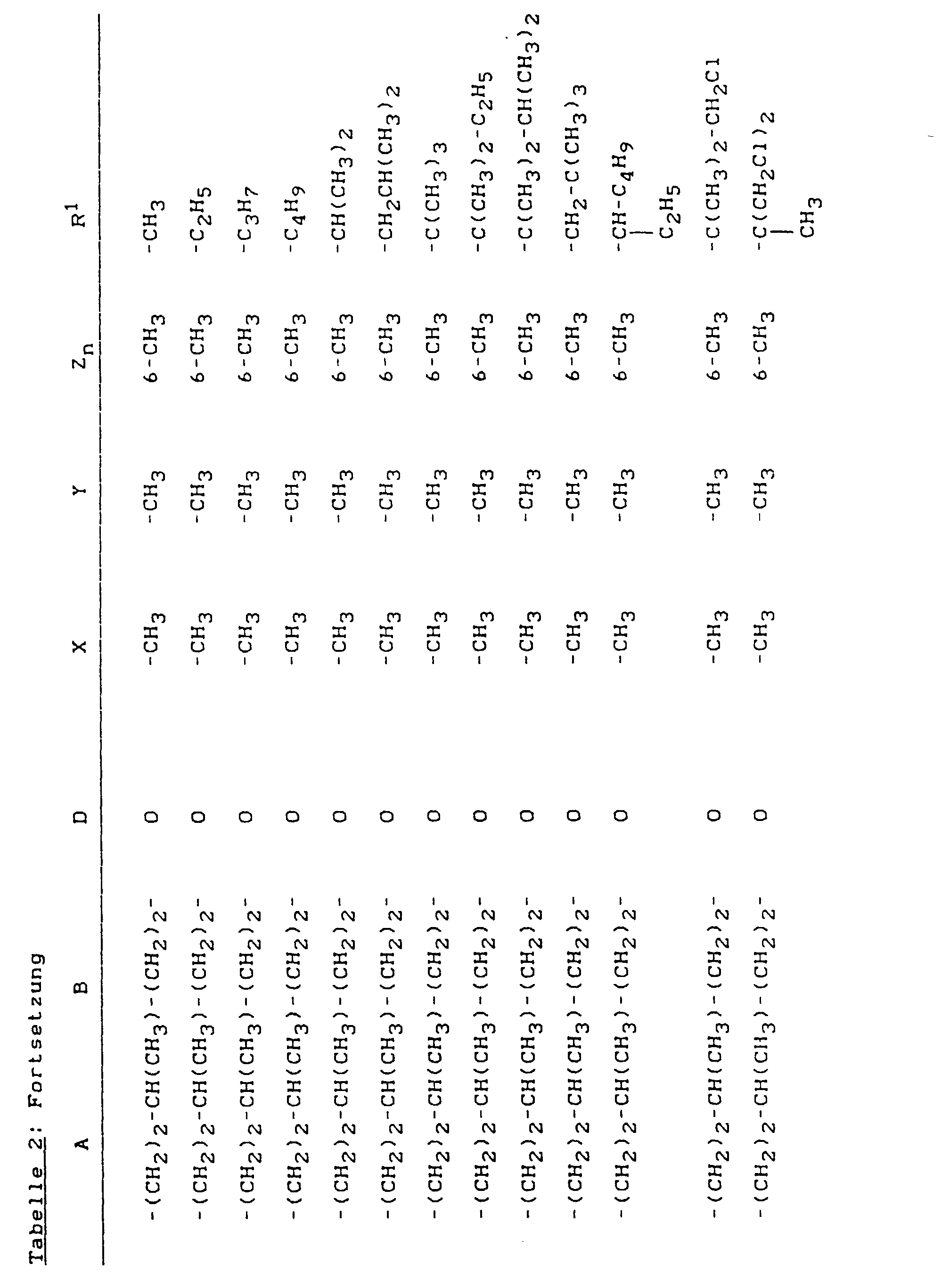

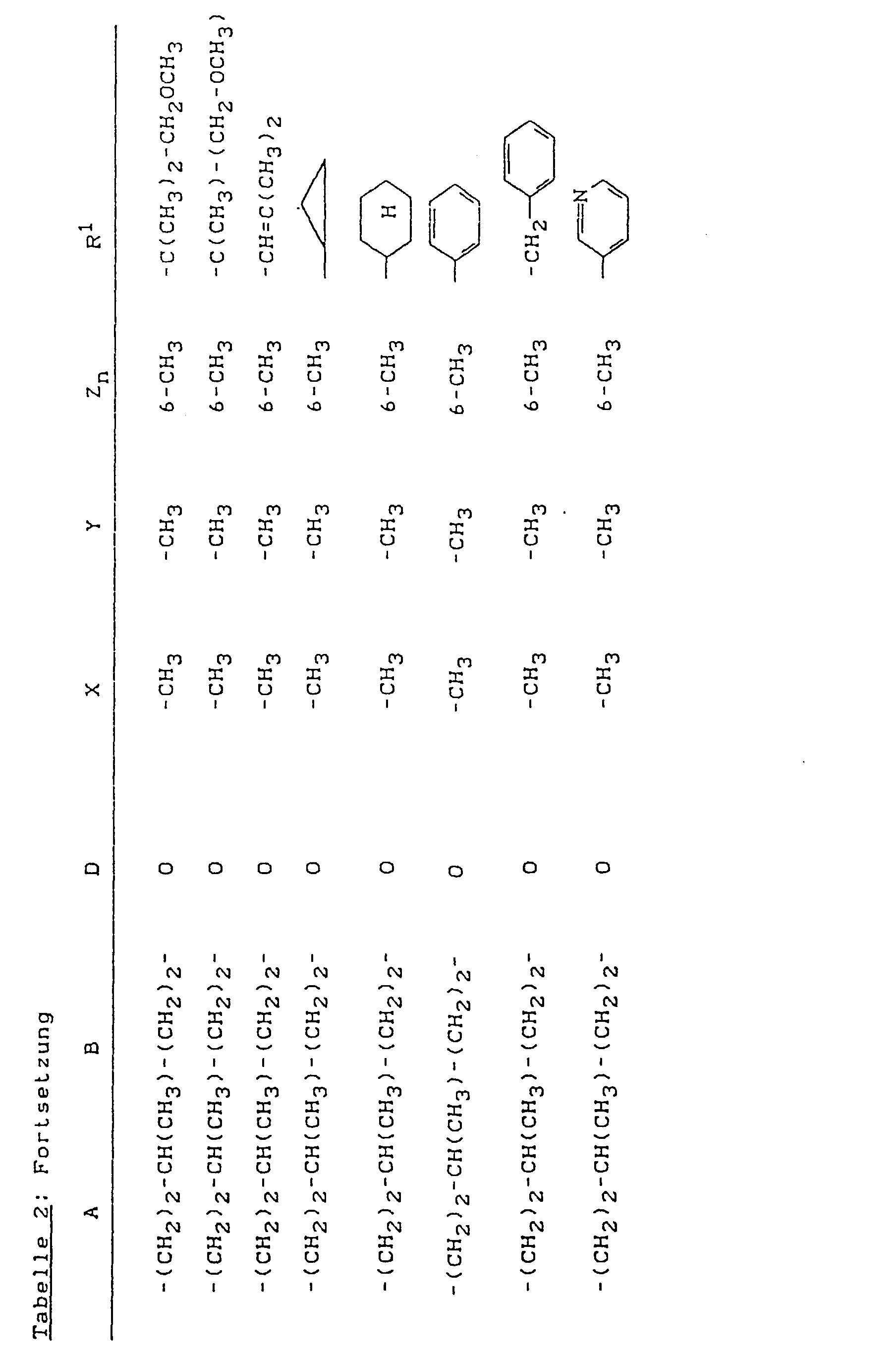

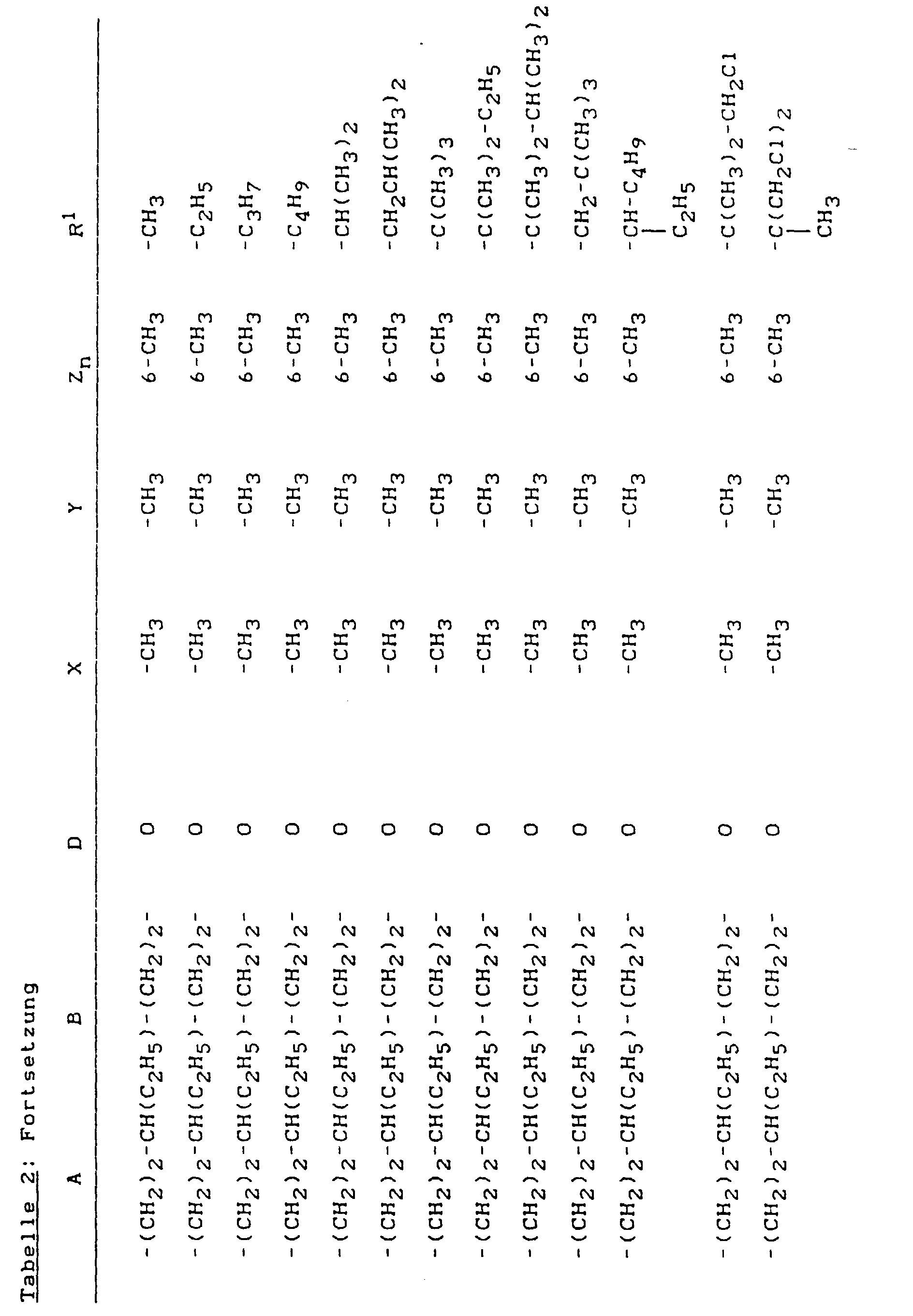

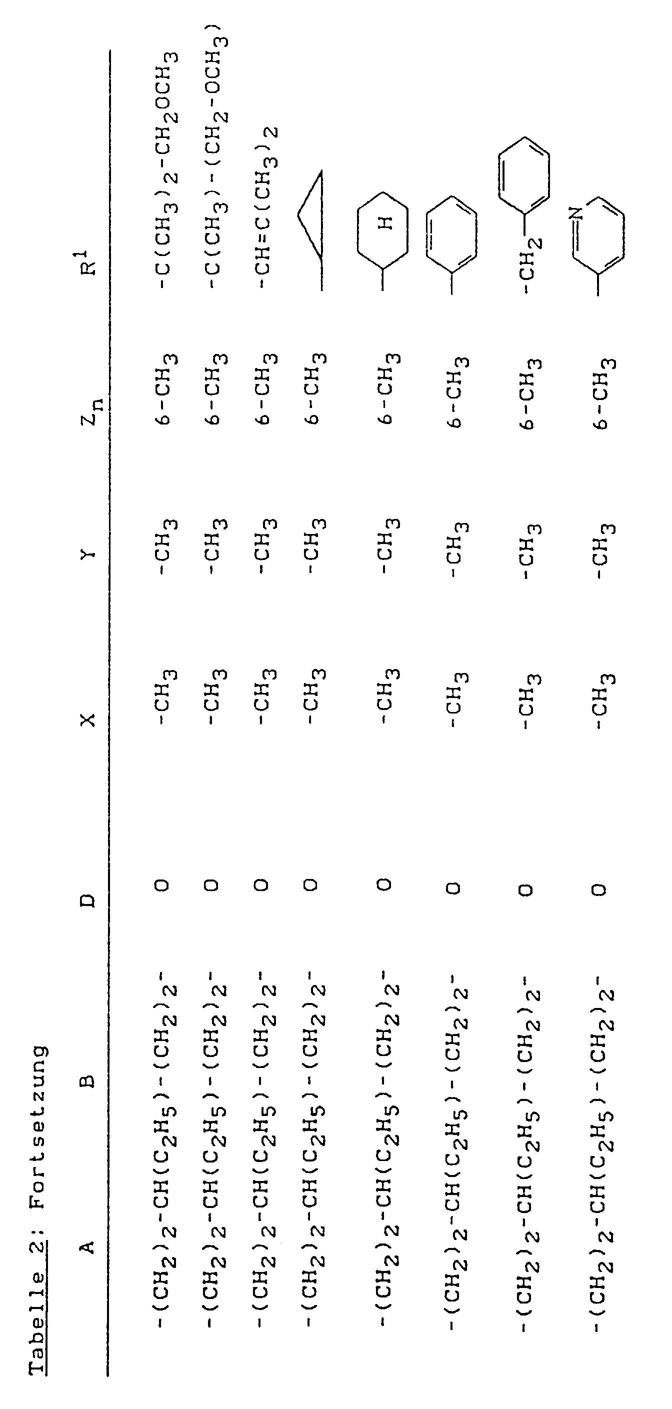

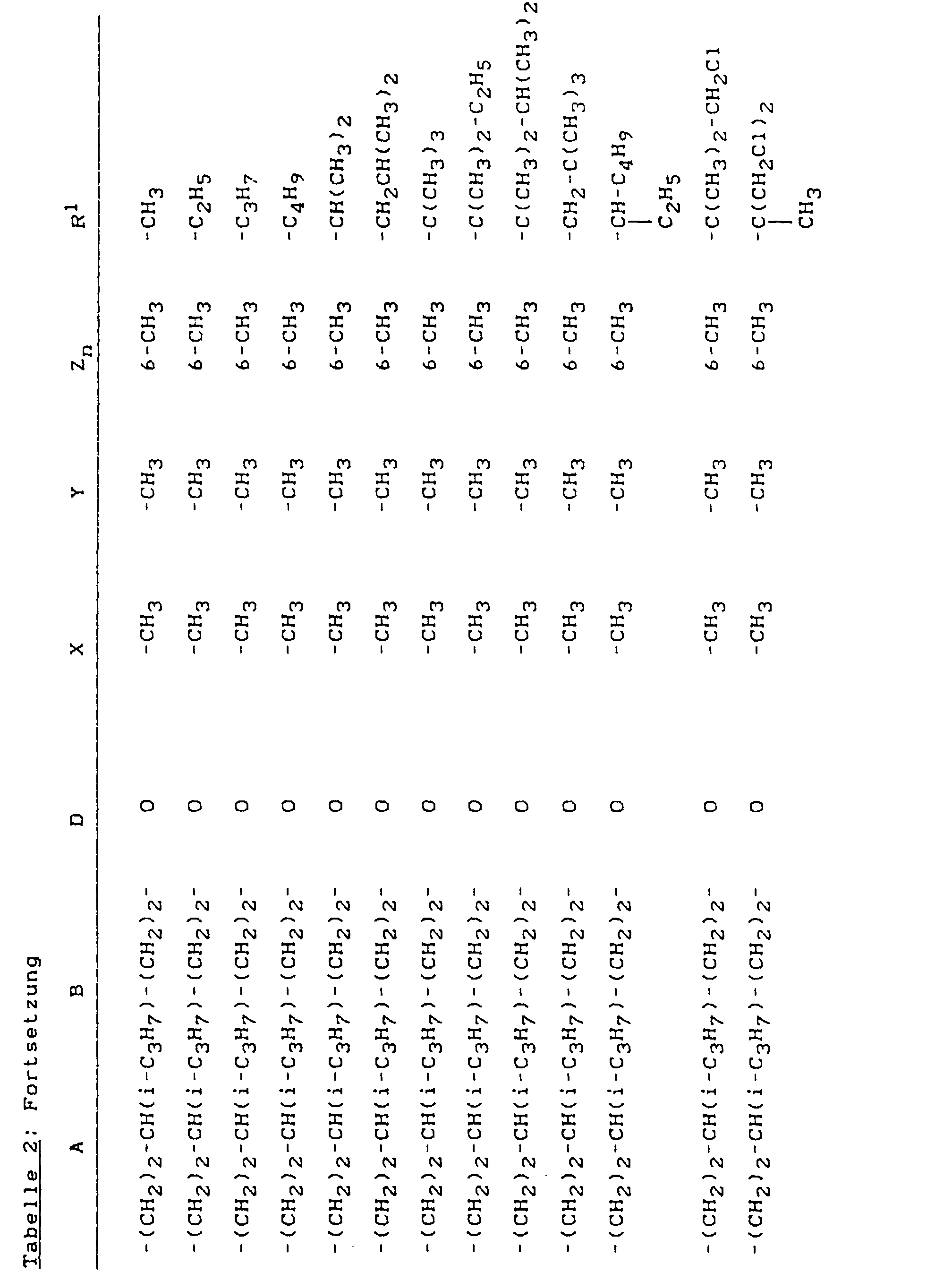

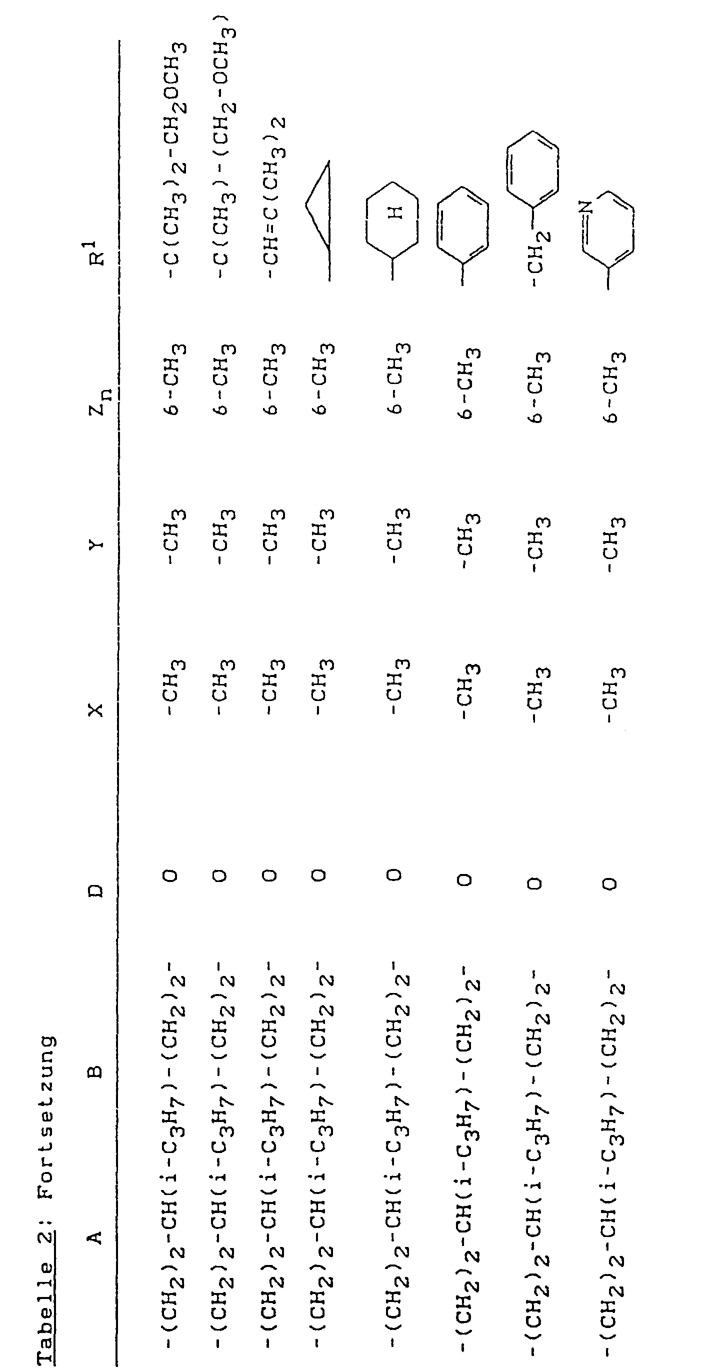

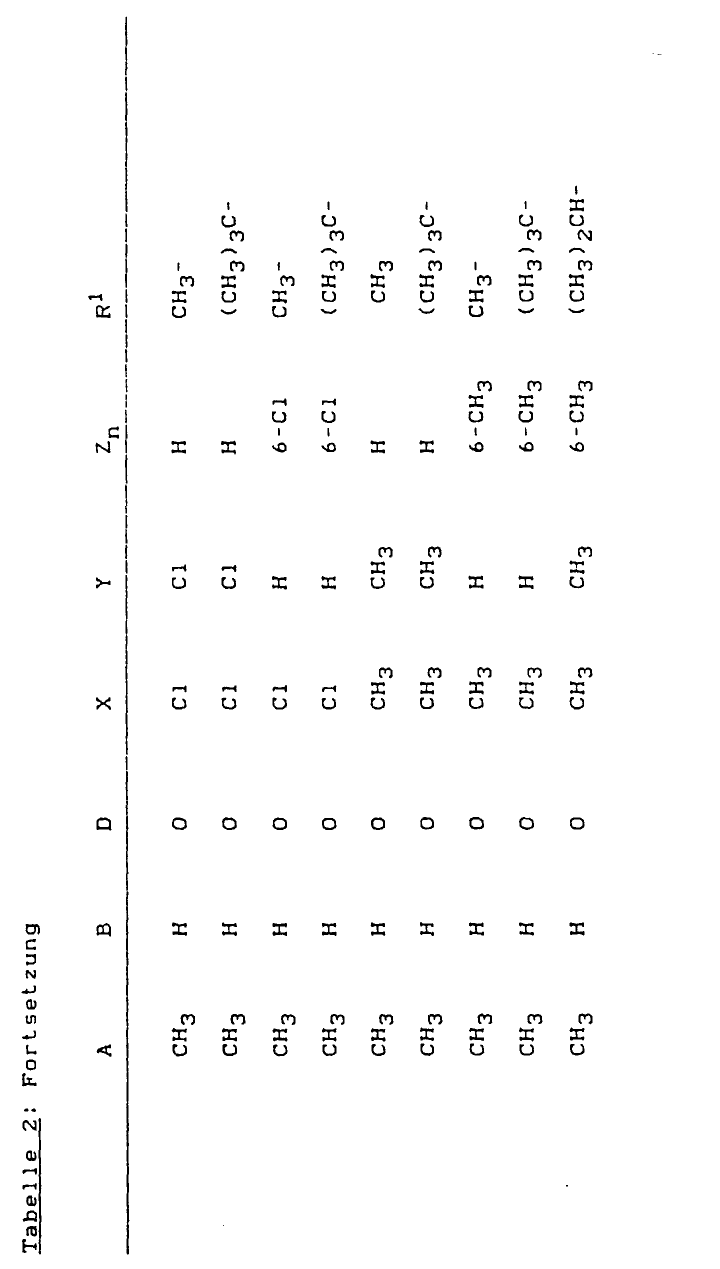

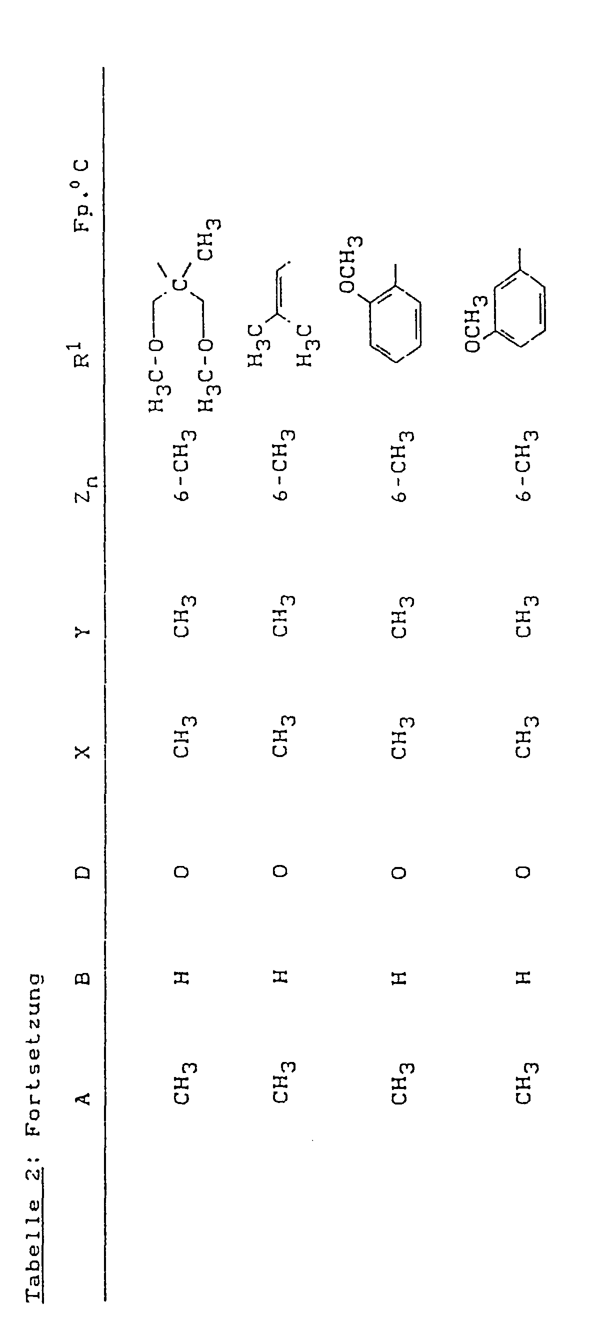

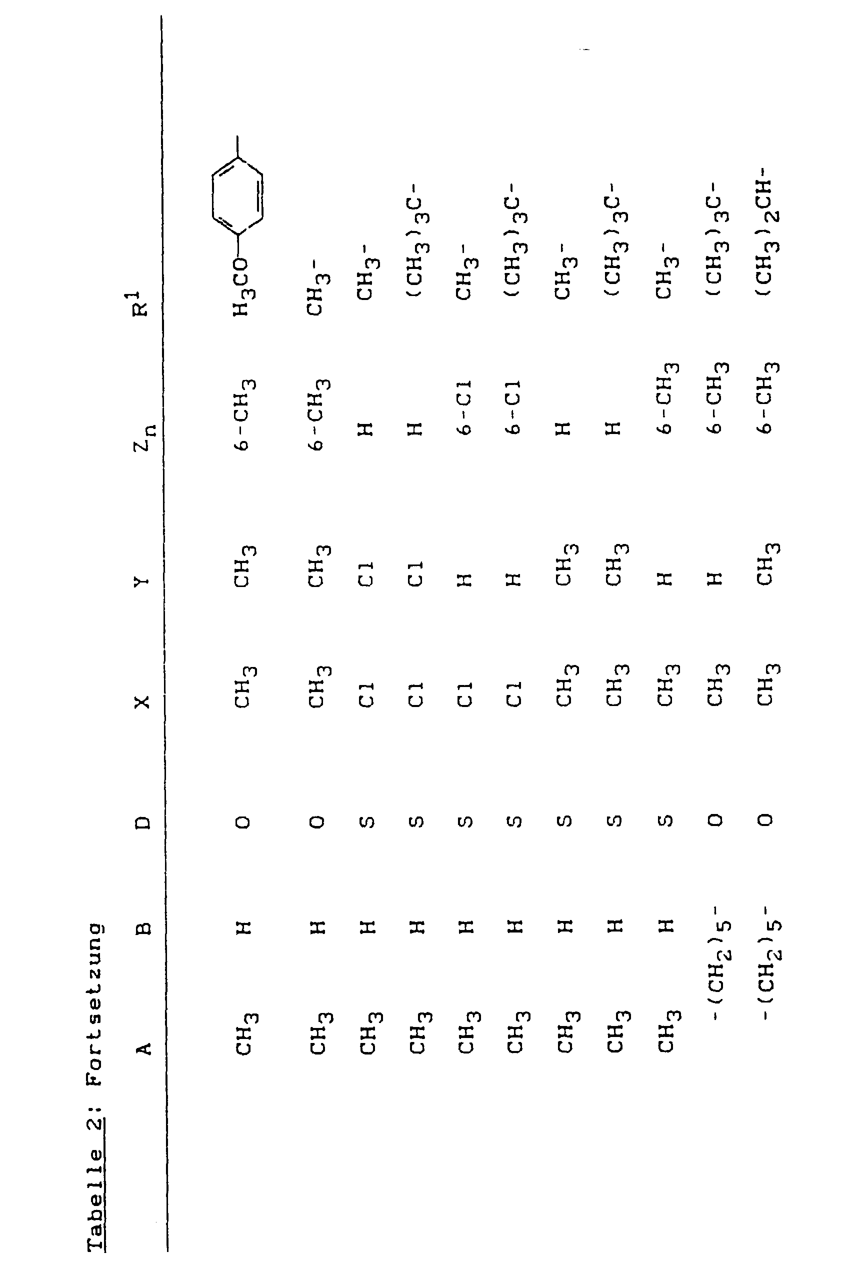

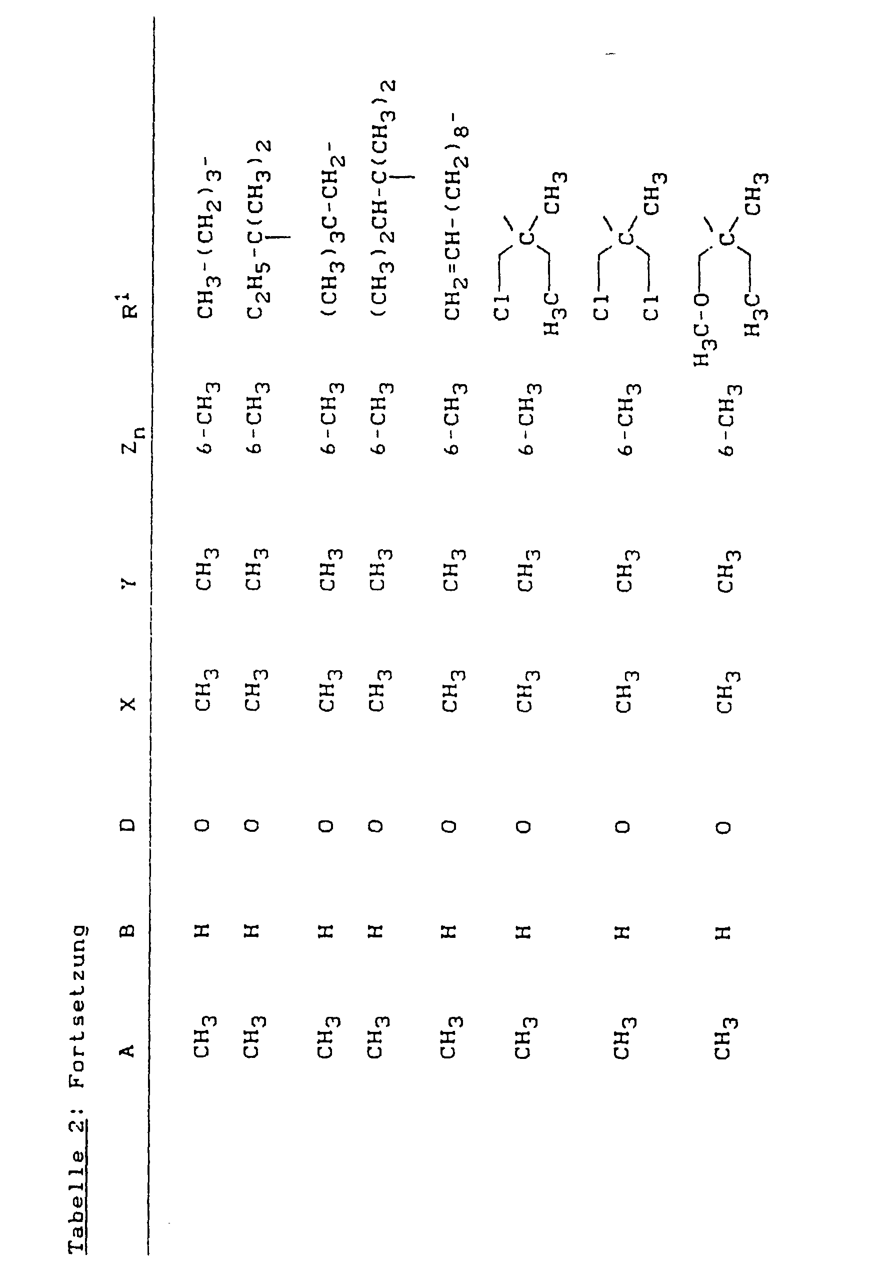

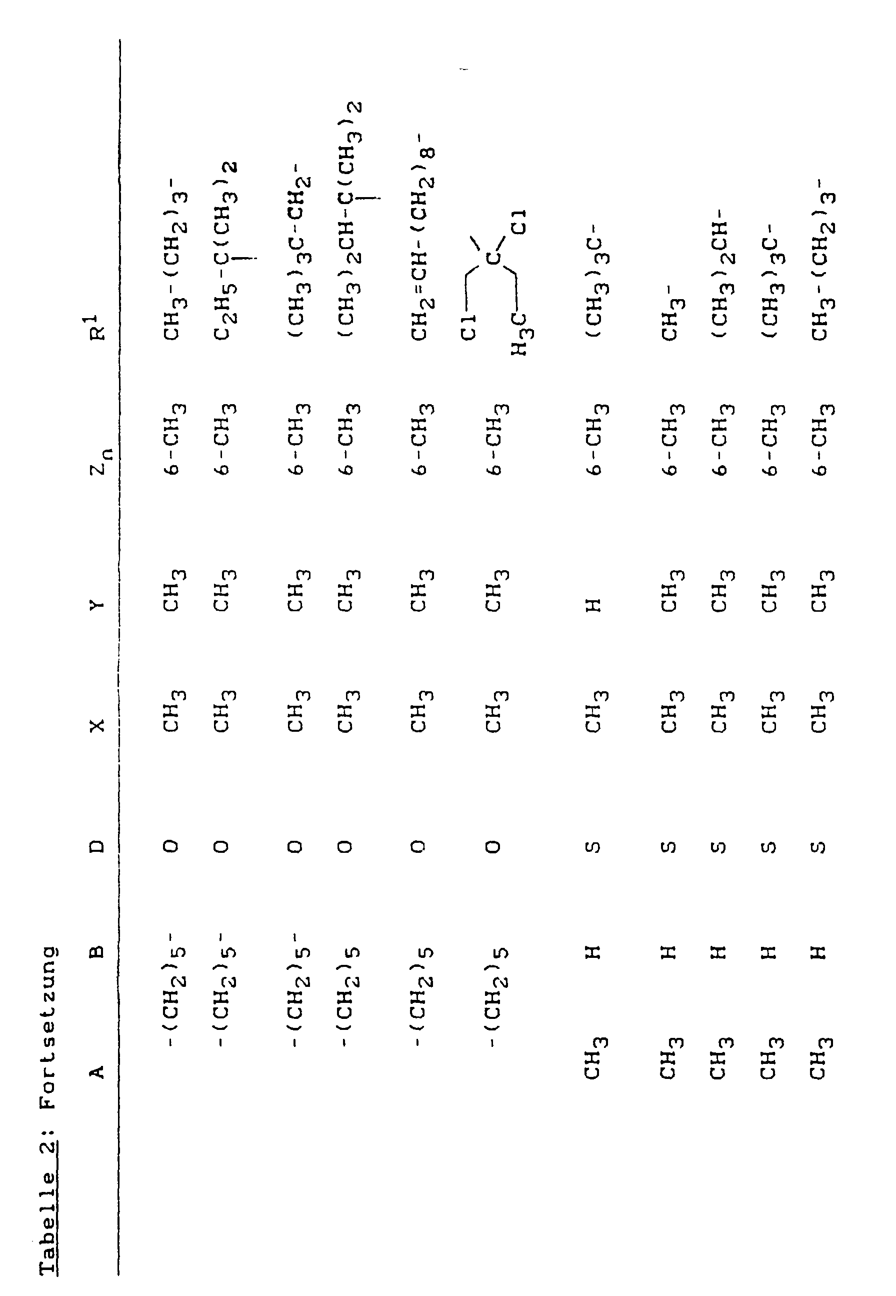

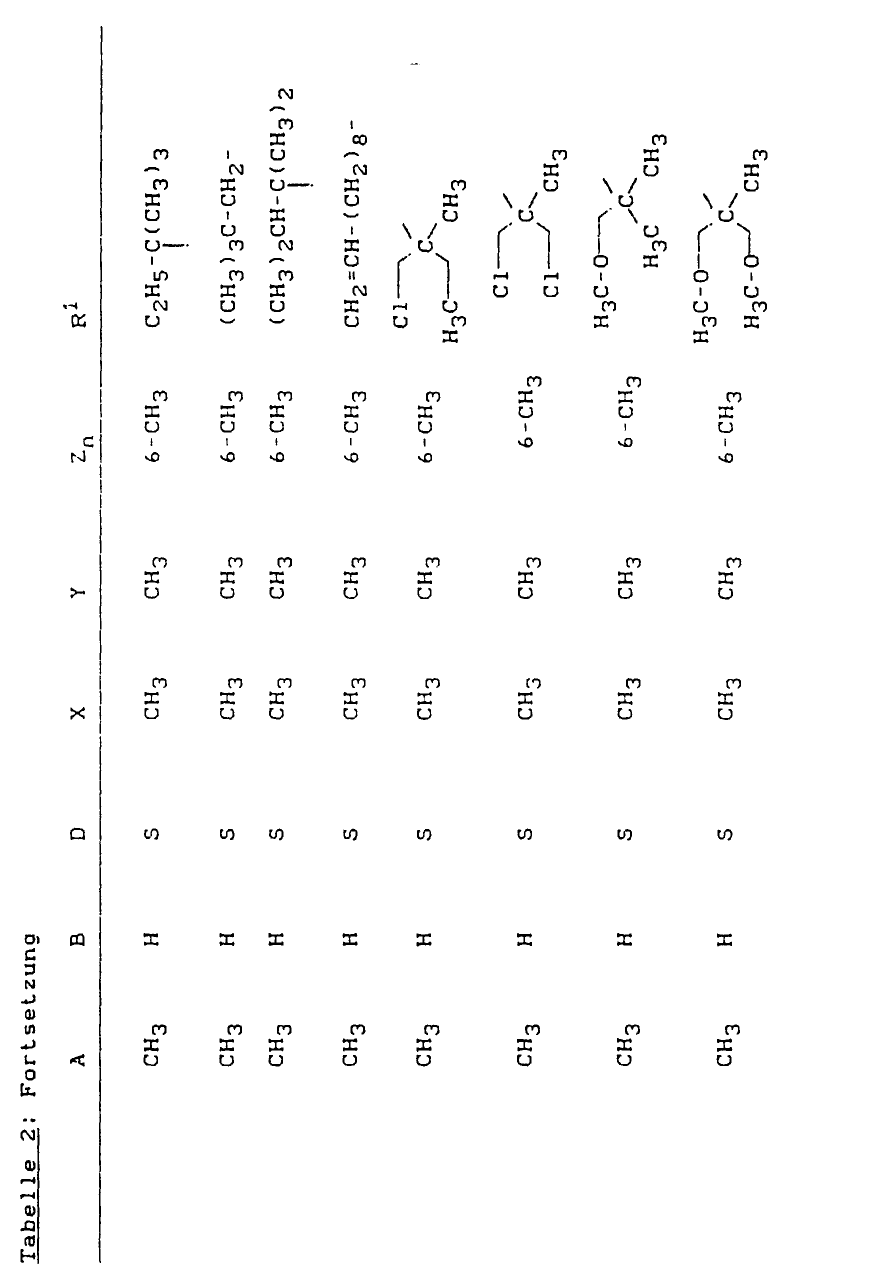

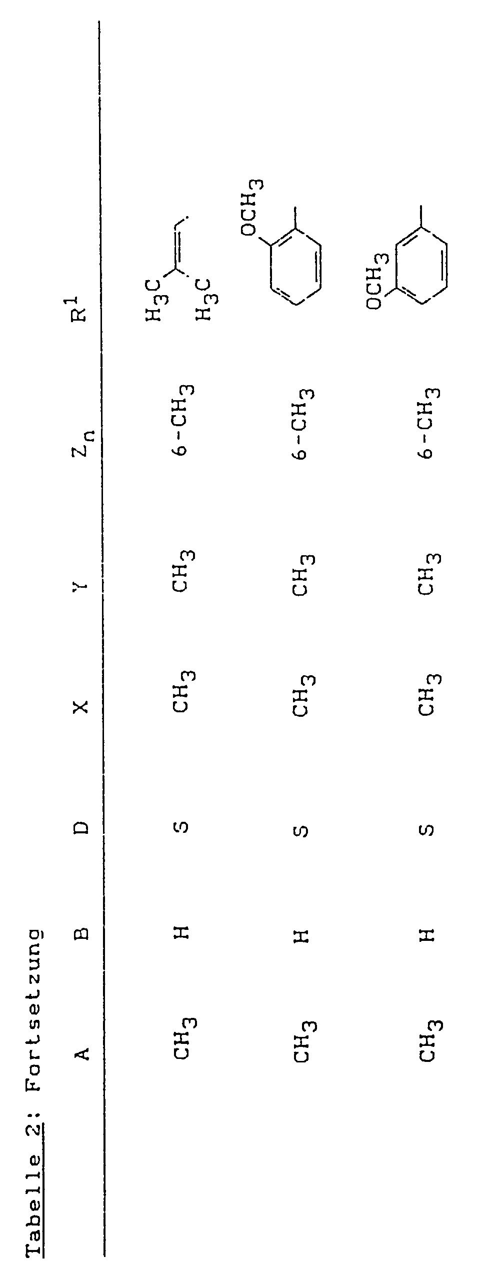

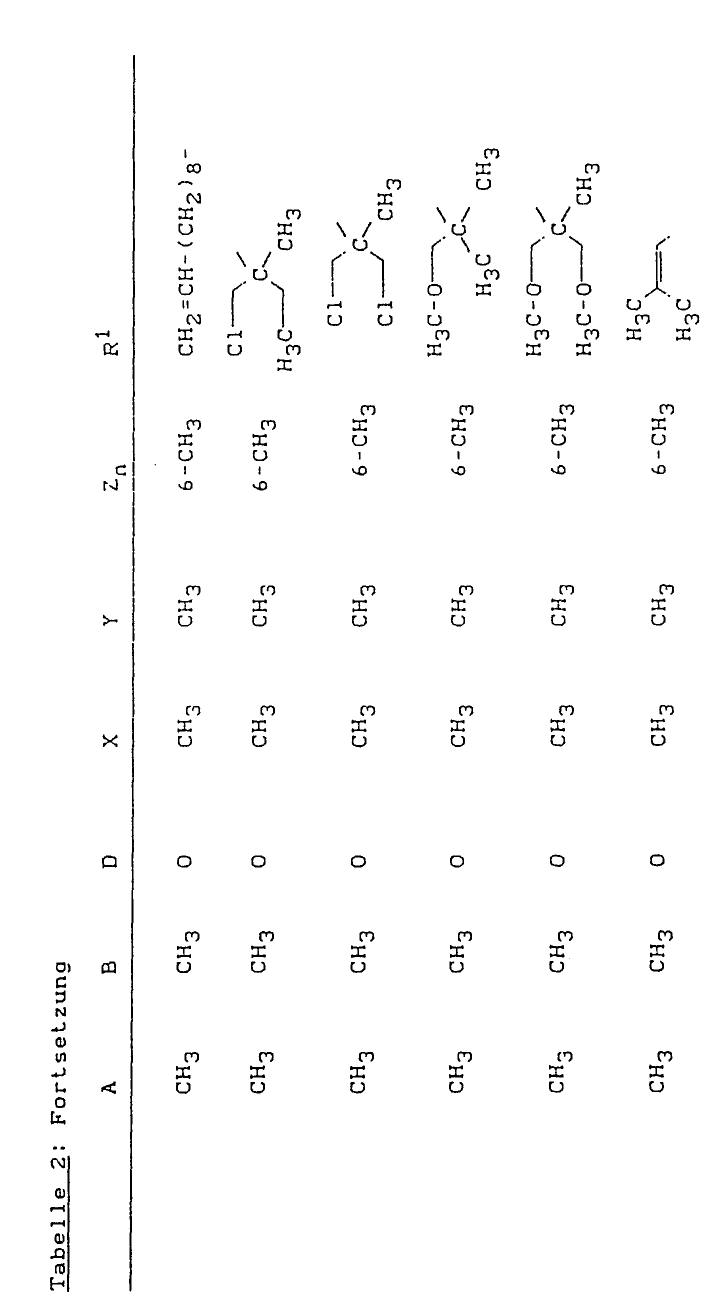

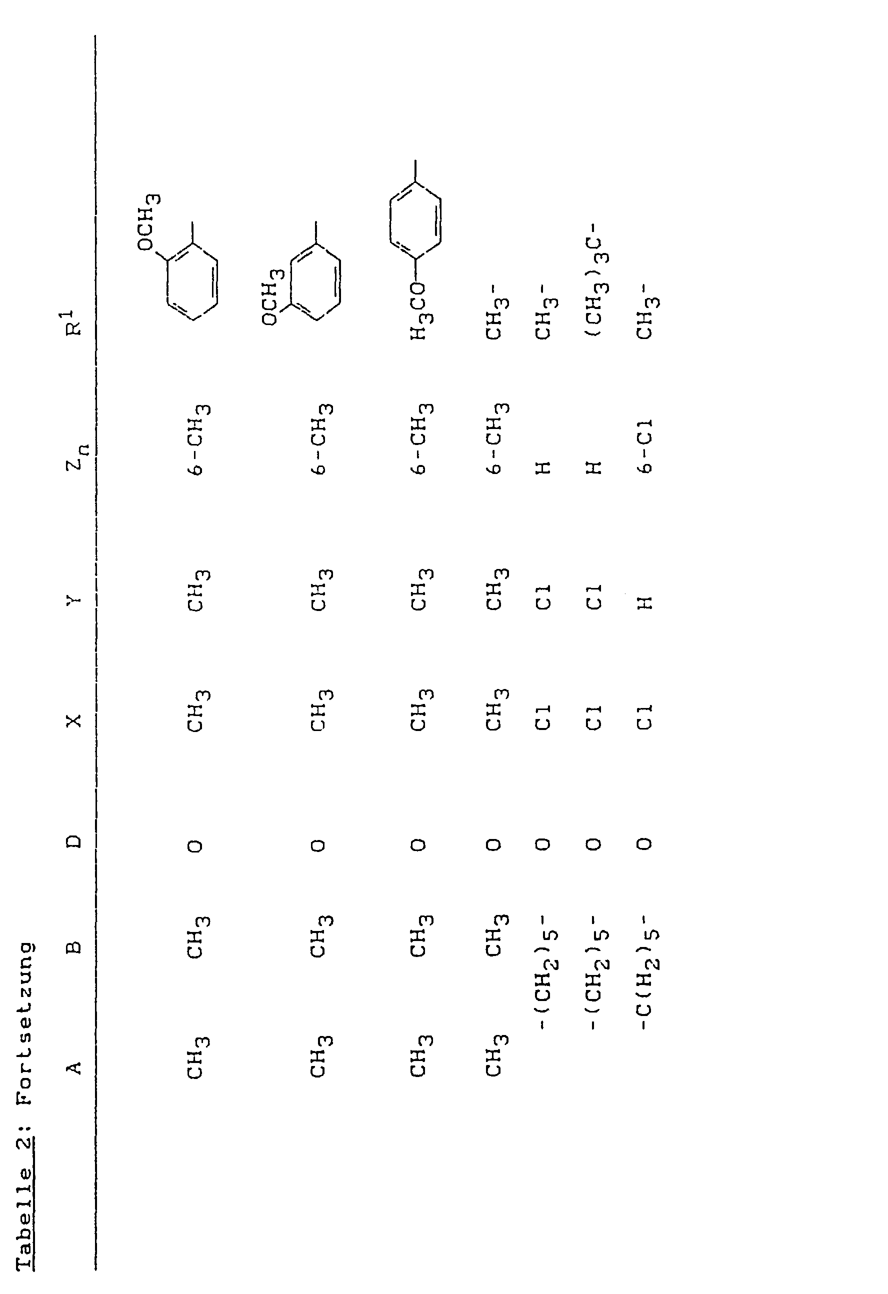

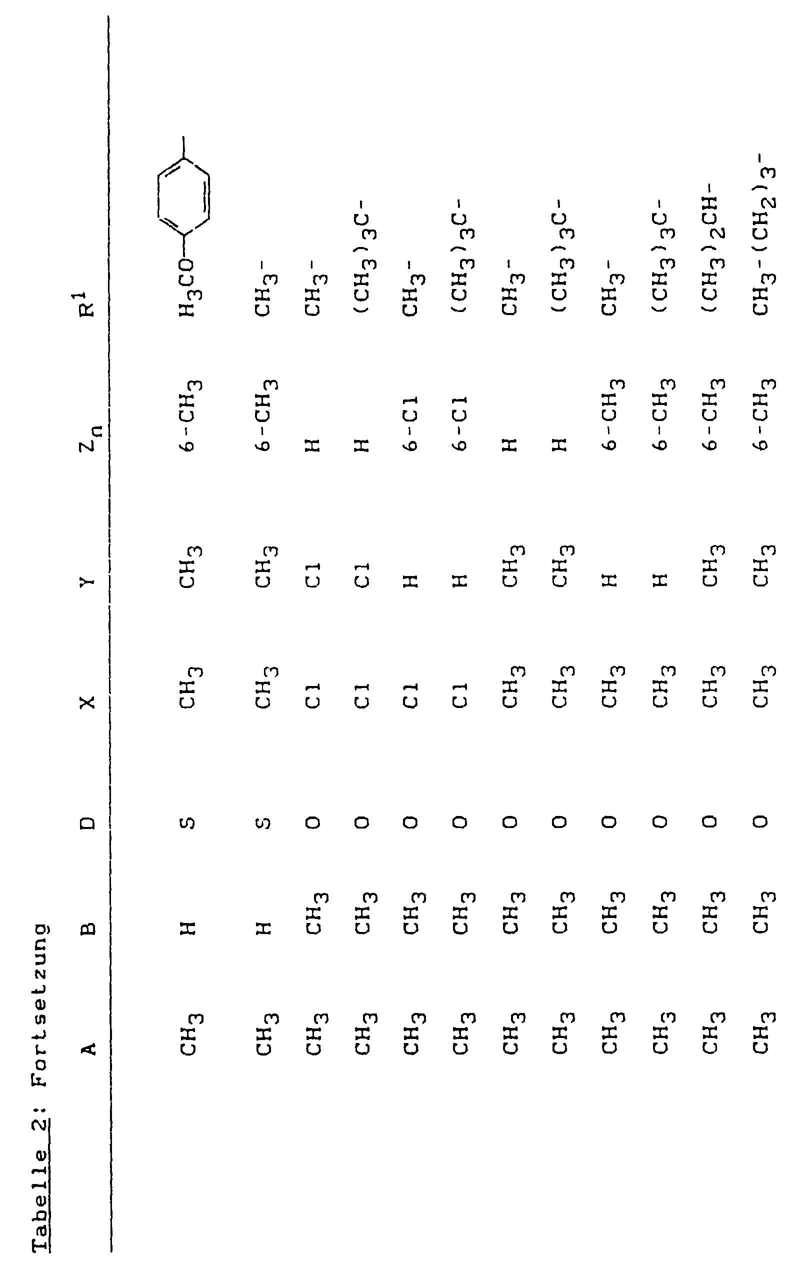

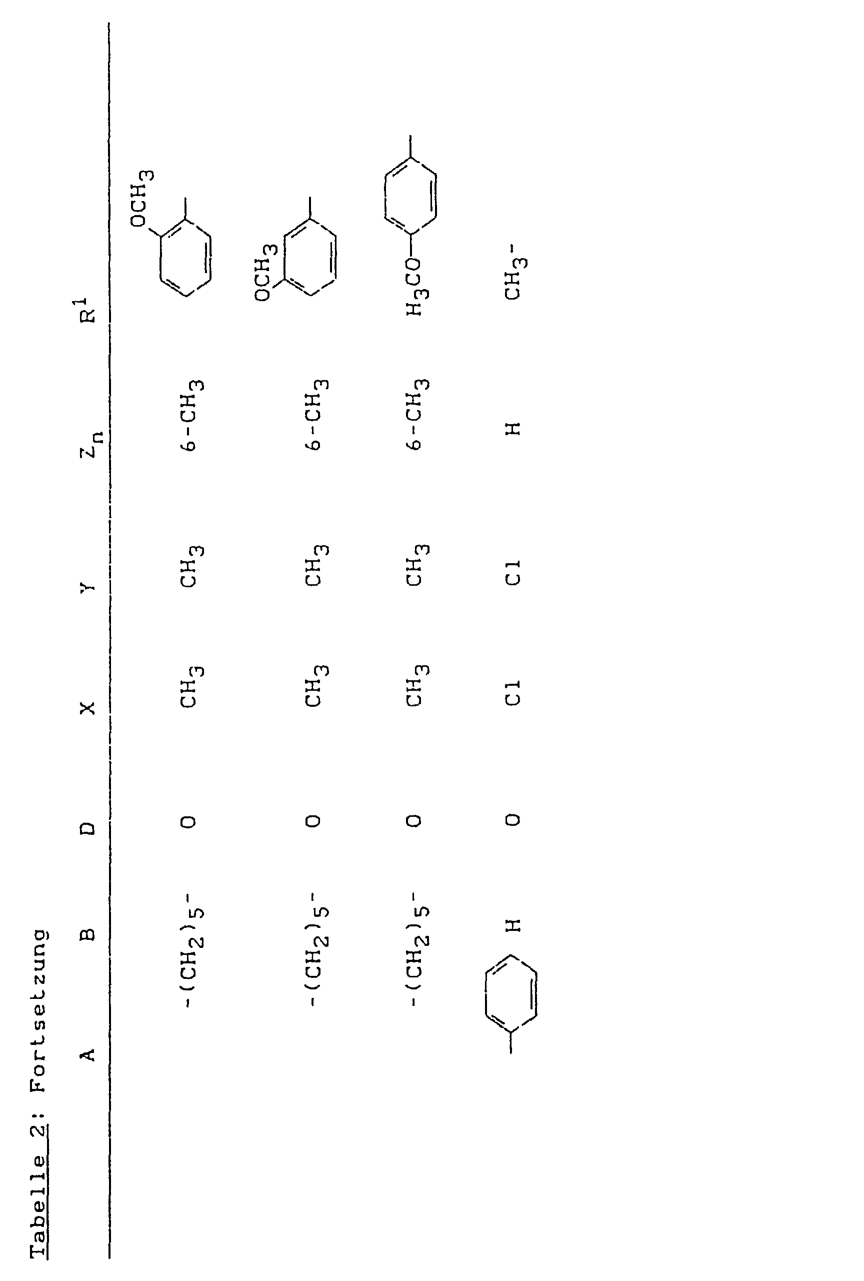

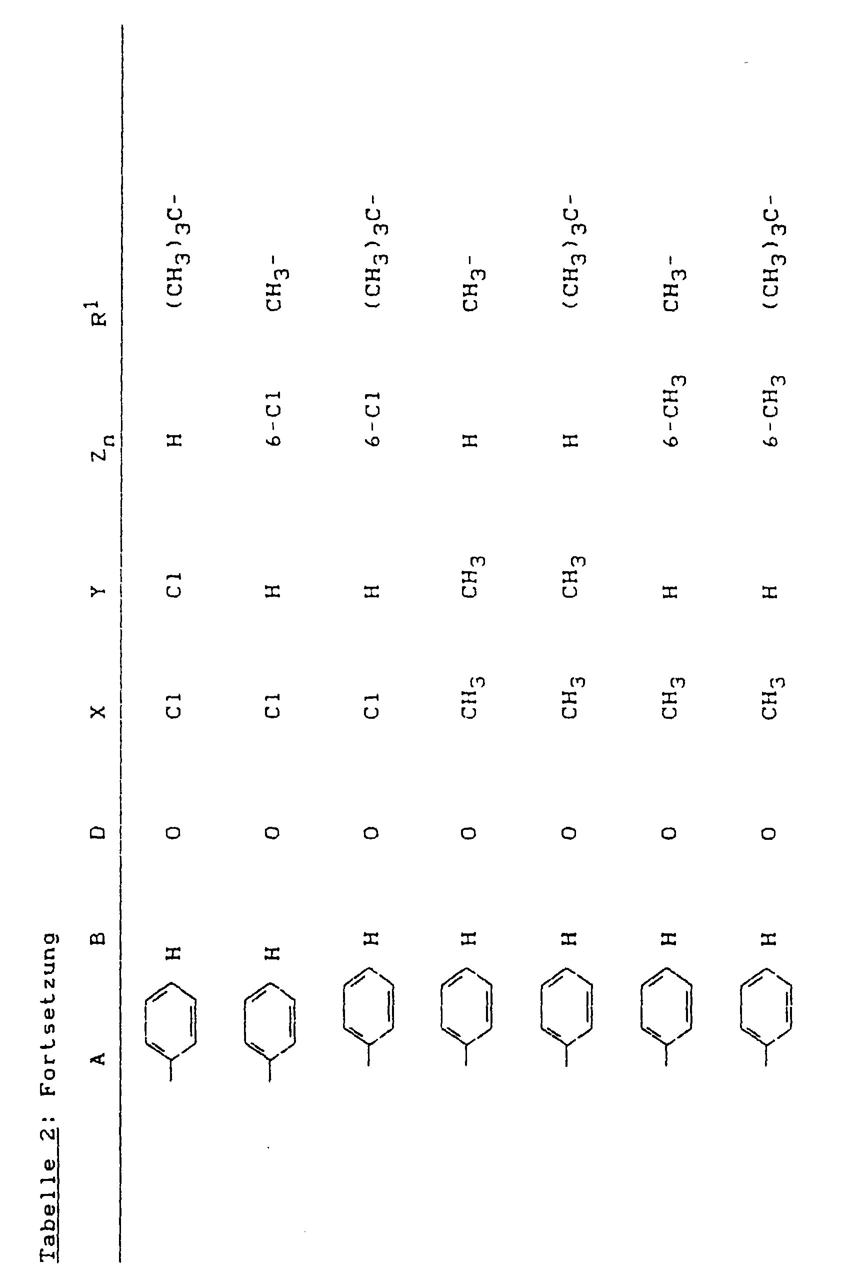

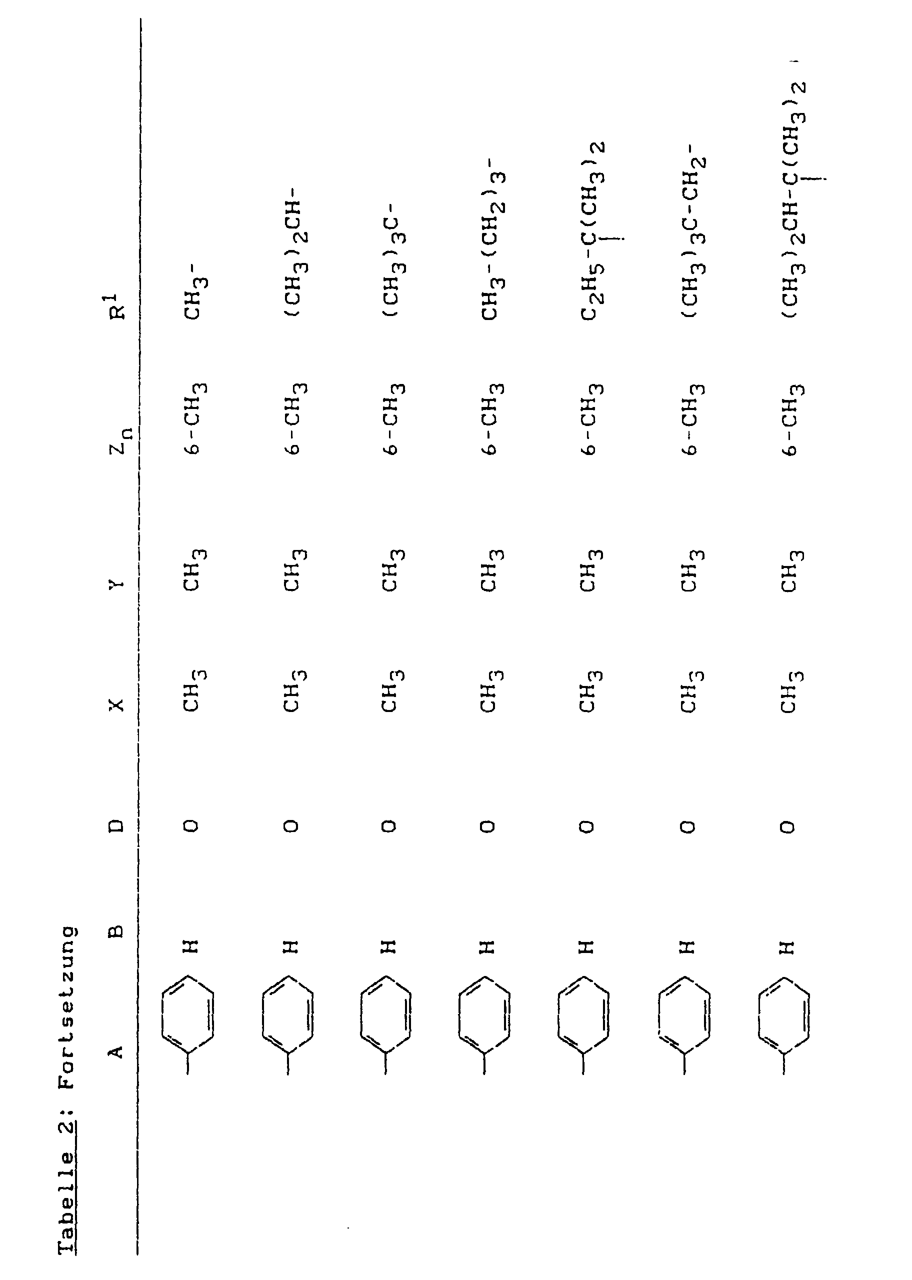

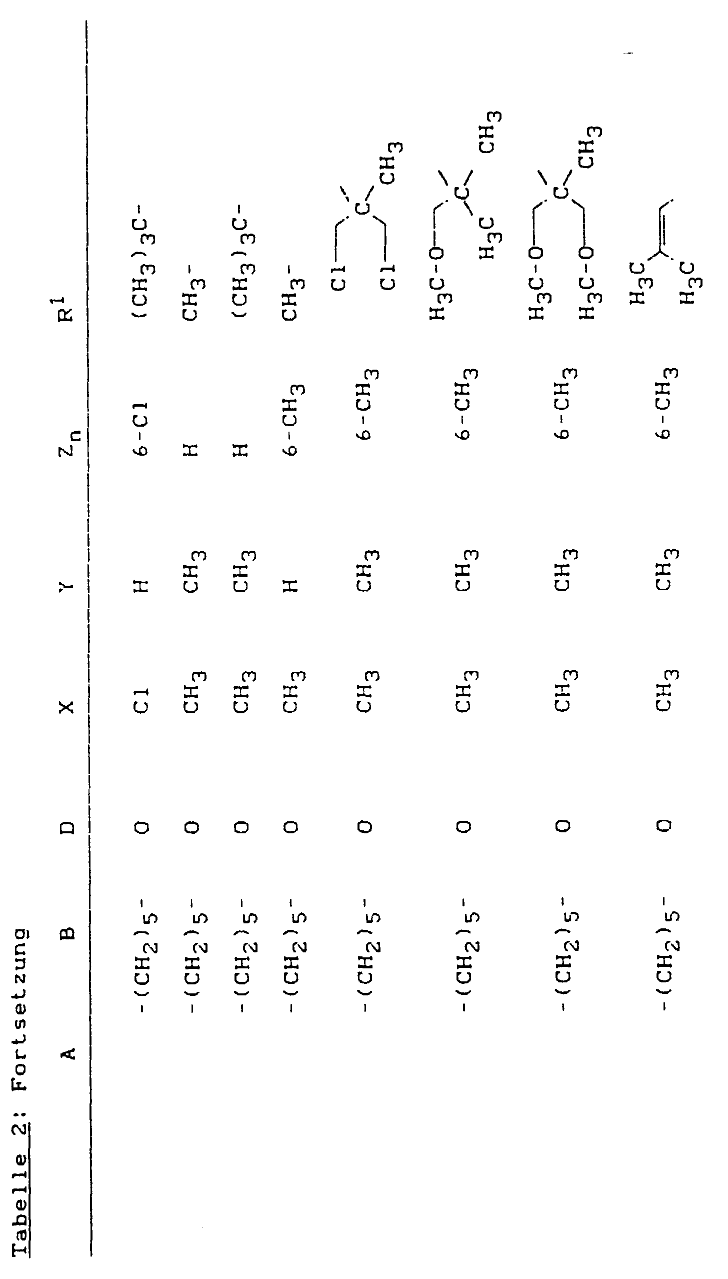

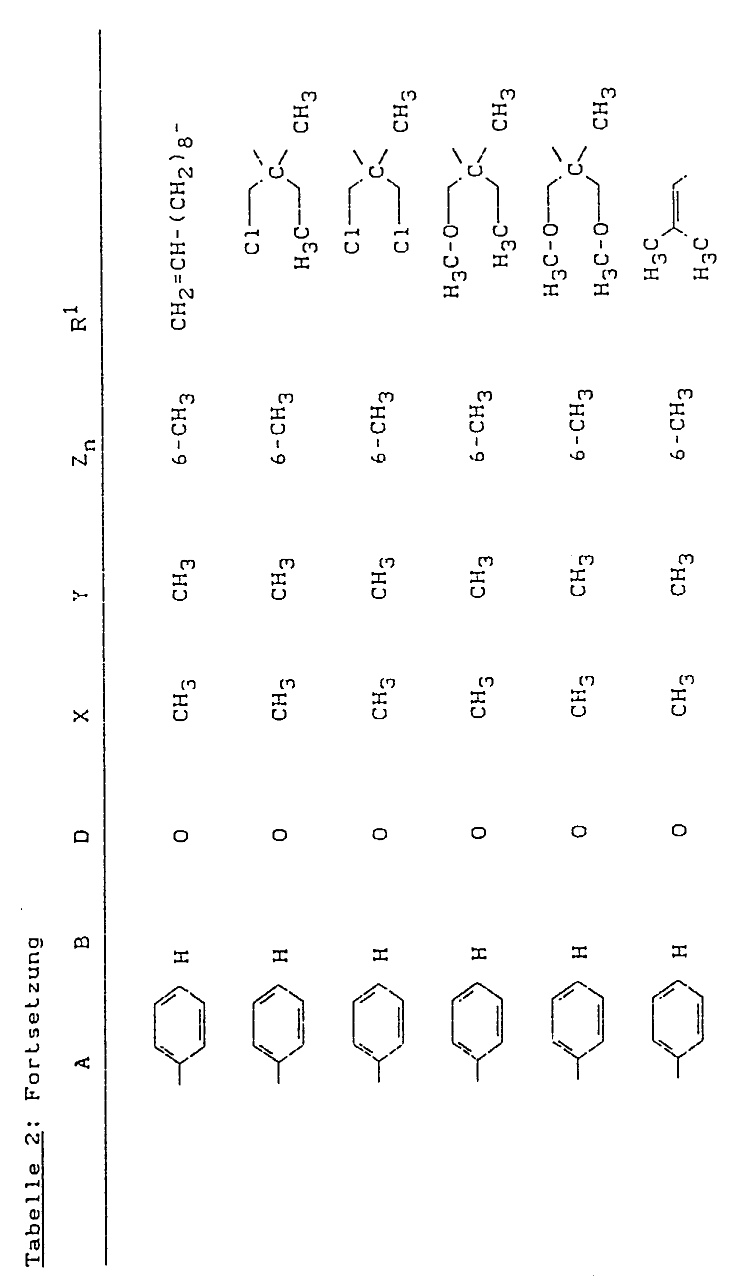

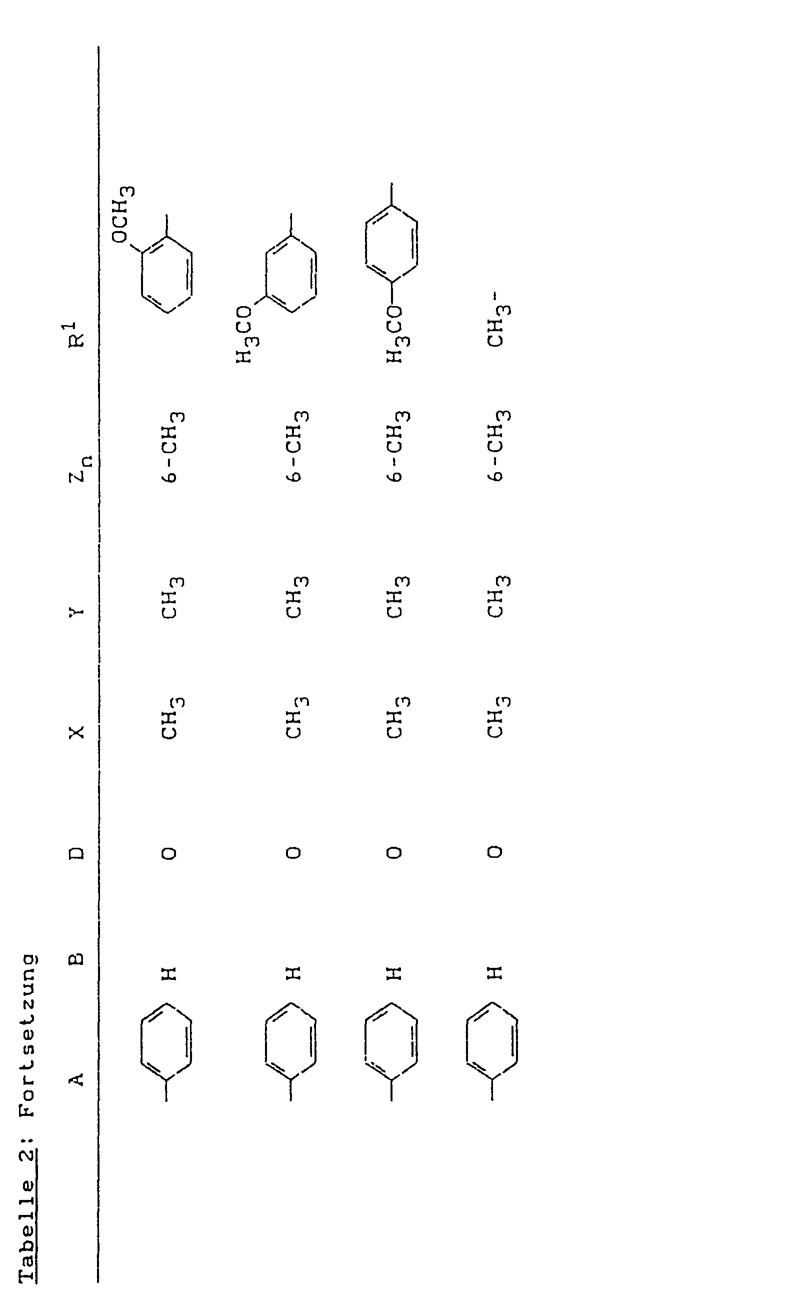

Im einzelnen seien außer den bei den Herstellungsbeispielen genannten Verbindungen die folgenden 3-Aryl-4-hydroxy-Δ³-dihydrofuran-Derivate der Formel (Ic) genannt:

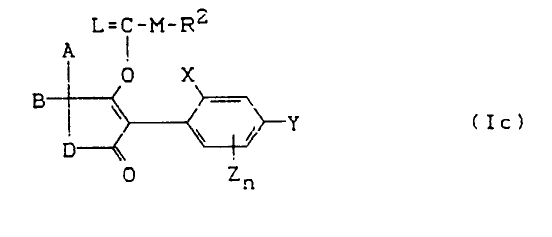

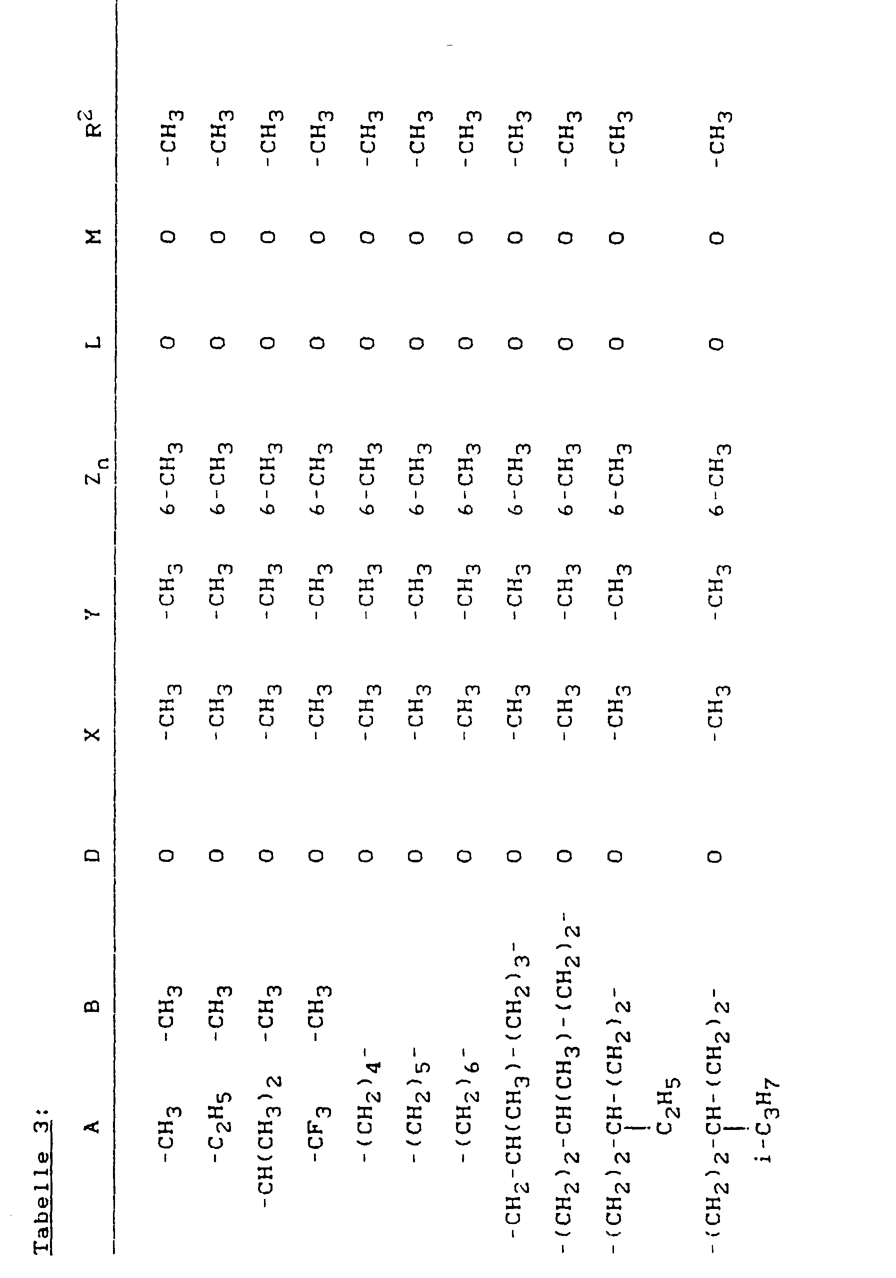

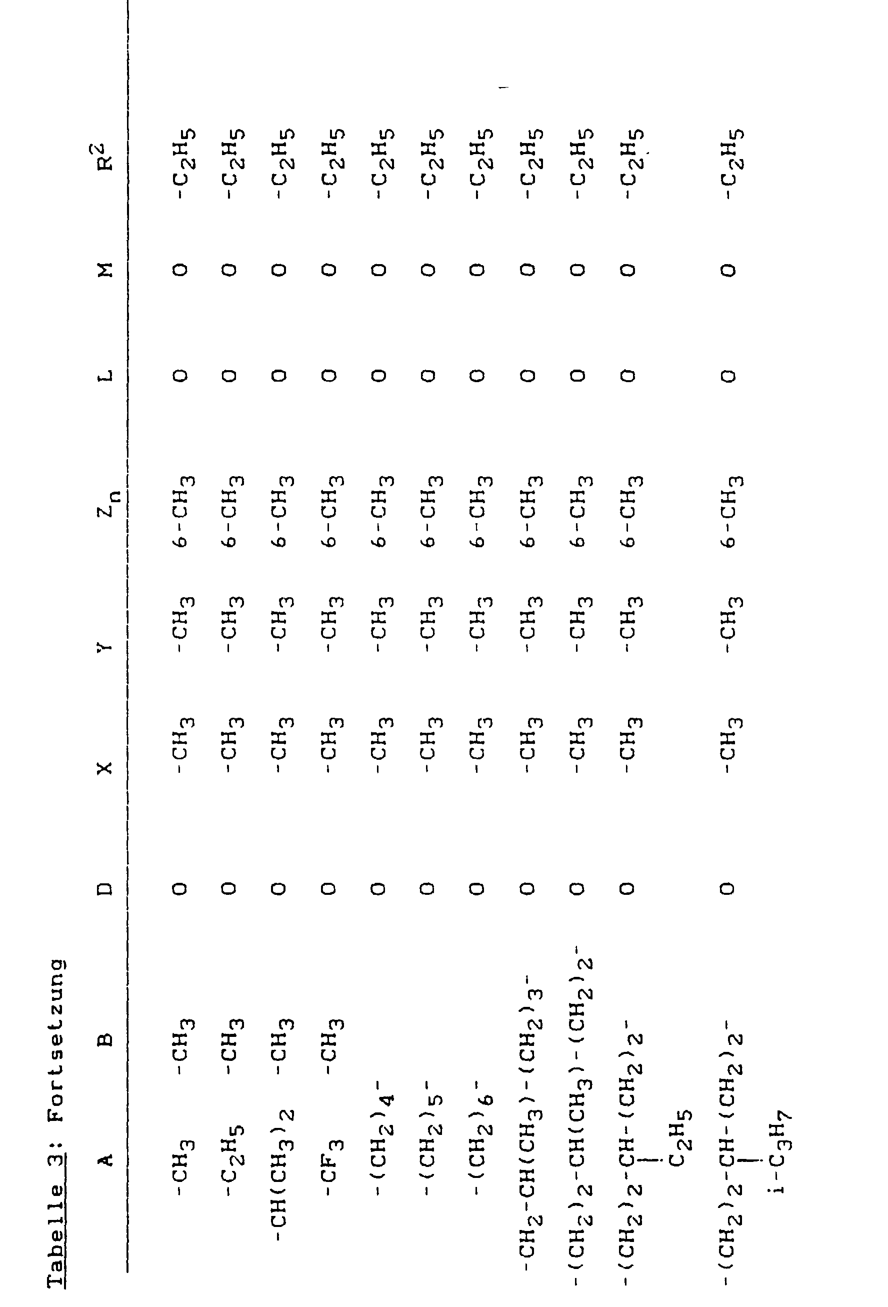

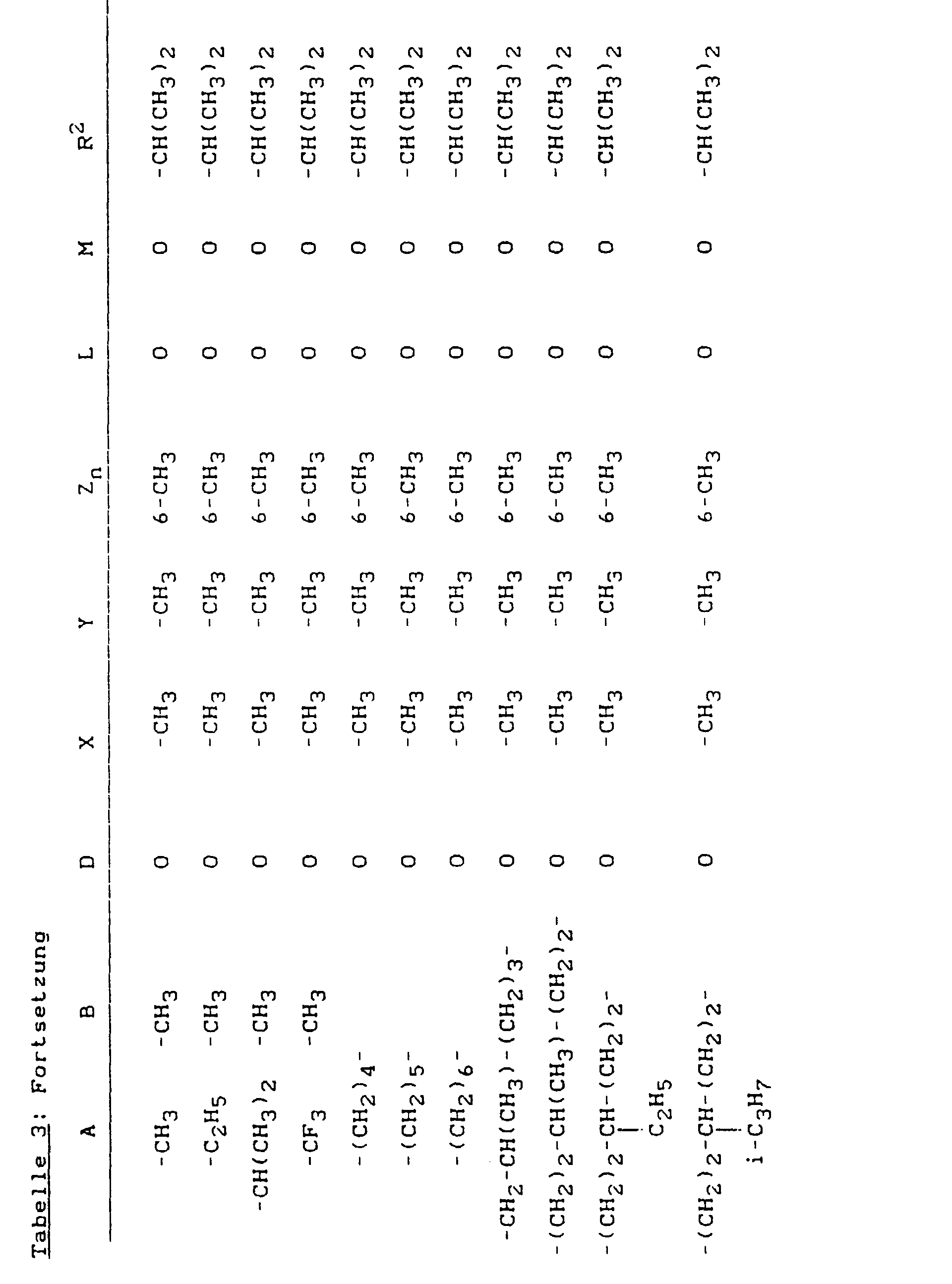

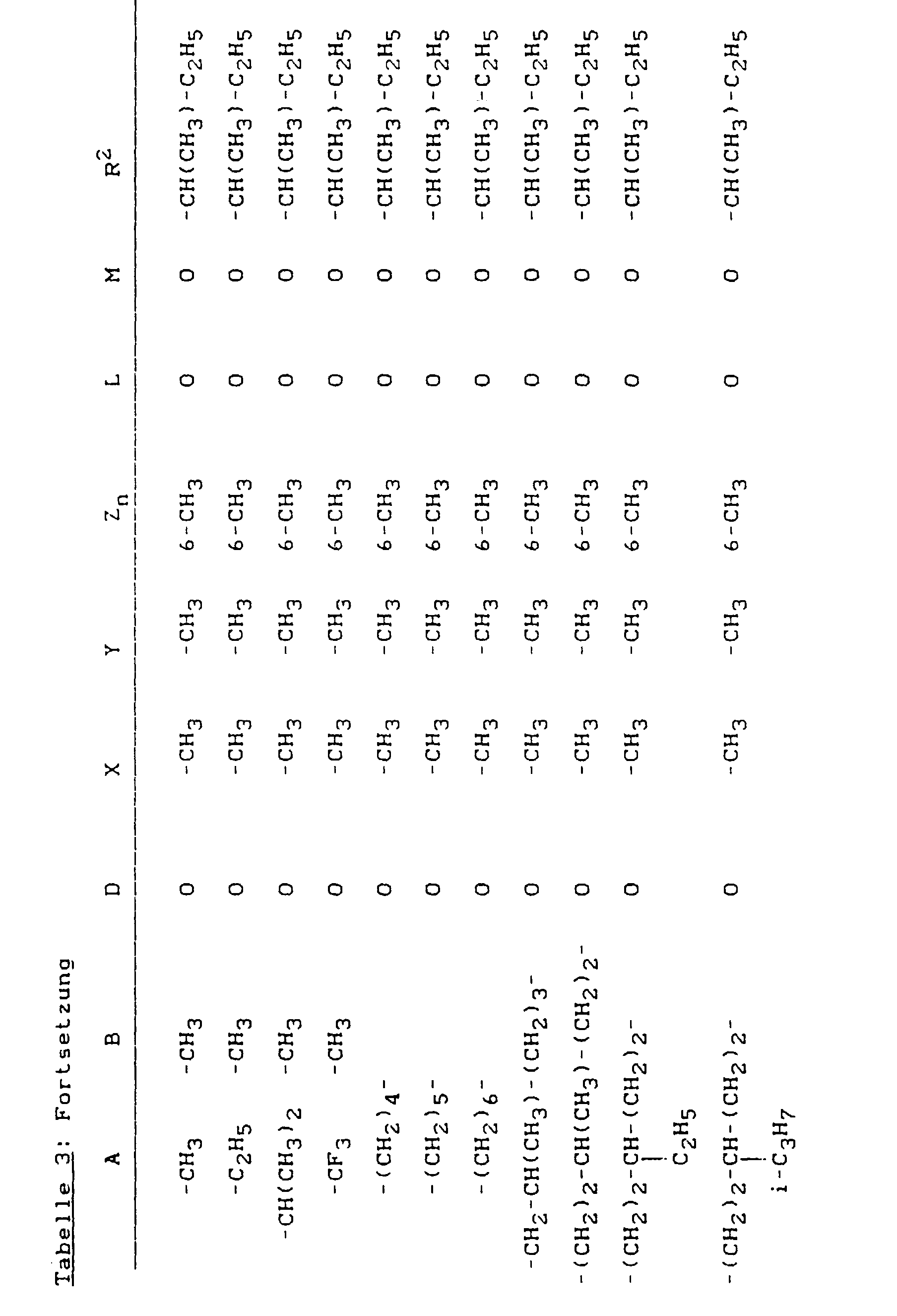

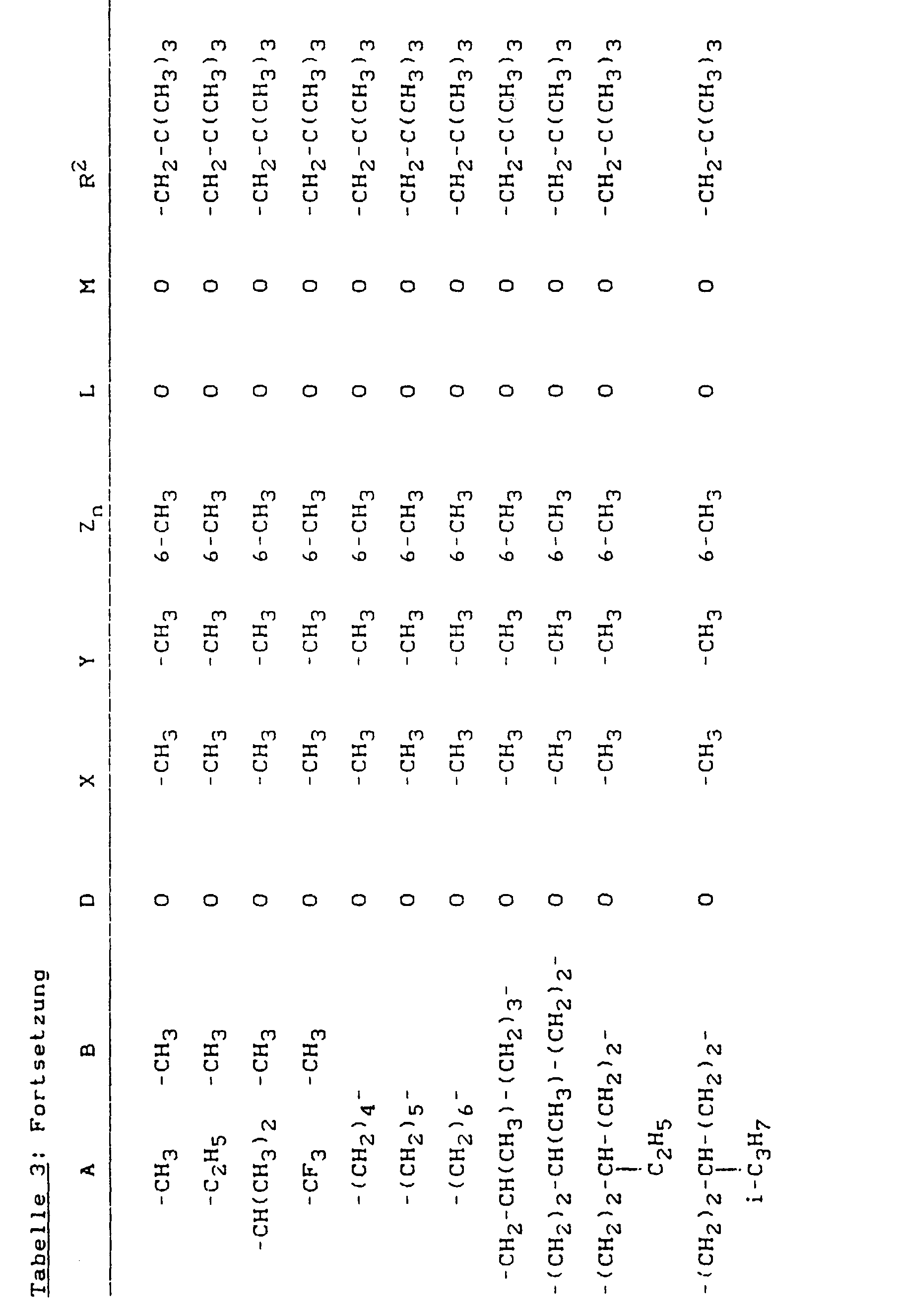

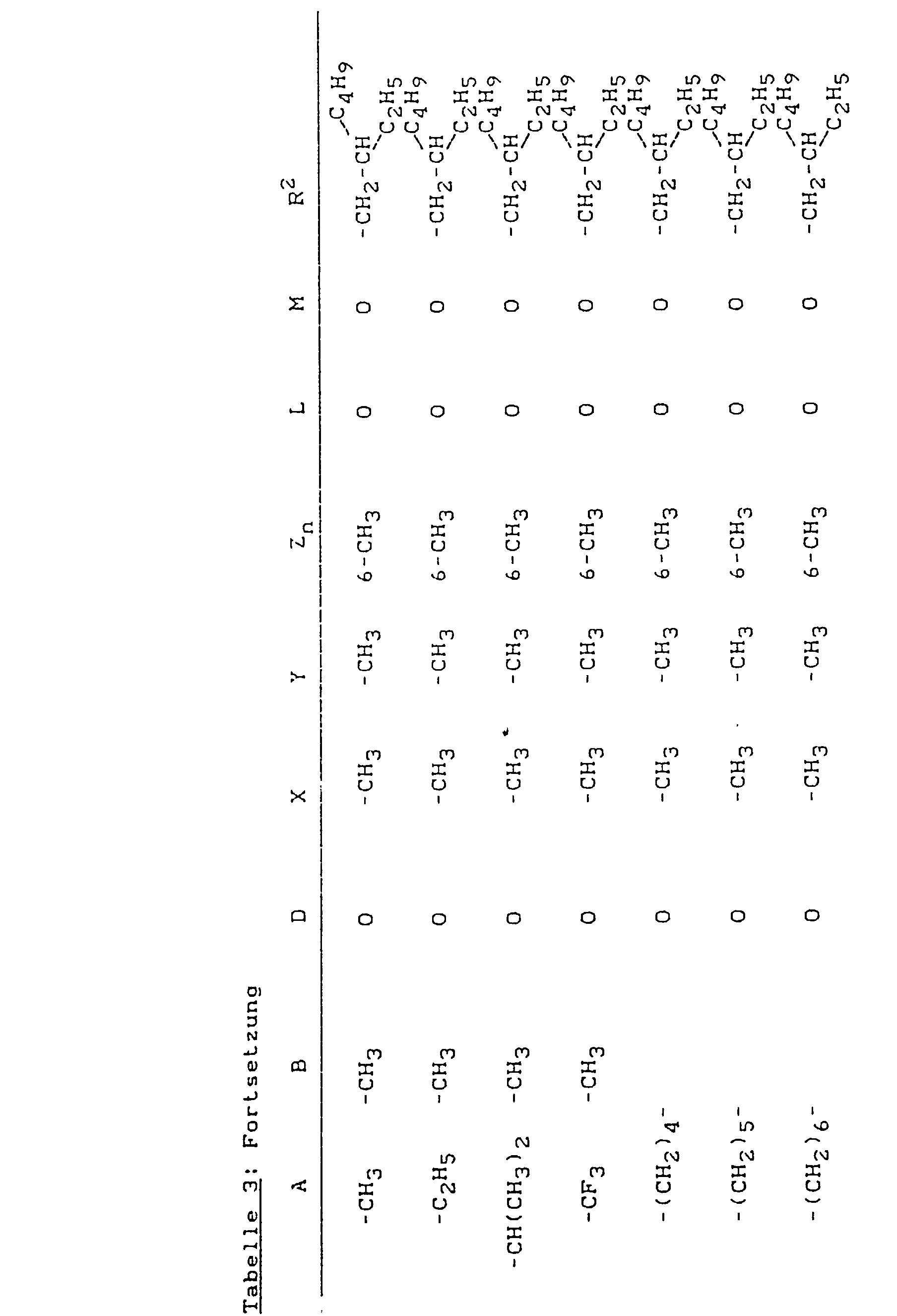

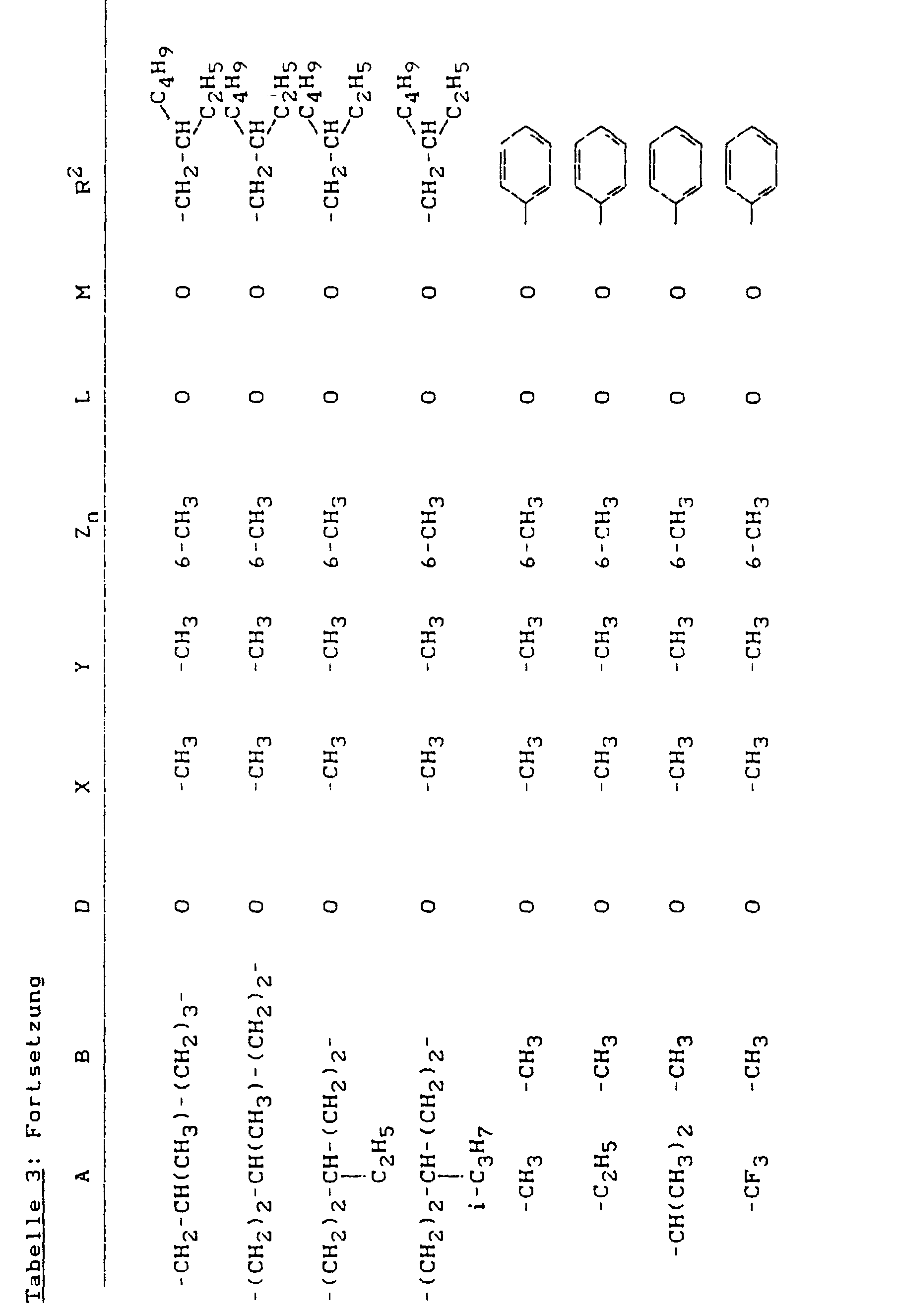

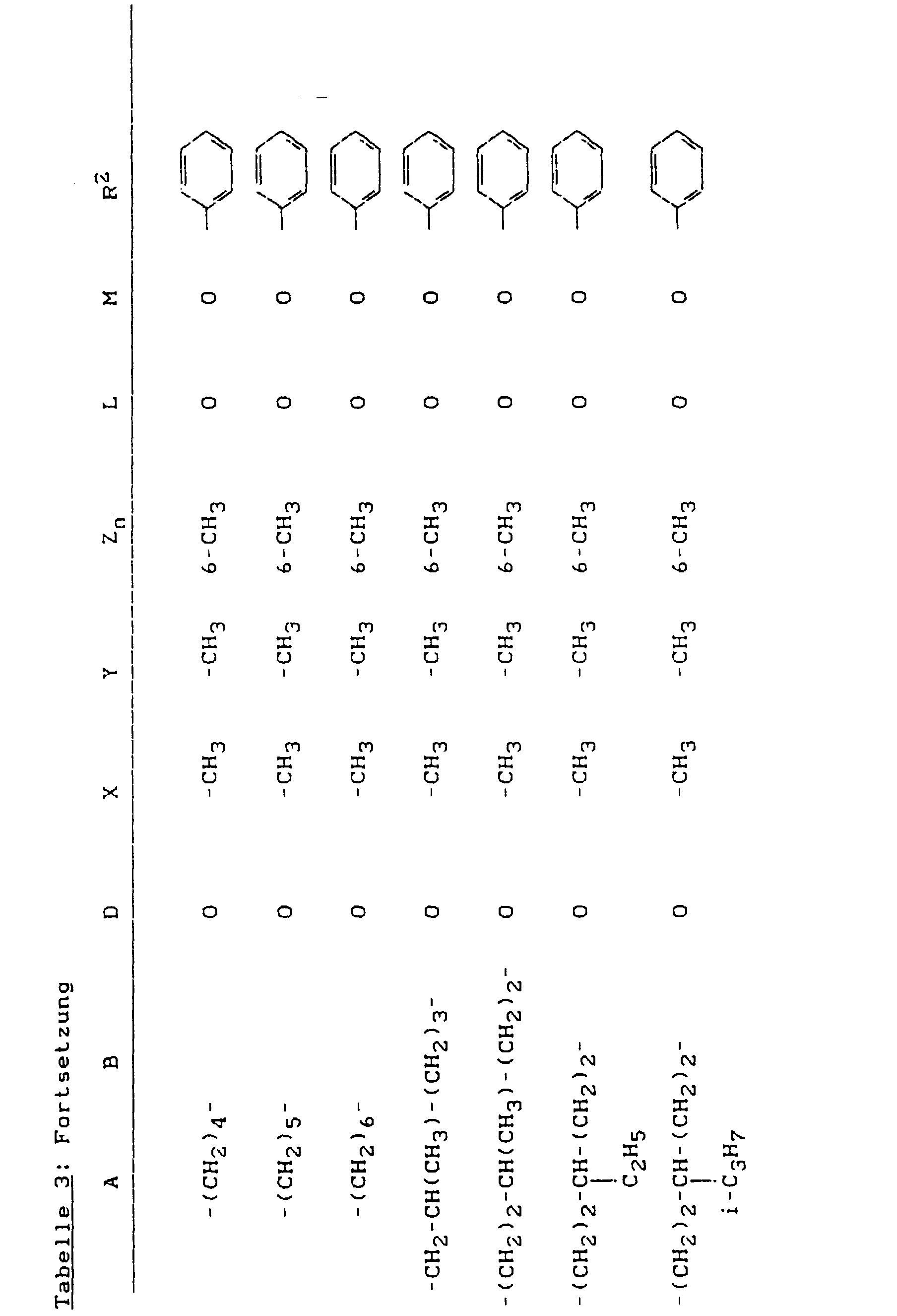

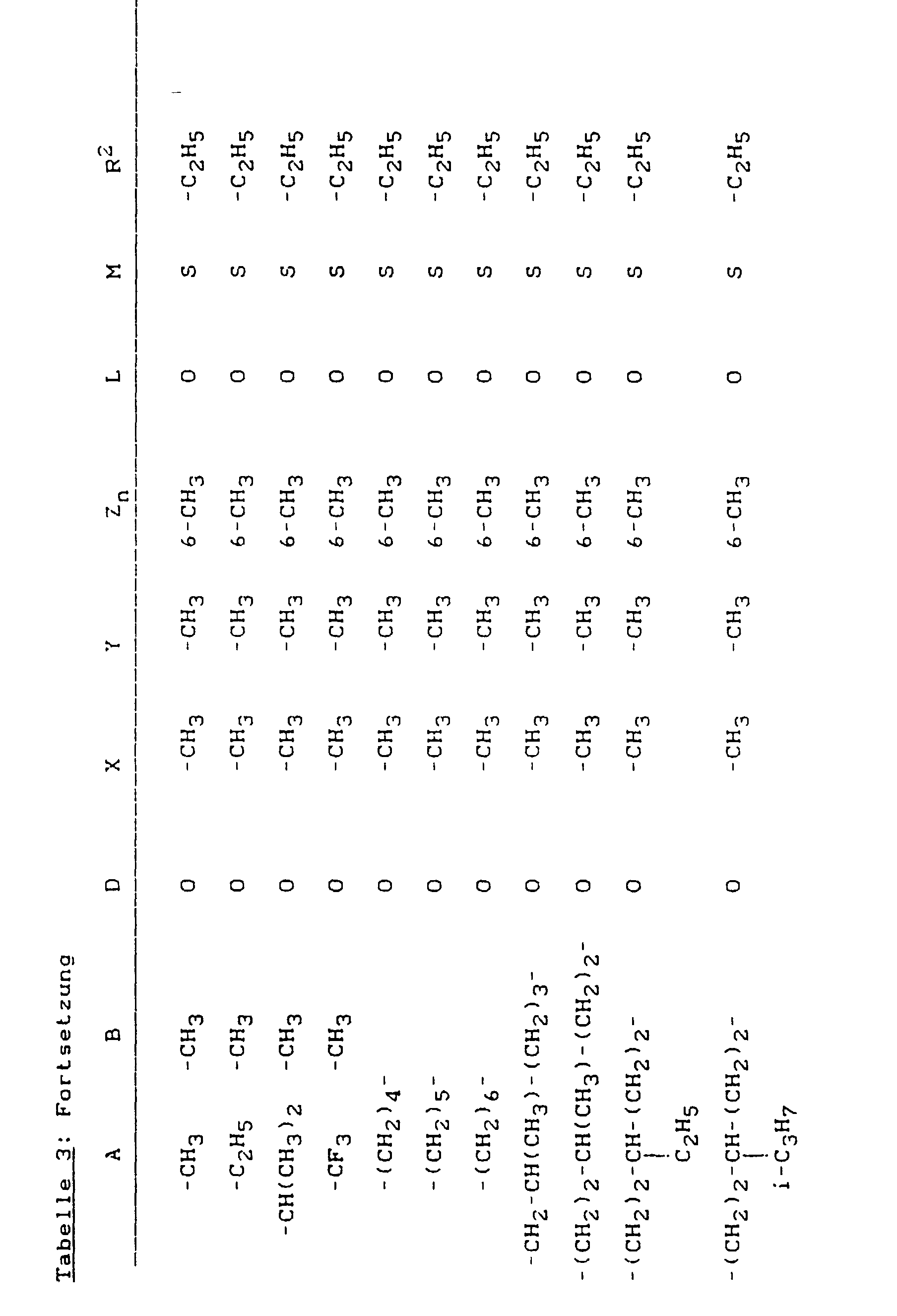

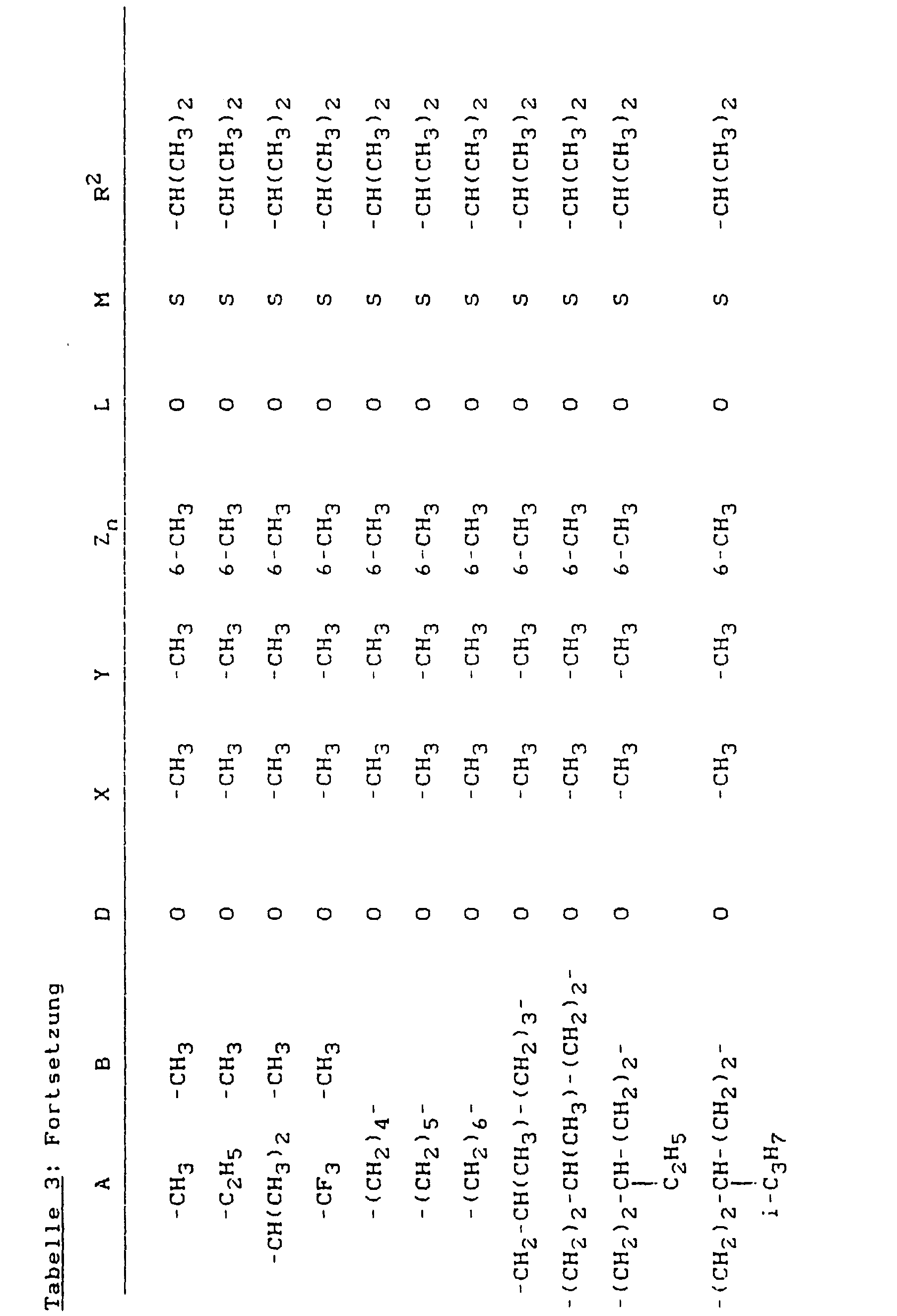

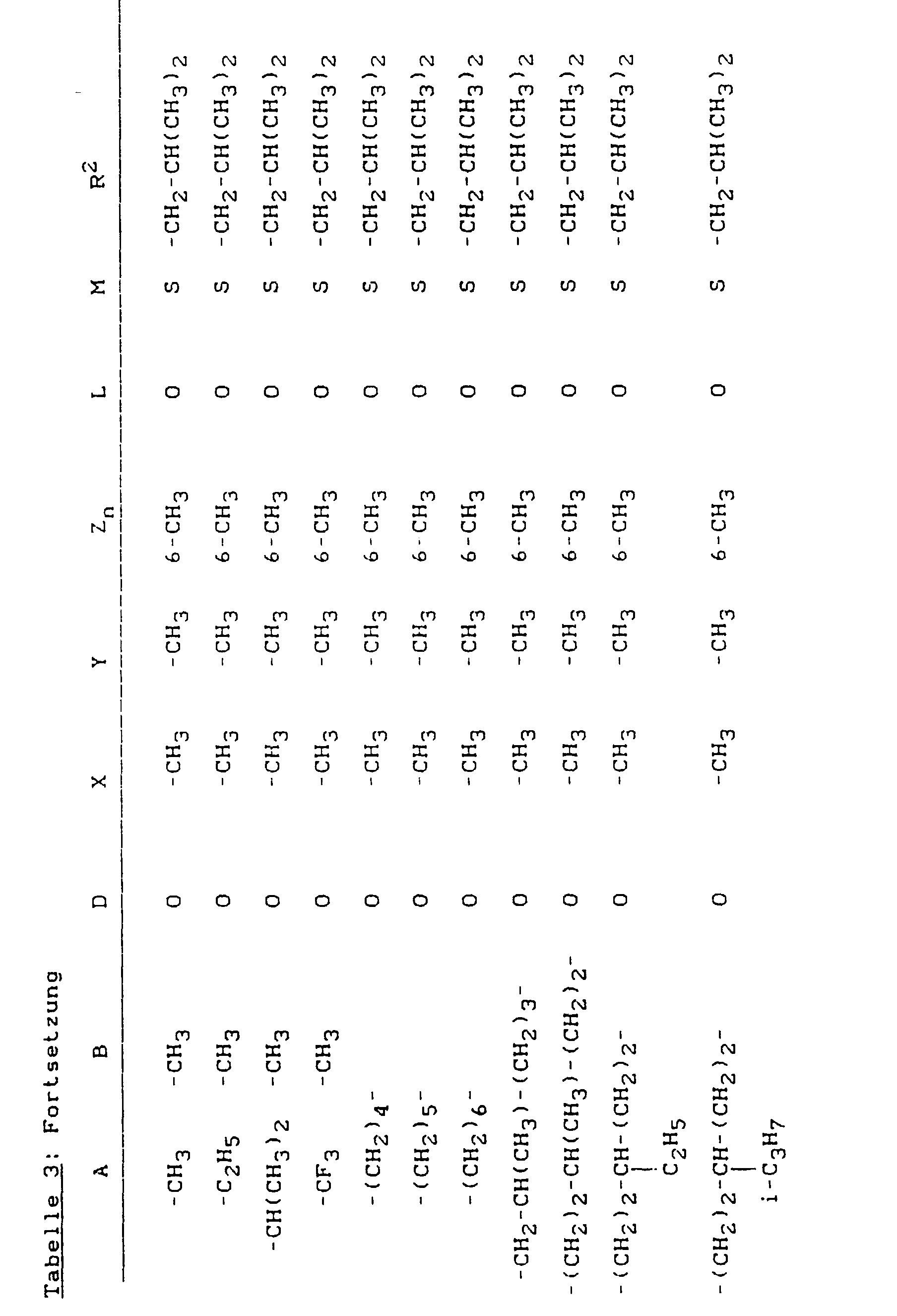

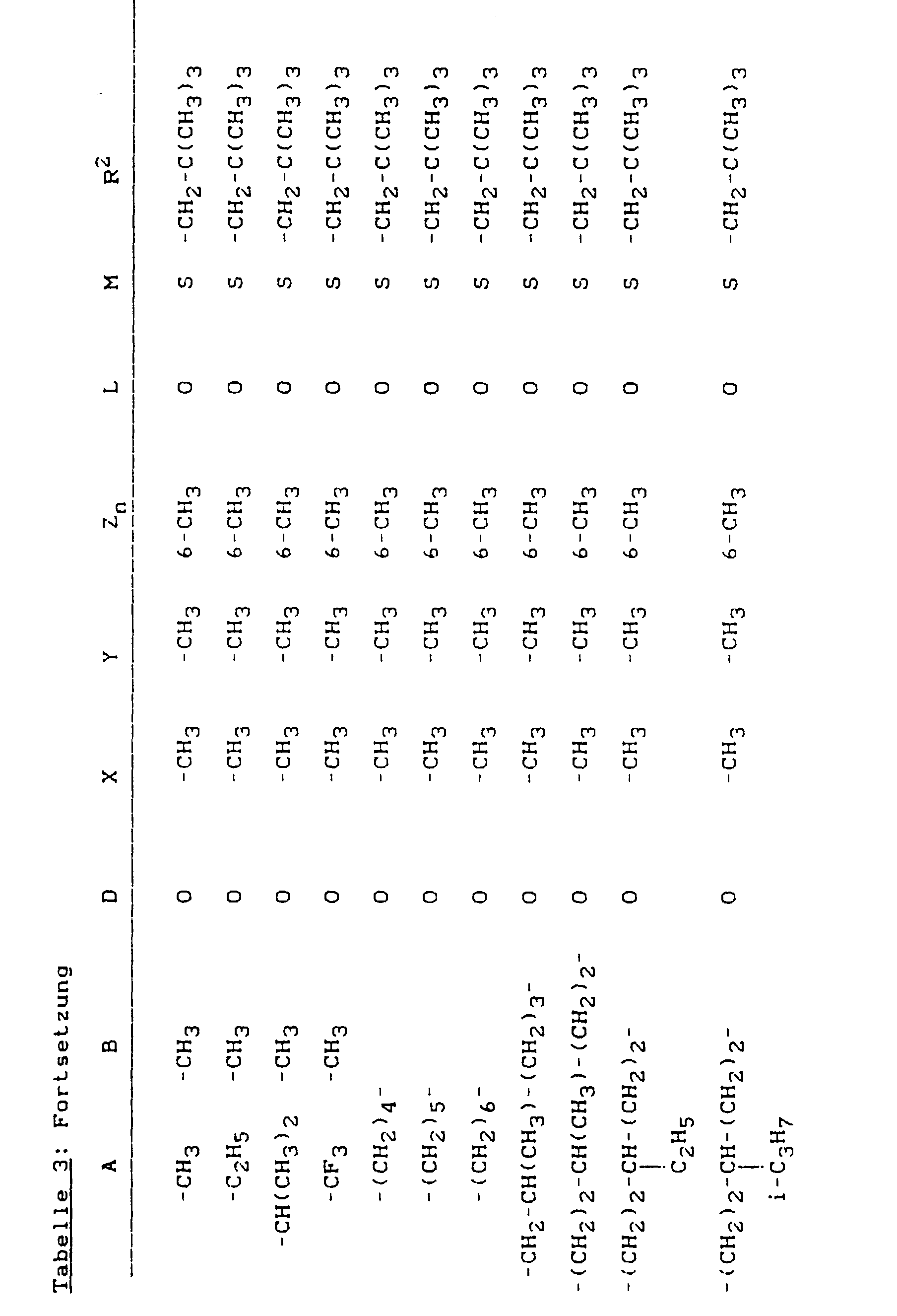

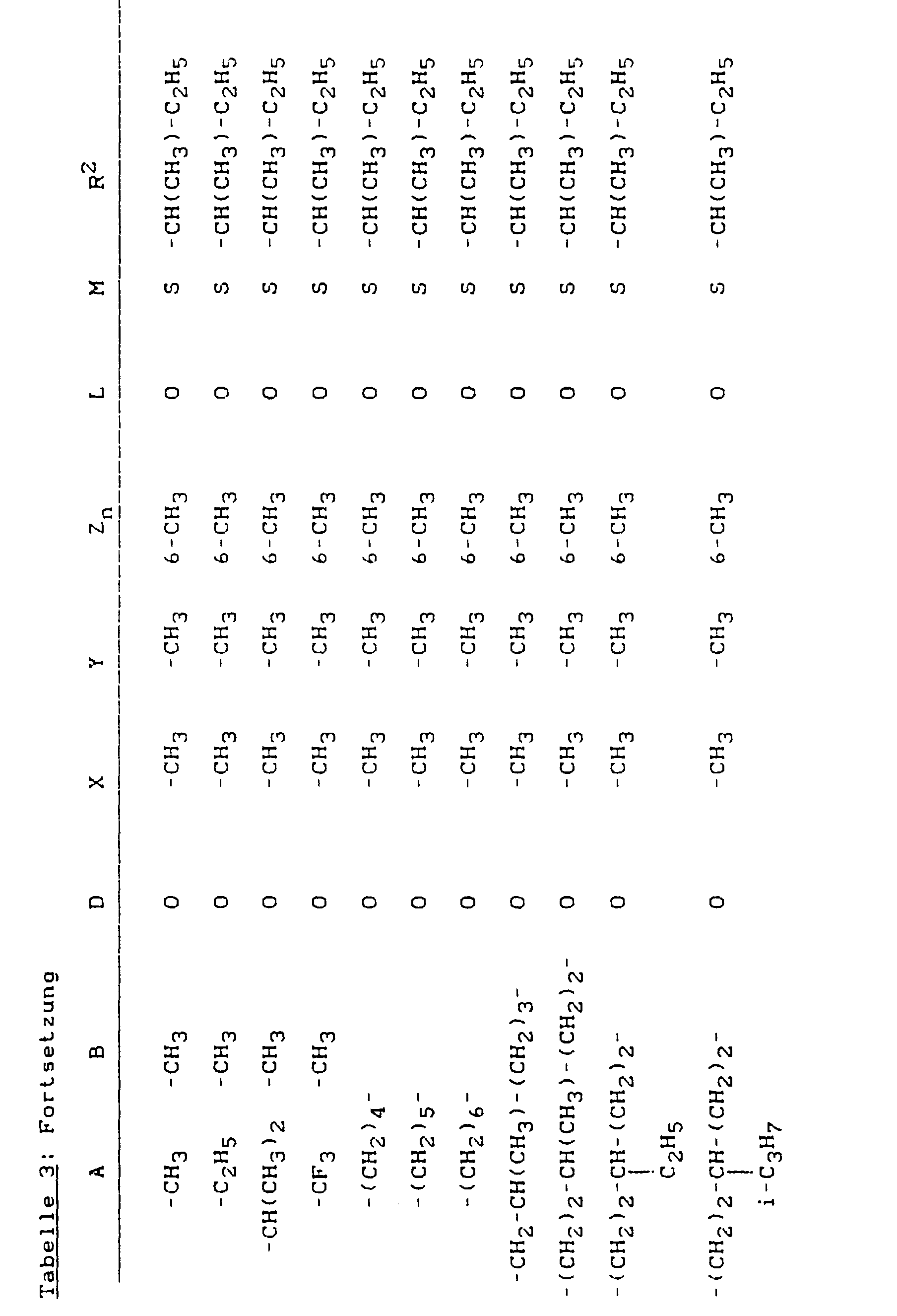

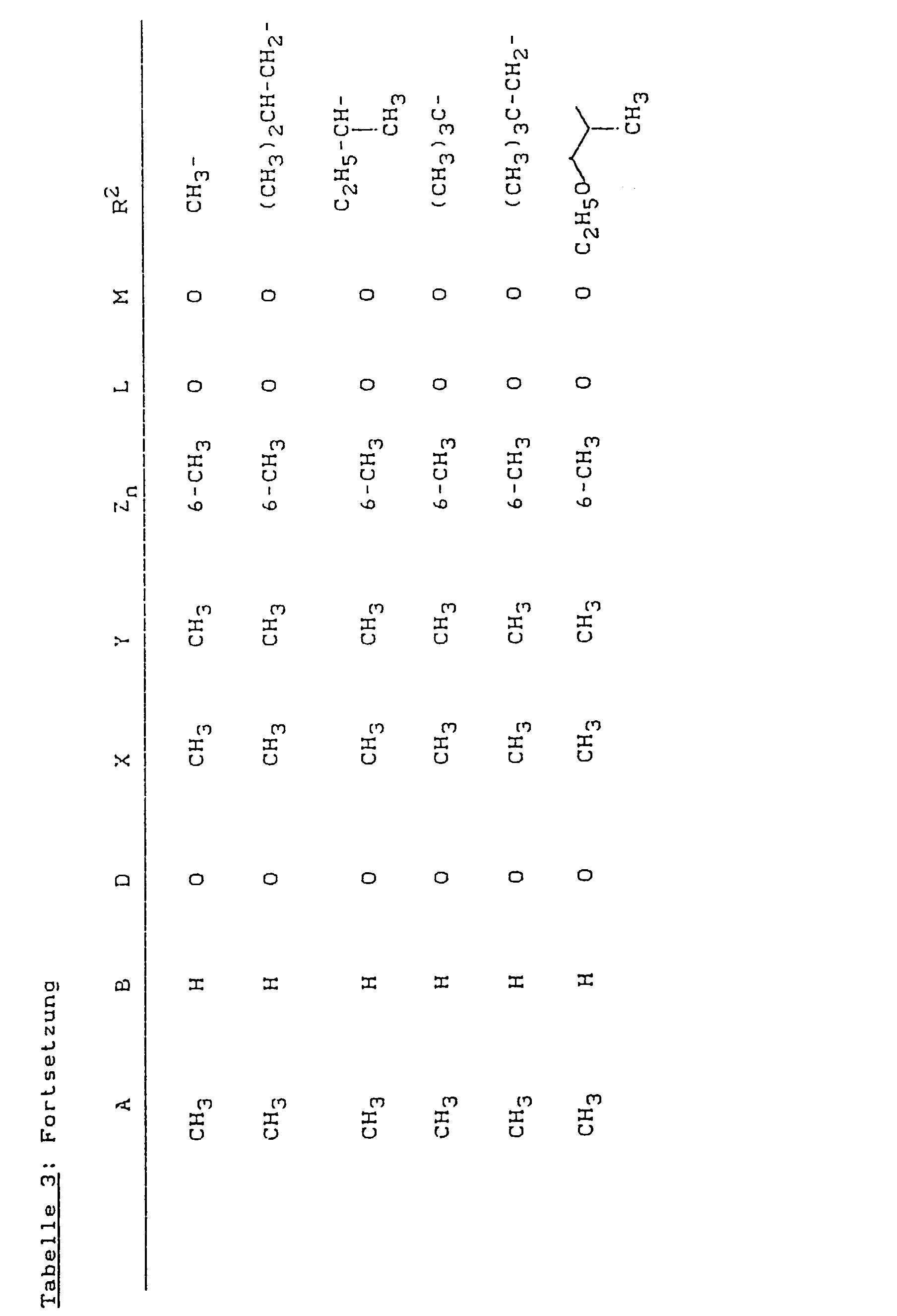

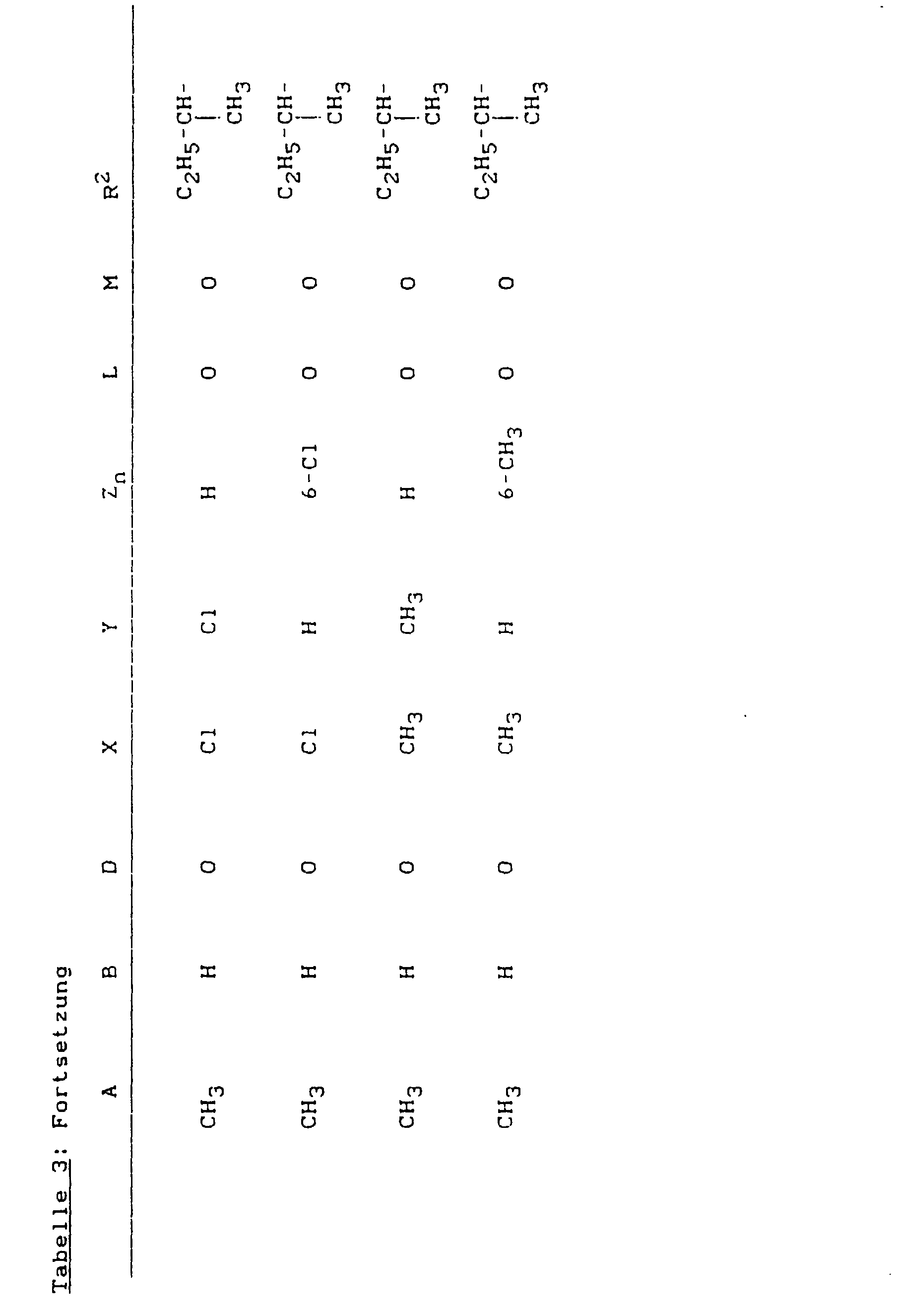

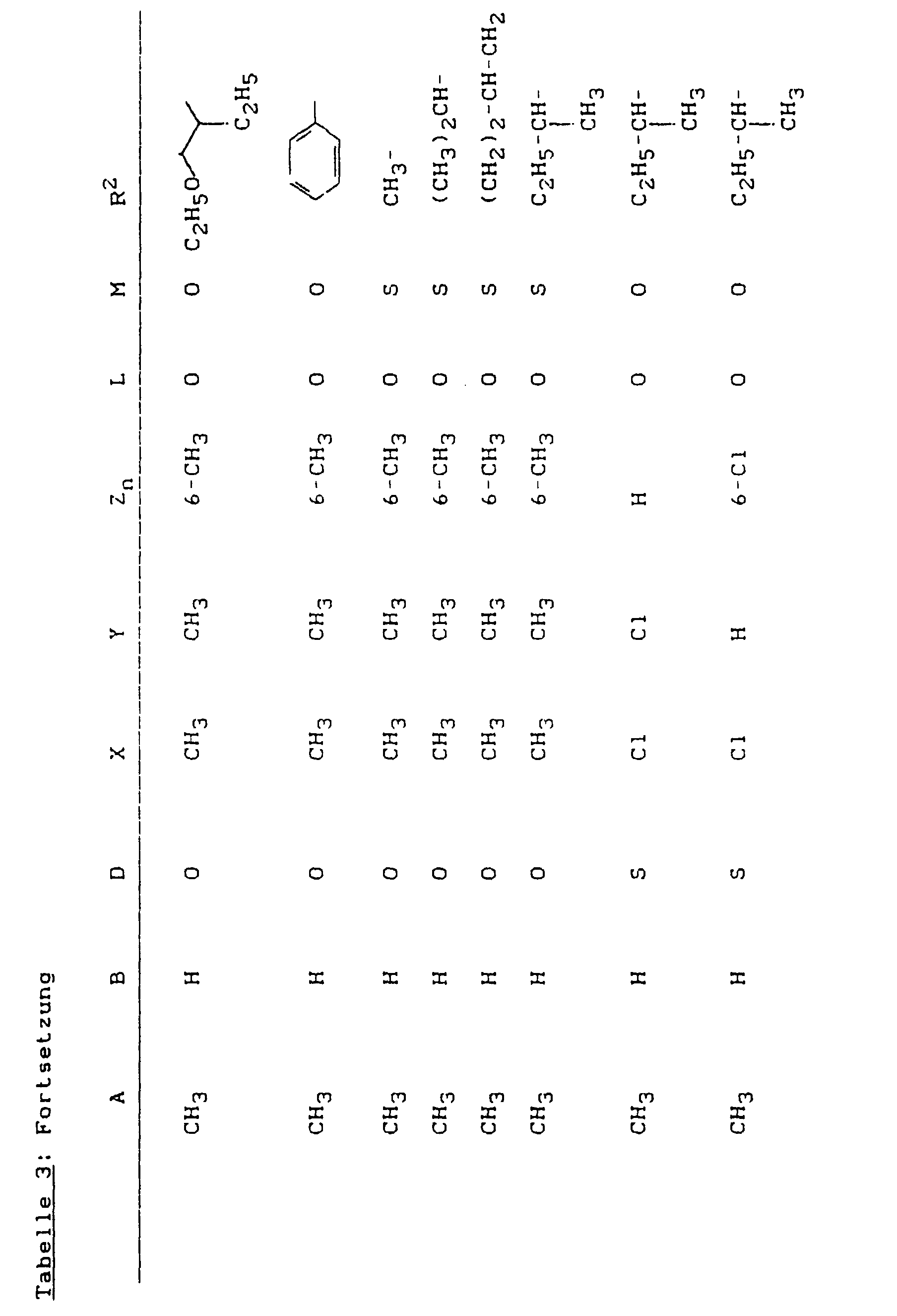

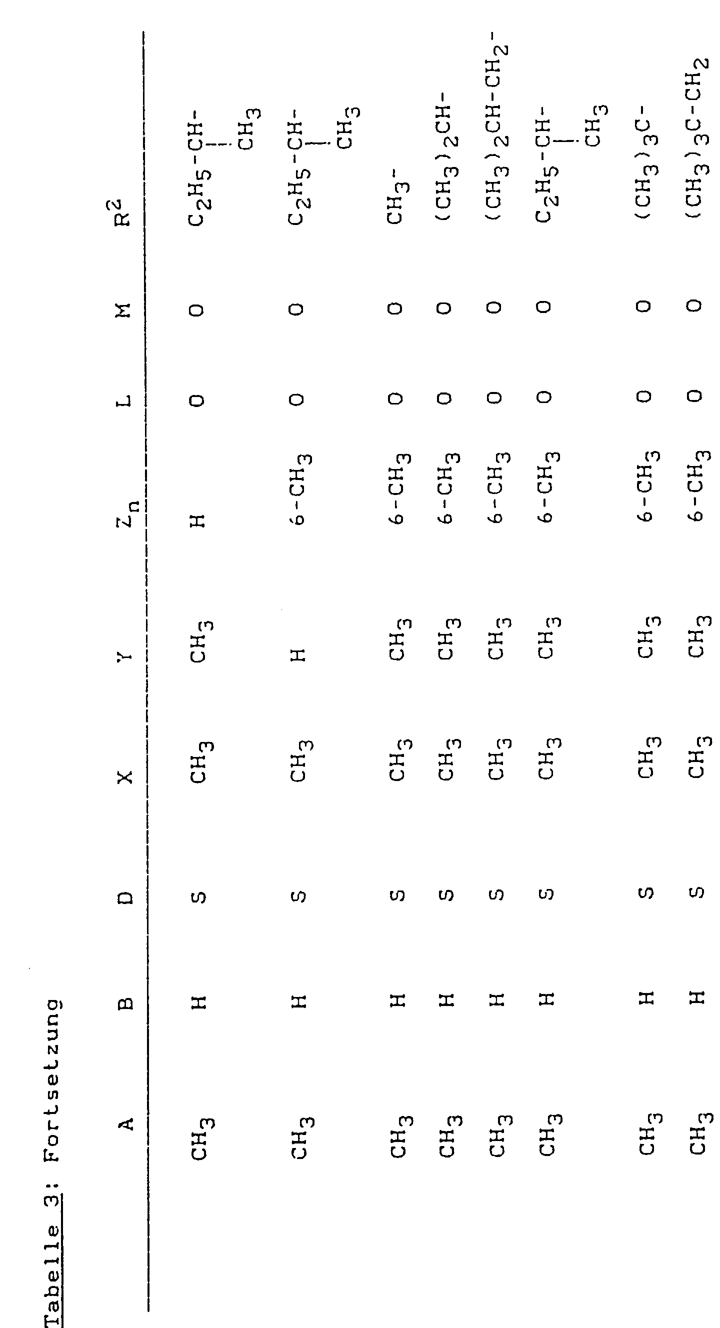

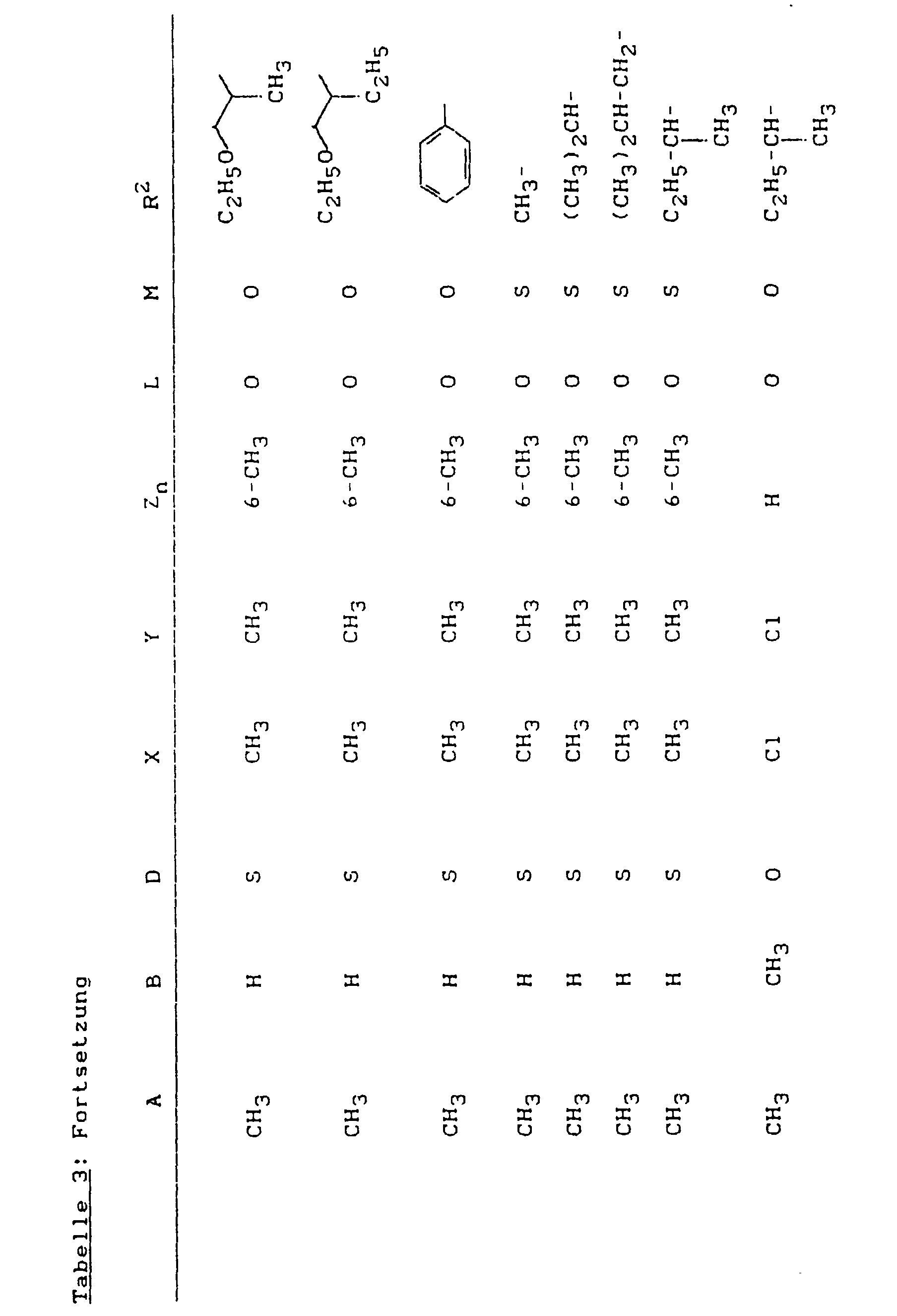

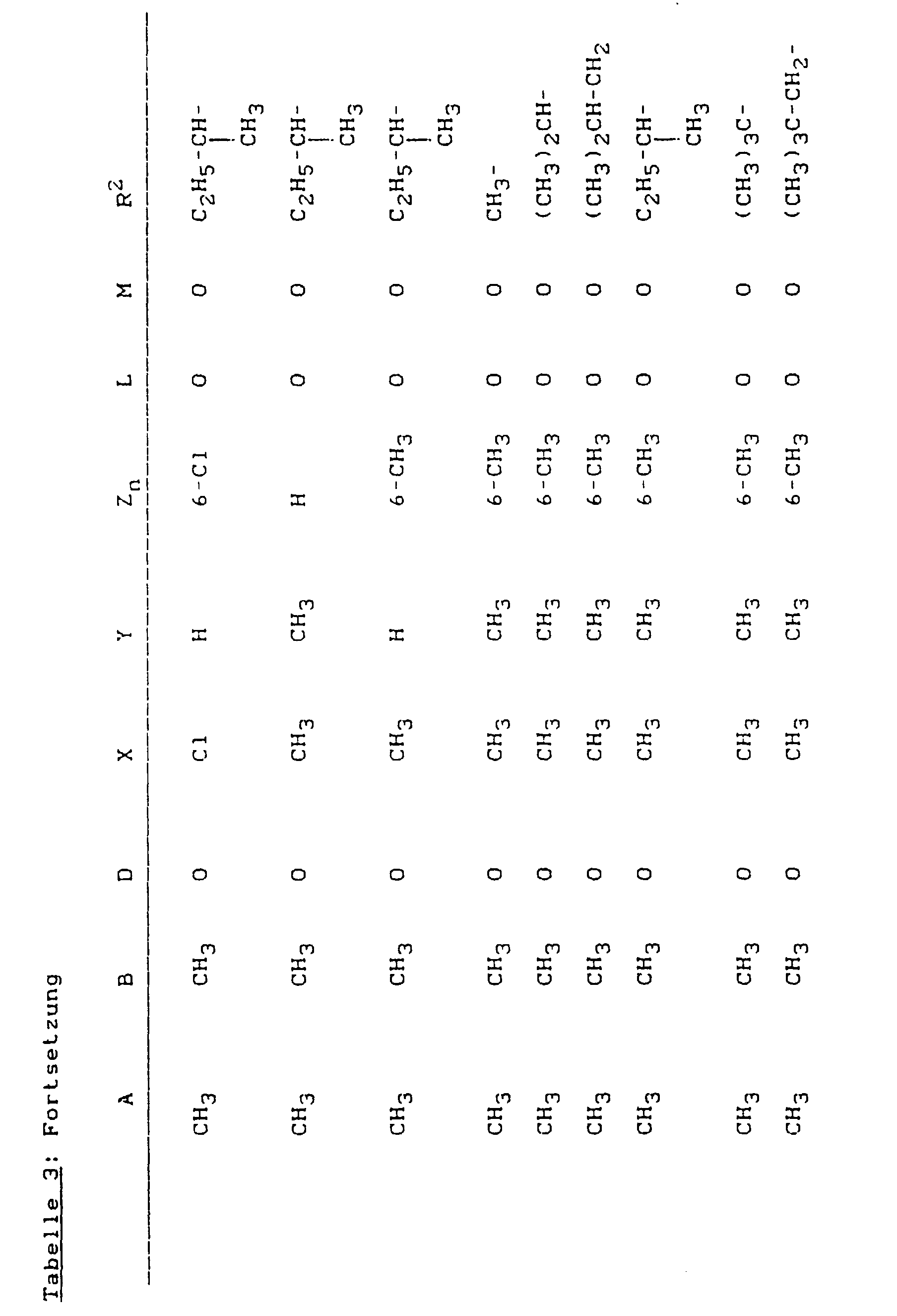

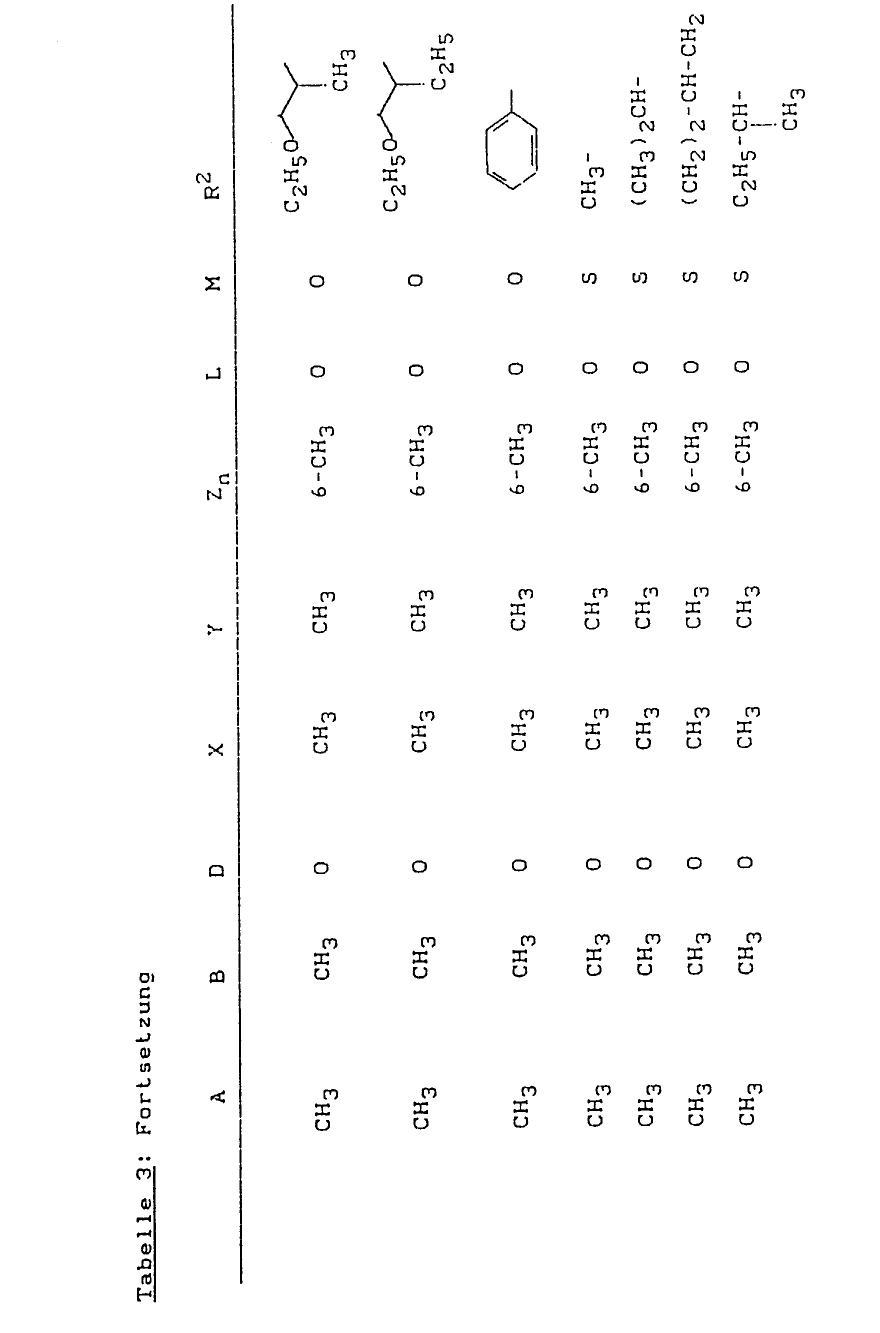

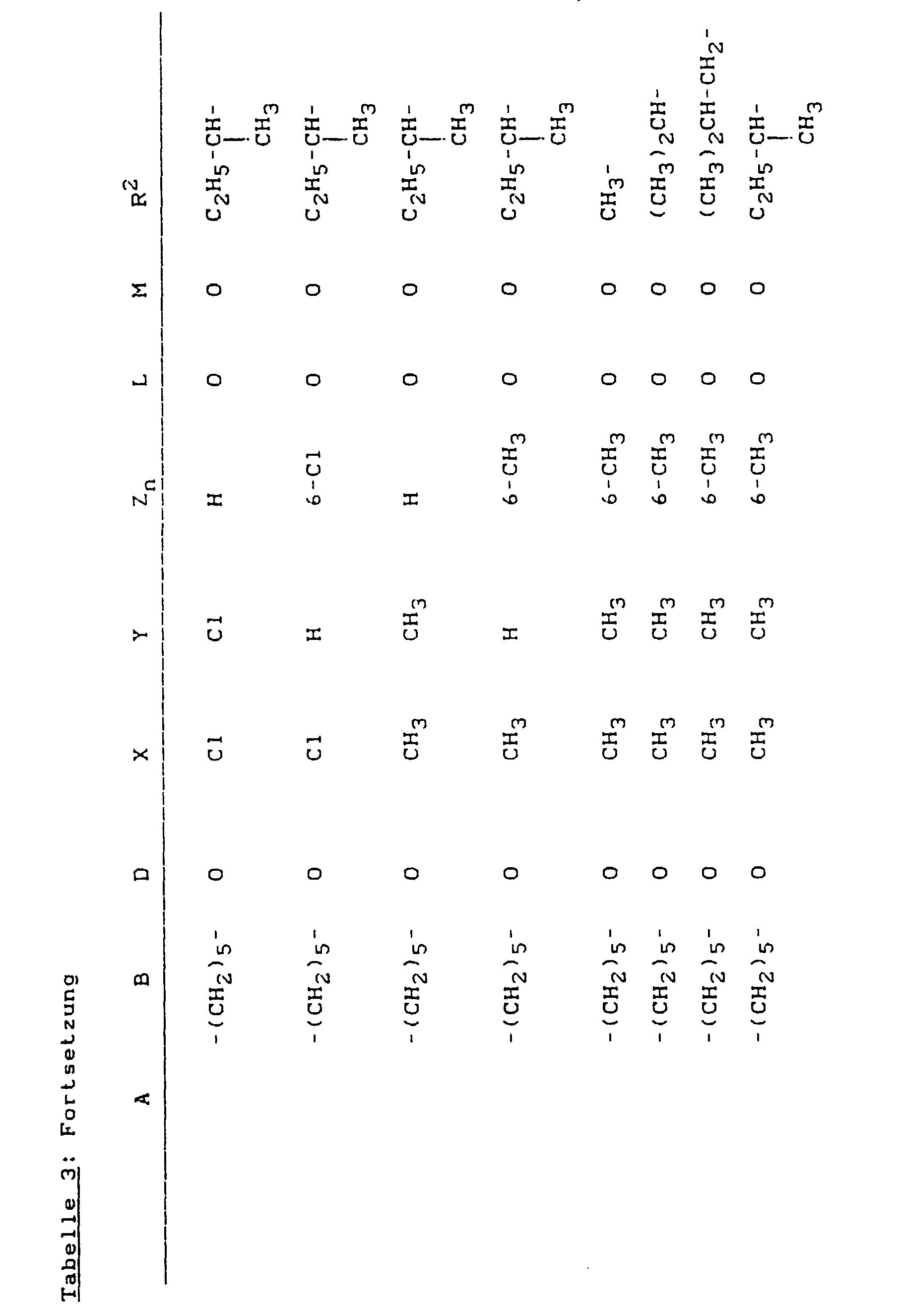

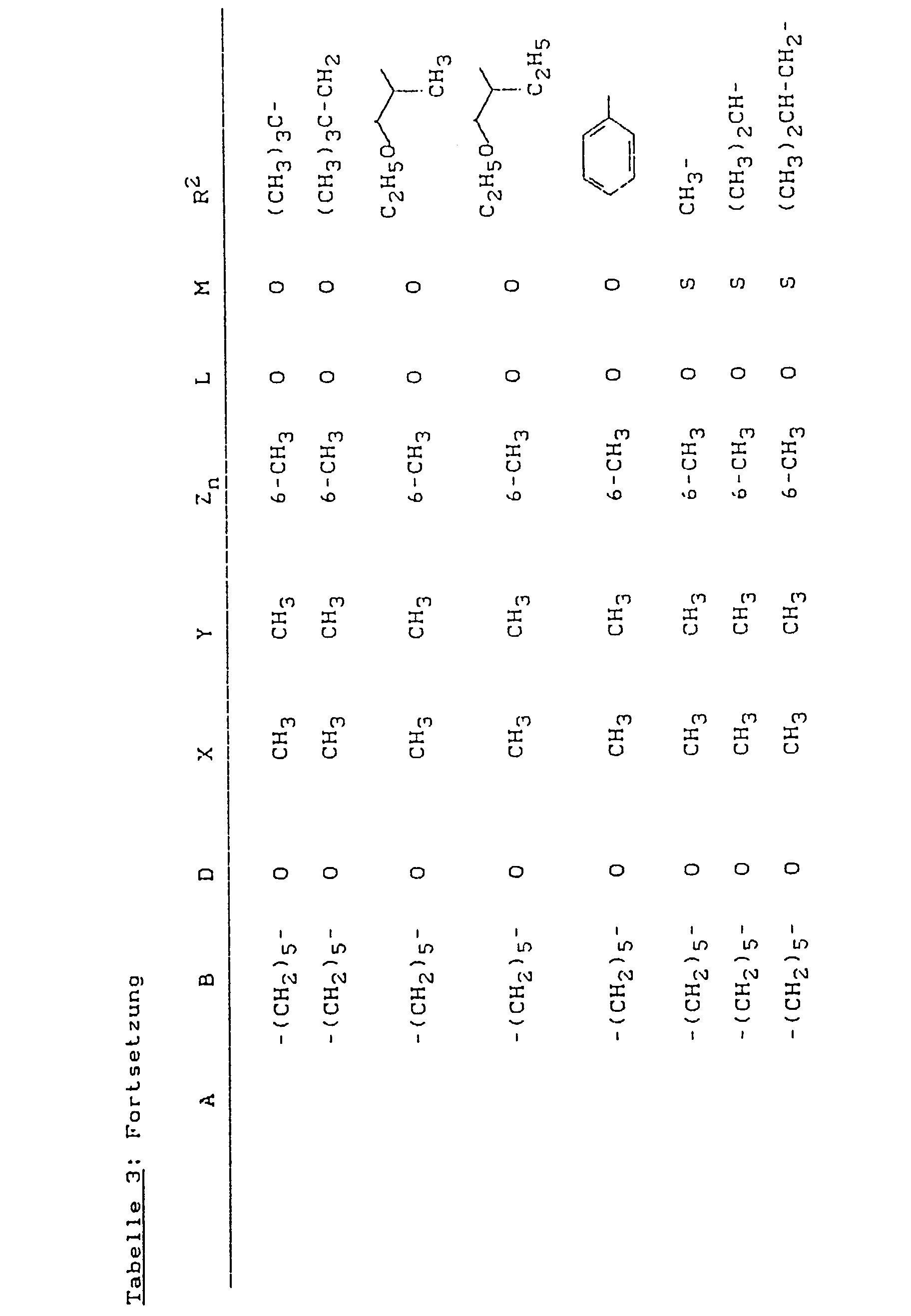

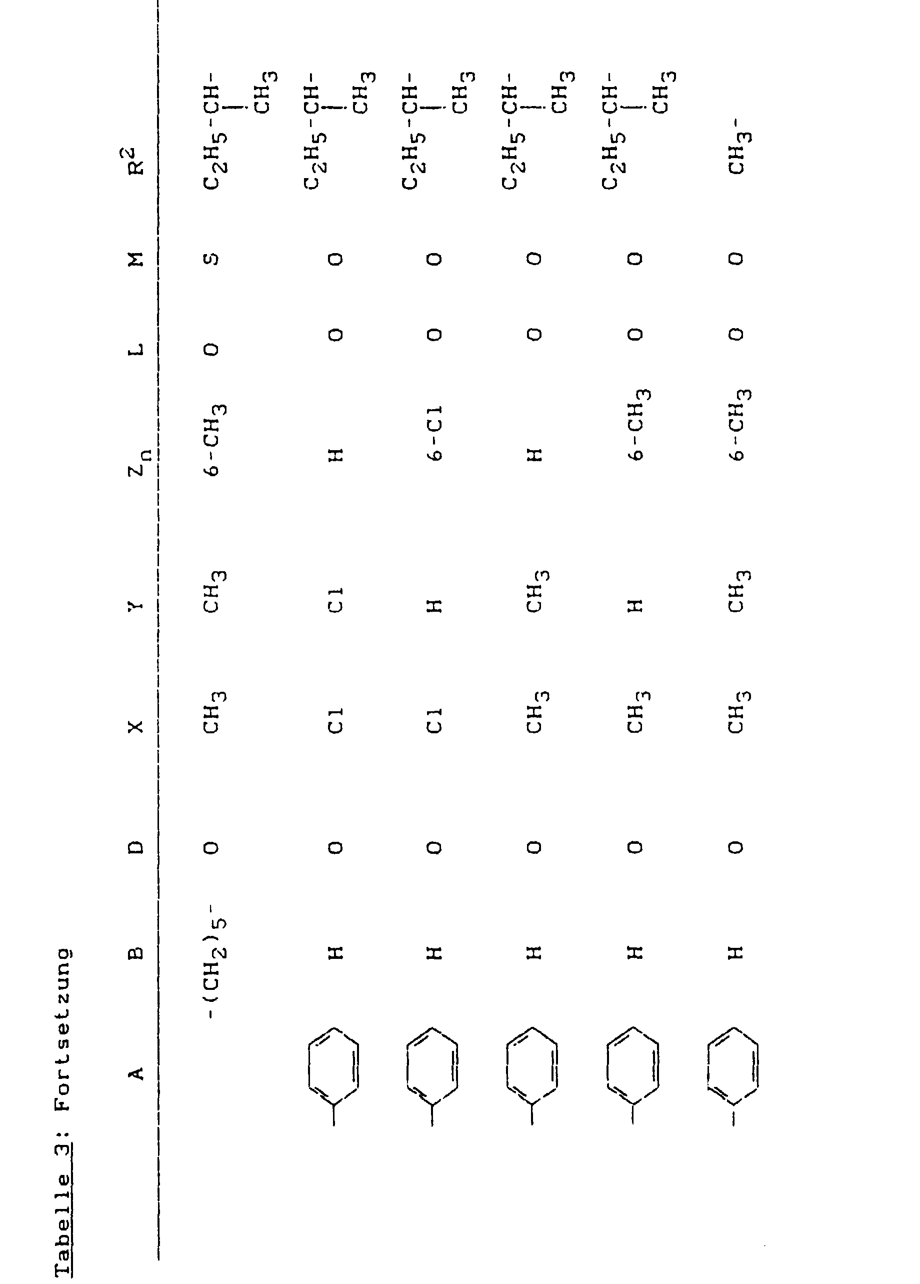

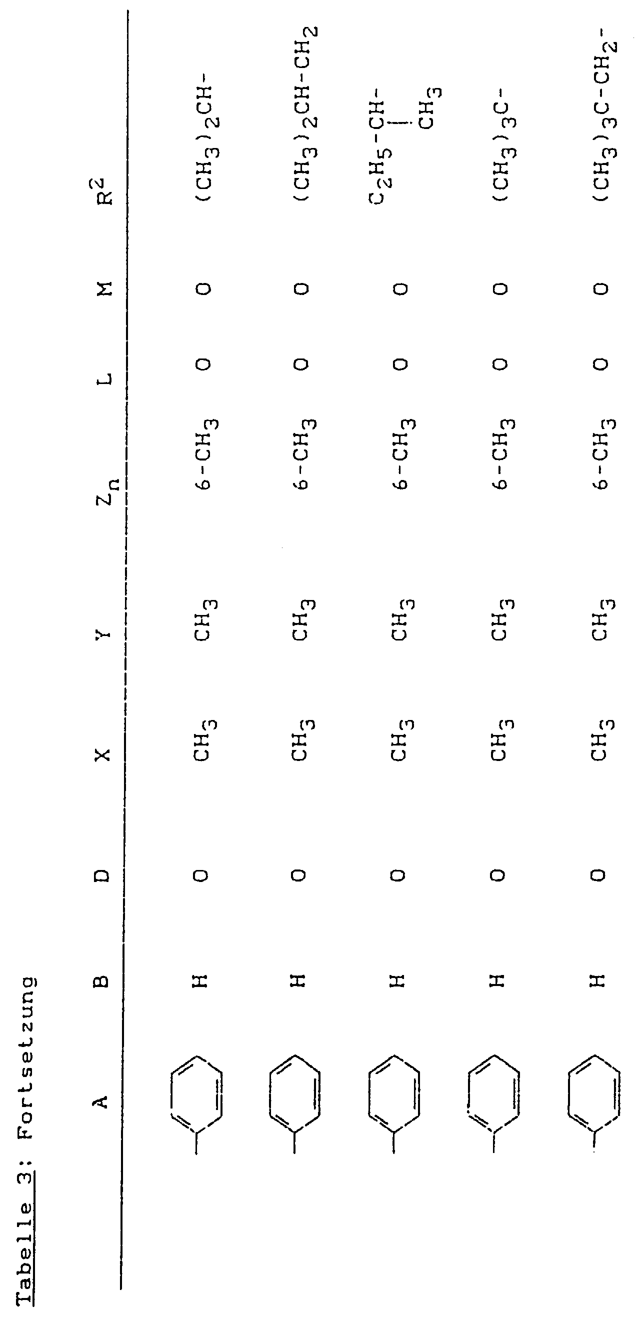

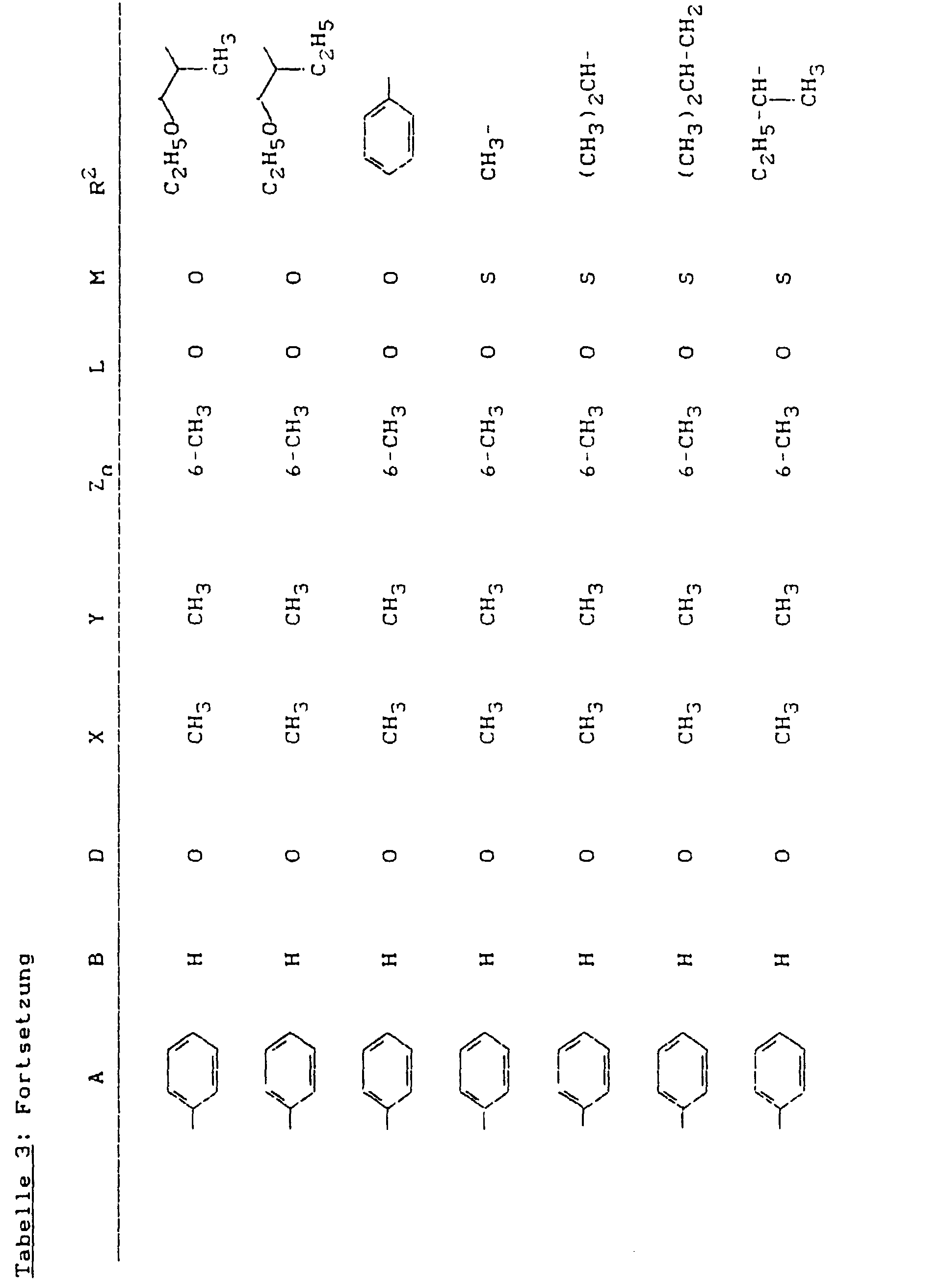

Im einzelnen seien außer den bei den Herstellungsbeispielen genannten Verbindungen die folgenden 3-Aryl-4-hydroxy-Δ³-dihydrofuran-Derivate der Formel (Id) genannt:

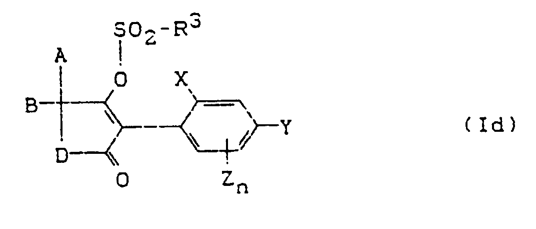

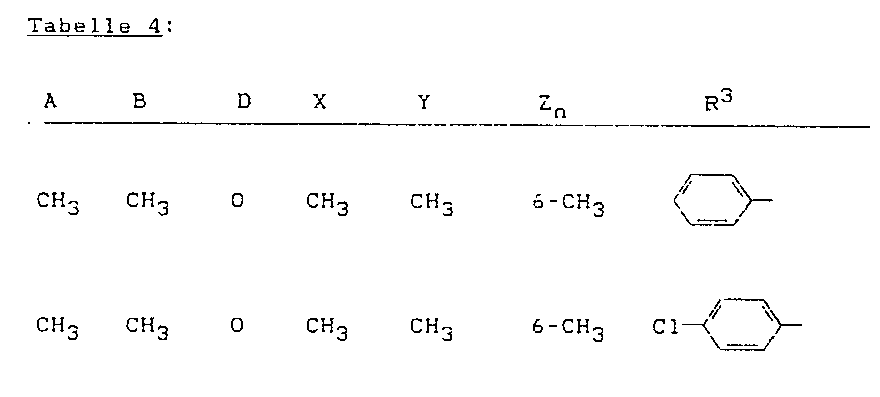

Im einzelnen seien außer den bei den Herstellungsbeispielen genannten Verbindungen die folgenden 3-Aryl-4-hydroxy-Δ³-dihydrofuran-Derivate der Formel (Ie) genannt:

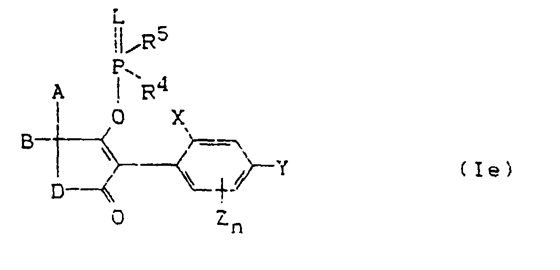

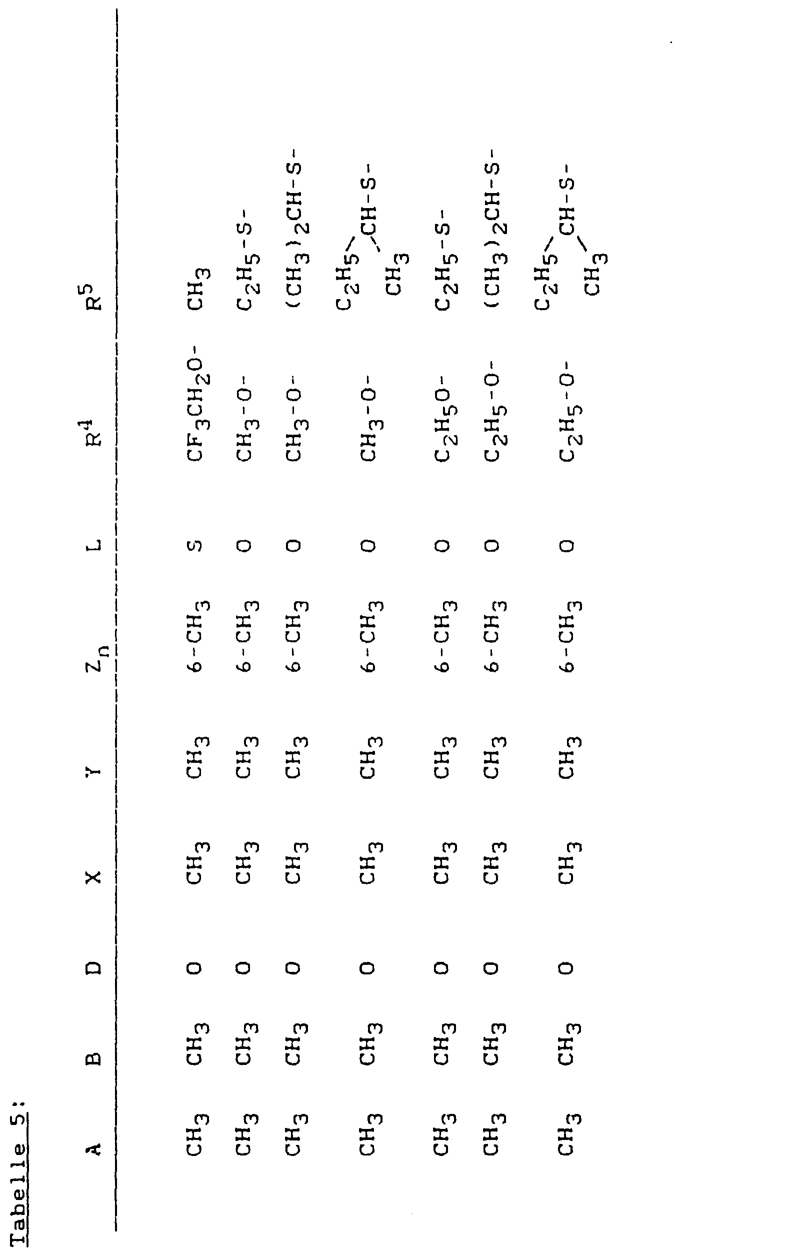

Im einzelnen seien außer den bei den Herstellungsbeispielen genannten Verbindungen die folgenden 3-Aryl-4-hydroxy-Δ³-dihydrofuran-Derivate der Formel (If) genannt:

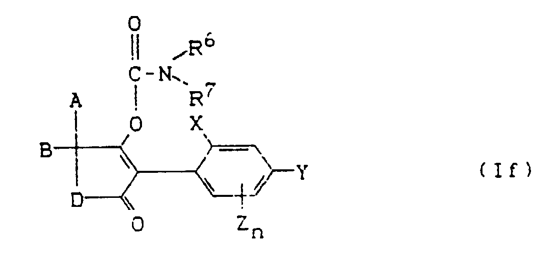

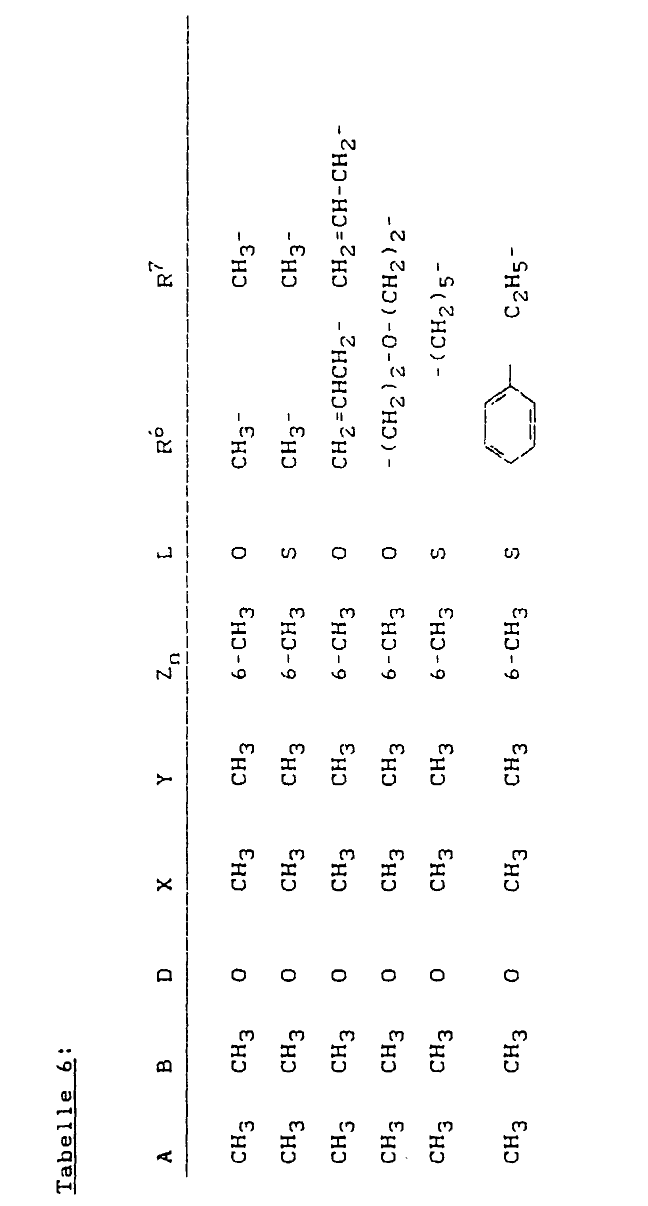

Im einzelnen seien außer den bei den Herstellungsbeispielen genannten Verbindungen die folgenden 3-Aryl-4-hydroxy-Δ³-dihydrofuran-Derivate der Formel (Ig) genannt:

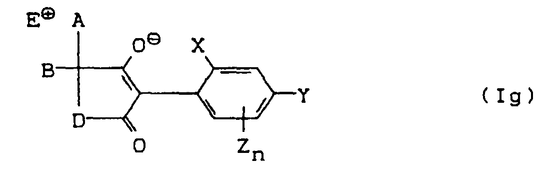

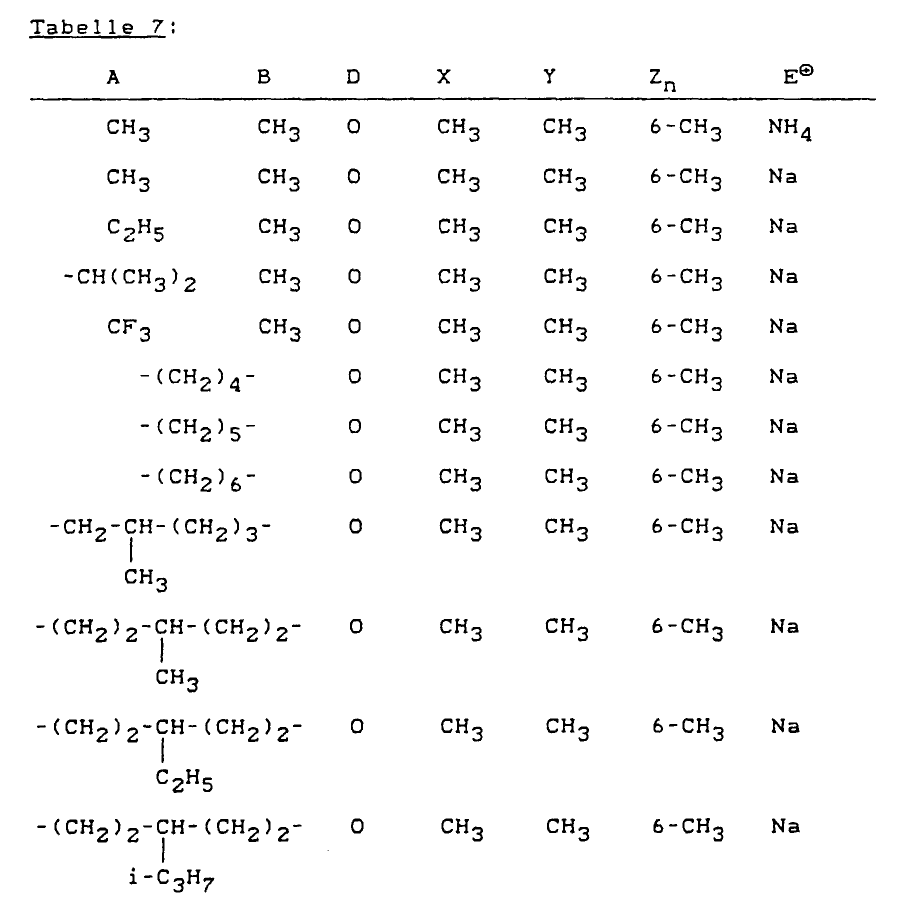

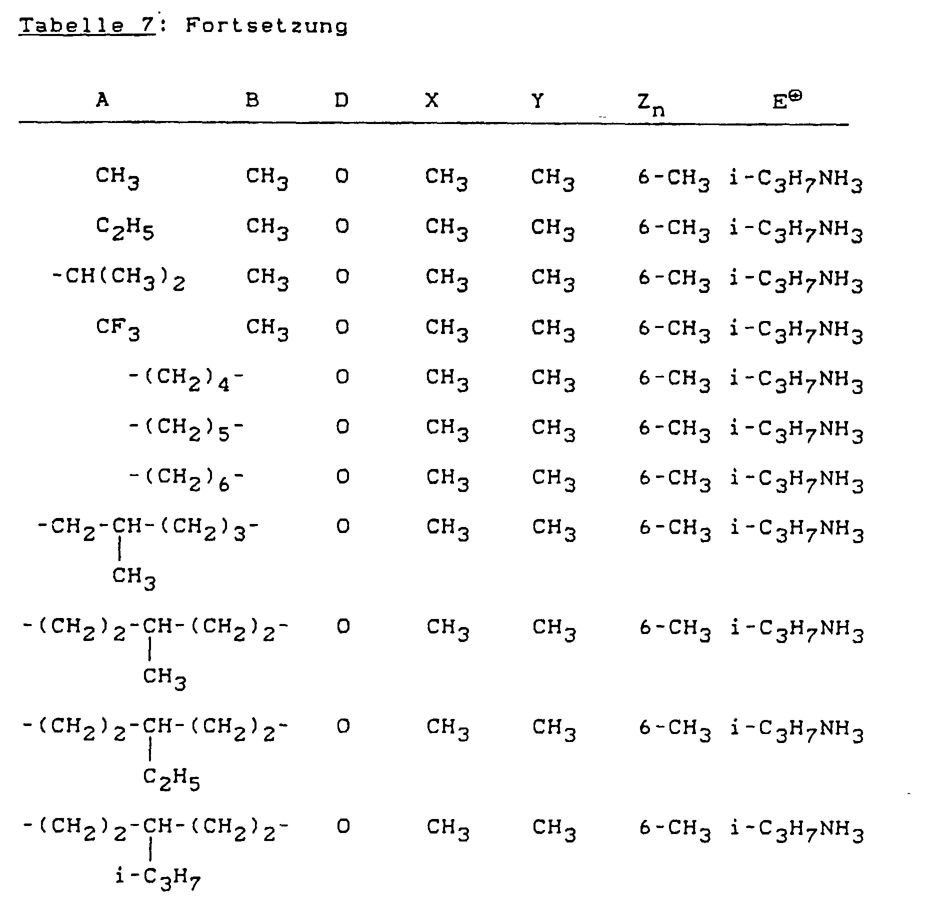

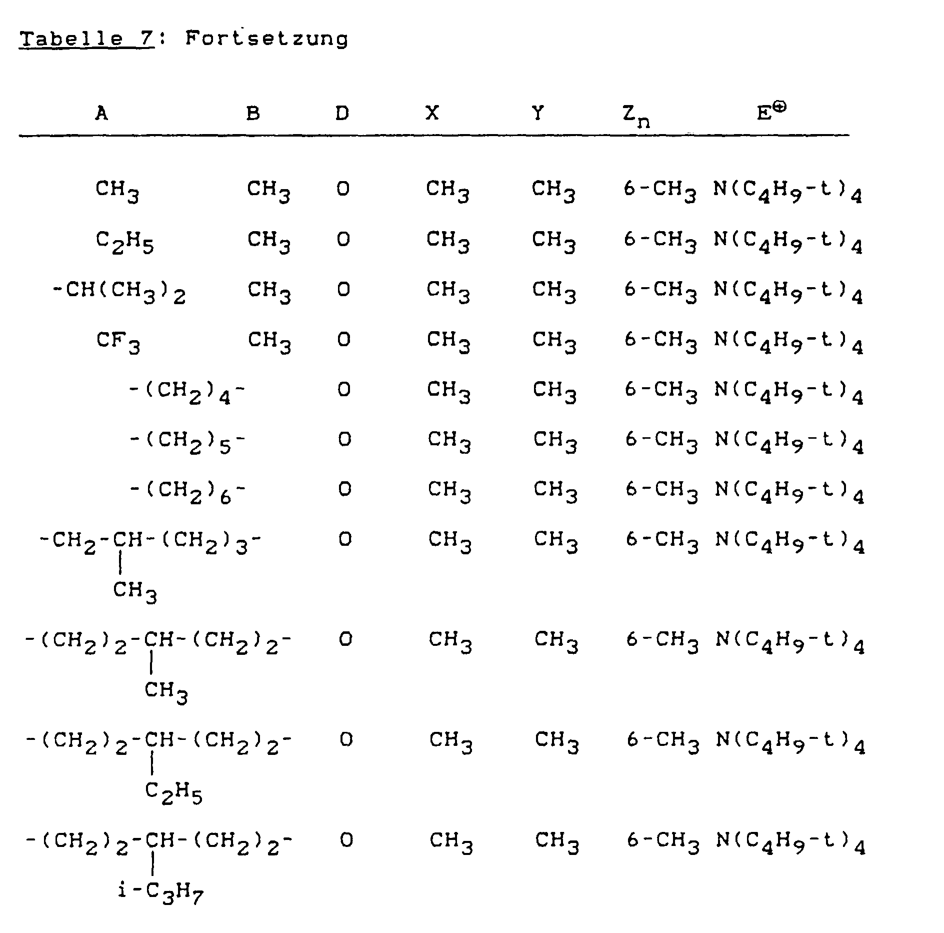

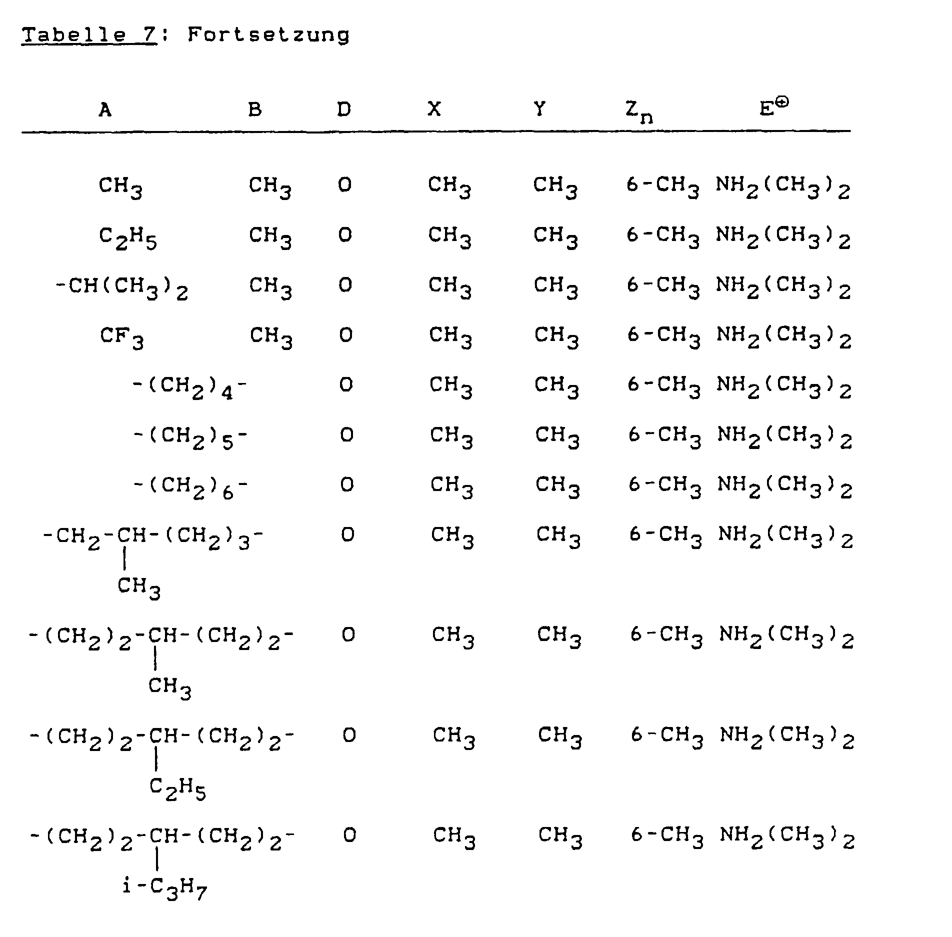

Verwendet man gemäß Verfahren (A) O-2,6-Dichlorphenylacetyl-hydroxyessigsäureethylester, so kann der Verlauf des erfindungsgemäßen Verfahrens durch folgendes Reaktionsschema wiedergegeben werden:

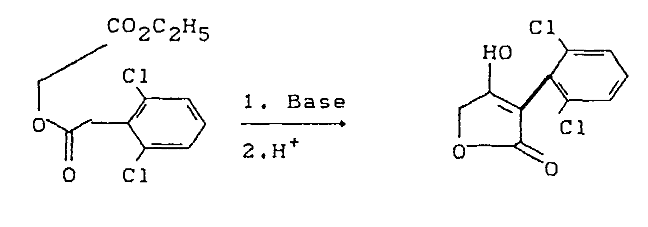

Verwendet man gemäß Verfahren (B) (Variante α) 3-(2,4,6 Trimethylphenyl)-4-hydroxy-5,5-dimethyl-Δ³-dihydrofuran-2-on und Pivaloylchlorid als Ausgangsstoff, so kann der Verlauf des erfindungsgemäßen Verfahrens durch folgendes Reaktionsschema wiedergegeben werden.

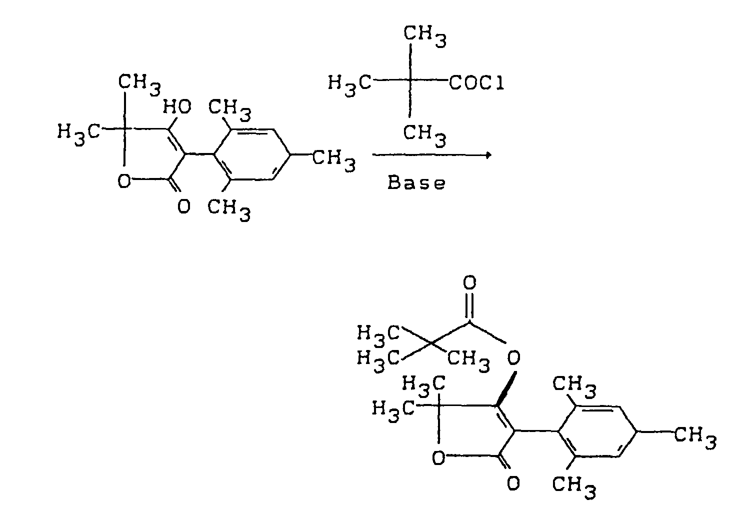

Verwendet man gemäß Verfahren B (Variante β) 3-(2,4,5-Trimethylphenyl)-4-hydroxy-5-phenyl-Δ³-dihydrofuran-2-on und Acetanhydrid als Ausgangsverbindungen, so kann der Verlauf des erfindungsgemäßen Verfahrens durch folgendes Reaktionsschema wiedergegeben werden.

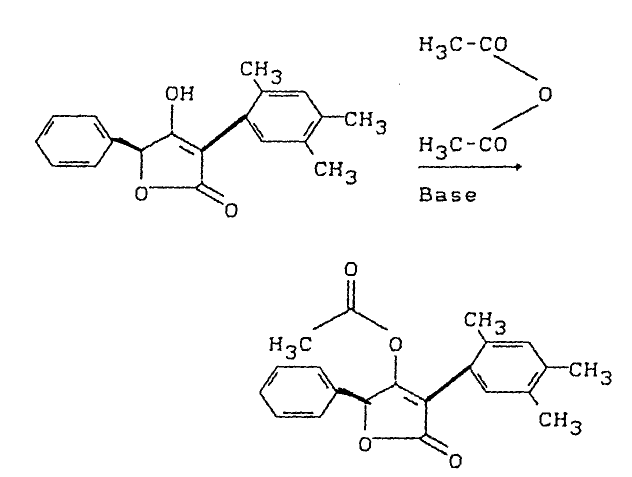

Verwendet man gemäß Verfahren C 3-(2,4-Dichlorphenyl)-4-hydroxy-5-methyl-Δ³-dihydrofuran-2-on und Chlorameisensäureethoxyethylester als Ausgangsverbindungen, so kann der Verlauf des erfindungsgemäßen Verfahrens durch folgendes Reaktionsschema wiedergegeben werden.

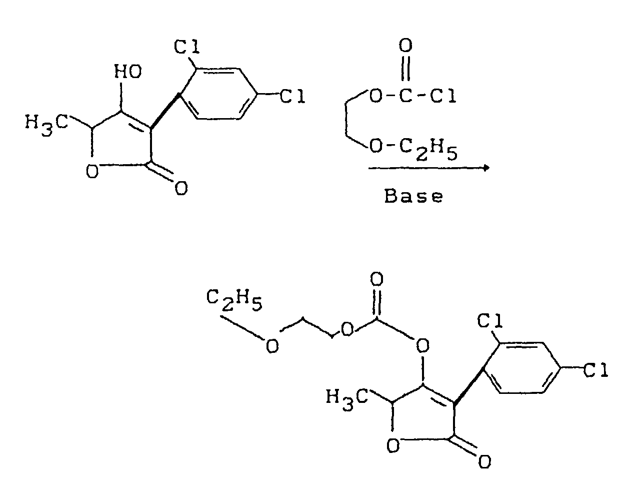

Verwendet man gemäß Verfahren (D_{α}) 3-(2,4,6-Trimethylphenyl)-4-hydroxy-5-methyl-Δ³-dihydro-thiophen-2-on und Chlormonothioameisensäuremethylester als Ausgangsprodukte, so kann der Reaktionsverlauf wie folgt wiedergegeben werden:

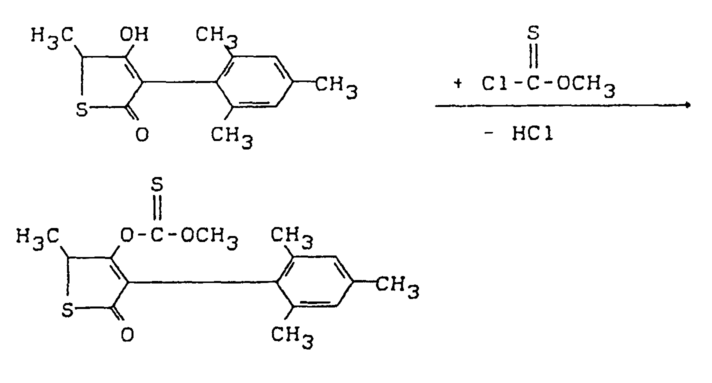

Verwendet man gemäß Verfahren (D_{β}) 3-(2,4,6-Trimethylphenyl)-4-hydroxy-5.5-pentamethylen-Δ³-dihydrofuran-2-on, Schwefelkohlenstoff und Methyl jodid als Ausgangskomponenten, so kann der Reaktionsverlauf wie folgt wiedergegeben werden:

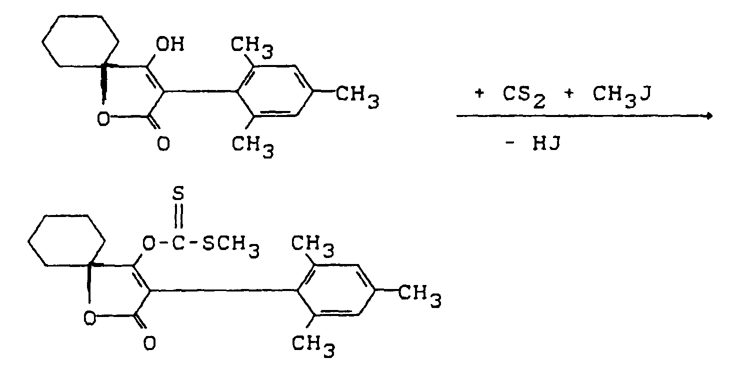

Verwendet man gemäß Verfahren (E) 3-(2,4,6-Trimethylphenyl)-4-hydroxy-5-methylmercaptomethyl-Δ³-dihydrofuran - 2-on und Methansulfonsäurechlorid als Ausgangsprodukt, so kann der Reaktionsverlauf durch folgendes Reaktionsschema wiedergegeben werden:

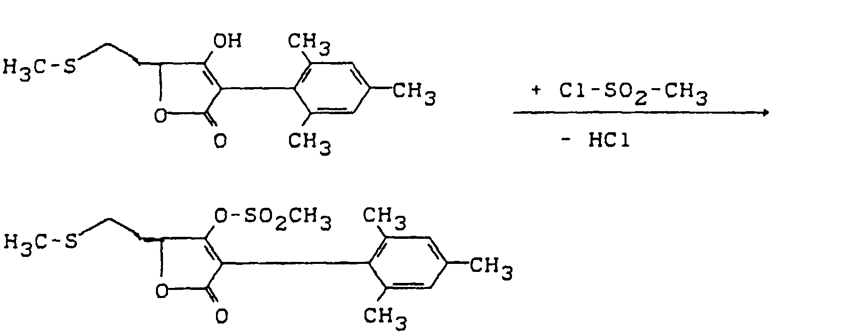

Verwendet man gemäß Verfahren (F) 3-(2,4,6-Trimethylphenyl)-4-hydroxy-5.5-dimethyl-Δ³-dihydro-furan-2-on und Methanthio-phosphonsäurechlorid-(2,2,2-trifluorethylester) als Ausgangsprodukte, so kann der Reaktionsverlauf durch folgendes Reaktionsschema wiedergegeben werden:

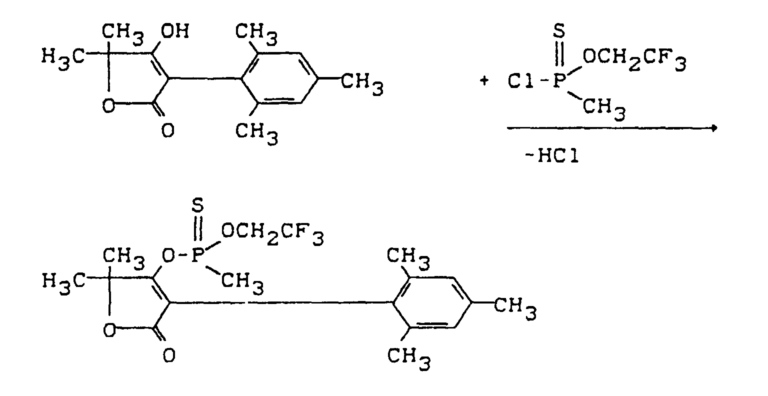

Verwendet man gemäß Verfahren (G_{α}) 3-(2,4,6-Trimethylphenyl)-4-hydroxy-5-tetramethylen-Δ³-dihydro-furan-2-on und Ethylisocyanat als Ausgangsprodukte, so kann der Reaktionsverlauf durch folgendes Reaktionsschema wiedergegeben werden:

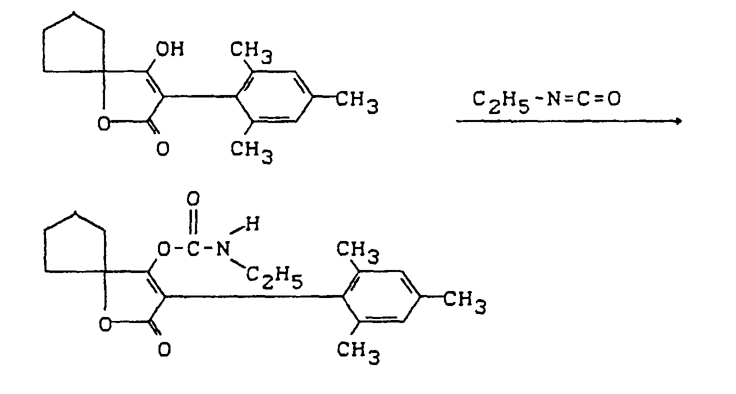

Verwendet man gemäß Verfahren (G_{β}) ₃-(2,4,6-Trimethylphenyl)-4-hydroxy-5-methyl-Δ³-dihydrofuran-2-on und Dimethylcarbamidsäurechlorid als Ausgangsprodukte, so kann der Reaktionsverlauf durch folgendes Schema wiedergegeben werden:

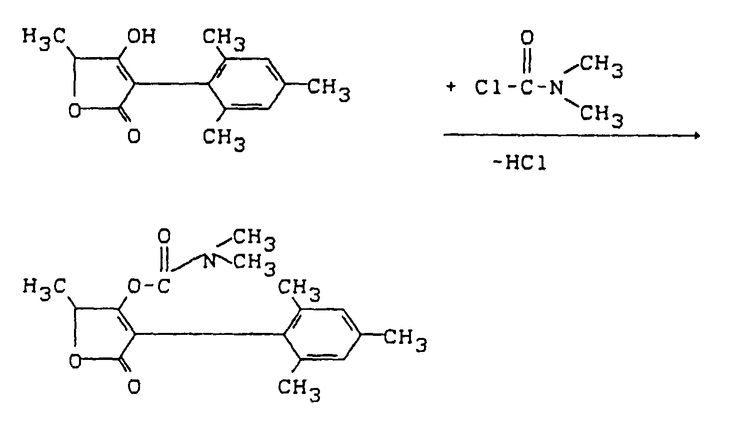

Verwendet man gemäß Verfahren (H) 3-(2,4,6-Trimethylphenyl)-4-hydroxy-5.5-dimethyl-Δ³-dihydro-furan-2-on und NaOH als Komponenten, so kann der Verlauf des erfindungsgemäßen Verfahrens durch folgendes Reaktionsschema wiedergegeben werden:

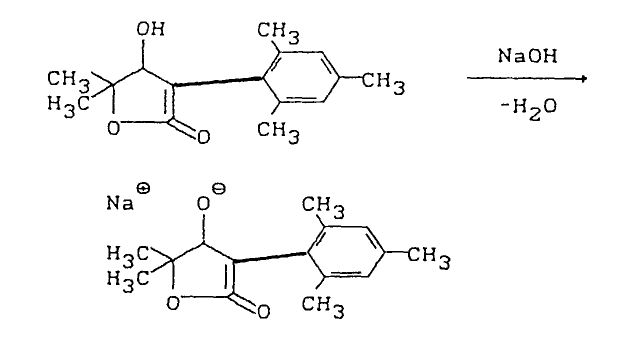

Die bei dem obigen Verfahren (A) als Ausgangsstoffe benötigten Verbindungen der Formel (II) 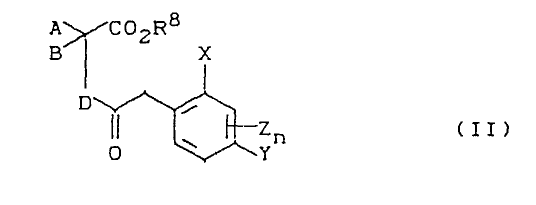 in welcher
A, B, D, X, Y, Z, n und R⁸ die oben angegebene Bedeutung haben sind bekannt oder lassen sich nach im Prinzip bekannten Methoden in einfacher Weise herstellen. So erhält man z.B. O-Acyl-α-hydroxycarbonsäureester der Formel (II), wenn man
a) 2-Hydroxycarbonsäure-(ester) bzw. 2-Mercaptocarbonsäure-(ester) der Formel (XIV) 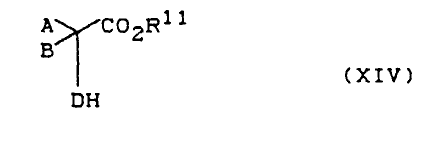 in welcher
- R¹¹: für Wasserstoff (XIVa) oder Alkyl (XIVb) steht
und
A, B und D die oben angegebene Bedeutung haben,
mit Phenylessigsäurehalogeniden der Formel (XV) 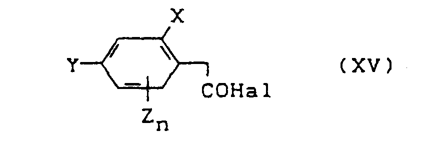 in welcher
- X, Y, Z und n: die oben angegebene Bedeutung haben
und
- Hal: für Chlor oder Brom steht,
acycliert (Chem. Reviews 52 237-416 (1953));
oder wenn man Thio- bzw. Hydroxycarbonsäuren der Formel (IIa), 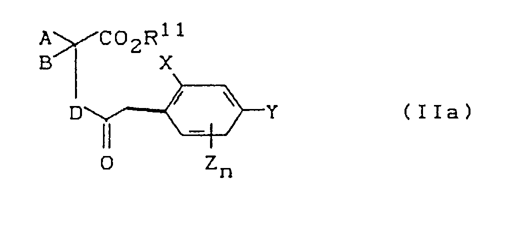 in welcher
A, B, D, X, Y, Z und n die oben angegebene Bedeutung haben
und
- R¹¹: für Wasserstoff steht,
verestert (Chem. Ind. (London) 1568 (1968).

Verbindungen der Formel (IIa) sind beispielsweise aus den Phenylessigsäurehalogeniden der Formel (XV) und Thio- bzw. Hydroxycarbonsäuren der Formel XIVa) erhältlich (Chem. Reviews 52 237-416 (1953).

Weiterhin erhält man Verbindungen der Formel (II), wenn man Phenylessigsäuren der Formel XVI 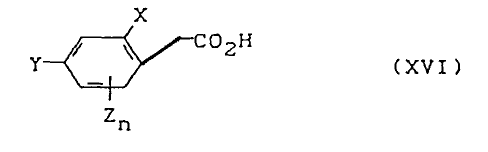 in welcher
X, Y, Z und n die oben angegebene Bedeutung haben
mit α-Halogencarbonsäureestern der Formel XVII 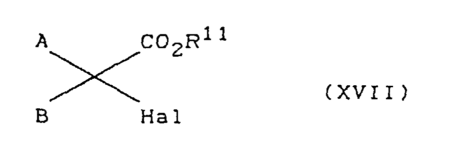 in welcher
A und B die oben angegebene Bedeutung haben,
- R¹¹: für Alkyl steht und
- Hal: für Chlor oder Brom steht
alkyliert.

Beispielhaft seien folgende Verbindungen der Formel (II) genannt:
O-(2,4-Dichlorphenyl-acetyl)-hydroxyessigsäureethylester
O-(2,6-Dichlorphenyl-acetyl)-hydroxyessigsäureethylester
O-(2,4,6-Trichlorphenyl-acetyl)-hydroxyessigsäureethylester
O-(2,4-Dimethylphenyl-acetyl)-hydroxyessigsäureethylester
O-(2,6-Dimethylphenyl-acetyl)-hydroxyessigsäureethylester
O-(2,4,6-Trimethylphenyl-acetyl)-hydroxyessigsäureethylester
O-(2,4-Dichlorphenyl-acetyl)-milchsäureethylester
O-(2,6-Dichlorphenyl-acetyl)-milchsäureethylester
O-(2,4,6-Trichlorphenyl-acetyl)-milchsäureethylester
O-(2,4-Dimethylphenyl-acetyl)-milchsäureethylester
O-(2,6-Dimethylphenyl-acetyl)-milchsäureethylester
O-(2,4,6-Trimethylphenyl-acetyl)-milchsäureethylester
O-(2,4-Dichlorphenyl-acetyl)-hydroxyisobuttersäureethylester
O-(2,6-Dichlorphenyl-acetyl)-hydroxyisobuttersäureethylester
O-(2,4,6-Trichlorphenyl-acetyl)-hydroxyisobuttersäureethylester
O-(2,4-Dimethylphenyl-acetyl)-hydroxyisobuttersäureethylester
O-(2,6-Dimethylphenyl-acetyl)-hydroxyisobuttersäureethylester
O-(2,4,6-Trimethylphenyl-acetyl)-hydroxyisobuttersäureethylester
O-(2,4-Dichlorphenyl-acetyl)-mandelsäureethylester
O-(2,6-Dichlorphenyl-acetyl)-mandelsäureethylester
O-(2,4,6-Trichlorphenyl-acetyl)-mandelsäureethylester
O-(2,4-Dimethylphenyl-acetyl)-mandelsäureethylester
O-(2,6-Dimethylphenyl-acetyl)-mandelsäureethylester
O-(2,4,6-Trimethylphenyl-acetyl)-mandelsäureethylester
O-(2,4-Dichlorphenyl-acetyl)-1-hydroxycyclohexancarbonsäureethylester
O-(2,6-Dichlorphenyl-acetyl)-1-hydroxycyclohexancarbonsäureethylester
O-(2,4,6-Trichlorphenyl-acetyl)-1-hydroxycyclohexancarbonsäureethylester
O-(2,4-Dimethylphenyl-acetyl)-1-hydroxycyclohexancarbonsäureethylester
O-(2,6-Dimethylphenyl-acetyl)-1-hydroxycyclohexancarbonsäureethylester
O-(2,4,6-Trimethylphenyl-acetyl)-1-hydroxycyclohexancarbonsäureethylester
O-(2,4-Dichlorphenyl-acetyl)-2-hydroxy-2-ethyl-buttersäureethylester
O-(2,6-Dichlorphenyl-acetyl)-2-hydroxy-2-ethylbuttersäureethylester
O-(2,4,6-Trichlorphenyl-acetyl)-2-hydroxy-2-ethylbuttersäureethylester
O-(2,4-Dimethylphenyl-acetyl)-2-hydroxy-2-ethylbuttersäureethylester
O-(2,6-Dimethylphenyl-acetyl)-2-hydroxy-2-ethylbuttersäureethylester
O-(2,4,6-Trimethylphenyl-acetyl)-2-hydroxy-2-ethylbuttersäureethylester

Beispielhaft seien folgende Verbindungen der Formel (II) genannt:
S-(2,4-Dichlorphenyl-acetyl)thioessigsäureethylester
S-(2,6-Dichlorphenyl-acetyl)-thioessigsäureethylester
S-(2,4,6-Trichlorphenyl-acetyl)-thioessigsäureethylester
S-(2,4-Dimethylphenyl-acetyl)-thioessigsäureethylester
S-(2,6-Dimethylphenyl-acetyl)-thioessigsäureethylester
S-(2,4,6-Trimethylphenyl-acetyl)-thioessigsäureethylester
S-(2,4-Dichlorphenyl-acetyl)-thiomilchsäureethylester
S-(2,6-Dichlorphenyl-acetyl)-thiomilchsäureethylester
S-(2,4,6-Trichlorphenyl-acetyl)-thiomilchsäureethylester
S-(2,4-Dimethylphenyl-acetyl)-thiomilchsäureethylester
S-(2,6-Dimethylphenyl-acetyl)-thiomilchsäureethylester
S-(2,4,6-Trimethylphenyl-acetyl)-thiomilchsäureethylester
S-(2,4-Dichlorphenyl-acetyl)-thioisobuttersäureethylester
S-(2,6-Dichlorphenyl-acetyl)-thioisobuttersäureethylester
S-(2,4,6-Trichlorphenyl-acetyl)-thioisobuttersäureethylester
S-(2,4-Dimethylphenyl-acetyl)-thioisobuttersäureethylester
S-(2,6-Dimethylphenyl-acetyl)-thioisobuttersäureethylester
S-(2,4,6-Trimethylphenyl-acetyl)-thioisobuttersäureethylester

Das Verfahren (A) ist dadurch gekennzeichnet, daß Verbindungen der Formel (II) in welcher A, B, D, X, Y, Z, n und R⁸ die oben angegebene Bedeutung haben, in Gegenwart von Basen einer intramolekularen Kondensation unterwirft.

Als Verdünnungsmittel können bei dem erfindungsgemäßen Verfahren (A) alle inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, iso-Propanol, Butanol, Isobutanol, tert.-Butanol eingesetzt werden.

Als Basen (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 oder TDA 1 eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetall-alkoholate, wie Natrium-methylat, Natriumethylat und Kalium-tert.-butylat einsetzbar.

Die Reaktionstemperaturen können bei der Durchführung des erfindungsgemäßen Verfahrens (A) innerhalb eines größeren Bereiches variiert werden. Im allgemeinen arbeitet man bei Temperaturen zwischen 0°C und 250°C, vorzugsweise zwischen 50°C und 150°C.

Das erfindungsgemäße Verfahren (A) wird im allgemeinen unter Normaldruck durchgeführt.

Bei der Durchführung des erfindungsgemäßen Verfahrens (A) setzt man die Reaktionskomponenten der Formeln (II) und die deprotonierenden Basen im allgemeinen in etwa äquimolaren Mengen ein. Es ist jedoch auch möglich, die eine oder andere Komponente in einem größeren Überschuß (bis zu 3 Mol) zu verwenden.
- Adogen 464 =: Methyltrialkyl(C₈-C₁₀)ammoniumchlorid
- TDA 1 =: Tris-(methoxyethoxyethyl)-amin

Das Verfahren (Bα) ist dadurch gekennzeichnet, daß man Verbindungen der Formel (Ia) mit Carbonsäurehalogeniden der Formel (III) umsetzt.

Als Verdünnungsmittel können bei dem erfindungsgemäßen Verfahren (Bα) bei Verwendung der Säurehalogenide alle gegenüber diesen Verbindungen inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon,weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan, Wenn die Hydrolysestabilität des Säurehalogenids es zuläßt, kann die Umsetzung auch in Gegenwart von Wasser durchgeführt werden.

Verwendet man die entsprechenden Carbonsäurehalogenide so kommen als Säurebindemittel bei der Umsetzung nach dem erfindungsgemäßen Verfahren (Bα) alle üblichen Säureakzeptoren in Betracht, Vorzugsweise verwendbar sind tertiäre Amine, wie Triethylamin, Pyridin, Diazabiyclooctan (DABCO), Diazabicycloundecan (DBU), Diazabicyclononen (DBN), Hüning-Base und N,N-Dimethylanilin, ferner Erdalkalimetalloxide, wie Magnesium- und Calciumoxid, außerdem Alkali- und Erdalkali-metall-carbonate, wie Natriumcarbonat, Kaliumcarbonat und Calciumcarbonat.

Die Reaktionstemperaturen können auch bei dem erfindungsgemäßen Verfahren (Bα) auch bei der Verwendung von Carbonsäurehalogeniden innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (Bα) werden die Ausgangsstoffe der Formel (Ia) und das Carbonsäurehalogenid der Formel (III) im allgemeinen in angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Das Verfahren (Bβ) ist dadurch gekennzeichnet, daß man Verbindungen der Formel (Ia) mit Carbonsäurehydriden der Formel (IV) umsetzt.

Verwendet man bei dem erfindungsgemäßen Verfahren (Bβ) als Reaktionskomponente der Formel (IV) Carbonsäureanhydride, so können als Verdünnungsmittel vorzugsweise diejenigen Verdünnungsmittel verwendet werden, die auch bei der Verwendung von Säurehalogeniden vorzugsweise in Betracht kommen. Im übrigen kann auch ein im Überschuß eingesetztes Carbonsäurehydrid gleichzeitig als Verdünnungsmittel fungieren.

Die Reaktionstemperaturen können bei dem erfindungsgemäßen Verfahren (Bβ) auch bei der Verwendung von Carbonsäureanhydriden innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens werden die Ausgangsstoffe der Formel (Ia) und das Carbonsäureanhydrid der Formel (IV) im allgemeinen in angenähert äquivalenten Mengen verwendet. Es ist jedoch auch möglich, das Carbonsäureanhydrid in einem größeren Überschuß (bis zu 5 Mol) einzusetzen. Die Aufarbeitung erfolgt nach üblichen Methoden.

Im allgemeinen geht man so vor, daß man Verdünnungsmittel und im Überschuß vorhandenes Carbonsäureanhydrid sowie die entstehende Carbonsäure durch Destillation oder durch Waschen mit einem organischen Lösungsmittel oder mit Wasser entfernt.

Das Verfahren (C) ist dadurch gekennzeichnet, daß man Verbindungen der Formel (Ia) mit Chlorameisensäureestern oder Chlorameisensäurethiolestern der Formel (V) umsetzt.

Verwendet man die entsprechenden Chlorameisensäureester bzw. Chlorameisensäurethiolester so kommen als Säurebindemittel bei der Umsetzung nach dem erfindungsgemäßen Verfahren (C) alle üblichen Säureakzeptoren in Betracht. Vorzugsweise verwendbar sind tertiäre Amine, wie Triethylamin, Pyridin, DABCO, DBC, DBA, Hünig-Base und N,N-Dimethyl-anilin, ferner Erdalkalimetalloxide, wie Magnesium- und Calcium-oxid, außerdem Alkali- und Erdalkalimetall-carbonate, wie Natriumcarbonat, Kaliumcarbonat und Calciumcarbonat.

Als Verdünnungsmittel können bei dem erfindungsgemäßen Verfahren (C) bei Verwendung der Chlorameisensäureester bzw. Chlorameisensäurethiolester alle gegenüber diesen Verbindungen inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüberhinaus Carbonsäureester, wie Ethylacetat, und auch stark polare Solventien, wie Dimethylsulfoxid und Sulfolan.

Bei Verwendung der Chlorameisensäureester bzw. Chlorameisensäurethiolester als Carbonsäure-Derivate der Formel (V) können die Reaktionstemperaturen bei der Durchführung des erfindungsgemäßen Verfahrens (C) innerhalb eines größeren Bereiches variiert werden. Arbeitet man in Gegenwart eines Verdünnungsmittels und eines Säurebindemittels, so liegen die Reaktionstemperaturen im allgemeinen zwischen -20°C und +100°C, vorzugsweise zwischen 0°C und 50°C.

Das erfindungsgemäße Verfahren (C) wird im allgemeinen unter Normaldruck durchgeführt.

Bei der Durchführung des erfindungsgemäßen Verfahrens (C) werden die Ausgangsstoffe der Formel (Ia) und der entsprechende Chlorameisensäureester bzw. Chlorameisensäurethiolester der Formel (V) im allgemeinen in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt dann nach üblichen Methoden. Im allgemeinen geht man so vor, daß man ausgefallene Salze entfernt und das verbleibende Reaktionsgemisch durch Abziehen des Verdünnungsmittels einengt.

Beim Herstellungsverfahren D_{α} setzt man pro Mol Ausgangsverbindung der Formel (Ia) ca. 1 Mol Chlormonothioameisensäureester bzw. Chlordithioameisensäureester der Formel (VII) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Ether, Amide, Alkohole, Sulfone, Sulfoxide.

Vorzugsweise werden Dimethylsulfoxid, Tetrahydrofuran, Dimethylformamid, Dimethylsulfid eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindung Ia dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Beim Herstellungsverfahren D_{β} setzt man pro Mol Ausgangsverbindung der Formel (II) die äquimolare Menge bzw. einen Überschuß Schwefelkohlenstoff zu. Man arbeitet hierbei vorzugsweise bei Temperaturen von 0 bis 50°C und insbesondere bei 20 bis 30°C.

Oft ist es zweckmäßig zunächst aus der Verbindung der Formel (II) durch Zusatz eines Deprotonierungsmittels (wie z.B. Kaliumtertiärbutylat oder Natriumhydrid) das entsprechende Salz herzustellen. Man setzt die Verbindung (II) solange mit Schwefelkohlenstoff um bis die Bildung der Zwischenverbindung abgeschlossen ist, z.B. nach mehrstündigem Rühren bei Raumtemperatur.

Die weitere Umsetzung mit dem Alkylhalogenid der Formel (VIII) erfolgt vorzugsweise bei 0 bis 70°C und insbesondere bei 20 bis 50°C. Hierbei wird mindestens die äquimolare Menge Alkylhalogenid eingesetzt.

Man arbeitet bei Normaldruck oder unter erhöhtem Druck, vorzugsweise bei Normaldruck.

Die Aufarbeitung erfolgt wiederum nach üblichen Methoden.

Beim Herstellungsverfahren E) setzt man pro Mol Ausgangsverbindung der Formel (Ia) ca. 1 Mol Sulfonsäurechlorid (VIII) bei 0 bis 150°C, vorzugsweise bei 20 bis 70°C um.

Als gegebenenfalls zugesetzte Verdünnungsmittel kommen alle inerten polaren organischen Lösungsmittel in Frage wie Ether, Amide, Nitrile, Alkohole, Sulfone, Sulfoxide.

Vorzugsweise werden Dimethylsulfoxid, Tetrahydrofuran, Dimethylformamid, Dimethylsulfid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung Ia dar, kann auf den weitern Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Beim Herstellungsverfahren E kann gegebenenfalls unter Phasen-Transfer-Bedingungen gearbeitet werden (W.J. Spillane et. al.; J. Chem. Soc., Perkin Trans I, (3) 677-9 (1982)). In diesem Fall setzt man pro Mol Ausgangsverbindung der Formel a) 0,3 bis 1,5 mol säurechlorid VIII, bevorzugt 0,5 mol bei 0° bis 150°C, vorzugsweise bei 20 bis 70°C um.

Als Phasen-Transfer-Katalysatoren können alle quartären Ammoniumsalze verwendet werden, vorzugsweise Tetraoctylammoniumbromid und Benzyltriethylammoniumchlorid. Als organische Lösungsmittel können in diesem Fall alle unpolaren inerten Lösungsmittel dienen, bevorzugt werden Benzol und Toluol eingesetzt.

Beim Herstellungsverfahren F) setzt man zum Erhalt von Verbindungen der Struktur (Ie) auf 1 Mol der Verbindung (Ia), 1 bis 2, vorzugsweise 1 bis 1,3 Mol der Phosphorverbindung der Formel (IX) bei Temperaturen zwischen - 40°C und 150°C, vorzugsweise zwischen -10 und 110°C Als gegebenenfalls zugesetzte Verdünnungsmittel kommen aller inerten, polaren organischen Lösungsmittel in Frage wie Ether, Amide, Nitrile, Alkohole, Sulfide, Sulfone, Sulfoxide etc.

Vorzugsweise werden Acetonitril, Dimethylsulfoxid, Tetrahydrofuran, Dimethylformamid, Dimethylsulfid eingesetzt.

Als gegebenenfalls zugesetzte Säurebindemittel kommen übliche anorganische oder organische Basen in Frage wie Hydroxide, Carbonate. Beispielhaft seien Natriumhydroxid, Natriumcarbonat, Kaliumcarbonat, Pyridin aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der organischen Chemie. Die Reinigung der anfallenden Endprodukte geschieht vorzugsweise durch Kristallisation, chromatographische Reinigung oder durch sogenanntes "Andestillieren", d.h. Entfernung der flüchtigen Bestandteile im Vakuum.

Beim Herstellungsverfahren G_{α} setzt man pro Mol Ausgangsverbindung der Formel Ia ca. 1 Mol Isocyanat der Formel (X) bei 0 bis 100°C, vorzugsweise bei 20 bis 50°C um.

Als gegebenenfalls zugesetzte Verdünnungsmittel kommen alle inerten organischen Lösungsmittel in Frage, wie Ether, Amide, Nitrile, Sulfone, Sulfoxide.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden. Es wird vorzugsweise bei Normaldruck gearbeitet.

Beim Herstellungsverfahren G_{β} setzt man pro Mol Ausgangsverbindung der Formel (Ia) ca. 1 Mol Carbamidsäurechlorid bzw. Thiocarbamidsäurechlorid der Formel (XI) bei 0 bis 150°C, vorzugsweise bei 20 bis 70°C um.

Als gegebenenfalls zugesetzte Verdünnungsmittel kommen aller inerten polaren organischen Lösungsmittel in Frage wie Ether, Amide, Alkohole, Sulfone, Sulfoxide.

Vorzugsweise werden Dimethylsulfoxid, Tetrahydrofuran, Dimethylformamid, Dimethylsulfid eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung Ia dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin aufgeführt.

Die Reaktion kann bei Normaldruck oder unter erhöhtem Druck durchgeführt werden, vorzugswiese wird bei Normaldruck gearbeitet. Die Aufarbeitung geschieht nach üblichen Methoden.

Das Verfahren (H) ist dadurch gekennzeichnet, daß man Verbindungen der Formel (Ia) mit Metallhydroxiden (XII) oder Aminen (XIII) umsetzt.

Als Verdünnungsmittel können bei dem erfindungsgemäßen Verfahren vorzugsweise Ether wie Tetrahydrofuran, Dioxan, Diethylether oder aber Alkohole wie Methanol, Ethanol, Isopropanol, aber auch Wasser eingesetzt werden. Das erfindungsgemäße Verfahren (H) wird im allgemeinen unter Normaldruck durchgeführt. Die Reaktionstemperaturen liegen im allgemeinen zwischen -20°C und 100°C, vorzugsweise zwischen 0°C und 50°C.

Bei der Durchführung des erfindungsgemäßen Verfahrens (H) werden die Ausgangsstoffe der Formel (Ia) bzw. (XII) oder (XIII) im allgemeinen in angenähert äquimolaren Mengen verwendet. Es ist jedoch auch möglich, die eine oder andere Komponente in einem größeren Überschuß (bis zu 2 Mol) einzusetzen. Im allgemeinen geht man so vor, daß man das Reaktionsgemisch durch Abziehen des Verdünnungsmittel einengt.

### Herstellungsbeispiele

### Beispiel Ia-1

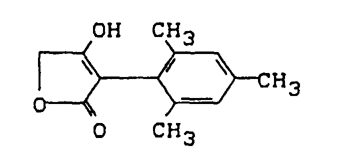

11,8 g (0,105 Mol) Kaliumtertiärbutylat werden bei 40°C in 100 ml tert. Butanol gelöst.

Anschließend läßt man 26 g 2,4,6-Trimethylphenylessigsäureethoxycarbonylmethylester, welcher in 50 ml tert. Butanol gelöst sind, bei 40°C unter Rühren zutropfen.

Man rührt in 600 ml Eiswasser ein, stellt mit 1N Salzsäure auf pH 2 ein, extrahiert mit Essigsäureethylester, wäscht zweimal mit Wasser, trocknet über Natriumsulfat und engt am Rotationsverdampfer ein.

Ausbeute: 6,82 g (30,3 % der Theorie) der Verbindung 3-(2,4,6-Trimethylphenyl)-4-hydroxy-Δ³-dihydrofuranon-2.

Schmelzpunkt (nach dem Umkristallisieren aus Methylenchlorid/n-Hexan) 154°C.

### Beispiel Ia-2

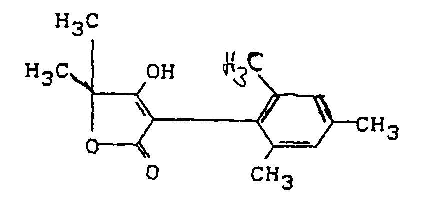

2,16 g (90 mmol) Natriumhydrid (80 %ig) wurden in 50 ml absolutem Toluol vorgelegt. Man arbeitet unter Argon-Atmosphäre. Es wird auf Rückflußtemperatur erhitzt. Dann läßt man unter Rückfluß 17,5 g (60 mmol) in 70 ml absolutem Toluol gelöste Verbindung der Formel 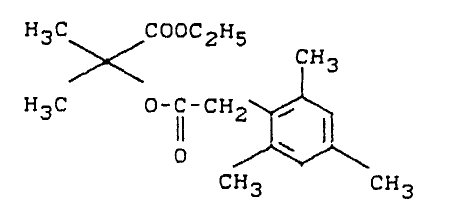 zutropfen und erhitzt 3 Stunden lang unter Rückfluß.

Zum Zwecke der Aufarbeitung wird die Lösung einrotiert, der Rückstand in Wasser aufgenommen und die Lösung angesäuert. Der dabei ausfallende Niederschlag wird in Methylenchlorid aufgenommen und die wäßrige Mutterlauge noch mehrfach extrahiert. Anschließend wird über Natriumsulfat getrocknet und am Rotationsverdampfer eingeengt.

Zur Reinigung suspendiert man heiß in 20 ml Chloroform, gibt unter Rückfluß 60 ml n-Hexan langsam zu, läßt langsam abkühlen, saugt ab und trocknet.

Ausbeute 4,66 g (= 32 % d. Th) der Verbindung 3-(2,4,6-Trimethylphenyl)-4-hydroxy-5,5-dimethyl-Δ³-dihydrofuranon-(2) vom Schmelzpunkt 254°C.

In Analogie zu den Herstellungsmethoden der Beispiele Ia-1 und Ia-2 wurden die folgenden Herstellungsbeispiele synthetisiert:

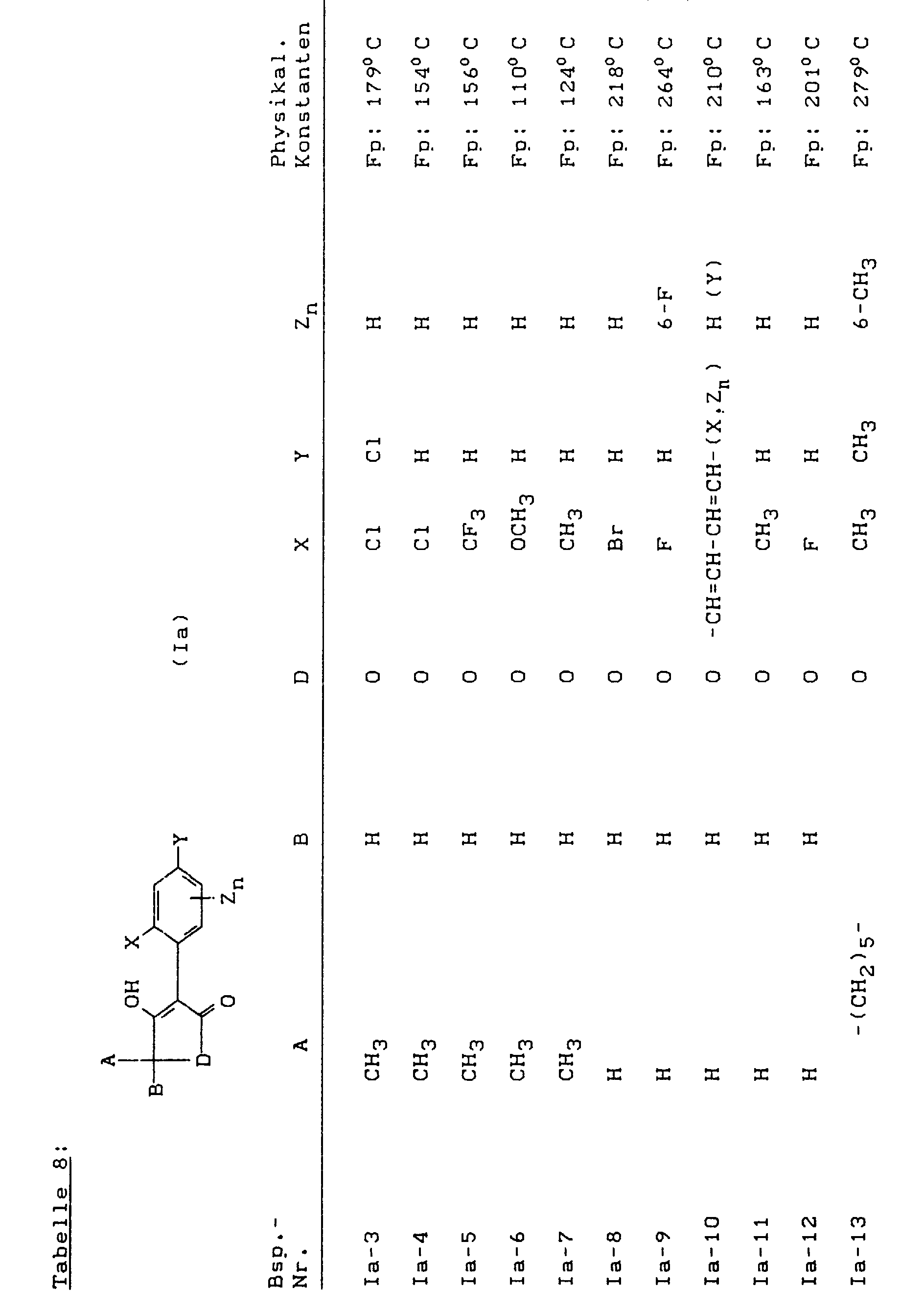

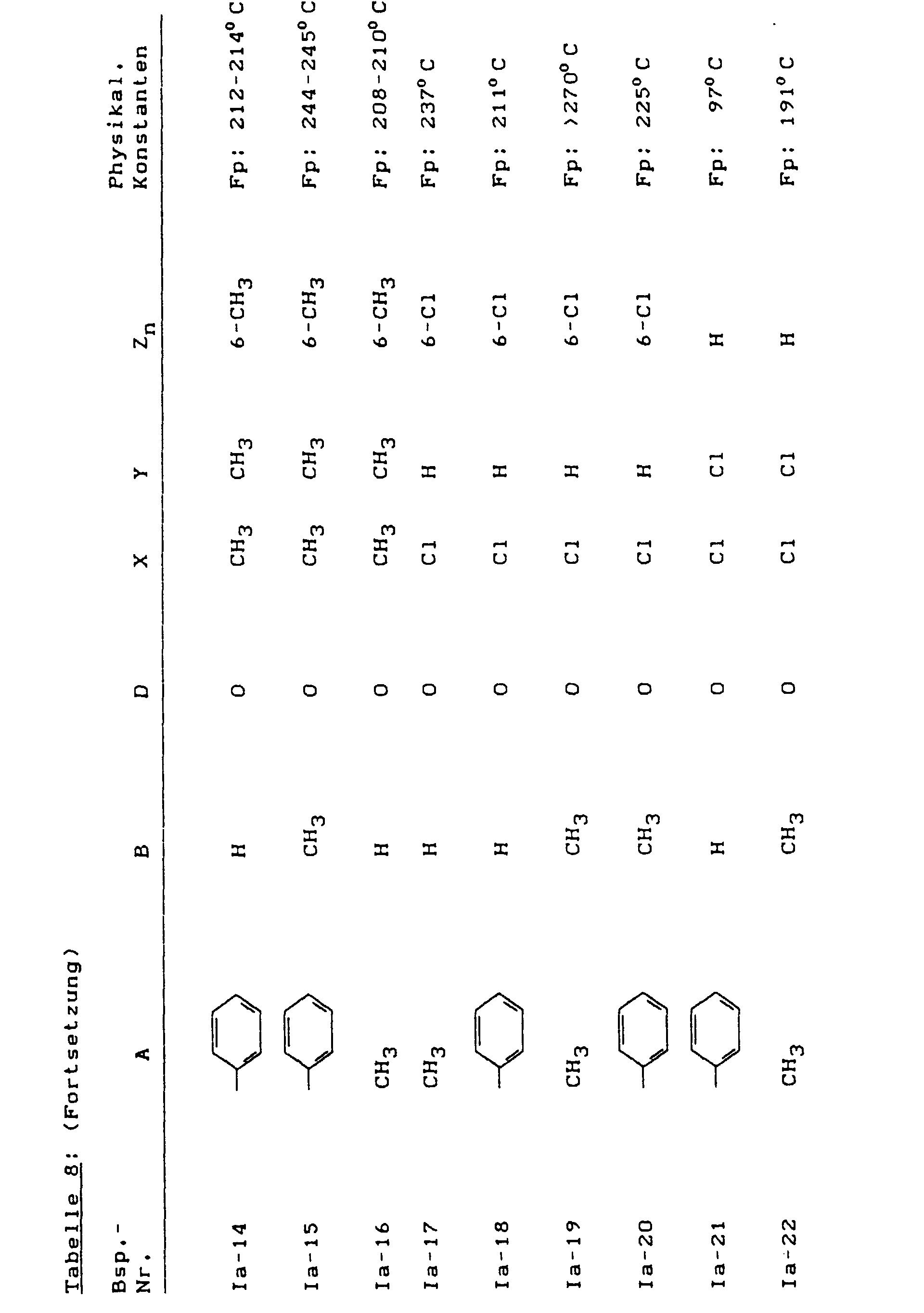

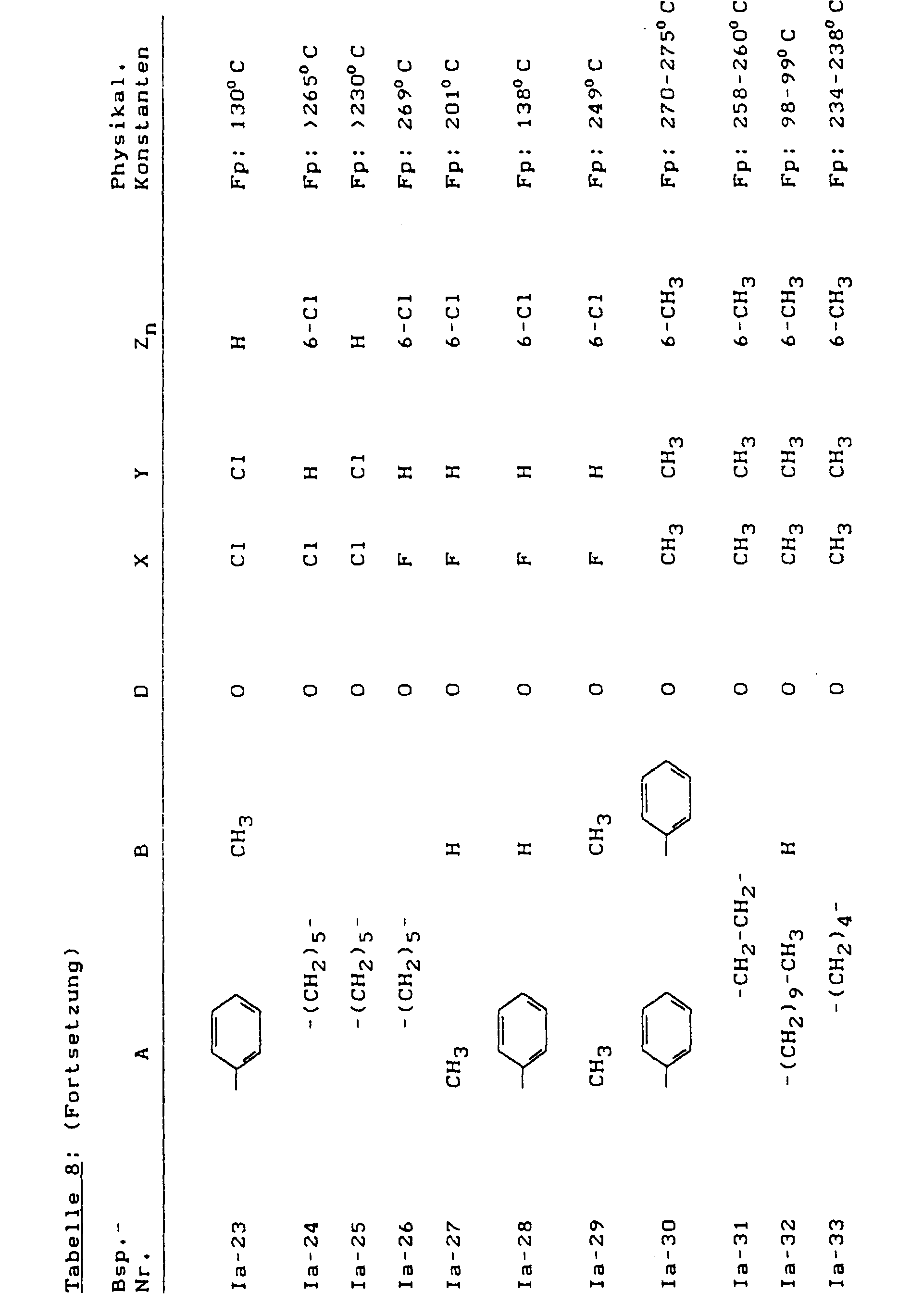

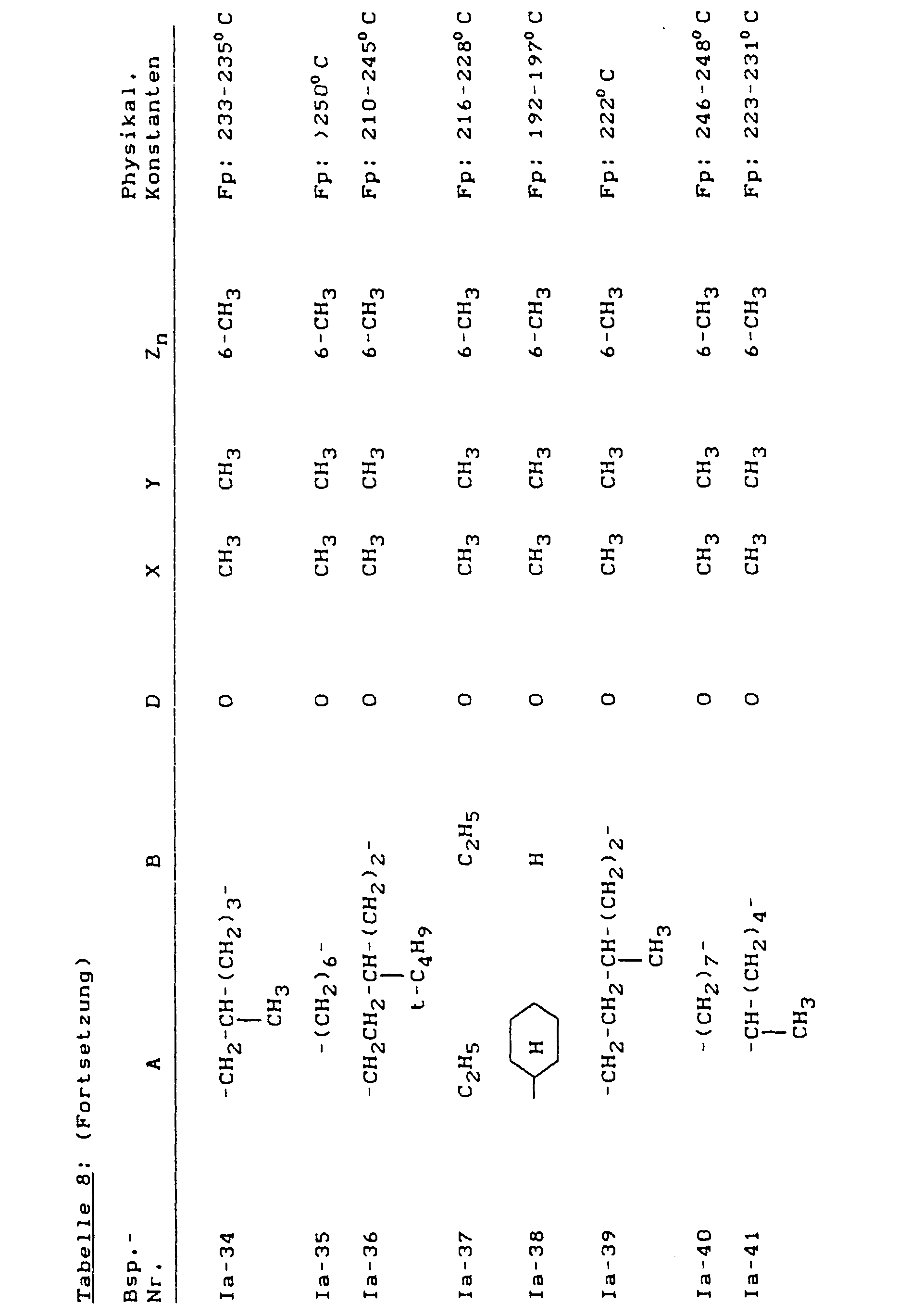

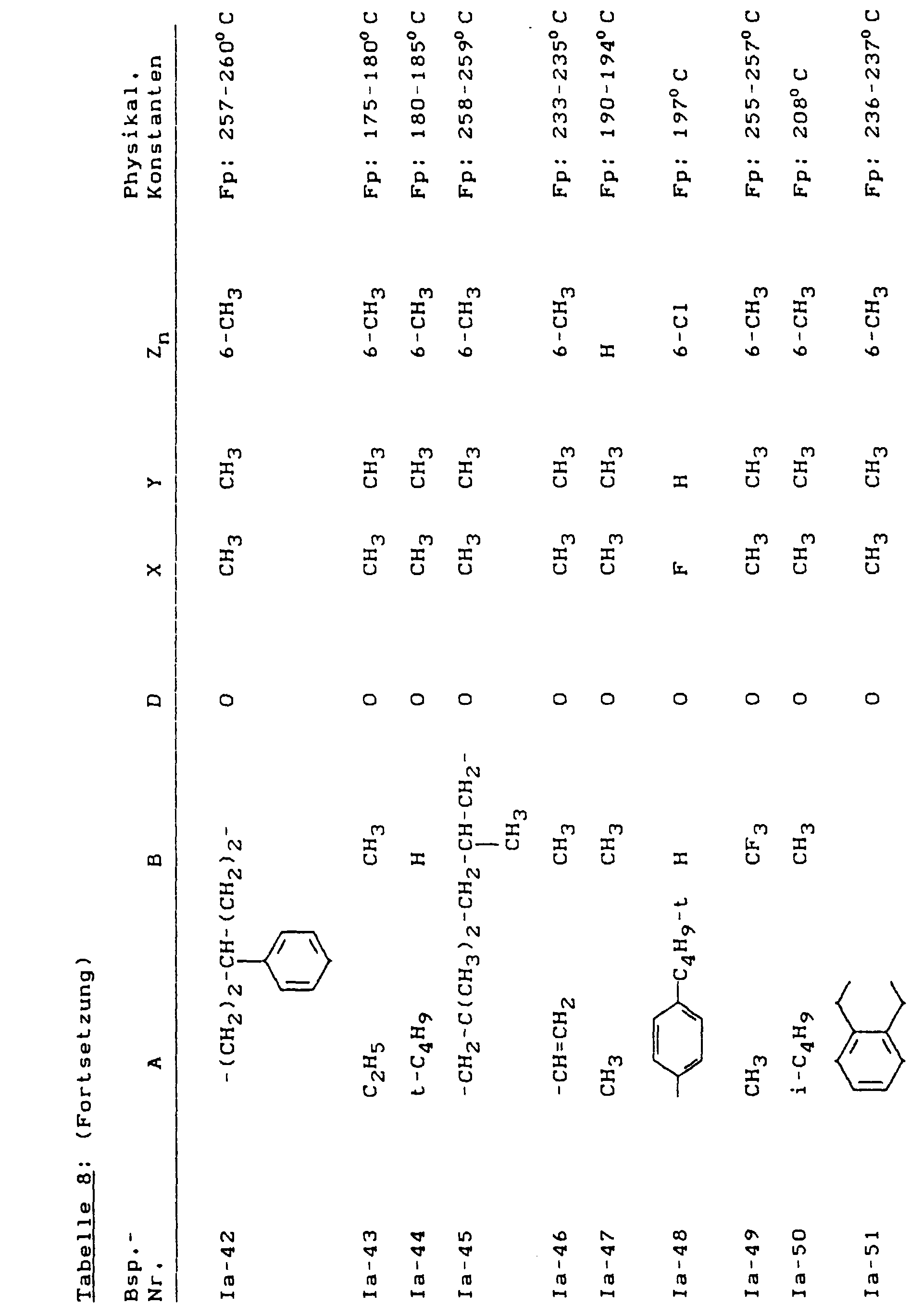

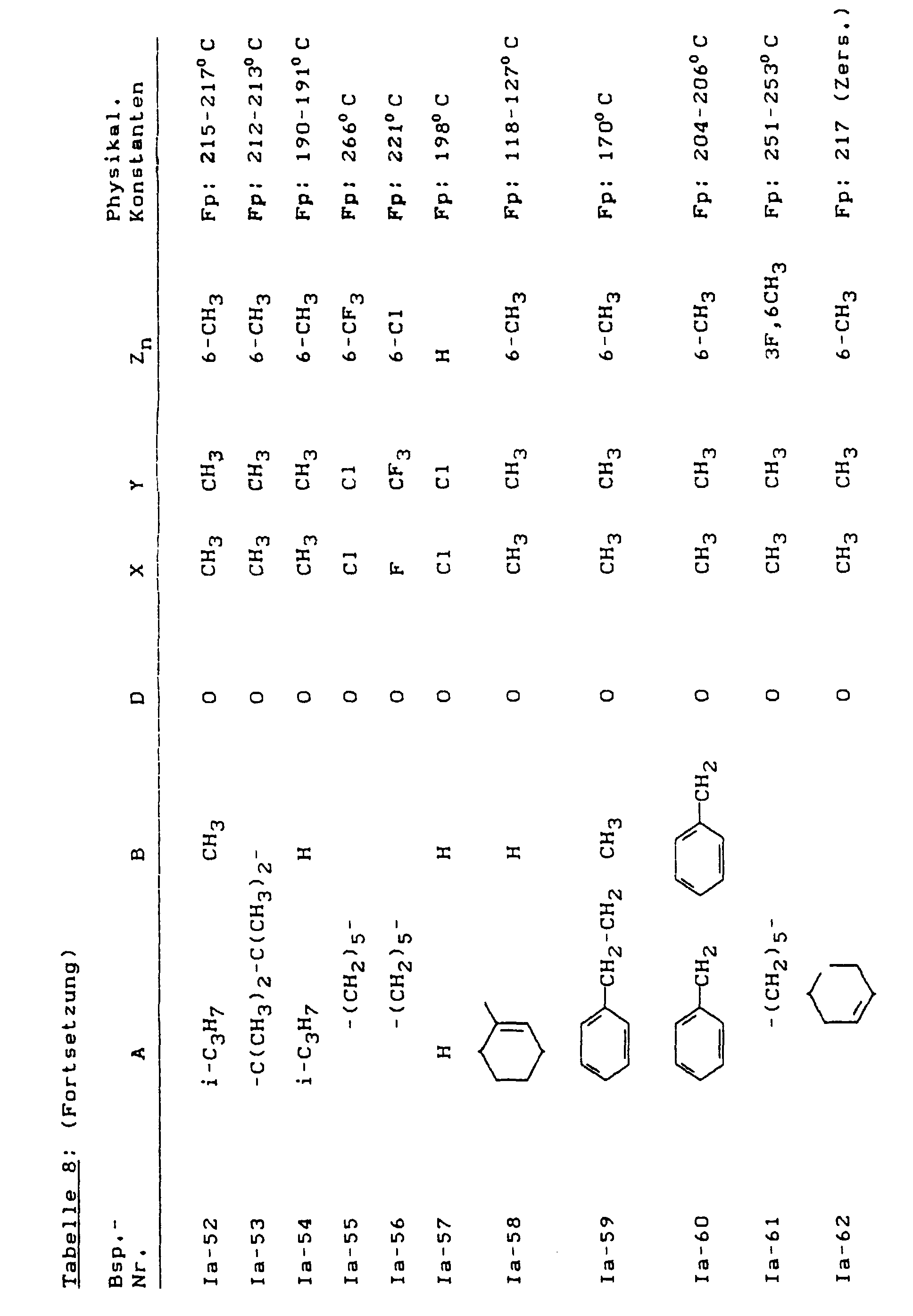

### Beispiel Ib-1

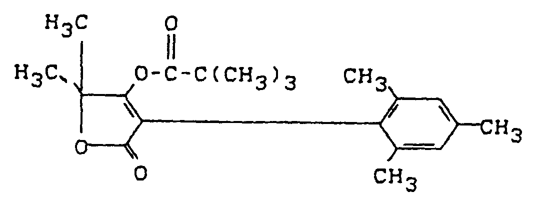

1,23 g (5 mmol) 3-(2,4,6-Trimethylphenyl)-4-hydroxy-5,5-dimethyl-Δ³ -dihydrofuranon-(2) werden in 20 ml absolutem Methylenchlorid vorgelegt. Dazu gibt man 0,61 g (6 mmol) Triethylamin, tropft bei 0-10°C eine Lösung von 0,72 g (6 mmol) Pivaloylchlorid in 5 ml abs. Methylenchlorid zu und rührt 1 h bei Raumtemperatur nach.

Zur Aufarbeitung wird die Lösung mit wäßriger Citronensäure und wäßriger Natriumhydrogencarbonatlösung gewaschen, über Natriumsulfat getrocknet und einrotiert.

Ausbeute: 1,43 g (87 % d.Th.) der Verbindung 3-(2,4,6-Trimethylphenyl)4-pivaloyloxy-5,5-dimethyl-Δ³-dihydrofuranon-(2) von Schmelzpunkt 82°C.

### Beispiel Ib-2

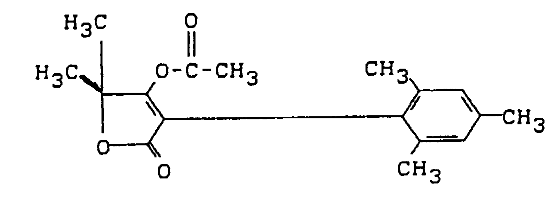

2,46 g (10 mmol) 3-(2,4,6-Trimethylphenyl)-4-hydroxy-5,5-dimethyl-Δ³-dihydrofuranon-(2) werden in 40 ml absolutem Methylenchlorid vorgelegt. Man setzt 1,11 g (11 mmol) Triethylamin zu, tropft bei 0-10°C eine Lösung von 0,86 g (11 mmol) Acetylchlorid in 10 ml abs. Methylenchlorid zu und läßt noch 1 h bei Raumtemperatur rühren.

Die Aufarbeitung erfolgt analog zu Beispiel 3.

Ausbeute: 2,55 g (88 % d. Th.) der Verbindung 3-(2,4,6-Trimethylphenyl)-4-acetyloxy-5,5-dimethyl-Δ³-dihydrofuranon-(2) vom Schmelzpunkt 160°C.

In Analogie zu den Hertellungsmethoden der Beispiele Ib-1 bis Ib-2 wurden die folgenden Herstellungsbeispiele synthetisiert:

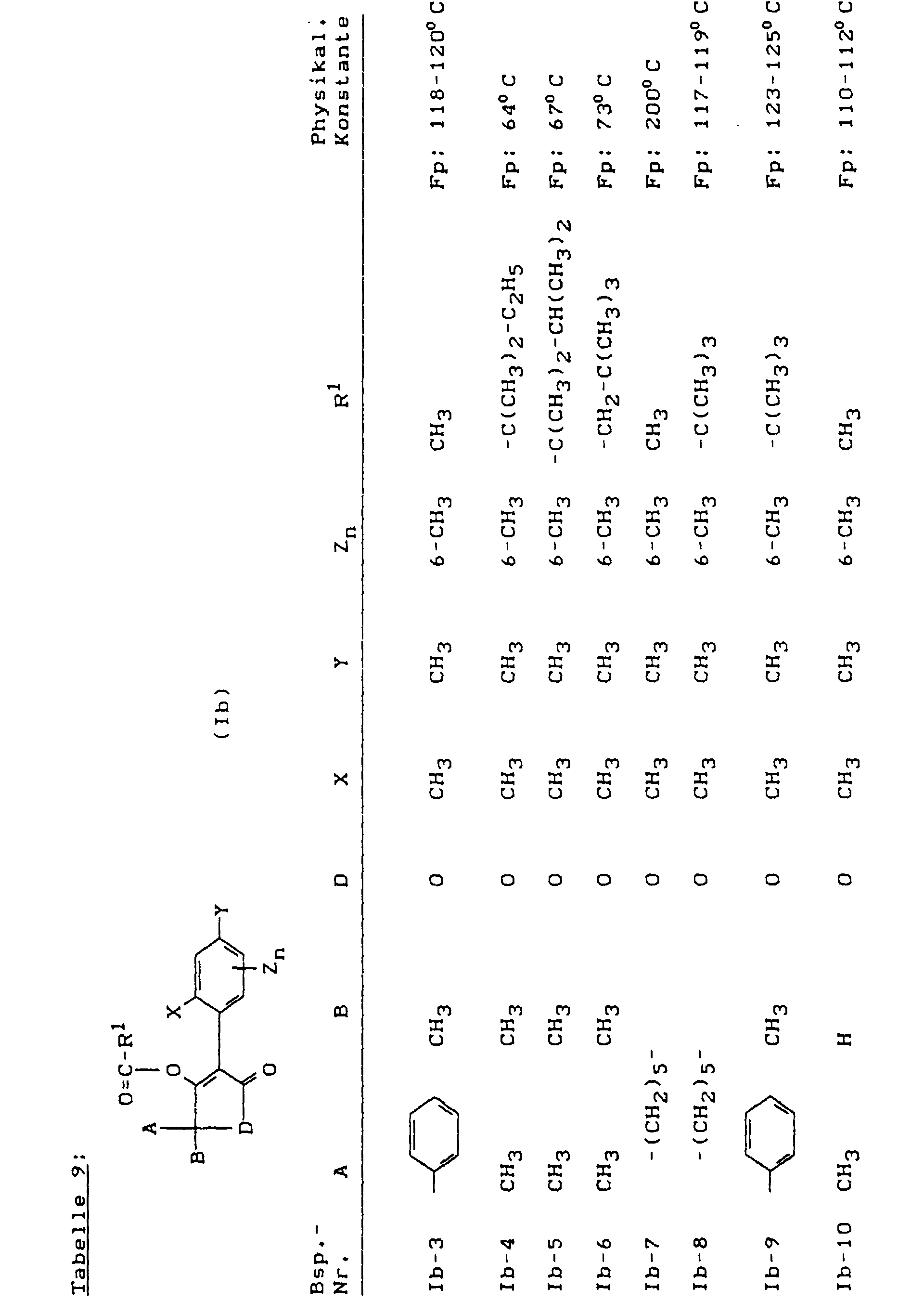

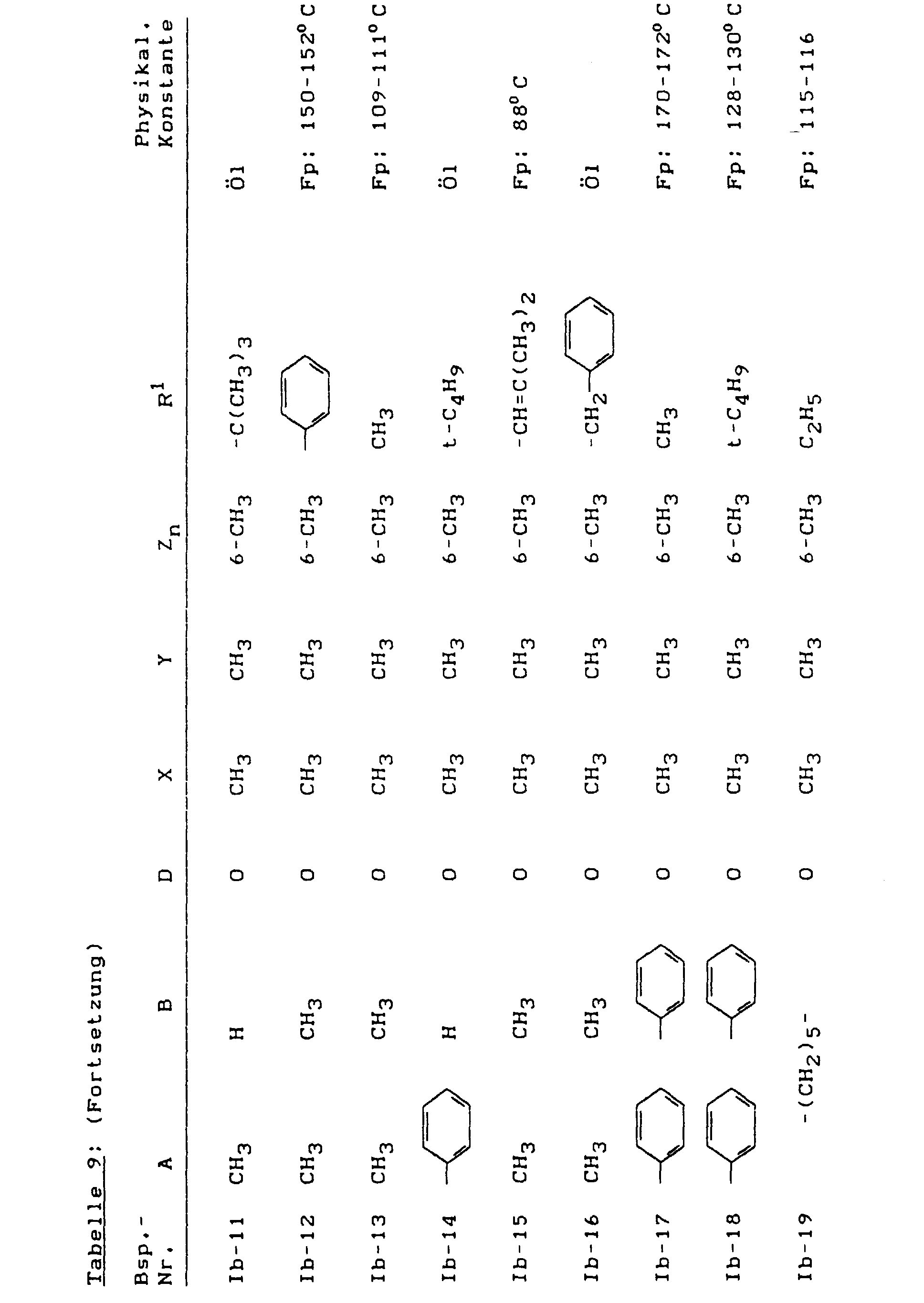

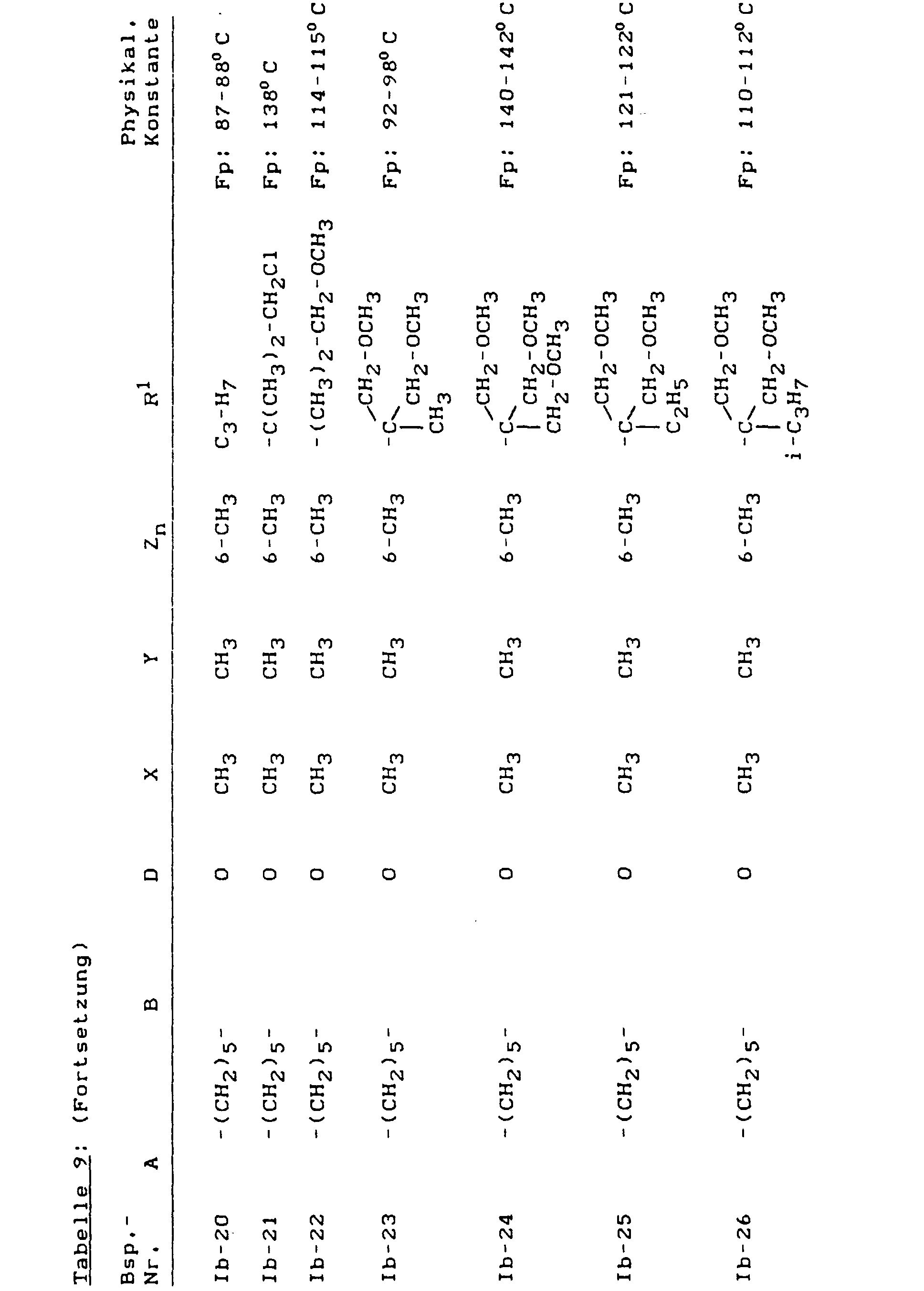

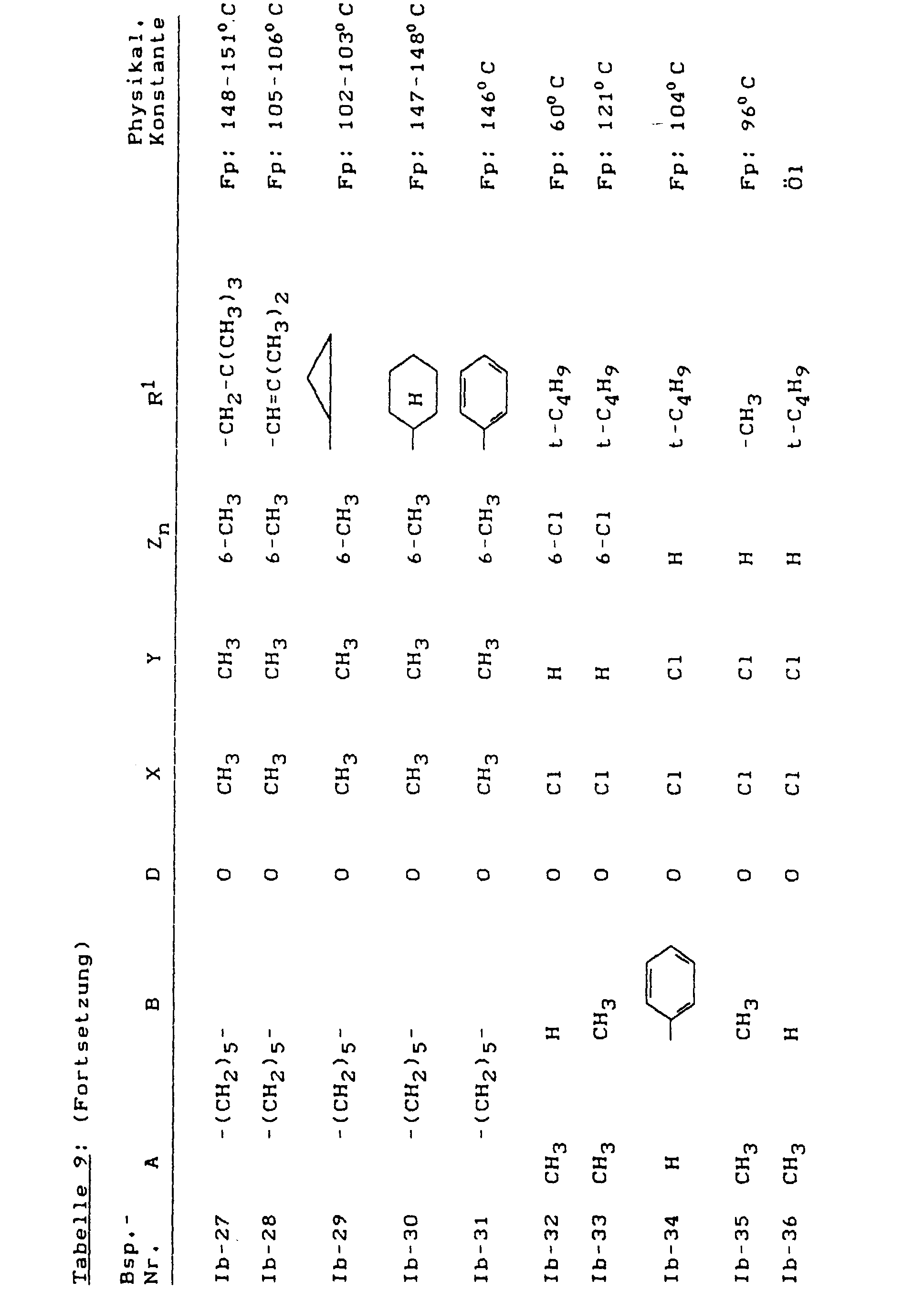

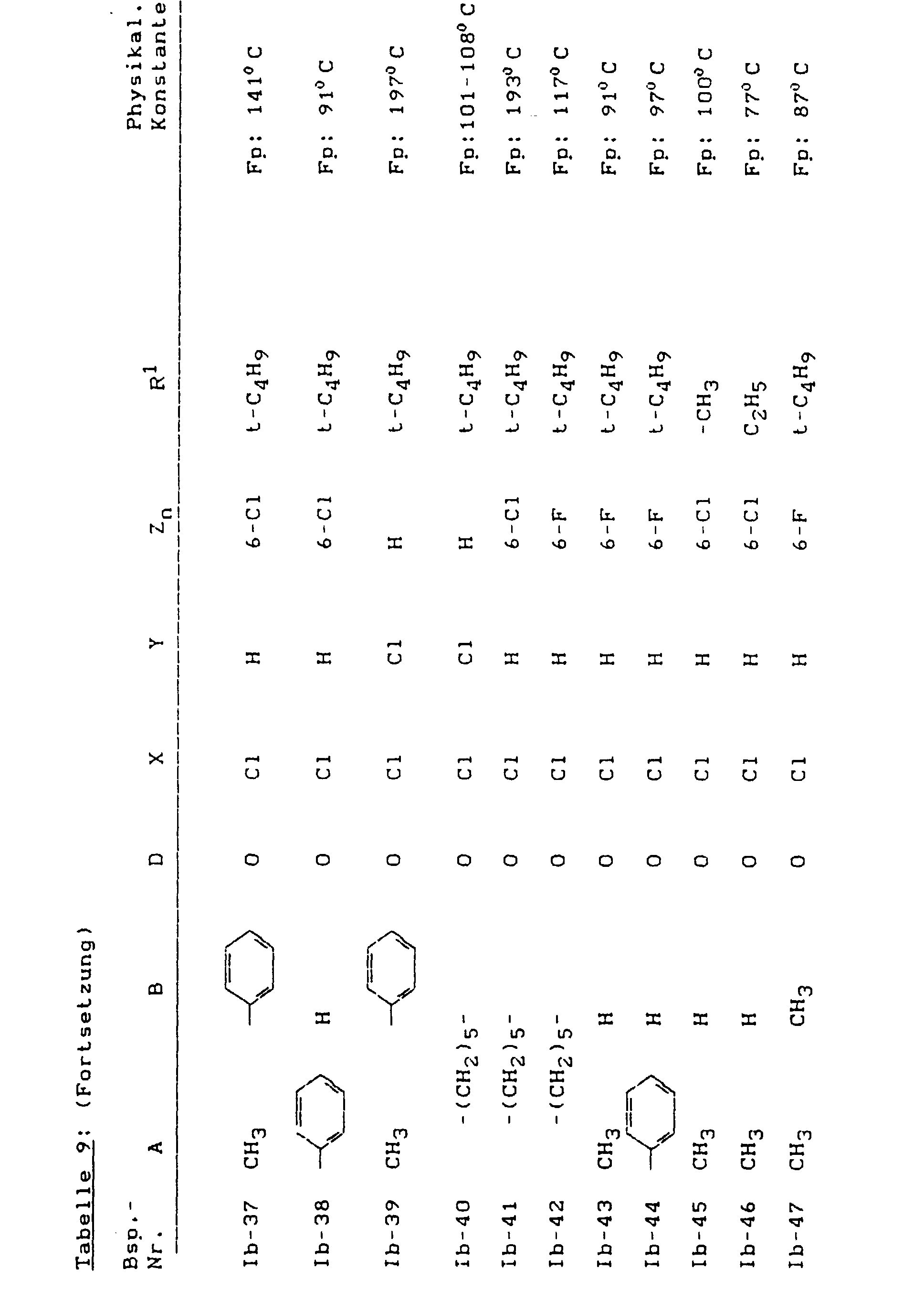

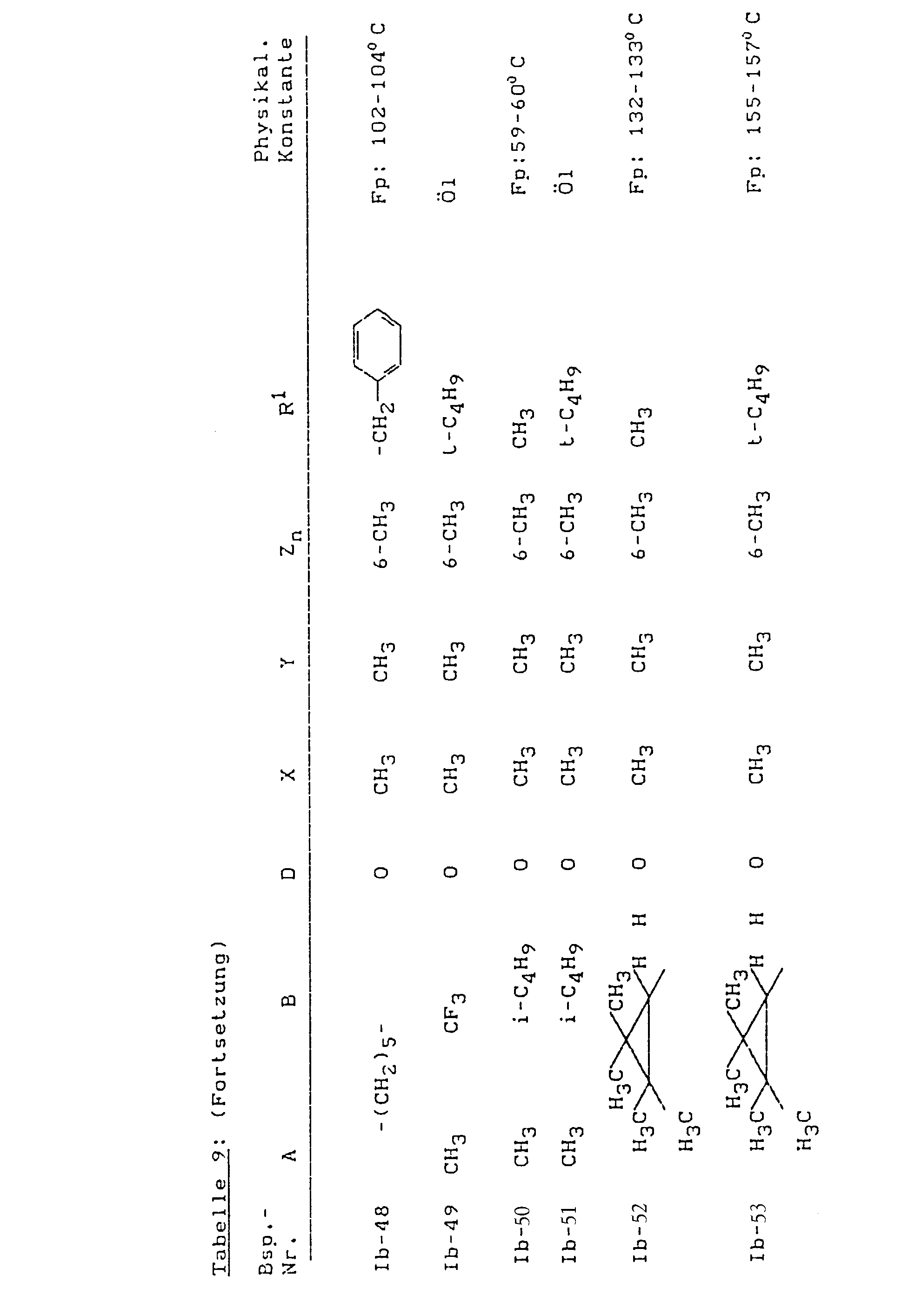

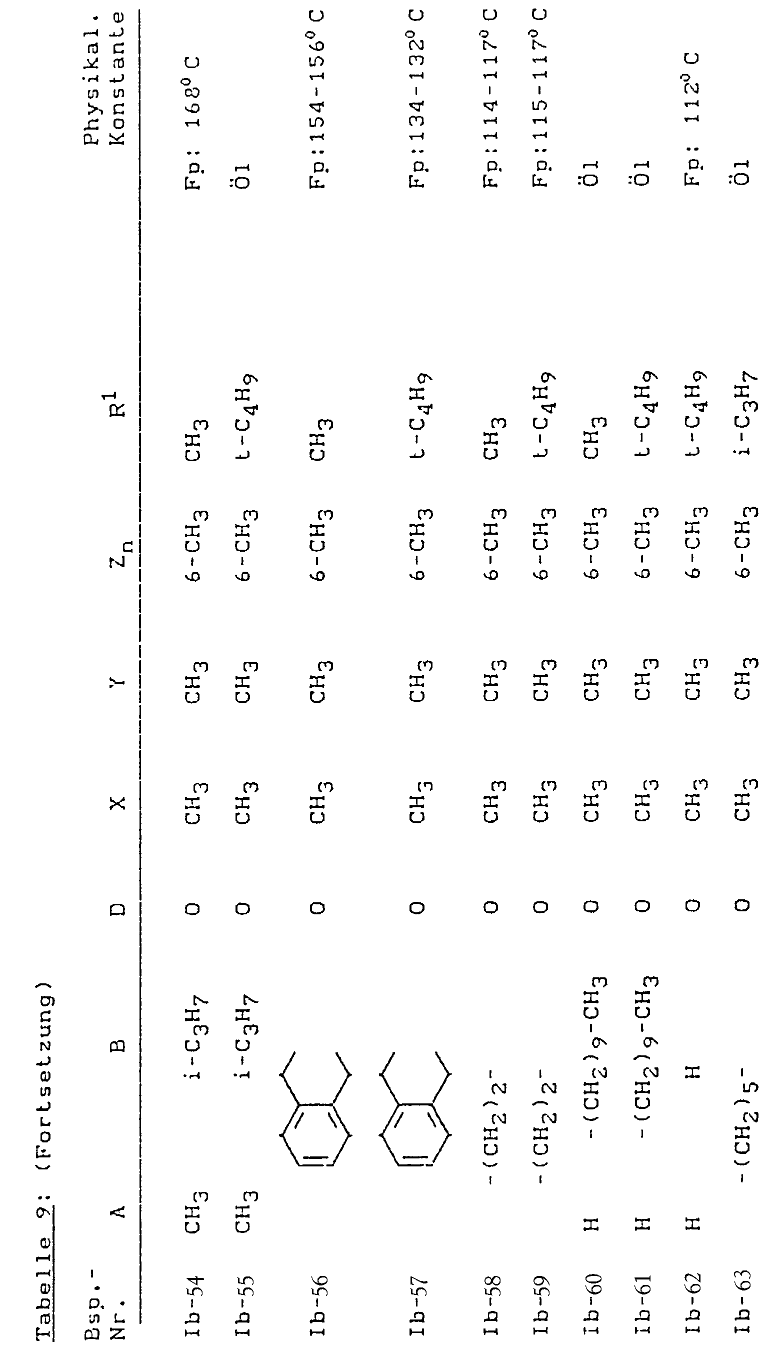

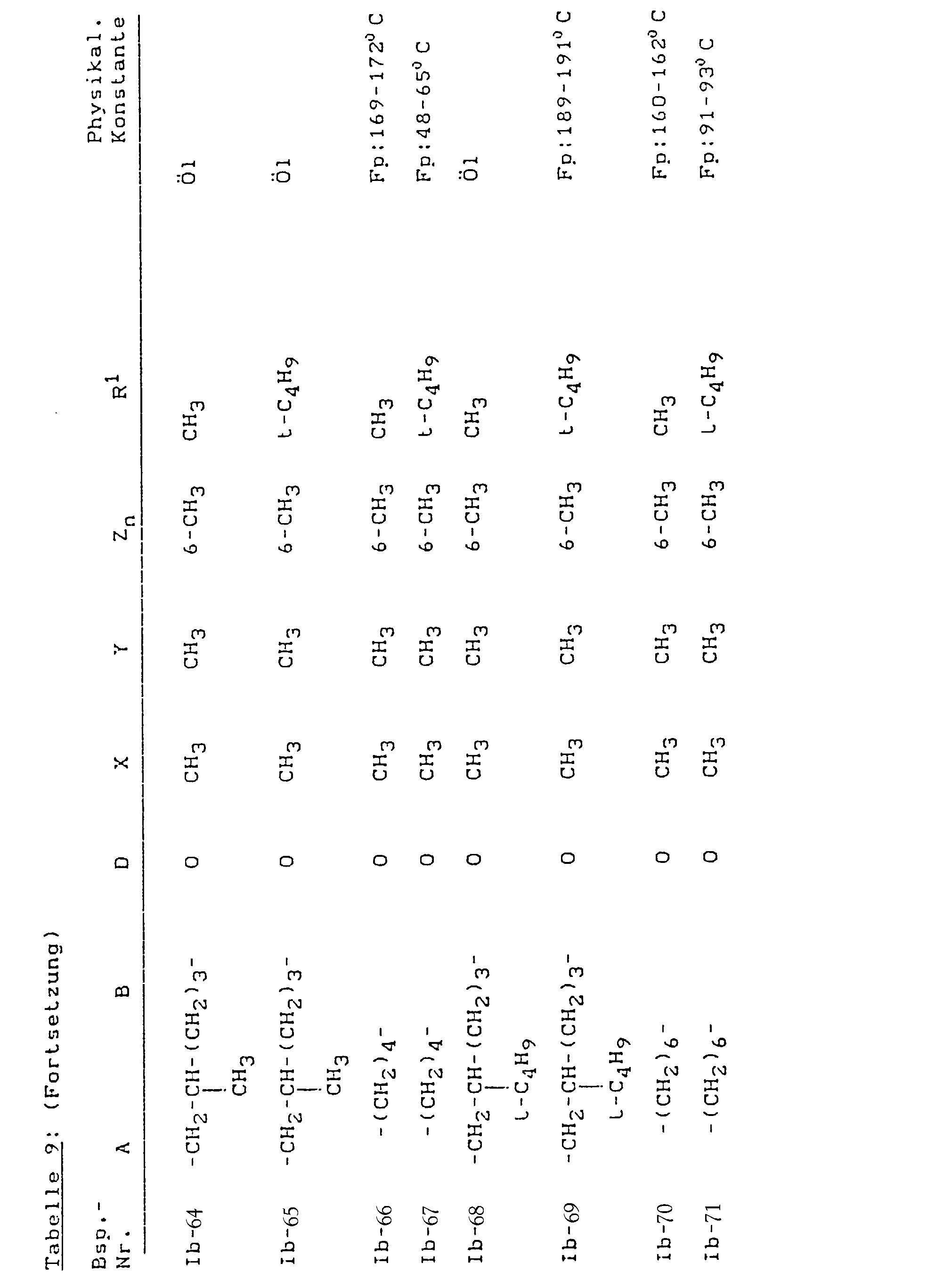

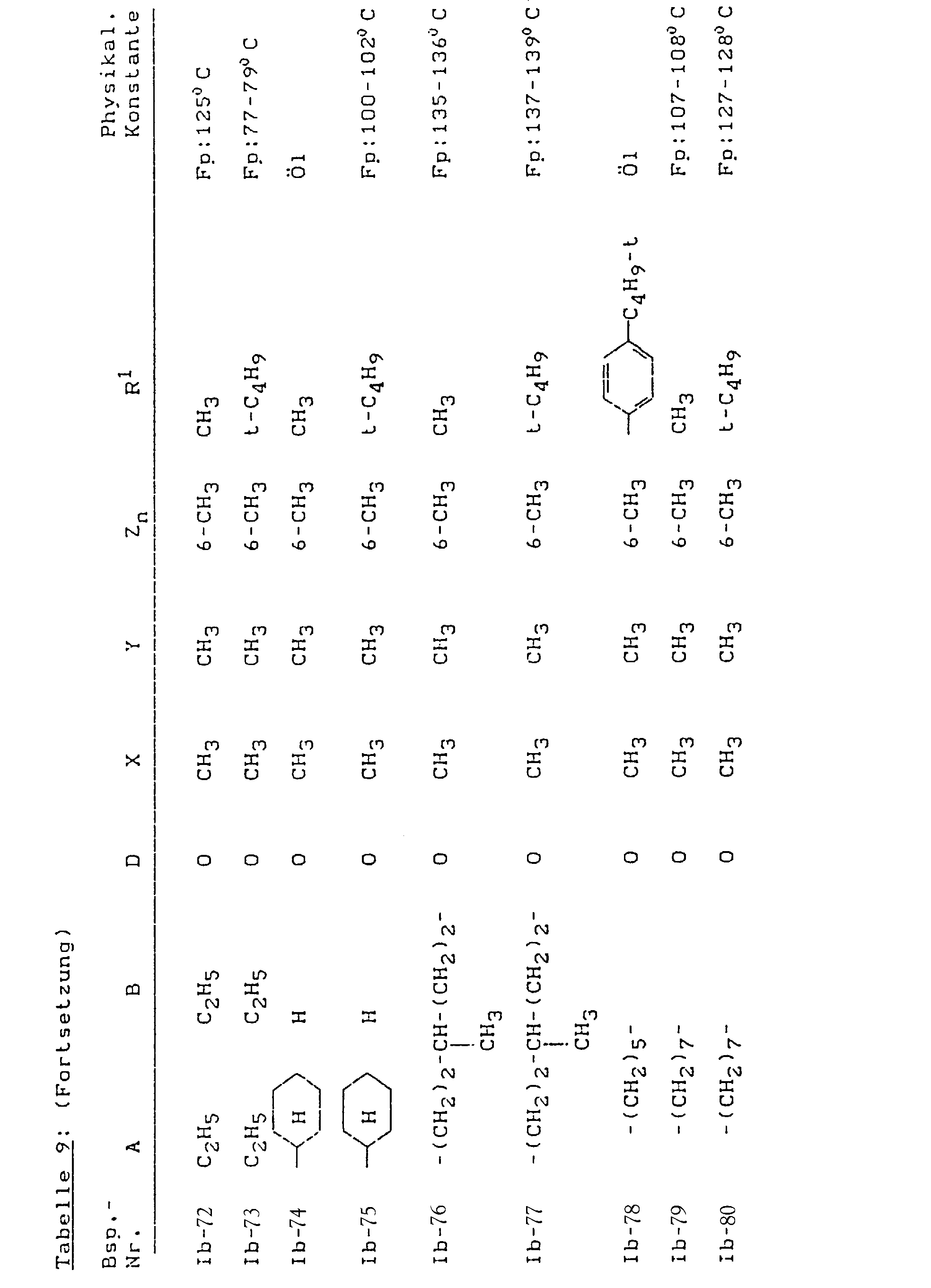

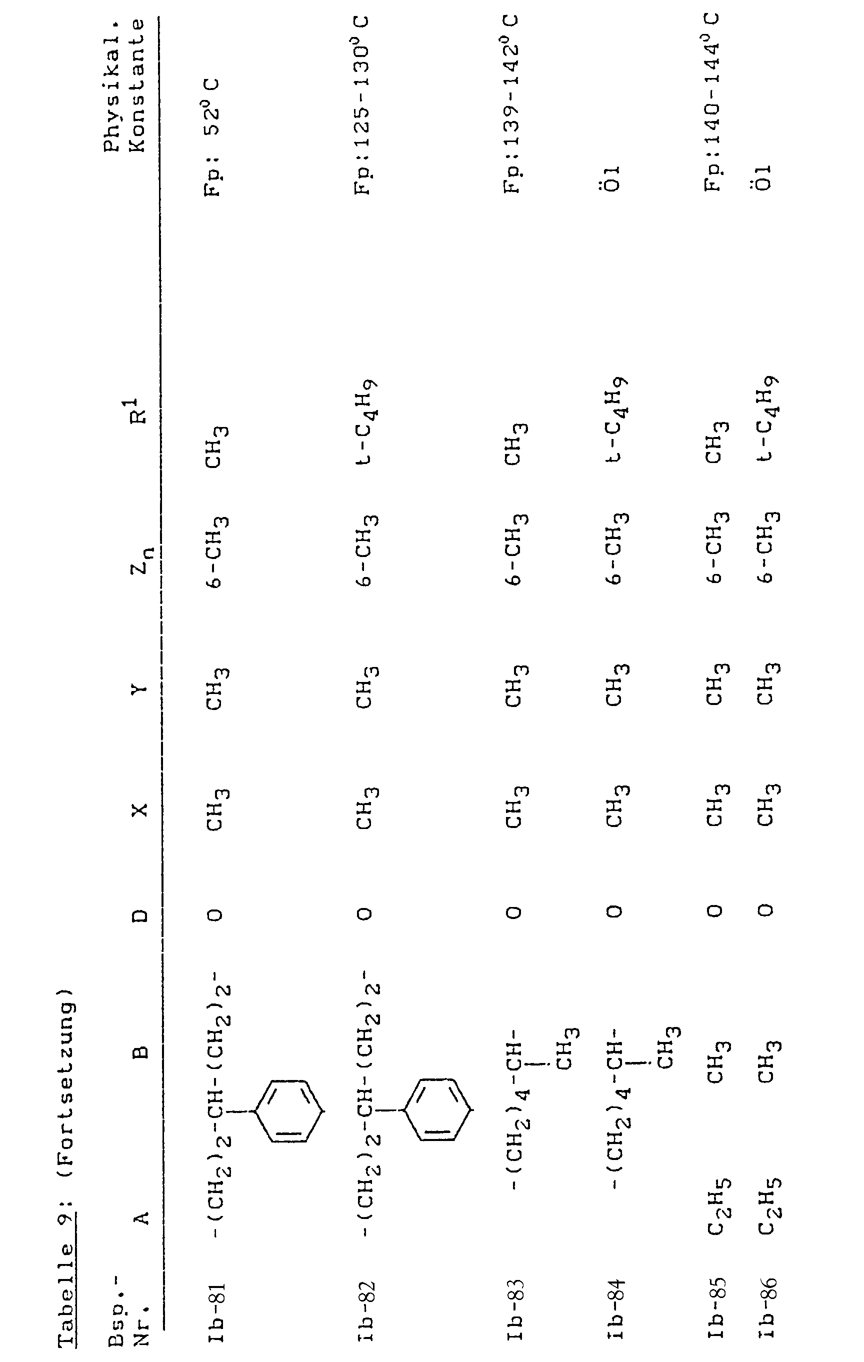

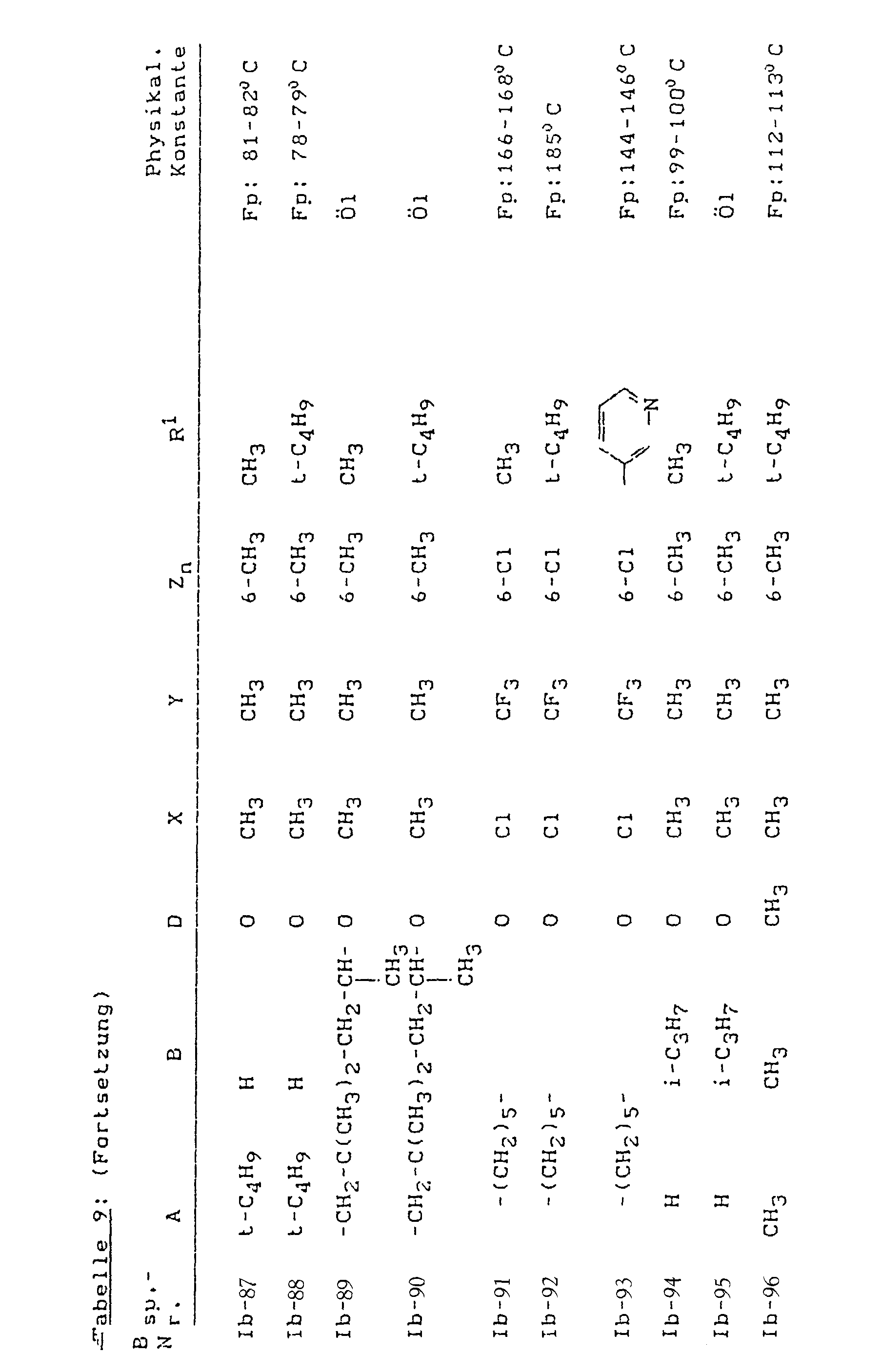

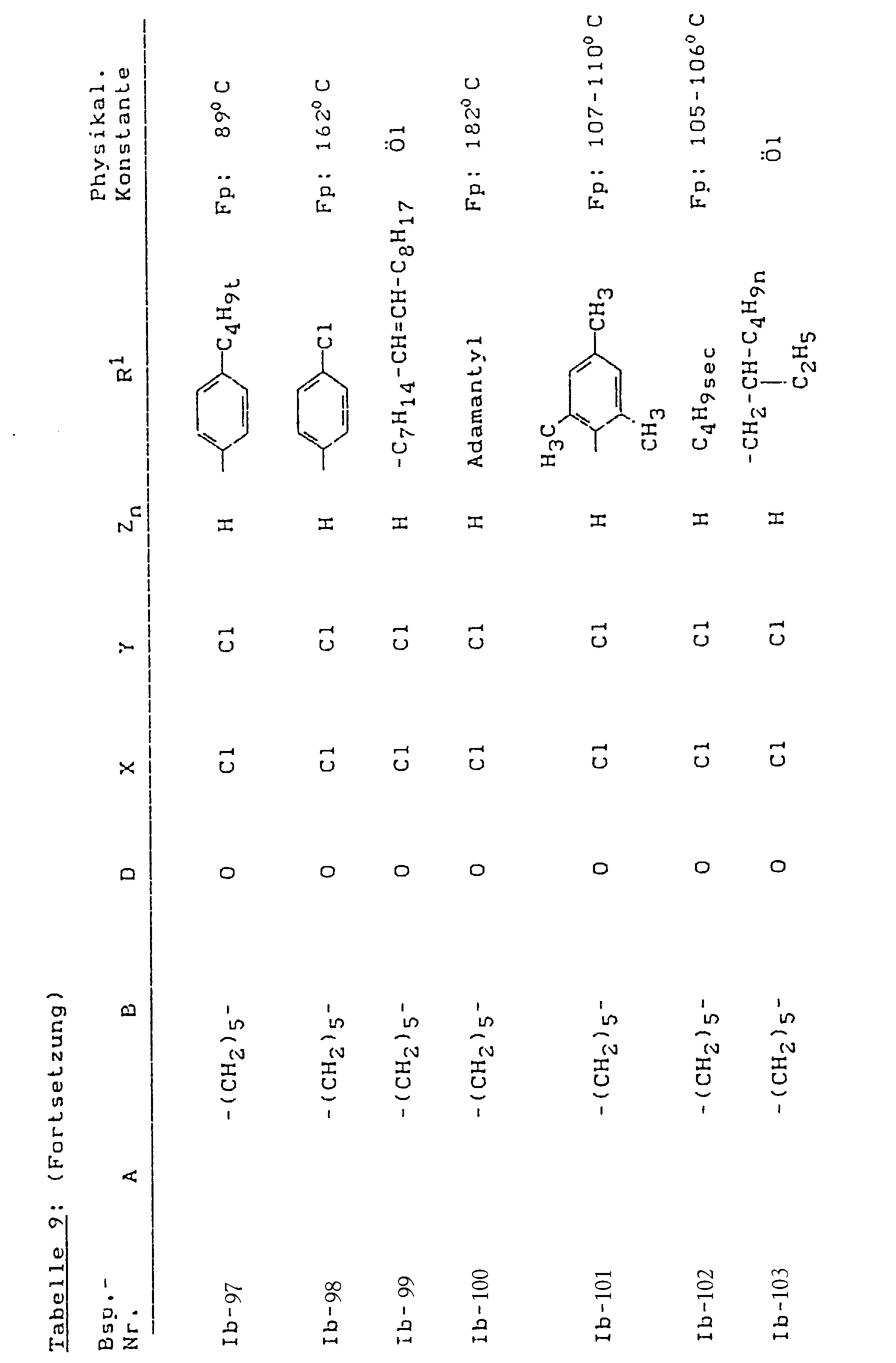

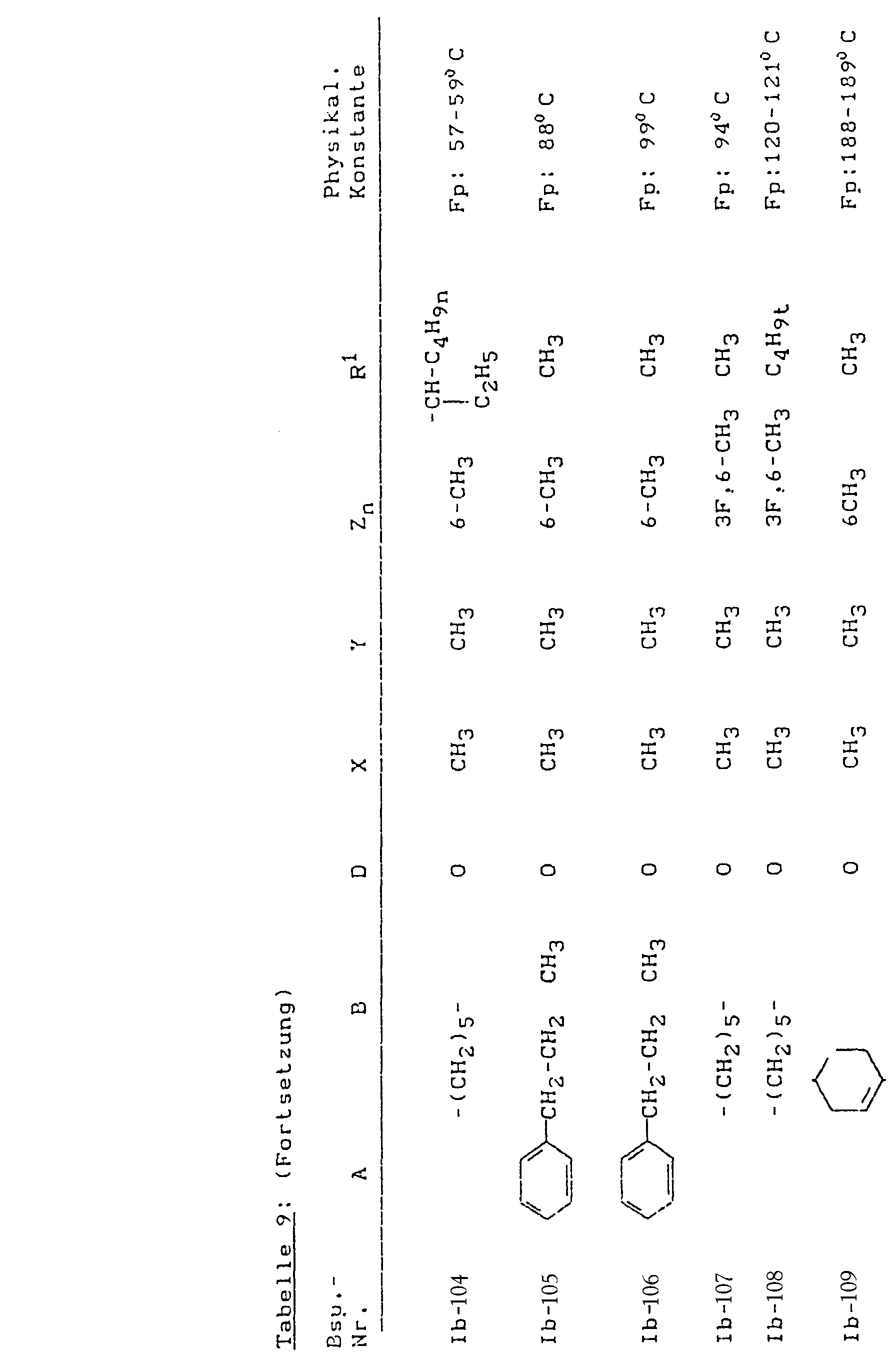

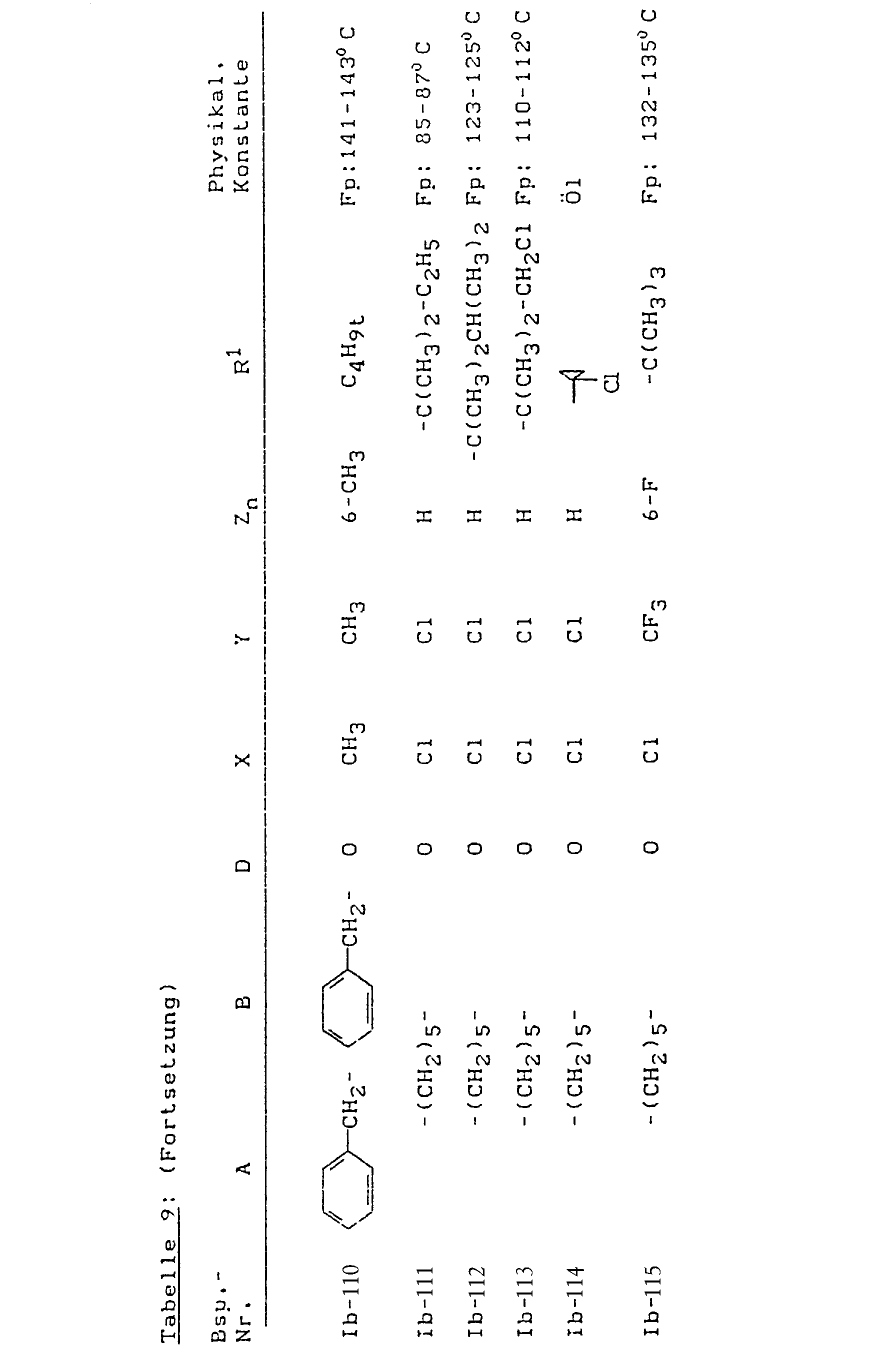

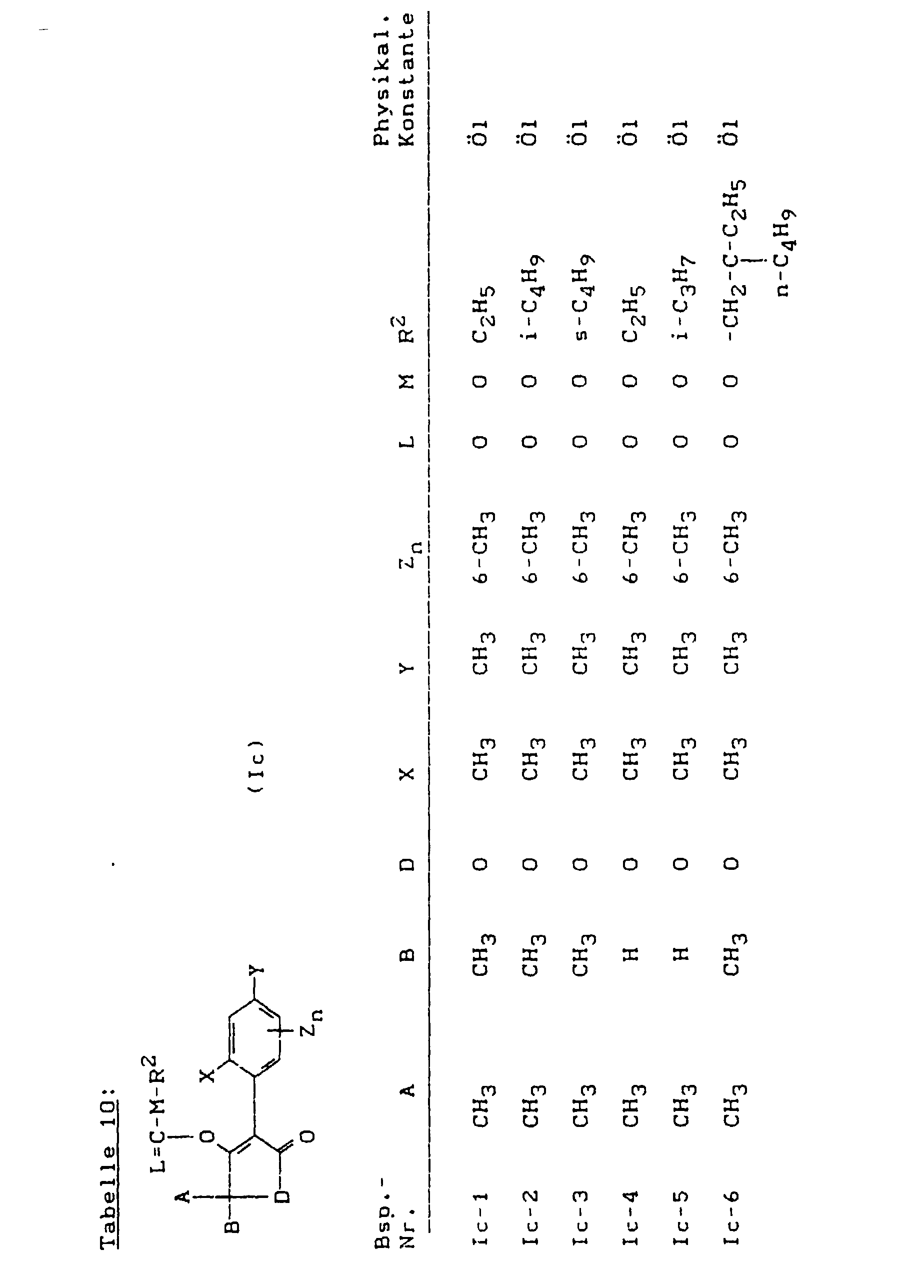

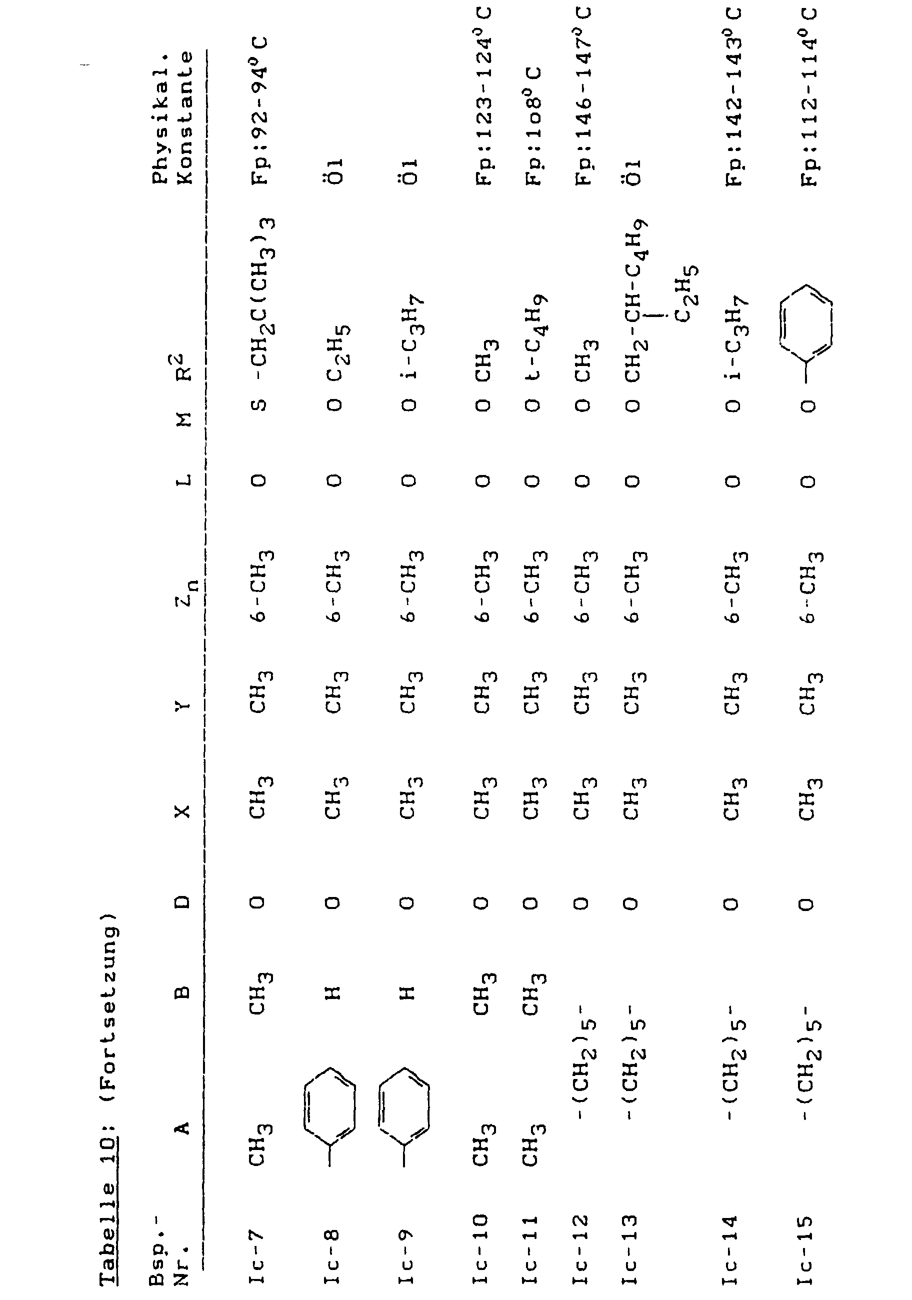

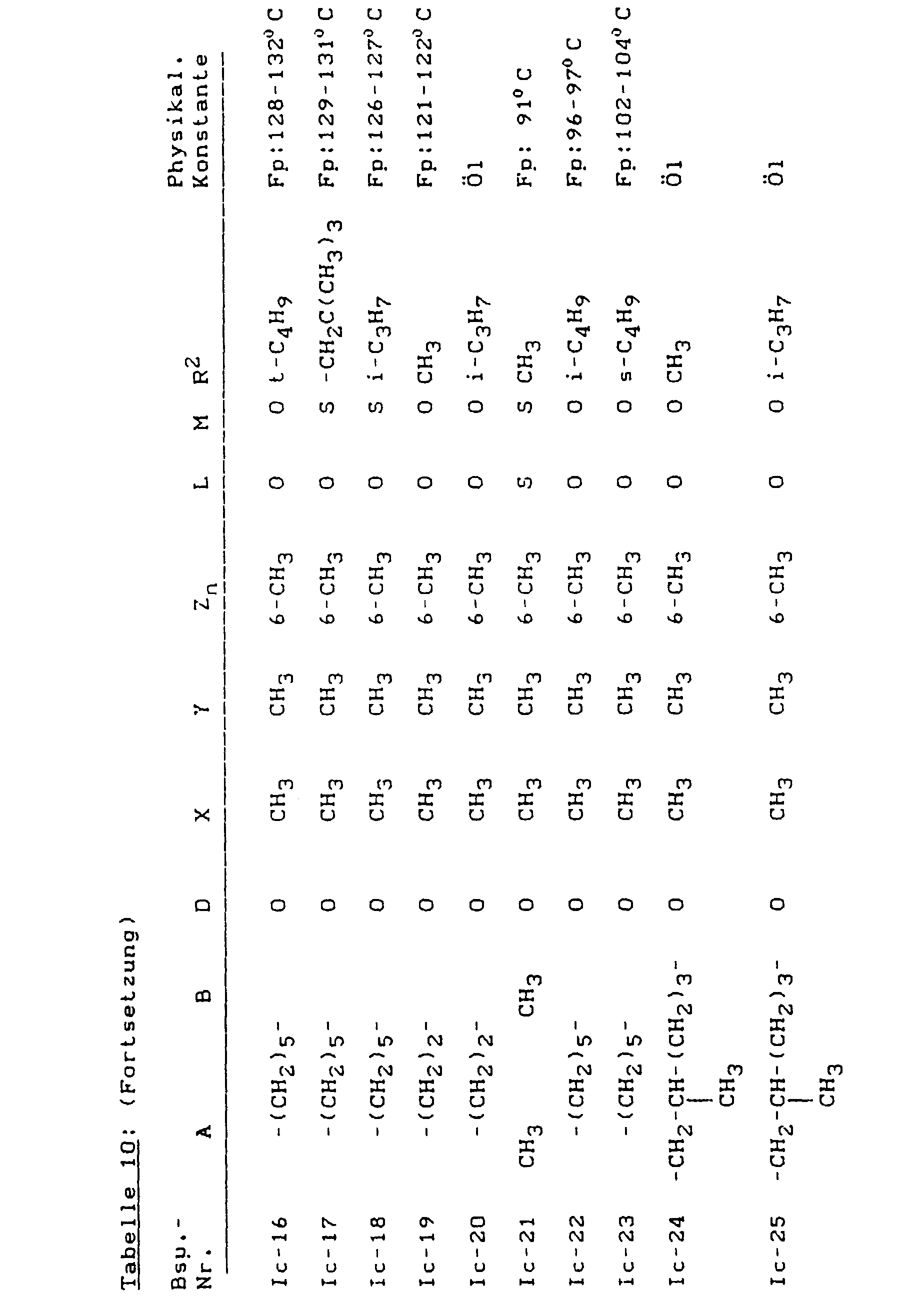

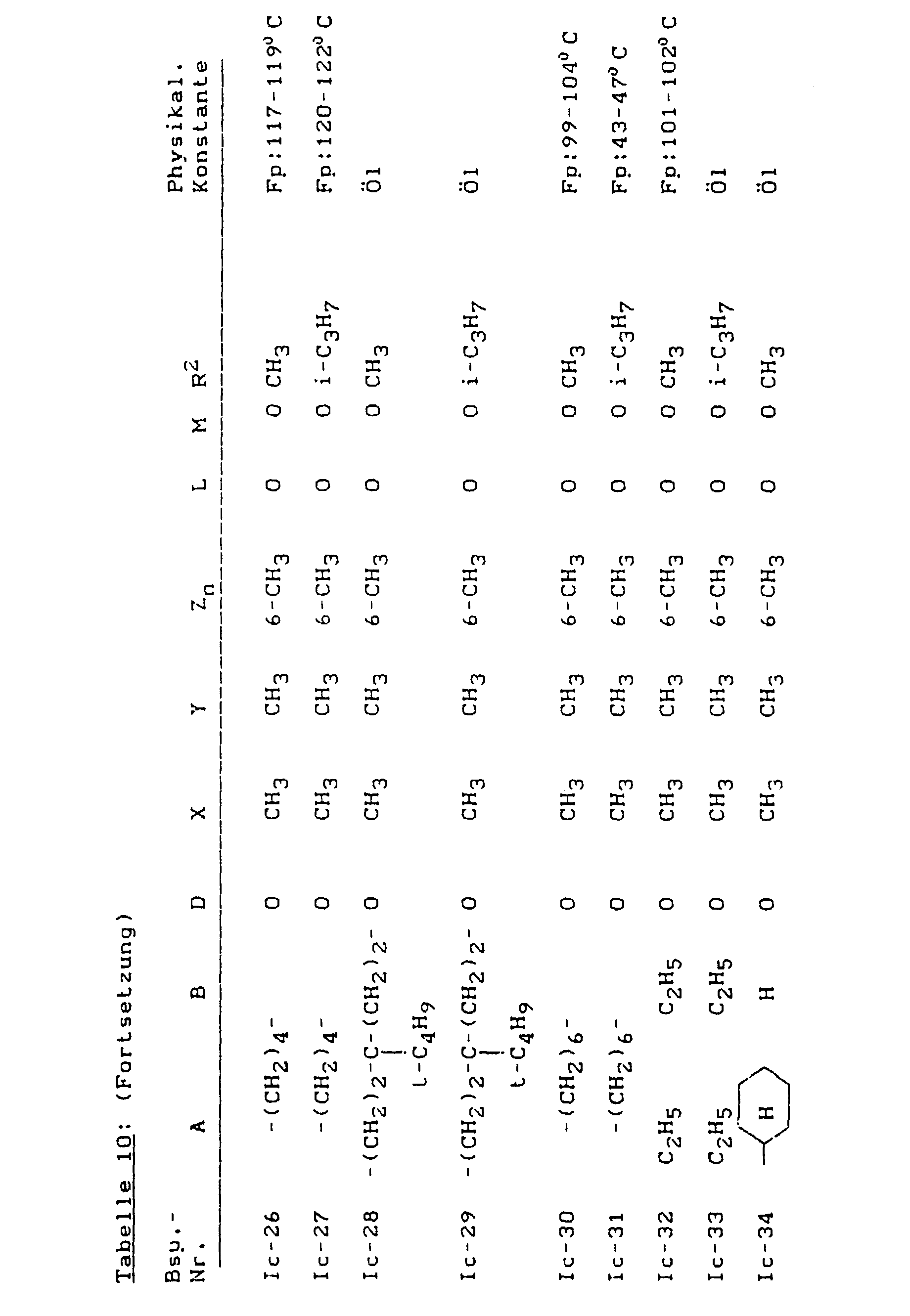

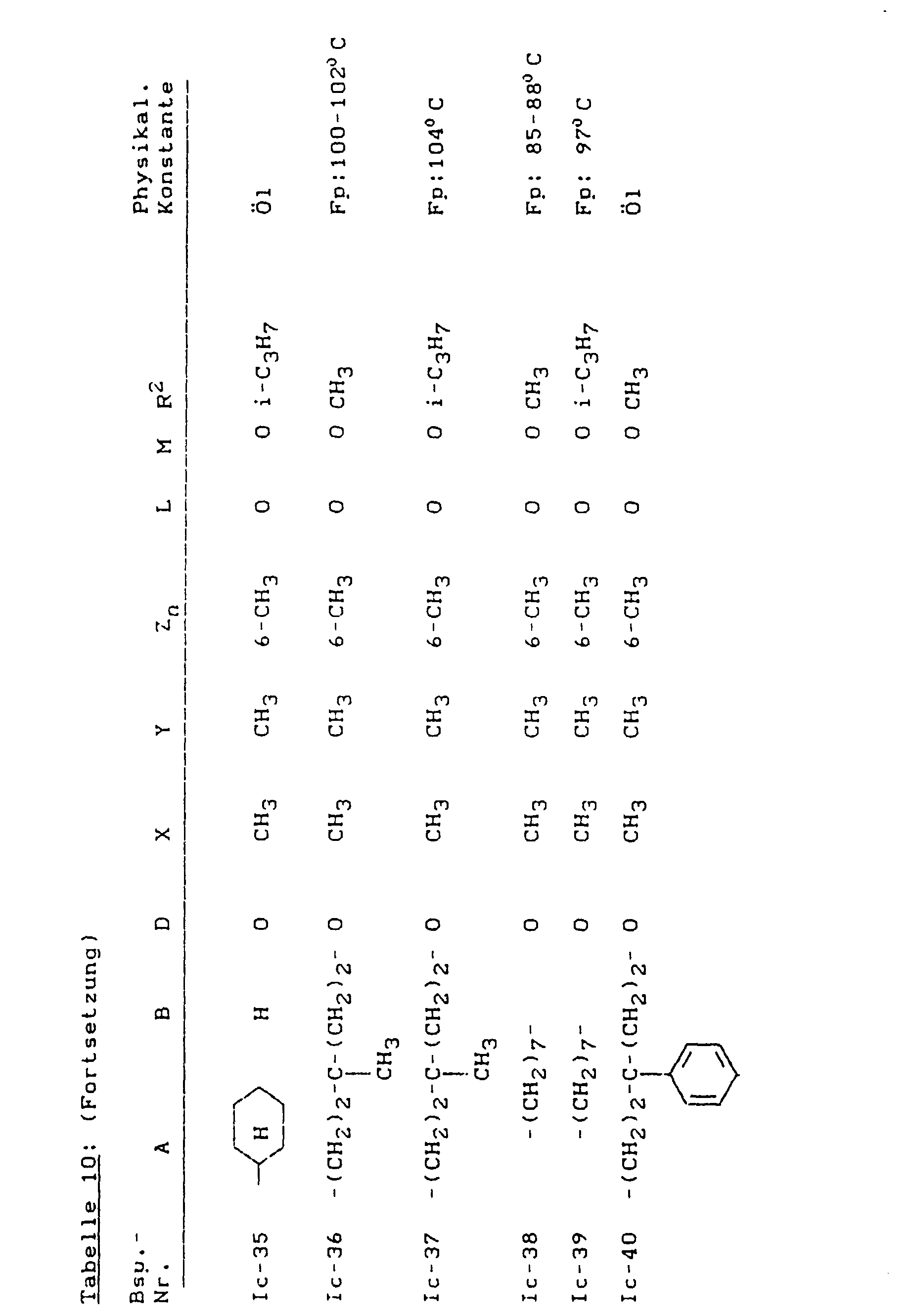

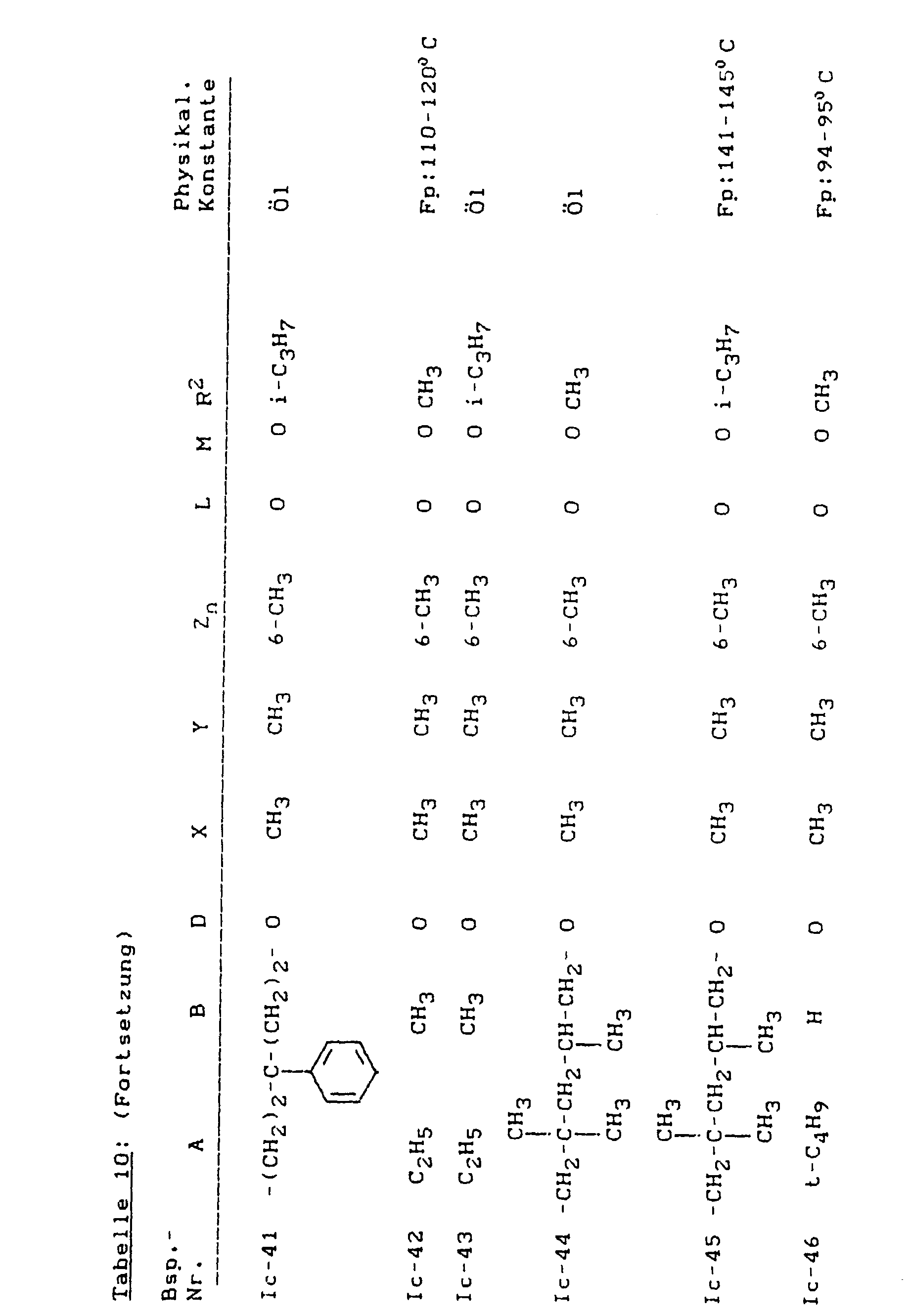

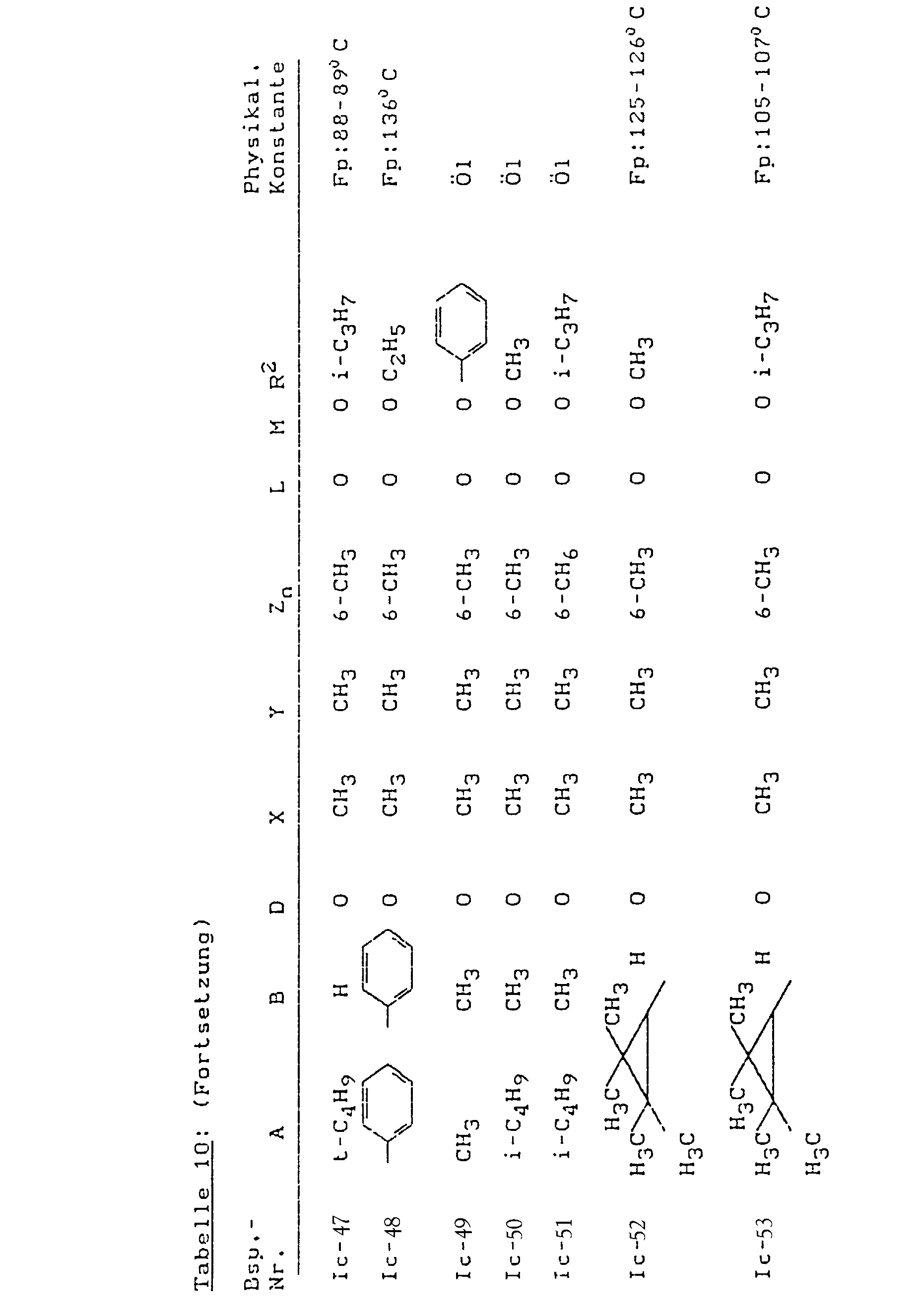

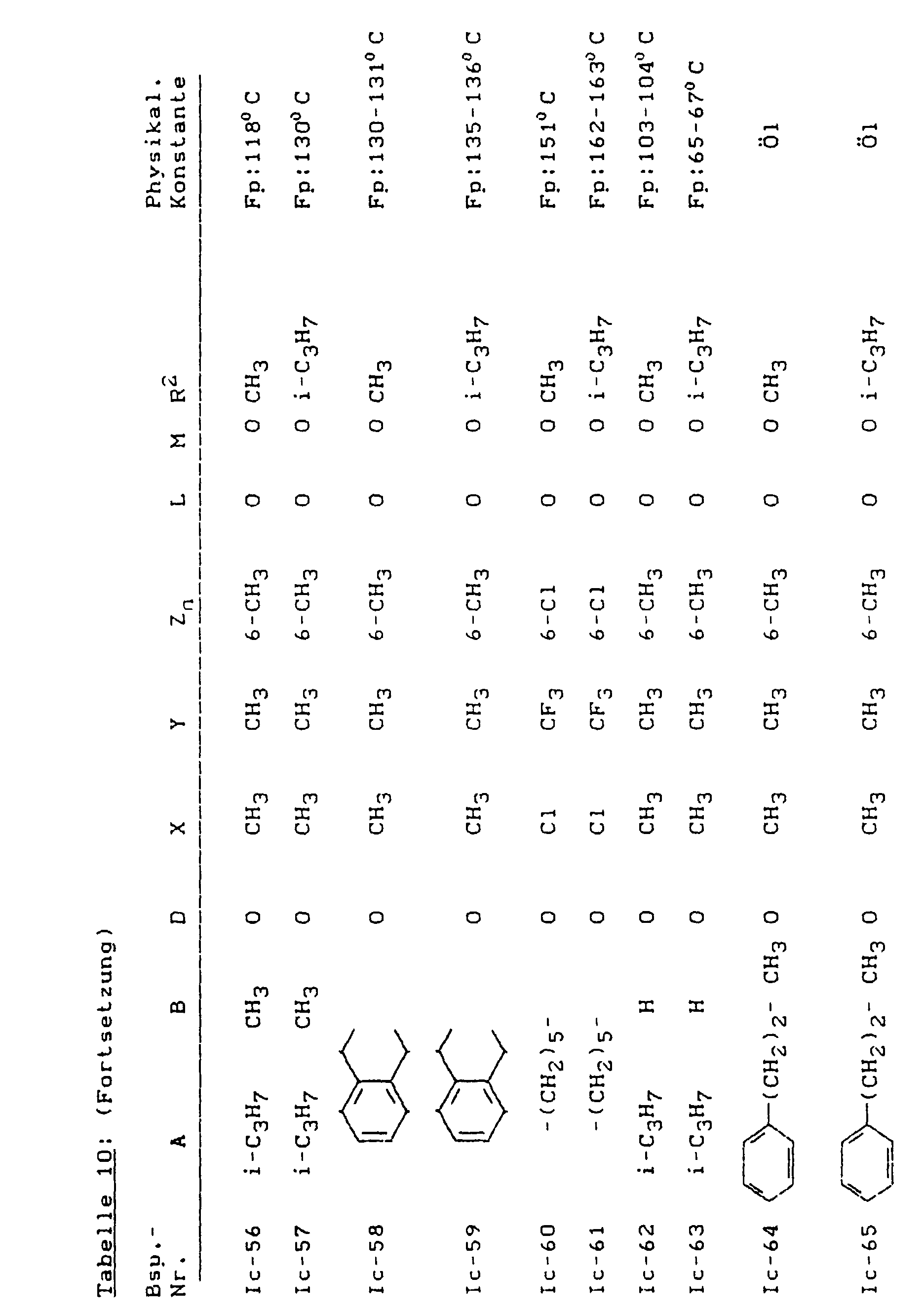

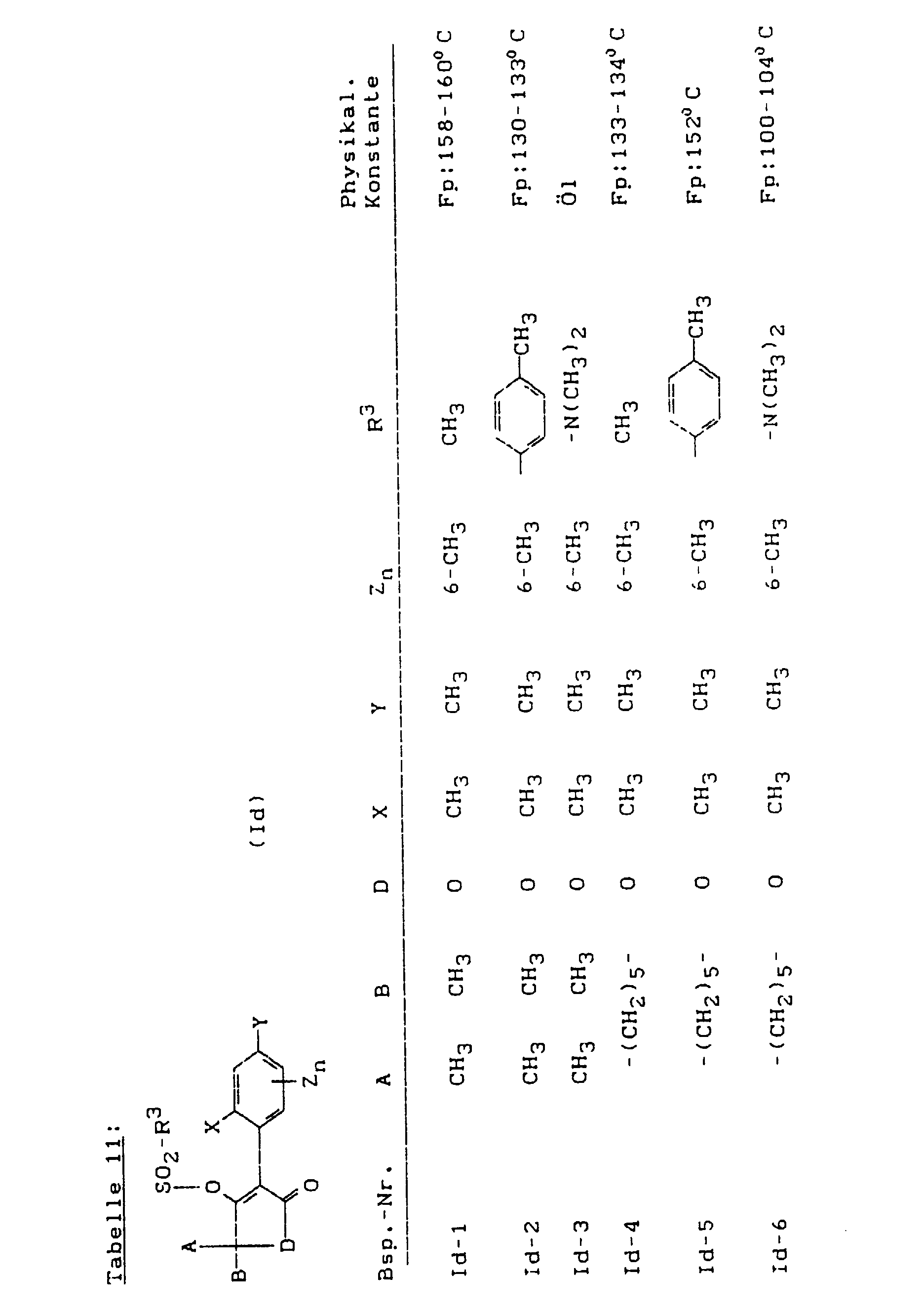

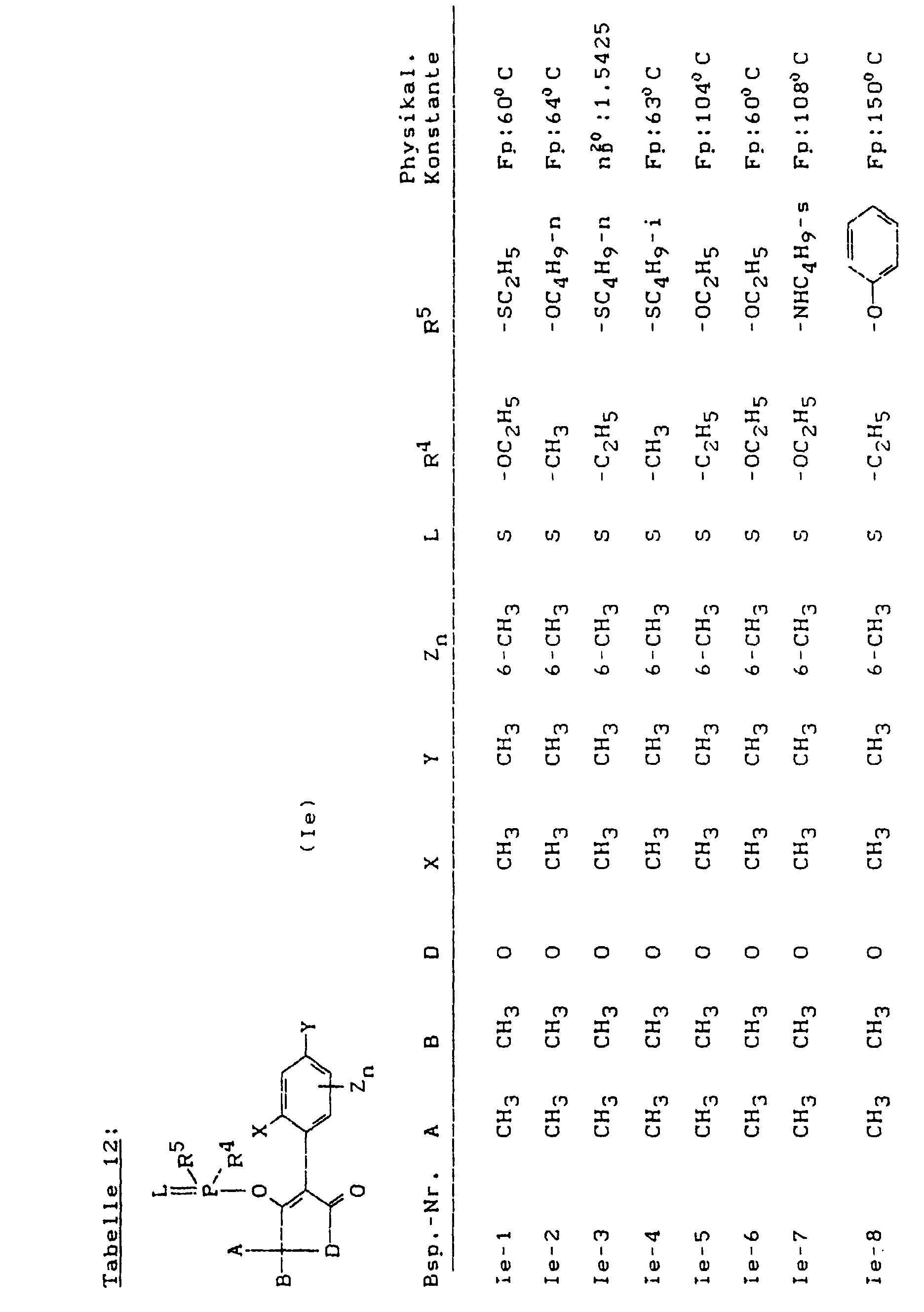

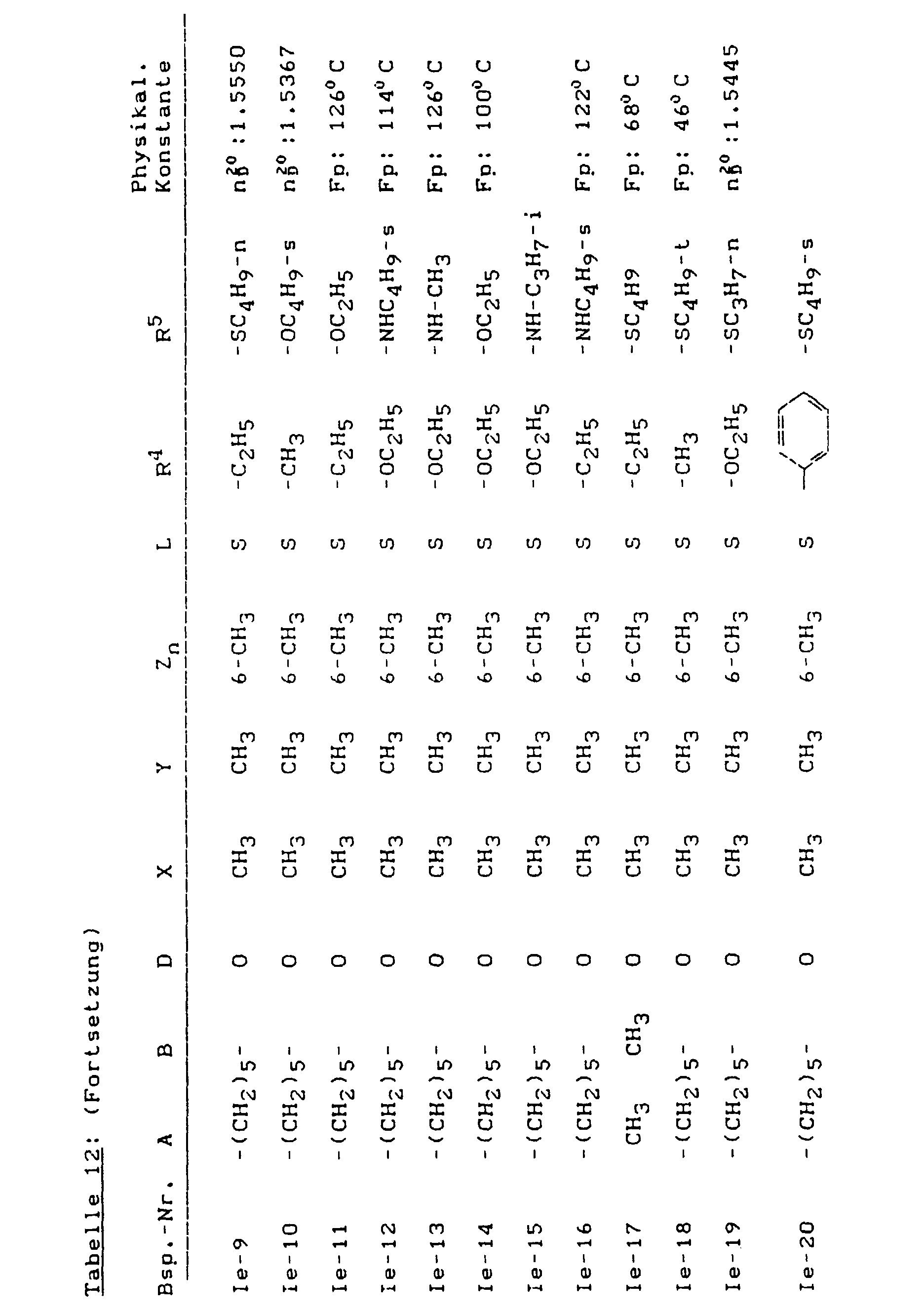

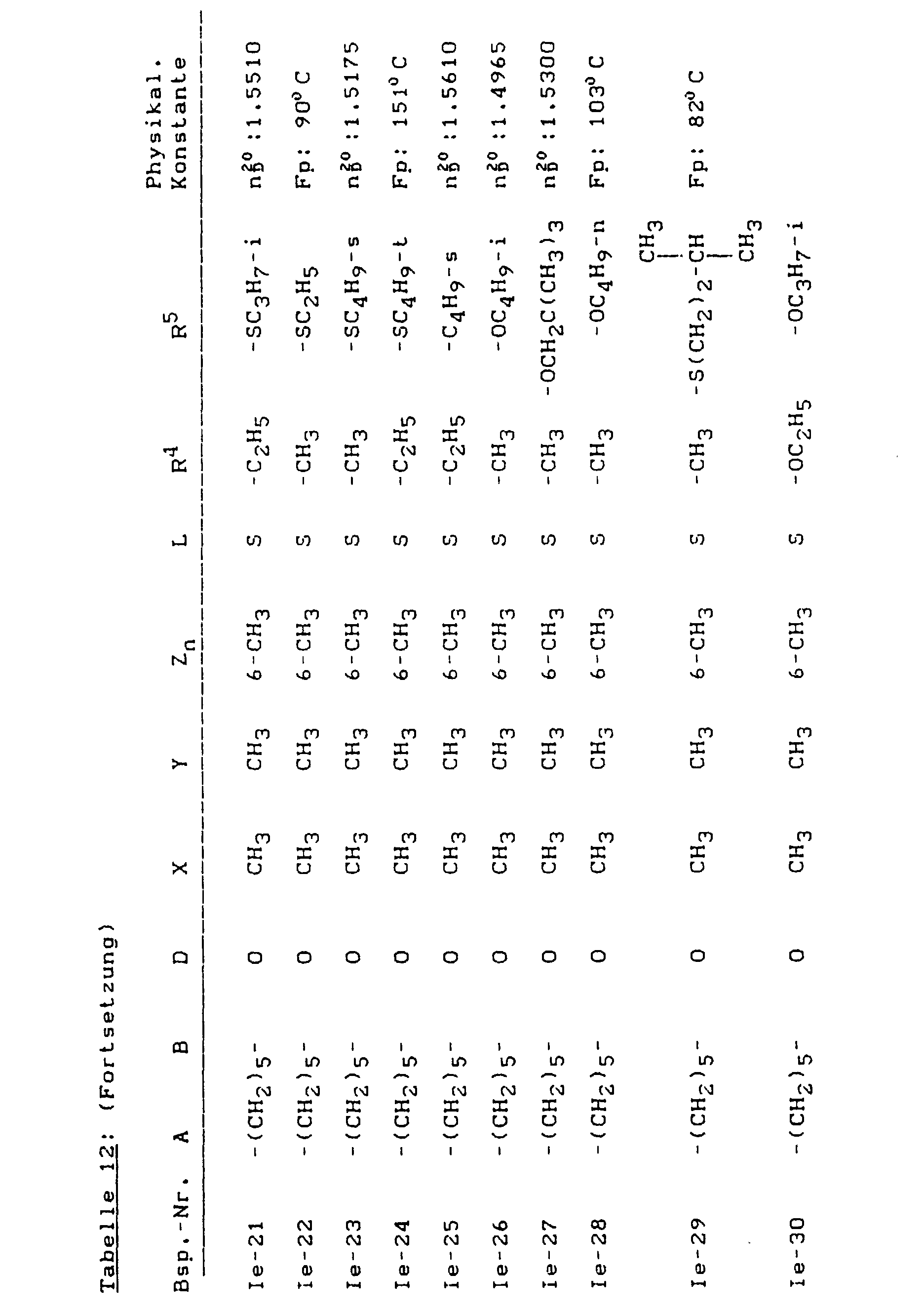

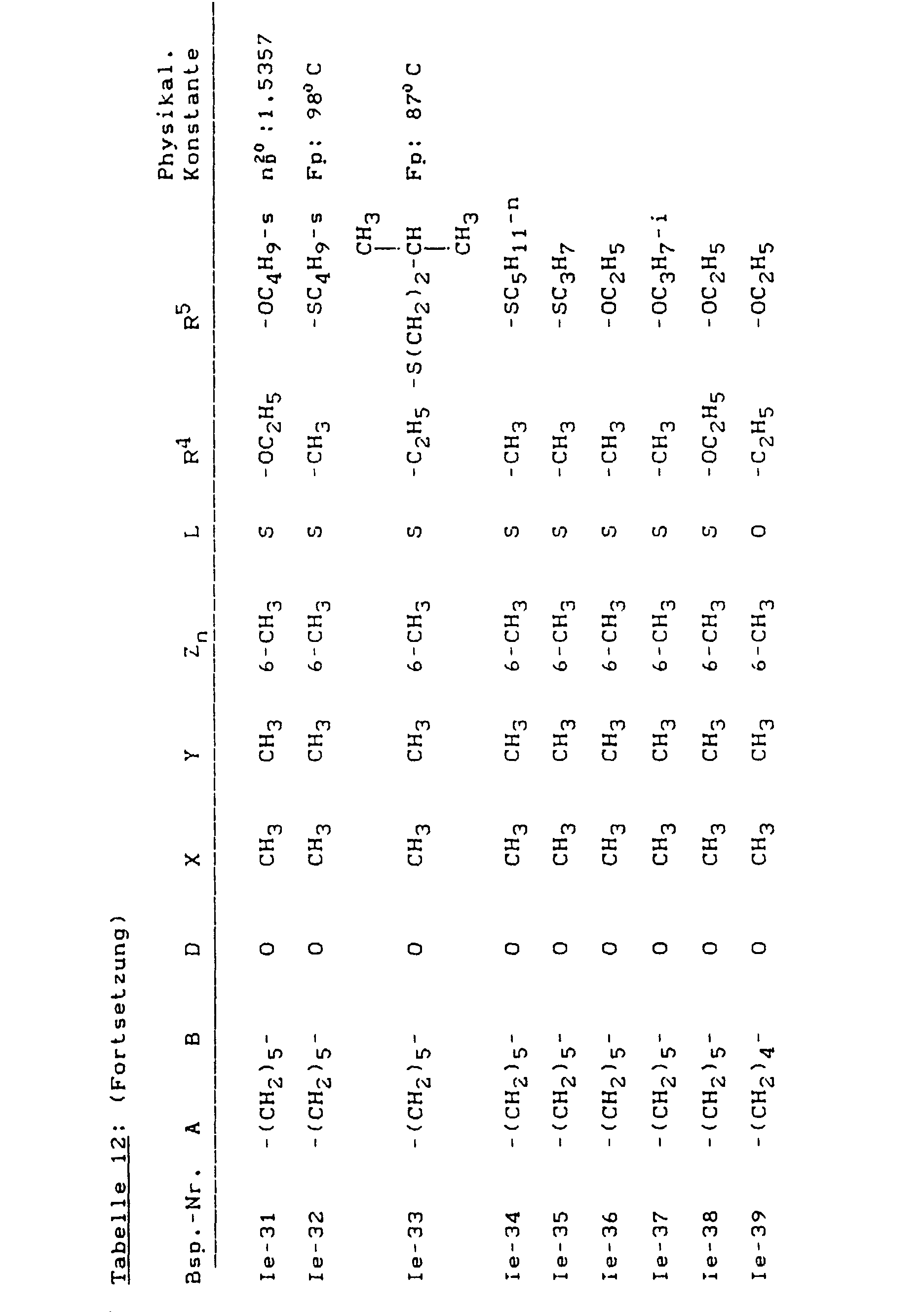

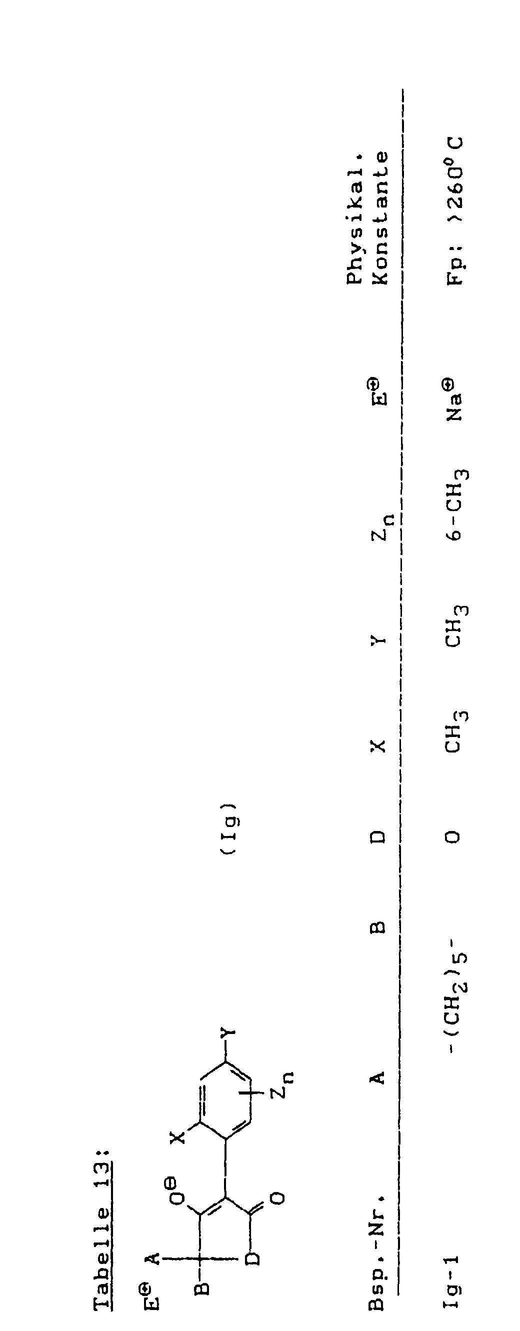

### Herstellung von Ausgangsverbindungen:

### Beispiel 1A

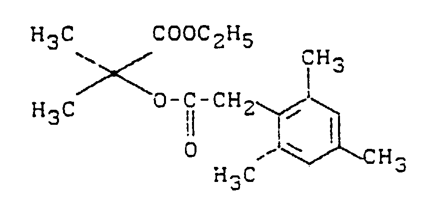

13,2 g (0,1 Mol) 2-Hydroxyisobuttersäureethylester werden in 200 ml abs. Methylenchlorid vorgelegt, 12,14 g (0,12 Mol) Triethylamin zugegeben und bei 0-10°C eine Lösung von 19,7 g (0,1 Mol) 2,4,6-Trimethylphenylessigsäurechlorid in 50 ml abs. Methylenchlorid zugetropft.

Nach 16 h Rühren bei Raumtemperatur wird die Lösung mit wäßriger Zitronensäure und wäßriger Natriumhydrogencarbonatlösung gewaschen, die organische Phase über Natriumsulfat getrocknet und einrotiert.

Ausbeute: 26,62 g (91 % d. Theorie) der Verbindung oben angegebener Formel. Die Verbindung fällt als Öl an.

### Beispiel 2A

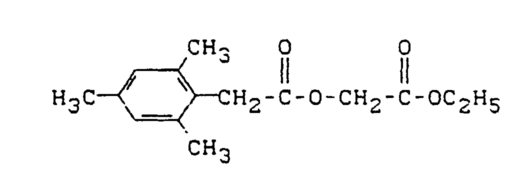

35,6 g (0,2 Mol) 2,4,6-Trimethylphenylessigsäure werden in 200 ml tert.- Butanol gelöst. Dazu werden 24,6 g (0,22 Mol) Kalium-tert.-butylat gegeben. Man läßt 15 Minuten rühren. Anschließend läßt man 34,9 g (0,2 Mol) Bromessigsäureeethylester zutropfen.

Nach dem Einrotieren wird mit Wasser/Methylenchlorid aufgenommen, extrahiert, über Natriumsulfat getrocknet und einrotiert.
Ausbeute: 38,8 g (74 % d.Theorie) der Verbindung O-(2,4,6-Trimethylphenylacetyl)-hydroxy-essigsäuremethylester vom Schmelzpunkt 154°C (umkristallisiert aus Methylenchlorid/n-Hexan-Gemisch).

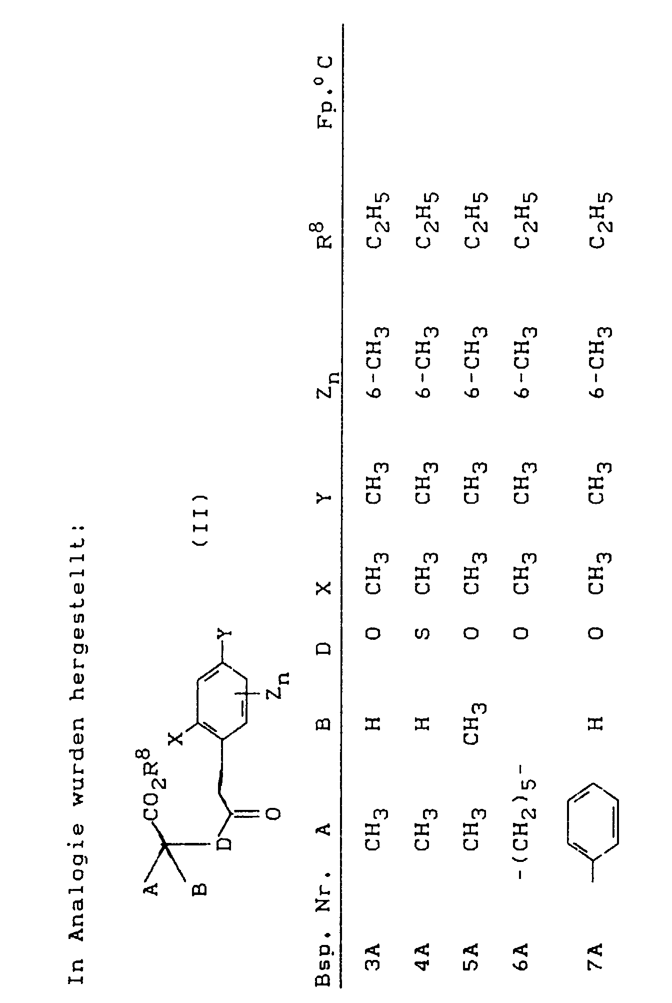

Die erfindungsgemäß verwendbaren Wirkstoffe eignen sich bei guter Pflanzenverträglichkeit und günstiger Warmblütertoxizität zur Bekämpfung von tierischen Schädlingen, insbesondere Insekten und Spinnentieren die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.

Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die erfindungsgemäßen verwendbaren Wirkstoffe wirken nicht nur gegen Pflanzen-, Hygiene- und Vorratsschädlinge, sondern auch auf dem veterinärmedizinischen Sektor gegen tierische Parasiten (Ektoparasiten und Endoparasiten) wie Schildzecken, Lederzecken, Räubemilben, Laufmilben, Fliegen (stechend und leckend), parasitierende Fliegenlarven; Läuse, Haarlinge, Federlinge, Flöhe und endoparasitisch lebende Würmer.

Die erfindungsgemäßen neuen Wirkstoffe können weiterhin als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.
Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser: mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid: als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden Herbiziden oder Fungiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Als Vergleichsverbindung aus dem Stand der Technik wurden bei den nachfolgenden biologischen Beispielen die Verbindung der Formel 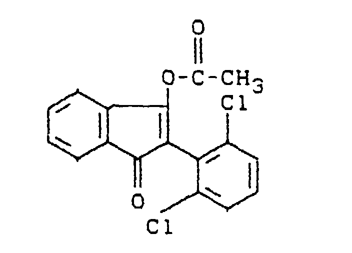 (bekannt aus US 3 954 998)
eingesetzt.

### Beispiel A

### Phaedon-Larven-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Merettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käfer-Larven abgetötet wurden: O % bedeutet, daß keine Käfer-Larven abgetötet wurden.

Bei diesem Test zeigen z.B. die folgenden Verbindungen der Herstellungsbeispiele überlegene Wirksamkeit gegenüber dem Stand der Technik: Ia-2, Ib-1, Ib-2.

### Beispiel B

### Nephotettix-Test

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryza sativa) werden durch Tauchen in die Wirkstoffzubereitung der gewünschten Konzentration behandelt und mit Larven der Grünen Reiszikade (Nephotettix cincticeps) besetzt, solange die Keimlinge noch feucht sind.

Nach der gewünschten Zeit wird die Abtötung in % bestimmt. Dabei bedeute 100 %, daß alle Zikaden abgetötet wurden: 0 % bedeutet, daß keine Zikaden abgetötet wurden.

Bei diesem Test zeigen z.B. die folgenden Verbindungen der Herstellungsbeispiele überlegene Wirksamkeit gegenüber dem Stand der Technik: Ia-2, Ib-1, Ib-2.

### Beispiel C

### Tetranychus-Test (OP-resistent)

- Lösungsmittel:: 7 Gewichtsteile Dimethylformamid
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit emulgatorhaltigem Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden mit einer Wirkstoffzubereitung der gewünschten Konzentration tropfnaß gespritzt.

Nach der gewünschten Zeit wird die Wirkung in % bestimmt. Dabei bedeutet 100 %, daß alle Spinnmilben abgetötet wurden: 0 % bedeutet, daß keine Spinnmilben abgetötet wurden.

Bei diesem Test zeigen die folgenden Verbindungen der Herstellungsbeispiele überlegene Wirksamkeit gegenüber dem Stand der Technik: Ia-2, Ib-1, Ib-2.

### Beispiel D

### Pre-emergence-Test / Gewächshaus

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

Bei diesem Test zeigt die folgende Verbindung der Herstellungsbeispiele überlegene Wirksamkeit gegenüber dem Stand der Technik: Ib-7.

Als Stand der Technik diente hier Fluortamone ((+)-5-(Methylamino)-2-phenyl-4-[3-(trifluormethyl)-phenyl]-3-(2H)-furanon, beschrieben z.B. in WO-A-86/02643.

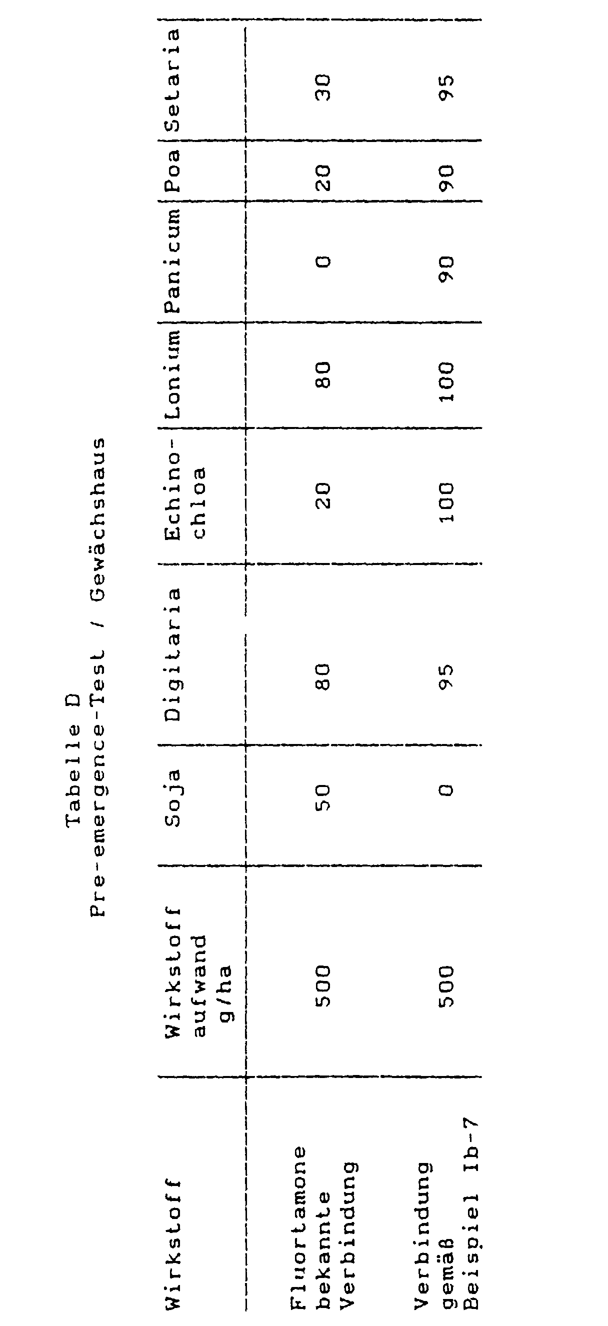

## Patentansprüche

1. Insektizide und akarizide Mittel, gekennzeichnet durch einen Gehalt an mindestens einem 3-Aryl-4-hydroxy-Δ³-dihydrofuranon- oder 3-Aryl-4-hydroxy-Δ³-dihydrothiophenon-Derivat der allgemeinen Formel (I) 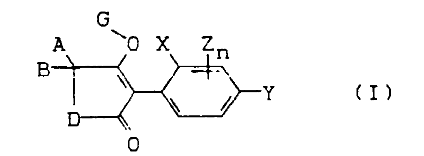 in welcher
X für C₁-C₆-Alkyl, Halogen, C₁-C₆-Alkoxy oder C₁-C₃-Halogenalkyl steht,
Y für Wasserstoff, C₁-C₆-Alkyl, Halogen, C₁-C₆-Alkoxy, C₁-C₃-Halogenalkyl steht,
Z für C₁-C₆-Alkyl, Halogen, C₁-C₆-Alkoxy steht,
n für eine Zahl von 0-3 steht,
oder wobei die Reste X und Z gemeinsam mit dem Phenylrest, an den sie gebunden sind, den Naphthalinrest der Formel 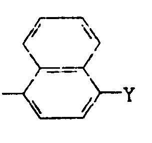 bilden,
in welchem Y die oben angegebene Bedeutung hat,
A und B gleich oder verschieden sind und für Wasserstoff oder gegebenenfalls durch Halogen substituiertes geradkettiges oder verzweigtes C₁-C₁₂-Alkyl, C₃-C₈-Alkenyl, C₃-C₈-Alkinyl, C₁-C₁₀-Alkoxy-C₂-C₈-alkyl, C₁-C₈-PolyalkoxyC₂-C₈-alkyl, C₁-C₁₀-Alkylthio-C₂-C₈-alkyl, Cycloalkyl mit 3-8 Ringatomen, das durch Sauerstoff und/oder Schwefel unterbrochen sein kann und gegebenenfalls durch Halogen, C₁-C₆-Alkyl, C₁-C₆-Halogenalkyl-, C₁-C₆-Alkoxy-, C₁-C₆-Halogenalkoxy, Nitro substituiertes Phenyl oder Phenyl-C1-C₆-alkyl steht,
oder worin
A und B gemeinsam mit dem Kohlenstoffatom, an das sie gebunden sind einen gesättigten oder ungesättigten, gegebenenfalls durch Sauerstoff und/oder Schwefel unterbrochenen und gegebenenfalls durch Halogen, C₁-C₆-Alkyl, C₁-C₆-Alkoxy, C₁-C₄-Halogenalkyl, C₁-C₄-Halogenalkoxy, C₁-C₄-Alkylthio oder gegebenenfalls substituiertes Phenyl substituierten oder gegebenenfalls benzokondensierten 3- bis 8-gliedrigen Ring bilden,
D für Sauerstoff oder Schwefel steht,
G für Wasserstoff (a) oder für die Gruppen 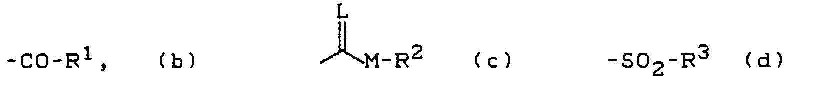 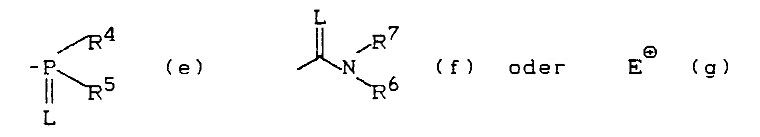 steht,
in welchen
E^{⊕} für ein Metallionäquivalent oder ein Ammoniumion steht,
L und M für Sauerstoff und/oder Schwefel steht,
R¹ für gegebenenfalls durch Halogen substituiertes: C₁-C₂₀-Alkyl, C₂-C₂₀-Alkenyl, C₂-C₈-Alkoxy-C₂-C₈-alkyl, C₁-C₈-Alkylthio-C₂-C₈-alkyl, C₁-C₈-Polyalkoxyl-C₂-C₈-alkyl oder Cycloalkyl mit 3-8 Ringatomen, das durch Sauerstoffund/oder Schwefelatome unterbrochen sein kann, steht,
für gegebenenfalls durch Halogen, Nitro, C₁-C₆-Alkyl, C₁-C₆-Alkoxy, C₁-C₆-Halogenalkyl, C₁-C₆-Halogenalkoxysubstituiertes Phenyl steht;
für gegebenenfalls durch Halogen-, C₁-C₆-Alkyl, C₁-C₆-Alkoxy-, C₁-C₆-Halogenalkyl-, C₁-C₆-Halogenalkoxy-substituiertes Phenyl-C₁-C₆-alkyl steht,
für gegebenenfalls durch Halogen und/oder C₁-C₆-Alkyl substituiertes Pyridyl, Pyrimidyl, Thiazolyl und Pyrazolyl steht,
für gegebenenfalls durch Halogen und/oder C₁-C₆-Alkyl-substituiertes Phenoxy-C₁-C₆-alkyl steht,
R² für gegebenenfalls durch Halogen substituiertes: C₁-C₂₀-Alkyl, C₂-C₂₀-Alkenyl, C₁-C₈-Alkoxy-C₂-C₈-alkyl, C₁-C₈-Polyalkoxy-C₂-C₈-alkyl steht,
für gegebenenfalls durch Halogen, Nitro, C₁-C₆-Alkyl, C₁-C₆-Alkoxy, C₁-C₆-Halogenalkylsubstituiertes Phenyl oder Benzyl steht,
R³, R⁴ und R⁵ unabhängig voneinander für gegebenenfalls durch Halogen substituiertes C₁-C₈-Alkyl, C₁-C₈-Alkoxy, C₁-C₈-Alkylamino, Di-(C₁-C₈)-Alkylamino, C₁-C₈-Alkylthio, C₂-C₅-Alkenylthio, C₂-C₅-Alkinylthio, C₃-C₇-Cycloalkylthio, für gegebenenfalls durch Halogen, Nitro, Cyano, C₁-C₄-Alkoxy, C₁-C₄-Halogenalkoxy, C₁-C₄-Alkylthio, C₁-C₄-Halogenalkyl-thio, C₁-C₄-Alkyl, C₁-C₄-Halogenalkyl substituiertes Phenyl, Phenoxy oder Phenylthio stehen,
R⁶ und R⁷ unabhängig voneinander für gegebenenfalls durch Halogen substituiertes C₁-C₂₀-Alkyl, C₁-C₂₀-Alkoxy, C₂-C₈-Alkenyl, C₁-C₂₀-Alkoxy-C₁-C₂₀-alkyl, für gegebenenfalls durch Halogen, C₁-C₂₀-Halogenalkyl, C₁-C₂₀-Alkyl oder C₁-C₂₀-Alkoxy substituiertes Phenyl, für gegebenenfalls durch Halogen, C₁-C₂₀-Alkyl, C₁₋C₂₀-Halogenalkyl oder C₁-C₂₀-Alkoxy substituiertes Benzyl steht oder zusammen für einen gegebenenfalls durch Sauerstoff unterbrochenen C₂-C₆-Alkylenring stehen,
mit Ausnahme folgender Verbindungen:
3-(2-Methoxyphenyl)-4-hydroxy-Δ³-dihydrofuranon-2,
3-(2-Chlorphenyl)-4-hydroxy-Δ³-dihydrofuranon-2,
3-(2-Fluorphenyl)-4-hydroxy-Δ³-dihydrofuranon-2,
sowie die enantiomerenreinen Formen von Verbindungen der Formel (I).

2. Insektizide und akarizide Mittel, gekennzeichnet durch einen Gehalt an mindestens einem 3-Aryl-4-hydroxy-Δ³-dihydrofuranon- oder 3-Aryl-4-hydroxy-Δ³-dihydrothiophenon-Derivat der Formel (I) gemäß Anspruch 1,
in welcher
X für C₁-C₄-Alkyl, Halogen, C₁-C₄-Alkoxy oder C₁-C₂-Halogenalkyl steht,
Y für Wasserstoff, C₁-C₆-Alkyl, Halogen, C₁-C₄-Alkoxy, C₁-C₂-Halogenalkyl steht,
Z für C₁-C₄-Alkyl, Halogen, C₁-C₄-Alkoxy steht,
n für eine Zahl von 0-3 steht,
oder wobei die Reste X und Z gemeinsam mit dem Phenylrest, an den sie gebunden sind, den Naphthalinrest der Formel 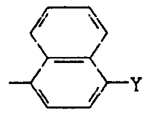 bilden,
in welchem Y die oben angegebene Bedeutung hat,
A und B gleich oder verschieden sind und für Wasserstoff oder gegebenenfalls durch Halogen substituiertes geradkettiges oder verzweigtes C₁-C₁₀-Alkyl, C₃-C₆-Alkenyl, C₃-C₆-Alkinyl, C₁-C₈-Alkoxy-C₂-C₆-alkyl, C₁-C₆-Polyalkoxy-C₂-C₆-alkyl, C₁-C₈-Alkylthio-C₂-C₆-alkyl, Cycloalkyl mit 3-7 Ringatomen, das durch 1-2 Sauerstoff und/oder Schwefelatomen unterbrochen sein kann und gegebenenfalls durch Halogen, C₁-C₄-Alkyl, C₁-C₄-Halogenalkyl, C₁-C₄-Alkoxy, Nitro substituiertes Phenyl oder PhenylC1-C₄-alkyl stehen,
oder worin
A und B gemeinsam mit dem Kohlenstoffatom, an das sie gebunden sind einen gesättigten oder ungesättigten, gegebenenfalls durch Sauerstoff und/oder Schwefel unterbrochenen und gegebenenfalls durch Halogen, C₁-C₅-Alkyl, C₁-C₅-Alkoxy, C₁-C₃-Halogenalkyl, C₁-C₄-Halogenalkoxy, C₁-C₃-Alkylthio oder gegebenenfalls durch Halogen, Alkyl, Alkoxy substituiertes Phenyl substituierten oder gegebenenfalls benzokondensierten 3- bis 8-gliedrigen Ring bilden,
D für Sauerstoff oder Schwefel steht,
G für Wasserstoff (a) oder für die Gruppen 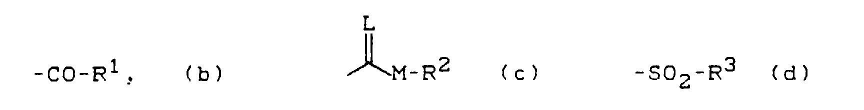 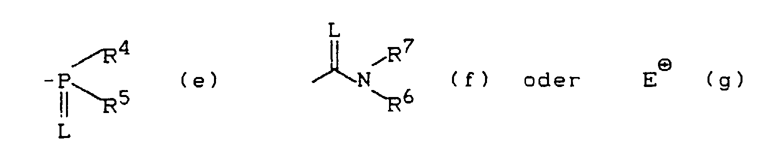
steht,
in welchen
E^{⊕} für ein Metallionäquivalent oder ein Ammoniumion steht,
L und M für Sauerstoff und/oder Schwefel steht,
R¹ für gegebenenfalls durch Halogen substituiertes: C₁-C₁₆-Alkyl, C₂-C₁₆-Alkenyl, C₁-C₆-Alkoxy-C₂-C₆-alkyl, C₁-C₁₆-Alkylthio-C₂-C₆-alkyl, C₁-C₆-Polyalkoxy-C₂-C₆-alkyl und Cycloalkyl mit 3-7 Ringatomen, das durch 1-2 Sauerstoff- und/oder Schwefelatome unterbrochen sein kann, steht,
für gegebenenfalls durch Halogen, Nitro, C₁-C₄-Alkyl, C₁-C₄-Alkoxy, C₁-C₃-Halogenalkyl, C₁-C₃-Halogenalkoxy-substituiertes Phenyl steht,
für gegebenenfalls durch Halogen, C₁-C₄-Alkyl, C₁-C₄-Alkoxy, C₁-C₃-Halogenalkyl, C₁-C₃-Halogenalkoxy-substituierets Phenyl-C₁-C₄-alkyl steht,
für gegebenenfalls durch Halogen und C₁-C₆-Alkyl-substituiertes Pyridyl, Pyrimidyl, Thiazolyl und Pyrazolyl steht,
für gegebenenfalls durch Halogen und C₁-C₄-Alkyl-substituiertes Phenoxy-C₁-C₅-alkyl steht,
R² für gegebenenfalls durch Halogen substituiertes: C₁-C₁₆-Alkyl, C₂-C₁₆-Alkenyl, C₁-C₁₆-Alkox-C₂-C₆-alkyl, C₁-C₆-Polyalkoxy-C₂-C₆-alkyl steht,
für gegebenenfalls durch Halogen, Nitro, C₁-C₄-Alkyl, C₁-C₃-Alkoxy, C₁-C₃-Halogenalkyl substituiertes Phenyl oder Benzyl steht,
R³, R⁴ und R⁵ unabhängig voneinander für gegebenenfalls durch Halogen substituiertes C₁-C₆-Alkyl, C₁-C₆-Alkoxy, C₁-C₆-Alkylamino, Di-(C₁-C₆)-Alkylamino, C₁-C₆-Alkylthio, C₃-C₄-Alkenylthio, C₂-C₄-Alkinylthio, C₃-C₆-Cycloalkylthio, für gegebenenfalls durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl, C₁-C₃-Halogenalkyl substiutiertes Phenyl, Phenoxy oder Phenylthio stehen,
R⁶ und R⁷ unabhängig voneinander für gegebenenfalls durch Halogen substituiertes C₁-C₂₀-Alkyl, C₁-C₂₀-Alkoxy, C₂-C₈-Alkenyl, C₁-C₂₀-Alkoxy-C₁-C₂₀-alkyl, für gegebenenfalls durch Halogen, C₁-C₅-Halogenalkyl, C₁-C₅-Alkyl oder C₁-C₅-Alkoxy substituiertes Phenyl, für gegebenenfalls durch Halogen, C₁-C₅-Alkyl, C₁-C₅-Halogenalkyl oder C₁-C₅-Alkoxy substituiertes Benzyl steht,
mit Ausnahme folgender Verbindungen:
3-(2-Methoxyphenyl)-4-hydroxy-Δ³-dihydrofuranon-2,
3-(3-Chlorphenyl)-4-hydraxy-Δ³-dihydrofuranon-2,
3-(2-Fluorphenyl)-4-hydroxy-Δ³-dihydrofuranon-2,
sowie die enantiomerenreinen Formen von Verbindungen der Formel (I).

3. Insektizide und akarizide Mittel, gekennzeichnet durch einen Gehalt an mindestens einem 3-Aryl-4-hydroxy-Δ³-dihydrofuranon- oder 3-Aryl-4-hydroxy-Δ³-dihydrothiophenon-Derivat der Formel (I) gemäß Anspruch 1,
in welcher
X für Methyl, Ethyl, Propyl, i-Propyl, Fluor, Chlor, Brom, Methoxy, Ethoxy und Trifluormethyl steht,
Y für Wasserstoff, Methyl, Ethyl, Propyl, i-Propyl, Butyl, i-Butyl, tert.-Butyl, Fluor, Chlor, Brom, Methoxy, Ethoxy und Trifluormethyl steht,
Z für Methyl, Ethyl, i-Propyl, Butyl, i-Butyl, tert.-Butyl, Fluor, Chlor, Brom, Methoxy und Ethoxy steht,
n für eine Zahl von 0-3 steht,
oder wobei die Reste X und Z gemeinsam mit dem Phenylrest, an den sie gebunden sind, den Naphthalinrest der Formel 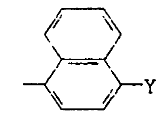 bilden,
in welchem Y die oben angegebene Bedeutung hat,
A und B gleich oder verschieden sind und für Wasserstoff, gegebenenfalls durch Halogen substituiertes geradkettiges oder verzweigtes C₁-C₈-Alkyl, C₃-C₄-Alkenyl, C₃-C₄-Alkinyl, C₁-C₆-Alkoxy-C₂-C₄-alkyl, C₁-C₄-Polyalkoxy-C₂-C₄-alkyl, C₁-C₆-Alkylthio-C₂-C₄-alkyl, Cycloalkyl mit 3-6 Ringatomen, das durch 1-2 Sauerstoff und/oder Schwefelatomen unterbrochen sein kann oder gegebenenfalls durch Fluor-, Chlor-, Brom-, Methyl-, Ethyl-, Propyl-, iso-Propyl-, Methoxy-, Ethoxy-, Trifluormethyl-, Nitro substituiertes Phenyl oder Phenyl-C₁-C₃-alkyl stehen,
oder worin
A und B gemeinsam mit dem Kohlenstoffatom, an das sie gebunden sind, einen gesättigten oder ungesättigten, gegebenenfalls durch Sauerstoff und/oder Schwefel unterbrochenen und gegebenenfalls durch Fluor, Chlor, C₁-C₄-Alkyl, C₁-C₄-Alkoxy, Trifluormethyl, C₁-C₂-Alkylthio oder gegebenenfalls durch Fluor, Chlor, Methyl, Methoxy substituiertes Phenyl substituierten oder gegebenenfalls benzokondensierten 3-bis 8-gliedrigen Ring bilden,
D für Sauerstoff oder Schwefel steht,
G für Wasserstoff (a) oder für die Gruppen 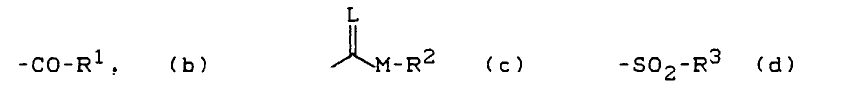 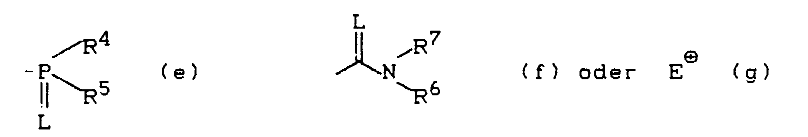
steht, in welchen
E^{⊕} für ein Metallionäquivalent oder ein Ammoniumion steht,
L und M für Sauerstoff und/oder Schwefel steht,
R¹ für gegebenenfalls durch Fluor oder Chlor substituiertes C₁-C₁₄-Alkyl, C₂-C₁₄-Alkenyl, C₁-C₄-Alkoxy-C₂-C₆-alkyl, C₁-C₄-Alkylthio-C₂-C₆-alkyl, C₁-C₄-Polyalkoxyl-C₂-C₄-alkyl und Cycloalkyl mit 3-6 Ringatomen, das durch 1-2 Sauerstoff- und/oder Schwefelatome unterbrochen sein kann, steht,
für gegebenenfalls durch Fluor, Chlor, Brom, Methyl, Ethyl, Propyl, i-Propyl, Methoxy, Ethoxy, Trifluormethyl, Trifluormethoxy, Nitrosubstituiertes Phenyl steht,
für gegebenenfalls durch Fluor, Chlor, Brom, Methyl, Ethyl, Propyl, i-Propyl, Methoxy, Ethoxy, Trifluormethyl, Trifluormethoxy substituiertes Phenyl-C₁-C₃-alkyl steht,
für gegebenenfalls durch Fluor, Chlor, Brom, Methyl, Ethylsubstituiertes Pyridyl, Pyrimidyl, Thiazolyl und Pyrazolyl steht,
für gegebenenfalls durch Fluor, Chlor, Methyl, Ethyl substituiertes Phenoxy-C₁-C₄-alkyl steht,
R² für gegebenenfalls durch Fluor oder Chlor substituiertes C₁-C₁₄-Alkyl, C₂-C₁₄-Alkenyl, C₁-C₄-Alkoxy-C₂-C₆-alkyl, C₁-C₄-Polyalkoxy-C₂-C₆-alkyl steht,
oder für gegebenenfalls durch Fluor, Chlor, Nitro, Methyl, Ethyl, Propyl, i-Propyl, Methoxy, Ethoxy, Trifluormethyl substituiertes Phenyl oder Benzyl steht,
R³, R⁴ und R⁵ unabhängig voneinander für gegebenenfalls durch Fluor oder Chlor substituiertes C₁-C₄-Alkyl, C₁-C₄-Alkoxy, C₁-C₄-Alkylamino, Di-(C₁-C₄-Alkyl)-amino, C₁-C₄-Alkylthio, für gegebenenfalls durch Fluor, Chlor, Brom, Nitro, Cyano, C₁-C₂-Alkoxy, C₁-C₄-Fluoralkoxy, C₁-C₂-Chloralkoxy, C₁-C₂-Alkylthio, C₁-C₂-Fluoralkylthio, C₁-C₂-Chloralkylthio, C₁-C₃-Alkyl substituiertes Phenyl, Phenoxy oder Phenylthio stehen,
R⁶ und R⁷ unabhängig voneinander für gegebenenfalls durch Fluor, Chlor, Brom substituiertes C₁-C₁₀-Alkyl, C₁-C₁₀-Alkoxy, C₁-C₁₀-Alkoxy-(C₁-C₁₀)alkyl, für gegebenenfalls durch Fluor, Chlor, Brom, C₁-C₂₀-Halogenalkyl, C₁-C₂₀-Alkyl oder C₁-C₄-Alkoxy substituiertes Phenyl, für gegebenenfalls durch Fluor, Chlor, Brom, C₁-C₄-Alkyl, C₁-C₄-Halogenalkyl oder C₁-C₄-Alkoxy substituiertes Benzyl steht,
mit Ausnahme folgender Verbindungen:
3-(2-Methoxyphenyl)-4-hydroxy-Δ³-dihydrofuranon-2,
3-(2-Chlorphenyl)-4-hydroxy-Δ³-dihydrofuranon-2,
3-(2-Fluorphenyl)-4-hydroxy-Δ³-dihydrofuranon-2,
sowie die enantiomerenreinen Formen von Verbindungen der Formel (I).

4. Verfahren zur Bekämpfung von Insekten und/oder Spinnentieren, dadurch gekennzeichnet, daß man 3-Aryl-4-hydroxy-Δ³-dihydrofuranon- oder 3-Aryl-4-hydroxy-Δ³-dihydrothiophenon-Derivate der Formel (I) gemäß Anspruch 1 auf Insekten und/oder Spinnentiere und/oder deren Lebensraum einwirken läßt.

5. Verwendung von 3-Aryl-4-hydroxy-Δ³-dihydrofuranon- oder 3-Aryl-4-hydroxy-Δ³ -dihydrothiophenon-Derivaten der Formel (I) gemäß Anspruch 1 zur Bekämpfung von Insekten und/oder Spinnentieren.

6. Verfahren zur Herstellung von insektiziden und/oder akariziden Mitteln, dadurch gekennzeichnet, daß man 3-Aryl-4-hydroxy-Δ³ -dihydrofuranon- oder 3- Aryl-4-hydroxy-Δ³-dihydrothiophenon-Derivate der Formel (I) gemäß Anspruch 1 mit Streckmitteln und/oder oberflächenaktiven Mitteln vermischt.

7. 3-Aryl-4-hydroxy-Δ³-dihydrofuranon- und 3-Aryl-4-hydroxy-Δ³-dihydrothiophenon-Derivate der allgemeinen Formel (I) 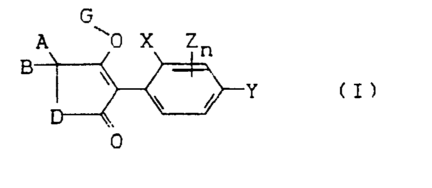 in welcher
X für C₁-C₆-Alkyl, Halogen, C₁-C₆-Alkoxy oder C₁-C₃-Halogenalkyl steht,
Y für Wasserstoff, C₁-C₆-Alkyl, Halogen, C₁-C₆-Alkoxy, C₁-C₃-Halogenalkyl steht,
Z für C₁-C₆-Alkyl, Halogen, C₁-C₆-Alkoxy steht,
n für eine Zahl von 0-3 steht,
oder wobei die Reste X und Z gemeinsam mit dem Phenylrest, an den sie gebunden sind, den Naphthalinrest der Formel 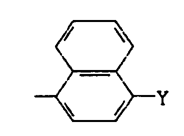 bilden,
in welchem Y die oben angegebene Bedeutung hat,
A und B gleich oder verschieden sind und für Wasserstoff oder gegebenenfalls durch Halogen substituiertes geradkettiges oder verzweigtes C₁-C₁₂-Alkyl, C₃-C₈-Alkenyl, C₃-C₈-Alkinyl, C₁-C₁₀-Alkoxy-C₂-C₈-alkyl, C₁-C₈-PolyalkoxyC₂-C₈-alkyl, C₁-C₁₀-Alkylthio-C₂-C₈-alkyl, Cycloalkyl mit 3-8 Ringatomen, das durch Sauerstoff und/oder Schwefel unterbrochen sein kann und gegebenenfalls durch Halogen, C₁-C₆-Alkyl, C₁-C₆-Halogenalkyl-, C₁-C₆-Alkoxy-, C₁-C₆-Halogenalkoxy, Nitro substituiertes Phenyl oder Phenyl-C1-C₆-alkyl steht,
oder worin
A und B gemeinsam mit dem Kohlenstoffatom, an das sie gebunden sind einen gesättigten oder ungesättigten, gegebenenfalls durch Sauerstoff und/oder Schwefel unterbrochenen und gegebenenfalls durch Halogen, C₁-C₆-Alkyl, C₁-C₆-Al-koxy, C₁-C₄-Halogenalkyl, C₁-C₄-Halogenalkoxy, C₁-C₄-Alkylthio oder gegebenenfalls substituiertes Phenyl substituierten oder gegebenenfalls benzokondensierten 3- bis 8-gliedrigen Ring bilden,
D für Sauerstoff oder Schwefel steht,
G für Wasserstoff (a) oder für die Gruppen 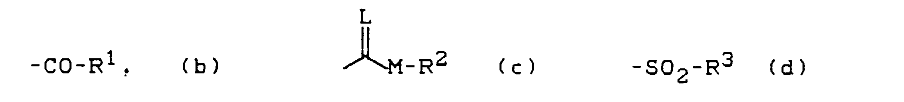 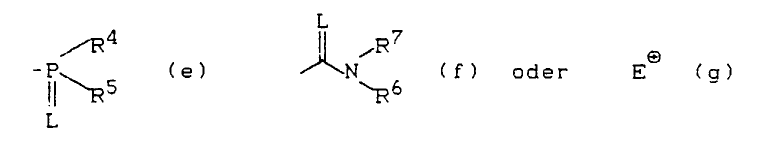
steht,
in welchen
E^{⊕} für ein Metallionäquivalent oder ein Ammoniumion steht,
L und M für Sauerstoff und/oder Schwefel steht,
R¹ für gegebenenfalls durch Halogen substituiertes: C₁-C₂₀-Alkyl, C₂-C₂₀-Alkenyl, C₁-C₈-Alkoxy-C₂-C₈-alkyl, C₁-C₈-Alkylthio-C₂-C₈-alkyl, C₁-C₈-Polyalkoxyl-C₂-C₈-alkyl oder Cycloalkyl mit 3-8 Ringatomen, das durch Sauerstoff- und/oder Schwefelatome unterbrochen sein kann, steht,
für gegebenenfalls durch Halogen, Nitro, C₁-C₆-Alkyl, C₁-C₆-Alkoxy, C₁-C₆-Halogenalkyl, C₁-C₆-Halogenalkoxysubstituiertes Phenyl steht;
für gegebenenfalls durch Halogen-, C₁-C₆-Alkyl, C₁-C₆-Alkoxy-, C₁-C₆-Halogenalkyl-, C₁-C₆-Halogenalkoxy-substituiertes Phenyl-C₁-C₆-alkyl steht,
für gegebenenfalls durch Halogen und/oder C₁-C₆-Alkyl substituiertes Pyridyl, Pyrimidyl, Thiazolyl und Pyrazolyl steht,
für gegebenenfalls durch Halogen und/oder C₁-C₆-Alkyl-substituiertes Phenoxy-C₁-C₆-alkyl steht,
R² für gegebenenfalls durch Halogen substituiertes: C₁-C₂₀-Alkyl, C₂-C₂₀-Alkenyl, C₁-C₈-Alkoxy-C₂-C₈-alkyl, C₁-C₈-Polyalkoxy-C₂-C₈-alkyl steht,
für gegebenenfalls durch Halogen, Nitro, C₁-C₆-Alkyl, C₁-C₆-Alkoxy, C₁-C₆-Halogenalkylsubstituiertes Phenyl oder Benzyl steht,
R³, R⁴ und R⁵ unabhängig voneinander für gegebenenfalls durch Halogen substituiertes C₁-C₈-Alkyl, C₁-C₈-Alkoxy, C₁-C₈-Alkylamino, Di-(C₁-C₈)-Alkylamino, C₁-C₈-Alkylthio, C₂-C₅-Alkenylthio, C₂-C₅-Alkinylthio, C₃-C₇-Cycloalkylthio, für gegebenenfalls durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl, C₁-C₄-Halogenalkyl substituiertes Phenyl, Phenoxy oder Phenylthio stehen,
R⁶ und R⁷ unabhängig voneinander für gegebenenfalls durch Halogen substituiertes C₁-C₂₀-Alkyl, C₁-C₂₀-Alkoxy, C₂-C₈-Alkenyl, C₁-C₂₀-Alkoxy-C₁-C₂₀-alkyl, für gegebenenfalls durch Halogen, C₁-C₂₀-Halogenalkyl, C₁-C₂₀-Alkyl oder C₁-C₂₀-Alkoxy substituiertes Phenyl, für gegebenenfalls durch Halogen, C₁-C₂₀-Alkyl, C₁-C₂₀-Halogenalkyl oder C₁-C₂₀-Alkoxy substituiertes Benzyl steht oder zusammen für einen gegebenenfalls durch Sauerstoff unterbrochenen C₂-C₆-Alkylenring stehen,
sowie die enantiomerenreinen Formen von Verbindungen der Formel (I),
mit Ausnahme folgender Verbindungen:
3-(2-Methoxyphenyl)-4-hydroxy-Δ³-dihydrofuranon-2,
3-(2-Chlorphenyl)-4-hydroxy-Δ³-dihydrofuranon-2,
3-(2-Fluorphenyl)-4-hydroxy-Δ³-dihydrofuranon-2,
und mit der Maßgabe, daß nicht gleichzeitig X für Halogen oder Halogenmethyl und 1 bis 3 gleichen oder verschiedenen Halogenatomen, Y für Wasserstoff und n für die Zahl 0 steht, wenn D für Sauerstoff und gleichzeitig R¹ für Alkylcarbonyl oder Halogenalkylcarbonyl steht und gleichzeitig A und B unabhängig voneinander für Wasserstoff, Alkyl oder jeweils unsubstituiertes oder einfach oder mehrfach, gleich oder verschieden substituiertes Phenyl oder Phenylalkyl stehen, wobei als Substituenten im Phenylteil ausgewählt sind: Nitro, Halogen, Alkyl, Alkoxy, Halogenalkyl oder Halogenalkoxy.

8. 3-Aryl-4-hydroxy-Δ³ -dihydrofuranon- und 3-Aryl-4-hydroxy-Δ³-dihydrothiophenon-Derivate der Formel (I) gemäß Anspruch 7, dadurch gekennzeichnet, daß es sich um eine der folgenden Strukturen (Ia) bis (Ia) handelt: 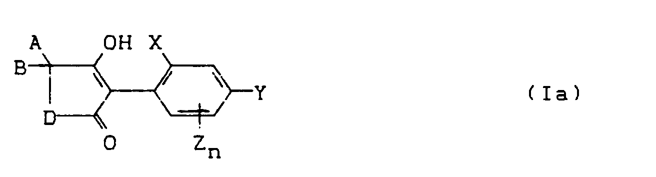 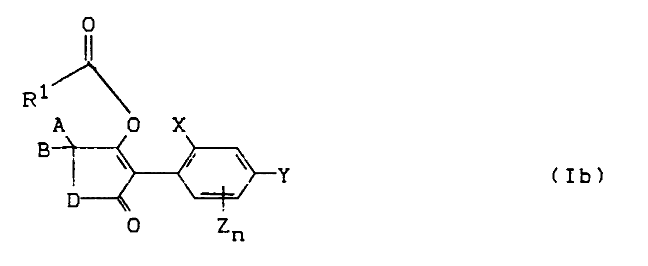 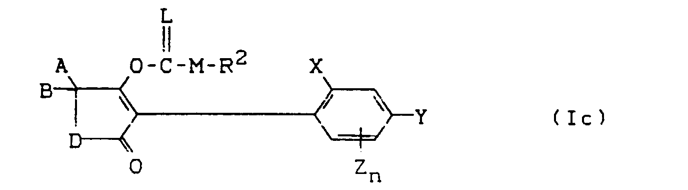 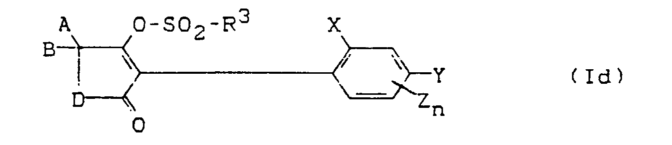 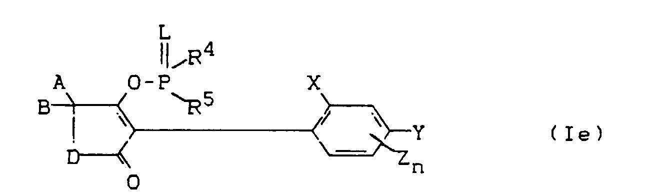 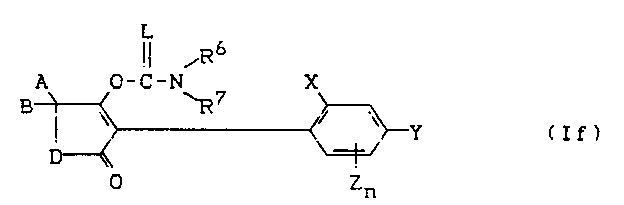 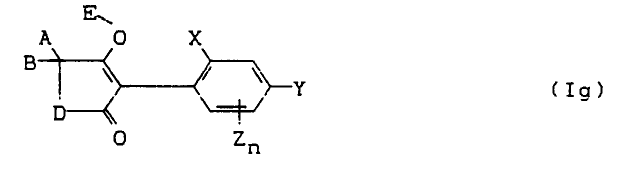 worin
A, B, D, E, L, M, X, Y, Zₙ, R¹, R², R³, R⁴, R⁵, R⁶ und R⁷ die in Anspruch 7 angegebenen Bedeutungen besitzen.

9. 3-Aryl-4-hydroxy-Δ³-dihydrofuranon- und 3-Aryl-4-hydroxy-Δ³-dihydrothiophenon-Derivate der Formel (I) gemäß Anspruch 7,
in welcher
X für C₁-C₄-Alkyl, Halogen, C₁-C₄-Alkoxy oder C₁-C₂-Halogenalkyl steht,
Y für Wasserstoff, C₁-C₆-Alkyl, Halogen, C₁-C₄-Alkoxy, C₁-C₂-Halogenalkyl steht,
Z für C₁-C₄-Alkyl, Halogen, C₁-C₄-Alkoxy steht,
n für eine Zahl von 0-3 steht,
oder wobei die Reste X und Z gemeinsam mit dem Phenylrest, an den sie gebunden sind, den Naphthalinrest der Formel 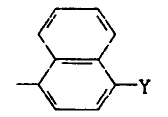 bilden,
in welchem Y die oben angegebene Bedeutung hat,
A und B gleich oder verschieden sind und für Wasserstoff oder gegebenenfalls durch Halogen substituiertes geradkettiges oder verzweigtes C₁-C₁₀-Alkyl, C₃-C₆-Alkenyl, C₃-C₆-Alkinyl, C₁-C₈-Alkoxy-C₂-C₆-alkyl, C₁-C₆-Polyalkoxy-C₂-C₆-alkyl, C₁-C₈-Alkylthio-C₂-C₆-alkyl, Cycloalkyl mit 3-7 Ringatomen, das durch 1-2 Sauerstoff und/oder Schwefelatomen unterbrochen sein kann und gegebenenfalls durch Halogen, C₁-C₄-Alkyl, C₁-C₄-Halogenalkyl, C₁-C₄-Alkoxy, Nitro substituiertes Phenyl oder Phenyl-C1-C₄-alkyl stehen,
oder worin
A und B gemeinsam mit dem Kohlenstoffatom, an das sie gebunden sind einen gesättigten oder ungesättigten, gegebenenfalls durch Sauerstoff und/oder Schwefel unterbrochenen und gegebenenfalls durch Halogen, C₁-C₅-Alkyl, C₁-C₅-Alkoxy, C₁-C₃-Halogenalkyl, C₁-C₄-Halogenalkoxy, C₁-C₃-Alkylthio oder gegebenenfalls durch Halogen, Alkyl, Alkoxy substituiertes Phenyl substituierten oder gegebenenfalls benzokondensierten 3- bis 8-gliedrigen Ring bilden,
D für Sauerstoff oder Schwefel steht,
G für Wasserstoff (a) oder für die Gruppen 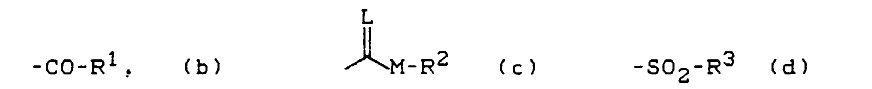 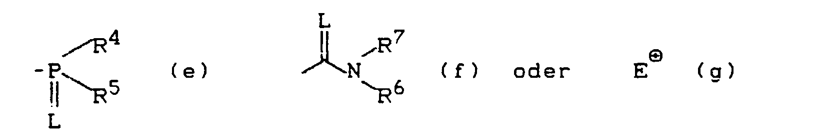
steht,
in welchen
E^{⊕} für ein Metallionäquivalent oder ein Ammoniumion steht,
L und M für Sauerstoff und/oder Schwefel steht,
R¹ für gegebenenfalls durch Halogen substituiertes: C₁-C₁₆-Alkyl, C₂-C₁₆-Alkenyl, C₁-C₆-Alkoxy-C₂-C₆-alkyl, C₁-C₁₆-Alkylthio-C₂-C₆-alkyl, C₁-C₆-Polyalkoxy-C₂-C₆-alkyl und Cycloalkyl mit 3-7 Ringatomen, das durch 1-2 Sauerstoff- und/oder Schwefelatome unterbrochen sein kann, steht,
für gegebenenfalls durch Halogen, Nitro, C₁-C₄-Alkyl, C₁-C₄-Alkoxy, C₁-C₃-Halogenalkyl, C₁-C₃-Halogenalkoxy-substituiertes Phenyl steht,
für gegebenenfalls durch Halogen, C₁-C₄-Alkyl, C₁-C₄-Alkoxy, C₁-C₃-Halogenalkyl, C₁-C₃-Halogenalkoxy-substituierets Phenyl-C₁-C₄-alkyl steht,
für gegebenenfalls durch Halogen und C₁-C₆-Alkyl-substituiertes Pyridyl, Pyrimidyl, Thiazolyl und Pyrazolyl steht,
für gegebenenfalls durch Halogen und C₁-C₄-Alkyl substituiertes Phenoxy-C₁-C₅-alkyl steht,
R² für gegebenenfalls durch Halogen substituiertes: C₁-C₁₆-Alkyl, C₂-C₁₆-Alkenyl, C₁-C₁₆-Alkox-C₂-C₆-alkyl, C₁-C₆-Polyalkoxy-C₂-C₆-alkyl steht,
für gegebenenfalls durch Halogen, Nitro, C₁-C₄-Alkyl, C₁-C₃-Alkoxy, C₁-C₃-Halogenalkylsubstituiertes Phenyl oder Benzyl steht,
R³, R⁴ und R⁵ unabhängig voneinander für gegebenenfalls durch Halogen substituiertes C₁-C₆-Alkyl, C₁-C₆-Alkoxy, C₁-C₆-Alkylamino, Di-(C₁-C₆)-Alkylamino, C₁-C₆-Alkylthio, C₃-C₄-Alkenylthio, C₂-C₄-Alkinylthio, C₃-C₆-Cycloalkylthio, für gegebenenfalls durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl, C₁-C₃-Halogenalkyl substiutiertes Phenyl, Phenoxy oder Phenylthio stehen,
R⁶ und R⁷ unabhängig voneinander für gegebenenfalls durch Halogen substituiertes C₁-C₂₀-Alkyl, C₁-C₂₀-Alkoxy, C₂-C₈-Alkenyl, C₁-C₂₀-Alkoxy-C₁-C₂₀-alkyl, für gegebenenfalls durch Halogen, C₁-C₅-Halogenalkyl, C₁-C₅-Alkyl oder C₁-C₅-Alkoxy substituiertes Phenyl, für gegebenenfalls durch Halogen, C₁-C₅-Alkyl, C₁-C₅-Halogenalkyl oder C₁-C₅-Alkoxy substituiertes Benzyl steht,
sowie die enantiomerenreinen Formen von Verbindungen der Formel (I),
mit Ausnahme folgender Verbindungen:
3-(2-Methoxyphenyl)-4-hydroxy-Δ³-dihydrofuranon-2,
3-(3-Chlorphenyl)-4-hydroxy-Δ³-dihydrofuranon-2,
3-(2-Fluorphenyl)-4-hydroxy-Δ³-dihydrofuranon-2,
und mit der Maßgabe, daß nicht gleichzeitig X für Halogen oder Halogenmethyl und 1 bis 3 gleichen oder verschiedenen Halogenatomen, Y für Wasserstoff und n für die Zahl 0 steht, wenn D für Sauerstoff und gleichzeitig R¹ für Alkylcarbonyl oder Halogenalkylcarbonyl steht und gleichzeitig A und B unabhängig voneinander für Wasserstoff, Alkyl oder jeweils unsubstituiertes oder einfach oder mehrfach, gleich oder verschieden substituiertes Phenyl oder Phenylalkyl stehen, wobei als Substituenten im Phenylteil ausgewählt sind: Nitro, Halogen, Alkyl, Alkoxy, Halogenalkyl oder Halogenalkoxy.

10. 3-Aryl-4-hydroxy-Δ³-dihydrofuranon- und 3-Aryl-4-hydroxy-Δ³-dihydrothiophenon-Derivate der Formel (I) gemäß Anspruch 7,
in welcher
X für Methyl, Ethyl, Propyl, i-Propyl, Fluor, Chlor, Brom, Methoxy, Ethoxy und Trifluormethyl steht,
Y für Wasserstoff, Methyl, Ethyl, Propyl, i-Propyl, Butyl, i-Butyl, tert.-Butyl, Fluor, Chlor, Brom, Methoxy, Ethoxy und Trifluormethyl steht,
Z für Methyl, Ethyl, i-Propyl, Butyl, i-Butyl, tert.-Butyl, Fluor, Chlor, Brom, Methoxy und Ethoxy steht,
n für eine Zahl von 0-3 steht,
oder wobei die Reste X und Z gemeinsam mit dem Phenylrest, an den sie gebunden sind, den Naphthalinrest der Formel 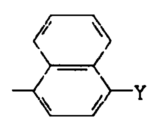 bilden,
in welchem Y die oben angegebene Bedeutung hat,
A und B gleich oder verschieden sind und für Wasserstoff, gegebenenfalls durch Halogen substituiertes geradkettiges oder verzweigtes C₁-C₈-Alkyl, C₃-C₄-Alkenyl, C₃-C₄-Alkinyl, C₁-C₆-Alkoxy-C₂-C₄-alkyl, C₁-C₄-Polyalkoxy-C₂-C₄-alkyl, C₁-C₆-Alkylthio-C₂-C₄-alkyl, Cycloalkyl mit 3-6 Ringatomen, das durch 1-2 Sauerstoff und/oder Schwefelatomen unterbrochen sein kann oder gegebenenfalls durch Fluor-, Chlor-, Brom-, Methyl-, Ethyl-, Propyl-, iso-Propyl-, Methoxy-, Ethoxy-, Trifluormethyl-, Nitro substituiertes Phenyl oder Phenyl-C₁-C₃-alkyl stehen,
oder worin
A und B gemeinsam mit dem Kohlenstoffatom, an das sie gebunden sind, einen gesättigten oder ungesättigten, gegebenenfalls durch Sauerstoff und/oder Schwefel unterbrochenen und gegebenenfalls durch Fluor, Chlor, C₁-C₄-Alkyl, C₁-C₄-Alkoxy, Trifluormethyl, C₁-C₂-Alkylthio oder gegebenenfalls durch Fluor, Chlor, Methyl, Methoxy substituiertes Phenyl substituierten oder gegebenenfalls benzokondensierten 3-bis 8-gliedrigen Ring bilden,
D für Sauerstoff oder Schwefel steht,
G für Wasserstoff (a) oder für die Gruppen 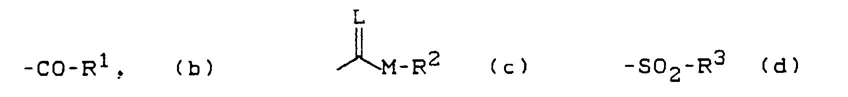 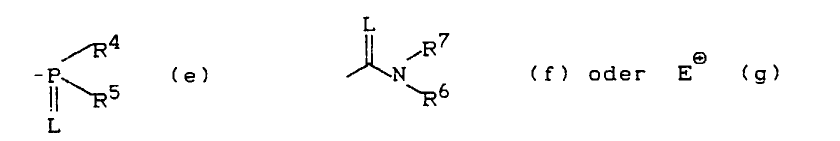
steht, in welchen
E^{⊕} für ein Metallionäquivalent oder ein Ammoniumion steht,
L und M für Sauerstoff und/oder Schwefel steht,
R¹ für gegebenenfalls durch Fluor oder Chlor substituiertes C₁-C₁₄-Alkyl, C₂-C₁₄-Alkenyl, C₁-C₄-Alkoxy-C₂-C₆-alkyl, C₁-C₄-Alkylthio-C₂-C₆-alkyl, C₁-C₄-Polyalkoxyl-C₂-C₄-alkyl und Cycloalkyl mit 3-6 Ringatomen, das durch 1-2 Sauerstoff- und/oder Schwefelatome unterbrochen sein kann, steht,
für gegebenenfalls durch Fluor, Chlor, Brom, Methyl, Ethyl, Propyl, 1-Propyl, Methoxy, Ethoxy, Trifluormethyl, Trifluormethoxy, Nitrosubstituiertes Phenyl steht,
für gegebenenfalls durch Fluor, Chlor, Brom, Methyl, Ethyl, Propyl, i-Propyl, Methoxy, Ethoxy, Trifluormethyl, Trifluormethoxy substituiertes Phenyl-C₁-C₃-alkyl steht,
für gegebenenfalls durch Fluor, Chlor, Brom, Methyl, Ethylsubstituiertes Pyridyl, Pyrimidyl, Thiazolyl und Pyrazolyl steht,
für gegebenenfalls durch Fluor, Chlor, Methyl, Ethyl substituiertes Phenoxy-C₁-C₄-alkyl steht,
R² für gegebenenfalls durch Fluor oder Chlor substituiertes C₁-C₁₄-Alkyl, C₂-C₁₄-Alkenyl, C₁-C₄-Alkoxy-C₂-C₆-alkyl, C₁-C₄-Polyalkoxy-C₂-C₆-alkyl steht,
oder für gegebenenfalls durch Fluor, Chlor, Nitro, Methyl, Ethyl, Propyl, i-Propyl, Methoxy, Ethoxy, Trifluormethyl substituiertes Phenyl oder Benzyl steht,
R³, R⁴ und R⁵ unabhängig voneinander für gegebenenfalls durch Fluor oder Chlor substituiertes C₁-C₄-Alkyl, C₁-C₄-Alkoxy, C₁-C₄-Alkylamino, Di-(C₁-C₄-Alkyl)-amino, C₁-C₄-Alkylthio, für gegebenenfalls durch Fluor, Chlor, Brom, Nitro, Cyano, C₁-C₂-Alkoxy, C₁-C₄-Fluoralkoxy, C₁-C₂-Chloralkoxy, C₁-C₂-Alkylthio, C₁-C₂-Fluoralkylthio, C₁-C₂-Chloralkylthio, C₁-C₃-Alkyl substituiertes Phenyl, Phenoxy oder Phenylthio stehen,
R⁶ und R⁷ unabhängig voneinander für gegebenenfalls durch Fluor, Chlor, Brom substituiertes C₁-C₁₀-Alkyl, C₁-C₁₀-Alkoxy, C₁-C₁₀-Alkoxy-(C₁-C₁₀)alkyl, für gegebenenfalls durch Fluor, Chlor, Brom, C₁-C₂₀-Halogenalkyl, C₁-C₂₀-Alkyl oder C₁-C₄-Alkoxy substituiertes Phenyl, für gegebenenfalls durch Fluor, Chlor, Brom, C₁-C₄-Alkyl, C₁-C₄-Halogenalkyl oder C₁-C₄-Alkoxy substituiertes Benzyl steht,
sowie die enantiomeren reinen Formen von Verbindungen der Formal (I),
mit Ausnahme folgender Verbindungen:
3-(2-Methoxyphenyl)-4-hydroxy-Δ³-dihydrofuranon-2,
3-(2-Chlorphenyl)-4-hydroxy-Δ³-dihydrofuranon-2,
3-(2-Fluorphenyl)-4-hydroxy-Δ³-dihydrofuranon-2,
und mit der Maßgabe, daß nicht gleichzeitig X für Halogen oder Halogenmethyl und 1 bis 3 gleichen oder verschiedenen Halogenatomen, Y für Wasserstoff und n für die Zahl 0 steht, wenn D für Sauerstoff und gleichzeitig R¹ für Alkylcarbonyl oder Halogenalkylcarbonyl steht und gleichzeitig A und B unabhängig voneinander für Wasserstoff, Alkyl oder jeweils unsubstituiertes oder einfach oder mehrfach, gleich oder verschieden substituiertes Phenyl oder Phenylalkyl stehen, wobei als Substituenten im Phenylteil ausgewählt sind: Nitro, Halogen, Alkyl, Alkoxy, Halogenalkyl oder Halogenalkoxy.

11. Verfahren zur Herstellung von 3-Aryl-4-hydroxy-Δ³-dihydrofuranon- und 3-Aryl-4-hydroxy-Δ³-dihydrothiophenon-Derivate der allgemeinen Formel (I) gemäß Anspruch 7 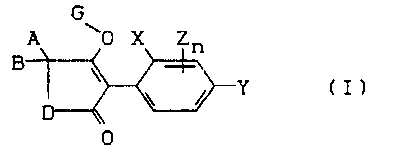 in welcher
A, B, D, G, X, Y, Z und n die in Anspruch 7 angegebene Bedeutung haben
dadurch gekennzeichnet,
daß man zum Erhalt von 3-Aryl-4-hydroxy-Δ³-dihydrofuranon- und 3-Aryl-4-hydroxy-Δ³-dihydrothiophenon-Derivaten der Formel (Ia) 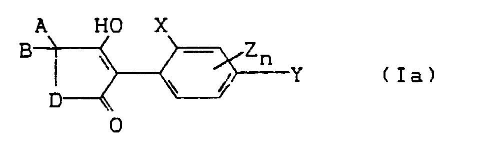 in welcher
A, B, D, X, Y, Z und n die oben angegebene Bedeutung haben,
entweder
(A) Carbonsäureester der Formel (II) 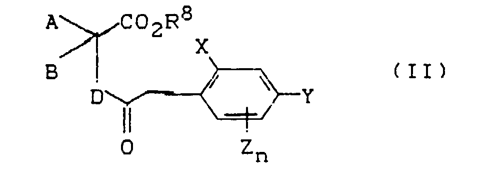 in welcher
A, B, D, X, Y, Z und n die oben angegebene Bedeutung haben
und
R⁸ für Alkyl steht,
in Gegenwart eines Verdünnungsmittels und in Gegenwart einer Base intramolekular kondensiert,
oder
(B) zum Erhalt von Verbindungen der Formel (Ib) 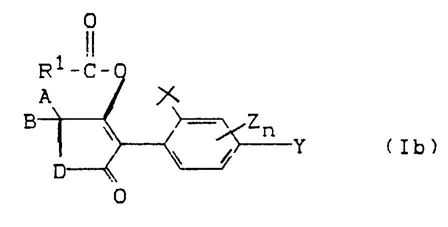 in welcher A, B, D, X, Y, Z, R¹ und n die oben angegebene Bedeutung haben,
Verbindungen der Formel (Ia), 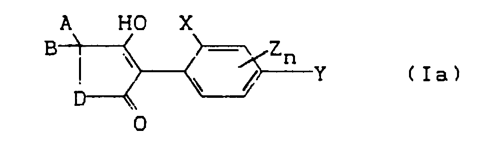 in welcher
A, B, D, X, Y, Z und n die oben angegebene Bedeutung haben,
α) mit Säurehalogeniden der allgemeinen Formel (III) 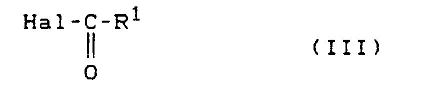 in welcher
R¹ die oben angegebene Bedeutung hat
und
Hal für Halogen, insbesondere Chlor und Brom steht,
gegebenenfalls in Gegenwart eines Verdünnungsmittels und gegebenenfalls in Gegenwart eines Säurebindemittels umsetzt
oder
β) mit Carbonsäureanhydriden der allgemeinen Formel (IV)
R¹-CO-O-CO-R¹ (IV)
in welcher
R¹ die oben angegebene Bedeutung hat,
gegebenenfalls in Gegenwart eines Verdünnungsmittels und gegebenenfalls in Gegenwart eines Säurebindemittels
umsetzt, oder daß man
(C) zum Erhalt von Verbindungen der Formel (Ic) 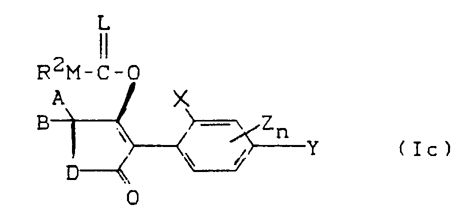 in welcher
A, B, D, X, Y, Z, R² und n die oben angegebene Bedeutung haben,
L für Sauerstoff
und
M für Sauerstoff oder Schwefel steht,
Verbindungen der Formel (Ia) 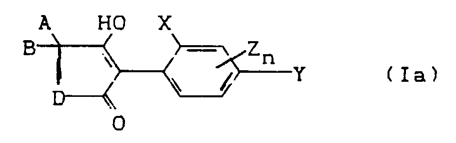 in welcher
A, B, D, X, Y, Z und n die oben angegebene Bedeutung haben
mit Chlorameisensäureester oder Chlorameisensäurethiolester der allgemeinen Formel (V)
R²-M-CO-Cl (V)
in welcher
R² und M die oben angegebene Bedeutung haben,
gegebenenfals in Gegenwart eines Verdünnungsmittels und gegebenenfalls in Gegenwart eines Säurebindemittels umsetzt, oder daß man
(D) zum Erhalt von Verbindungen der Formel (Ic) 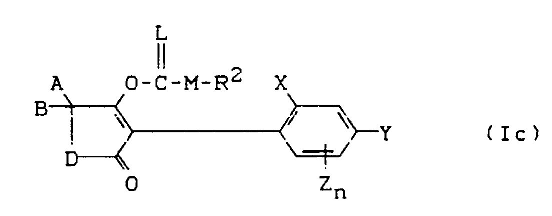 in welcher
A, B, D, R², X, Y, Z und n die oben angegebene Bedeutung haben,
L für Schwefel
und
M für Sauerstoff oder Schwefel steht,
Verbindungen der Formel (Ia) 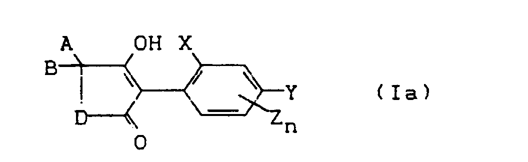 in welcher
A, B, D, X, Y, Z und n die oben angegebene Bedeutung haben
α) mit Chlormonothioameisensäureestern oder Chlordithioameisensäureestern der allgemeinen Formel (VI) 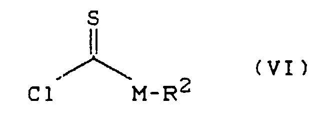 in welcher
M und R² die oben angegebene Bedeutung haben
gegebenenfalls in Gegenwart eines Verdünnungsmittels und gegebenenfalls in Gegenwart eines Säurebindemittels umsetzt,
oder
β) mit Schwefelkohlenstoff und anschließend mit Alkylhalogeniden der allgemeinen Formel (VII)
R²-Hal (VII)
in welcher
R² die oben angegebene Bedeutung hat
und
Hal für Chlor, Brom, Jod
steht, umsetzt,
oder daß man
(E) zum Erhalt von Verbindungen der Formel (Id) 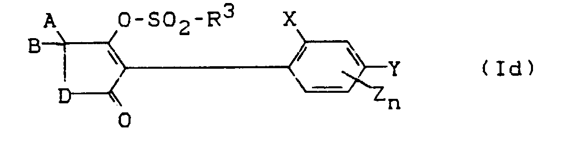 in welcher
A, B, D, X, Y, Z, R³ und n die oben angegebene Bedeutung haben,
Verbindungen der Formel (Ia) 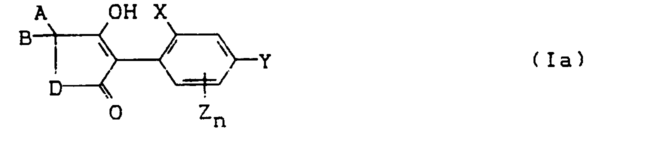 in welcher
A, B, D, X, Y, Z und n die oben angegebene Bedeutung haben,
mit Sulfonsäurechloriden der allgemeinen Formel (VIII)
R³-SO₂-Cl (VIII)
in welcher
R³ die oben angegebene Bedeutung hat
gegebenenfalls in Gegenwart eines Verdünnungsmittels und gegebenenfalls in Gegenwart eines Säurebindemittels,
umsetzt, oder daß man
(F) zum Erhalt von Verbindungen der Formel (Ie) 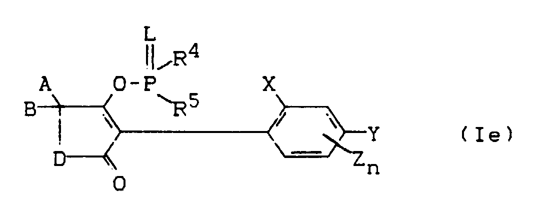 in welcher
A, B, D, L, X, Y, Z, R⁴, R⁵ und n die oben angegebene Bedeutung haben.
Verbindungen der Formel (Ia) 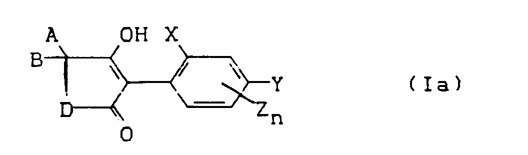 in welcher
A, B, D, X, Y, Z und n die oben angegebene Bedeutung haben
mit Phosphorverbindungen der allgemeinen Formel (IX) 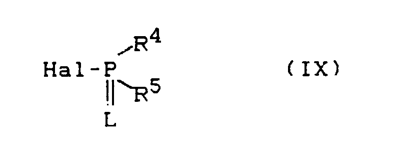 in welcher
L, R⁴ und R⁵ die oben angegebene Bedeutung haben
und
Hal für Halogen steht,
gegebenenfalls in Gegenwart eines Verdünnungsmittels und gegebenenfalls in Gegenwart eines Säurebindemittels umsetzt, oder daß man
(G) zum Erhalt von Verbindungen der Formel (If) 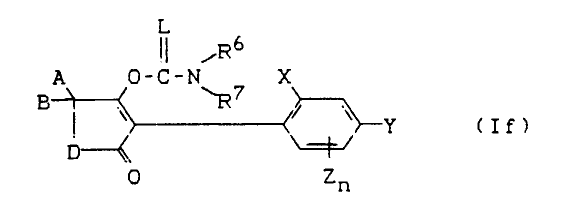 in welcher
A, B, D, L, X, Y, Z, R⁶, R⁷ und n die oben angegebene Bedeutung haben,
Verbindungen der Formel (Ia), 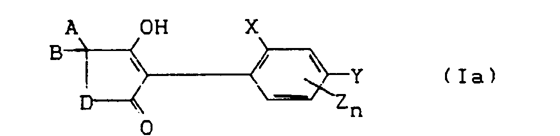 in welcher
A, B, D, X, Y, Z und n die oben angegebene Bedeutung haben
α) mit Isocyanaten der allgemeinen Formel (X)
R⁶-N=C=O (X)
in welcher
R⁶ die oben angegebene Bedeutung hat
gegebenenfalls in Gegenwart eines Verdünnungsmittels und gegebenenfalls in Gegenwart eines Katalysators umsetzt,
oder
β) mit Carbamidsäurechloriden oder Thiocarbamidsäurechloriden der allgemeinen Formel (XI) 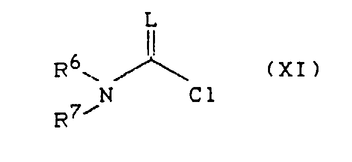 in welcher
L, R⁶ und R⁷ die oben angegebene Bedeutung haben,
gegebenenfalls in Gegenwart eines Verdünnungsmittels und gegebenenfalls in Gegenwart eines Säurebindemittels
umsetzt, oder daß man
(H) zum Erhalt von Verbindungen der Formel (Ig) 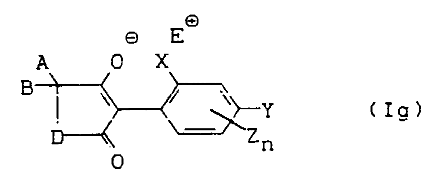 in welcher
X, Y, Z, A B, D und n die oben angegebene Bedeutung haben,
und E^{⊕} für ein Metallionäquivalent oder für ein Ammoniumion steht,
Verbindungen der Formel (Ia) 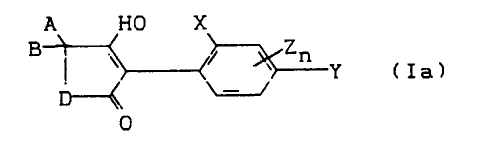 in welcher
X, Y, Z, A, B, D und n die oben angegebene Bedeutung haben, mit Metallhydroxiden oder Aminen der allgemeinen Formeln (XII) und (XIII) 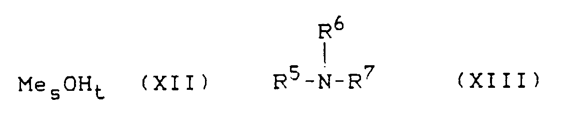 in welchen
Me für ein- oder zweiwertige Metallionen
s und t für die Zahl 1 oder 2 und
R⁵, R⁶ und R⁷ unabhängig voneinander für Wasserstoff und Alkyl
stehen,
gegebenenfalls in Gegenwart eines Verdünnungsmittels, umsetzt.

12. Herbizide Mittel, gekennzeichnet durch einen Gehalt an mindestens einem 3-Aryl-4-hydroxy-Δ³-dihydrofuranon-oder 3-Aryl-4-hydroxy-Δ³ -dihydrothiophenon-Derivat der Formel (I) gemäß Anspruch 7.

13. Verfahren zur Bekämpfung von Unkräutern, dadurch gekennzeichnet, daß man 3-Aryl-4-hydroxy-Δ³-dihydrofuranon- oder 3- Aryl-4-hydroxy-Δ³-dihydrothiophenon-Derivate der Formel (I) gemäß Anspruch 7 auf Unkräuter und/oder deren Lebensraum einwirken läßt.

14. Verwendung von 3-Aryl-4-hydroxy-Δ³-dihydrofuranonoder 3-Aryl-4-hydroxy-Δ³-dihydrothiophenon-Derivaten der Formel (I) gemäß Anspruch 7 zur Bekämpfung von Unkräutern.

15. Verfahren zur Herstellung von herbiziden Mitteln, dadurch gekennzeichnet, daß man 3-Aryl-4-hydroxy-Δ³-dihydrofuranon- oder 3-Aryl-4-hydroxy-Δ³-dihydrothiophenon-Derivate der Formel (I) gemäß Anspruch 7 mit Streckmitteln und/oder oberflächenaktiven Mitteln vermischt.

## Claims

1. Insecticidal and acaricidal compositions, characterised in that they comprise at least one 3-aryl-4-hydroxy-Δ³-dihydrofuranone or 3-aryl-4-hydroxy-Δ³-dihydrothiophenone derivative of the general formula (I) 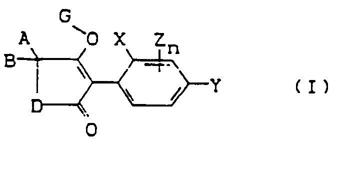 in which
X represents C₁-C₆-alkyl, halogen, C₁-C₆-alkoxy or C₁-C₃-halogenoalkyl,
Y represents hydrogen, C₁-C₆-alkyl, halogen, C₁-C₆-alkoxy or C₁-C₃-halogenoalkyl,
Z represents C₁-C₆-alkyl, halogen or C₁-C₆-alkoxy,
n represents a number from 0 to 3,
or the radicals X and Z together with the phenyl radical to which they are bonded form the naphthalene radical of the formula 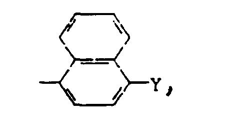 in which Y has the abovementioned meaning,
A and B are identical or different and represent hydrogen, or represent optionally halogen-substituted straight-chain or branched C₁-C₁₂-alkyl, C₃-C₈-alkenyl, C₃-C₈-alkinyl, C₁-C₁₀-alkoxy-C₂-C₈-alkyl, C₁-C₈-polyalkoxy-C₂-C₈-alkyl, C₁-C₁₀-alkylthio-C₂-C₈-alkyl or cycloalkyl which has 3 to 8 ring atoms and which can be interrupted by oxygen and/or sulphur, or represent phenyl or phenyl-C₁-C₆-alkyl, each of which is optionally substituted by halogen, C₁-C₆-alkyl, C₁-C₆-halogenoalkyl, C₁-C₆-alkoxy, C₁-C₆-halogenoalkoxy or nitro,
or
A and B together with the carbon atom to which they are bonded form a saturated or unsaturated 3-to 8-membered ring which is optionally interrupted by oxygen and/or sulphur and optionally substituted by halogen, C₁-C₆-alkyl, C₁-C₆-alkoxy, C₁-C₄-halogenoalkyl, C₁-C₄-halogenoalkoxy, C₁-C₄-alkylthio or optionally substituted phenyl or is optionally benzofused,
D represents oxygen or sulphur,
G represents hydrogen (a) or the groups 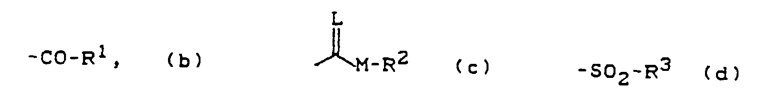 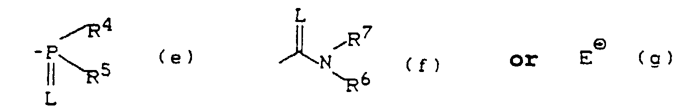
in which
E^{⊕} represents a metal ion equivalent or an ammonium ion,
L and M represent oxygen and/or sulphur,
R¹ represents optionally halogen-substituted C₁-C₂₀-alkyl, C₂-C₂₀-alkenyl, C₁-C₈-alkoxy-C₂-C₈-alkyl, C₁-C₈-alkylthio-C₂-C₈-alkyl, C₁-C₈-polyalkoxy-C₂-C₈-alkyl or cycloalkyl which has 3-8 ring atoms and which can be interrupted by oxygen and/or sulphur atoms,
or represents phenyl which is optionally substituted by halogen, nitro, C₁-C₆-alkyl, C₁-C₆-alkoxy, C₁-C₆-halogenoalkyl or C₁-C₆-halogenoalkoxy;
or represents phenyl-C₁-C₆-alkyl which is optionally substituted by halogen, C₁-C₆-alkyl, C₁-C₆-alkoxy, C₁-C₉-halogenoalkyl or C₁-C₆-halogenoalkoxy,
or represents pyridyl, pyrimidyl, thiazolyl and pyrazolyl, each of which is optionally substituted by halogen and/or C₁-C₆-alkyl,
or represents phenoxy-C₁-C₆-alkyl which is optionally substituted by halogen and/or C₁-C₆-alkyl,
R² represents C₁-C₂₀-alkyl, C₂-C₂₀-alkenyl, C₁-C₈-alkoxy-C₂-C₈-alkyl or C₁-C₈-polyalkoxy-C₂-C₈-alkyl each of which is optionally substituted by halogen,
or represents phenyl or benzyl, each of which is optionally substituted by halogen, nitro, C₁-C₆-alkyl, C₁-C₆-alkoxy or C₁-C₆-halogenoalkyl,
R³, R⁴ and R⁵ independently of one another represent C₁-C₈-alkyl, C₁-C₈-alkoxy, C₁-C₈-alkylamino, di(C₁-C₈)-alkylamino, C₁-C₈-alkylthio, C₂-C₅-alkenylthio, C₂-C₅-alkinylthio or C₃-C₇-cycloalkylthio, each of which is optionally substituted by halogen, or represent phenyl, phenoxy or phenylthio, each of which is optionally substituted by halogen, nitro, cyano, C₁-C₄-alkoxy, C₁-C₄-halogenoalkoxy, C₁-C₄-alkylthio, C₁-C₄-halogenoalkylthio, C₁-C₄-alkyl or C₁-C₄-halogenoalkyl,
R⁶ and R⁷ independently of one another represent C₁-C₂₀-alkyl, C₁-C₂₀-alkoxy, C₂-C₈-alkenyl or C₁-C₂₀-alkoxy-C₁-C₂₀-alkyl, each of which is optionally substituted by halogen, or represent phenyl which is optionally substituted by halogen, C₁-C₂₀-halogenoalkyl, C₁-C₂₀-alkyl or C₁-C₂₀-alkoxy, or represent benzyl which is optionally substituted by halogen, C₁-C₂₀-alkyl, C₁-C₂₀-halogenoalkyl or C₁-C₂₀-alkoxy, or together represent a C₂-C₆-alkylene ring which is optionally interrupted by oxygen,
with the exception of the following compounds:
3-(2-methoxyphenyl)-4-hydroxy-Δ³-dihydrofuran-2-one,
3-(2-chlorophenyl)-4-hydroxy-Δ³-dihydrofuran-2-one,
3-(2-fluorophenyl)-4-hydroxy-Δ³-dihydrofuran-2-one,
and the enantiomerically pure forms of compounds of the formula (I).

2. Insecticidal and acaricidal compositions, characterised in that they comprise at least one 3-aryl-4-hydroxy-Δ³-dihydrofuranone or 3-aryl-4-hydroxy-Δ³-dihydrothiophenone derivative of the formula (I) according to Claim 1,
in which
X represents C₄-C₄-alkyl, halogen, C₁-C₄-alkoxy or C₁-C₂-halogenoalkyl,
Y represents hydrogen, C₁-C₆-alkyl, halogen, C₁-C₄-alkoxy or C₁-C₂-halogenoalkyl,
Z represents C₁-C₄-alkyl, halogen or C₁-C₄-alkoxy,
n represents a number from 0 to 3,
or the radicals X and Z together with the phenyl radical to which they are bonded form the naphthalene radical of the formula 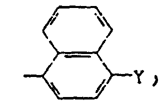 in which Y has the abovementioned meaning,
A and B are identical or different and represent hydrogen, or represent optionally halogen-substituted straight-chain or branched C₁-C₁₀-alkyl, C₃-C₆-alkenyl, C₃-C₆-alkinyl, C₁-C₈-alkoxy-C₂-C₆-alkyl,C₁-C₆-polyalkoxy-C₂-C₆-alkyl, C₁-C₈-alkylthio-C₂-C₆-alkyl or cycloalkyl which has 3 to 7 ring atoms and which can be interrupted by 1-2 oxygen and/or sulphur atoms, or represent phenyl or phenyl-C₁-C₄-alkyl, each of which is optionally substituted by halogen, C₁-C₄-alkyl, C₁-C₄-halogenoalkyl, C₁-C₄-alkoxy or nitro,
or
A and B together with the carbon atom to which they are bonded form a saturated or unsaturated 3-to 8-membered ring which is optionally interrupted by oxygen and/or sulphur and optionally substituted by halogen, C₁-C₅-alkyl, C₁-C₅-alkoxy, C₁-C₃-halogenoalkyl, C₁-C₄-halogenoalkoxy, C₁-C₃-alkylthio or optionally halogen-, alkyl- or alkoxy-substituted phenyl or is optionally benzo-fused,
D represents oxygen or sulphur,
G represents hydrogen (a) or the groups 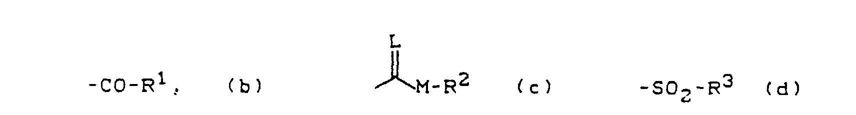 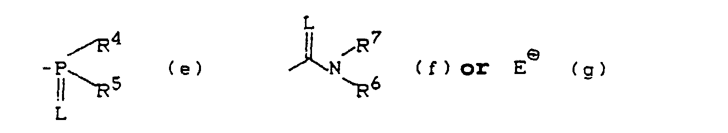
in which
E^{⊕} represents a metal ion equivalent or an ammonium ion,
L and M represent oxygen and/or sulphur,
R¹ represents optionally halogen-substituted C₁-C₁₆-alkyl, C₂-C₁₆-alkenyl, C₁-C₆-alkoxy-C₂-C₆-alkyl, C₁-C₁₆-alkylthio-C₂-C₆-alkyl, C₁-C₆-polyalkoxy-C₂-C₆-alkyl and cycloalkyl which has 3-7 ring atoms and which can be interrupted by 1-2 oxygen and/or sulphur atoms,
or represents phenyl which is optionally substituted by halogen, nitro, C₁-C₄-alkyl, C₁-C₄-alkoxy, C₁-C₃-halogenoalkyl or C₁-C₃-halogenoalkoxy,
or represents phenyl-C₁-C₄-alkyl which is optionally substituted by halogen, C₁-C₄-alkyl, C₁-C₄-alkoxy, C₁-C₃-halogenoalkyl or C₁-C₃-halogenoalkoxy,
or represents pyridyl, pyrimidyl, thiazolyl and pyrazolyl, each of which is optionally substituted by halogen and C₁-C₆-alkyl,
or represents phenoxy-C₁-C₅-alkyl which is optionally substituted by halogen and C₁-C₄-alkyl,
R² represents optionally halogen-substituted C₁-C₁₆-alkyl, C₂-C₁₆-alkenyl, C₁-C₁₆-alkoxy-C₂-C₆-alkyl or C₁-C₆-polyalkoxy-C₂-C₆-alkyl,
or represents phenyl or benzyl, each of which is optionally substituted by halogen, nitro, C₁-C₄-alkyl, C₁-C₃-alkoxy or C₁-C₃-halogenoalkyl,
R³, R⁴ and R⁵ independently of one another represent C₁-C₆-alkyl, C₁-C₆-alkoxy, C₁-C₆-alkylamino, di-(C₁-C₆) -alkylamino, C₁-C₆-alkylthio, C₃-C₄-alkenylthio, C₂-C₄-alkinylthio or C₃-C₆-cycloalkylthio, each of which is ptionally substituted by halogen, or represent phenyl, phenoxy or phenylthio, each of which is optionally substituted by fluorine, chlorine, bromine, nitro, cyano, C₁-C₃-alkoxy, C₁-C₃-halogenoalkoxy, C₁-C₃-alkylthio, C₁-C₃-halogenoalkylthio, C₁-C₃-alkyl or C₁-C₃-halogenoalkyl,
R⁶ and R⁷ independently of one another represent C₁-C₂₀-alkyl, C₁-C₂₀-alkoxy, C₂-C₈-alkenyl or C₁-C₂₀-alkoxy-C₁-C₂₀-alkyl, each of which is optionally substituted by halogen, or represent phenyl which is optionally substituted by halogen, C₁-C₅-halogenoalkyl, C₁-C₅-alkyl or C₁-C₅-alkoxy, or represent benzyl which is optionally substituted by halogen, C₁-C₅-alkyl, C₁-C₅-halogenoalkyl or C₁-C₅-alkoxy,
with the exception of the following compounds:
3-(2-methoxyphenyl)-4-hydroxy-Δ³-dihydrofuran-2-one,
3-(2-chlorophenyl)-4-hydroxy-Δ³-dihydrofuran-2-one,
3-(2-fluorophenyl)-4-hydroxy-Δ³-dihydrofuran-2-one,
and the enantiomerically pure forms of compounds of the formula (I).

3. Insecticidal and acaricidal compositions, characterised in that they comprise at least one 3-aryl-4-hydroxy-Δ³-dihydrofuranone or 3-aryl-4-hydroxy-Δ³-dihydrothiophenone derivative of the formula (I) according to Claim 1,
in which
X represents methyl, ethyl, propyl, i-propyl, fluorine, chlorine, bromine, methoxy, ethoxy and trifluoromethyl,
Y represents hydrogen, methyl, ethyl, propyl, i-propyl, butyl, i-butyl, tert-butyl, fluorine, chlorine, bromine, methoxy, ethoxy and trifluoromethyl,
Z represents methyl, ethyl, i-propyl, butyl, i-butyl, tert-butyl, fluorine, chlorine, bromine, methoxy and ethoxy,
n represents a number from 0 to 3,
or the radicals X and Z together with the phenyl radical to which they are bonded form the napthalene radical of the formula 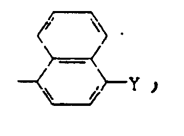 in which Y has the abovementioned meaning,
A and B are identical or different and represent hydrogen or represent optionally halogen-substituted straight-chain or branched C₁-C₈-alkyl, C₃-C₄-alkenyl, C₃-C₄-alkinyl, C₁-C₆-alkoxy-C₂-C-₄-alkyl, C₁-C₄-polyalkoxy-C₂-C₄-alkyl, C₁-C₆-alkylthio-C₂-C₄-alkyl or cycloalkyl which has 3 to 6 ring atoms and which can be interrupted by 1-2 oxygen and/or sulphur atoms, or represent phenyl or phenyl-C₁-C₃-alkyl, each of which is optionally substituted by fluorine, chlorine, bromine, methyl, ethyl, propyl, iso-propyl, methoxy, ethoxy, trifluoromethyl or nitro,
or
A and B together with the carbon atom to which they are bonded form a saturated or unsaturated 3-to 8-membered ring which is optionally interrupted by oxygen and/or sulphur and optionally substituted by fluorine, chlorine, C₁-C₄-alkyl, C₁-C₄-alkoxy, trifluoromethyl, C₁-C₂-alkylthio or optionally fluorine-, chlorine-, methyl- or methoxy-substituted phenyl or is optionally benzo-fused,
D represents oxygen or sulphur,
G represents hydrogen (a) or the groups 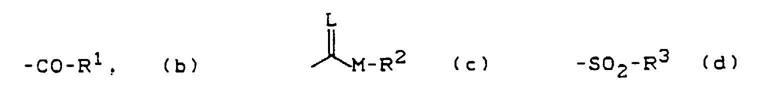 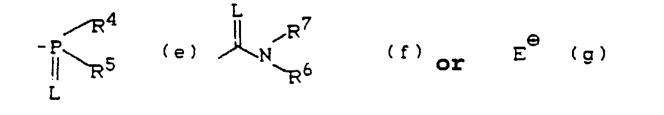
in which
E^{⊕} represents a metal ion equivalent or an ammonium ion,
L and M represent oxygen and/or sulphur,
R¹ represents optionally fluorine- or chlorine-substituted C₁-C₁₄-alkyl, C₂-C₁₄alkenyl, C₁-C₄-alkoxy-C₂-C₆-alkyl, C₁-C₄-alkylthio-C₂-C₆-alkyl, C₁-C₄-polyalkoxy-C₂-C₄-alkyl and cycloalkyl which has 3-6 ring atoms and which can be interrupted by 1-2 oxygen and/or sulphur atoms,
or represents phenyl which is optionally substituted by fluorine, chlorine, bromine, methyl, ethyl, propyl, i-propyl, methoxy, ethoxy, trifluoromethyl, trifluoromethoxy or nitro,
or represents phenyl-C₁-C₃-alkyl which is optionally substituted by fluorine, chlorine, bromine, methyl, ethyl, propyl, i-propyl, methoxy, ethoxy, trifluoromethyl or trifluoromethoxy,
or represents pyridyl, pyrimidyl, thiazolyl and pyrazolyl, each of which is optionally substituted by fluorine, chlorine, bromine, methyl or ethyl,
or represents phenoxy-C₁-C₄-alkyl which is optionally substituted by fluorine, chlorine, methyl or ethyl,
R² represents C₁-C₁₄-alkyl, C₂-C₁₄-alkenyl, C₁-C₄-alkoxy-C₂-C₆-alkyl or C₁-C₄-polyalkoxy-C₂-C₆-alkyl, each of which is optionally substituted by fluorine or chlorine,
or represents phenyl or benzyl, each of which is optionally substituted by fluorine, chlorine, nitro, methyl, ethyl, propyl, i-propyl, methoxy, ethoxy or trifluoromethyl,
R³, R⁴ and R⁵ independently of one another represent C₁-C₄-alkyl, C₁-C₄-alkoxy, C₁-C₄-alkylamino, di-(C₁-C₄-alkyl)-amino or C₁-C₄-alkylthio, each of which is optionally substituted by fluorine or chlorine, or represent phenyl, phenoxy or phenylthio, each of which is optionally substituted by fluorine, chlorine, bromine, nitro, cyano, C₁-C₂-alkoxy, C₁-C₄-fluoroalkoxy, C₁-C₂-chloroalkoxy, C₁-C₂-alkylthio, C₁-C₂-fluoroalkylthio, C₁-C₂-chloroalkylthio or C₁-C₃-alkyl,
R⁶ and R⁷ independently of one another represent C₁-C₁₀-alkyl, C₁-C₁₀-alkoxy or C₁-C₁₀-alkoxy(C₁-C₁₀)-alkyl, each of which it optionally substituted by fluorine, chlorine or bromine, or represent phenyl which is optionally substituted by fluorine, chlorine, bromine, C₁-C₂₀-halogenoalkyl, C₁-C₂₀-alkyl or C₁-C₄-alkoxy, or represent benzyl which is optionally substituted by fluorine, chlorine, bromine, C₁-C₄-alkyl, C₁-C₄-halogenoalkyl or C₁-C₄-alkoxy,
with the exception of the following compounds:
3-(2-methoxyphenyl)-4-hydroxy-Δ³-dihydrofuran-2-one,
3-(2-chlorophenyl)-4-hydroxy-Δ³-dihydrofuran-2-one,
3-(2-fluorophenyl)-4-hydroxy-Δ³-dihydrofuran-2-one,
and the enantiomerically pure forms of compounds of the formula (I).

4. Method of combating insects and/or arachnids, characterised in that 3-aryl-4-hydroxy-Δ³-dihydrofuranone or 3-aryl-4-hydroxy-Δ³-dihydrothiophenone derivatives of the formula (I) according to Claim 1 are allowed to act on insects and/or arachnids and/or their environment.

5. Use of 3-aryl-4-hydroxy-Δ³-dihydrofuranone or 3-aryl-4-hydroxy-Δ³-dihydrothiophenone derivatives of the formula (I) according to Claim 1 for combating insects and/or arachnids.

6. Process for the preparation of insecticidal and/or acaricidal agents, characterised in that 3-aryl-4-hydroxy-Δ³-dihydrofuranone or 3-aryl-4-hydroxy-Δ³-dihydrothiophenone derivatives of the formula (I) according to Claim 1 are mixed with extenders and/or surface-active agents.

7. 3-Aryl-4-hydroxy-Δ³-dihydrofuranone and 3-aryl-4-hydroxy-Δ³-dihydrothiophenone derivatives of the general formula (I) 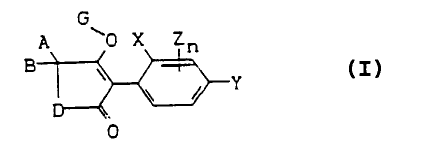 in which
X represents C₁-C₆-alkyl, halogen, C₁-C₆-alkoxy or C₁-C₃-halogenoalkyl,
Y represents hydrogen, C₁-C₆-alkyl, halogen, C₁-C₆-alkoxy or C₁-C₃-halogenoalkyl,
Z represents C₁-C₆-alkyl, halogen or C₁-C₆-alkoxy,
n represents a number from 0 to 3,
or the radicals X and Z together with the phenyl radical to which they are bonded form the naphthalene radical of the formula 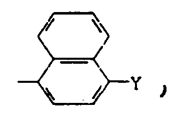 in which Y has the abovementioned meaning,
A and B are identical or different and represent hydrogen, or represent optionally halogen-substituted straight-chain or branched C₁-C₁₂-alkyl, C₃-C₈-alkenyl, C₃-C₈-alkinyl, C₁-C₁₀-alkoxy-C₂-C₈-alkyl,C₁-C₈-polyalkoxy-C₂-C₈-alkyl, C₁-C₁₀-alkylthio-C₂-C₈-alkyl or cycloalkyl which has 3 to 8 ring atoms and which can be interrupted by oxygen and/or sulphur, or represent phenyl or phenyl-C₁-C₆-alkyl, each of which is optionally substituted by halogen, C₁-C₆-alkyl, C₁-C₆-halogenoalkyl, C₁-C₆-alkoxy, C₁-C₆-halogenoalkoxy or nitro,
or
A and B together with the carbon atom to which they are bonded form a saturated or unsaturated 3- to 8-membered ring which is optionally interrupted by oxygen and/or sulphur and optionally substituted by halogen, C₁-C₆-alkyl, C₁-C₆-alkoxy, C₁-C₄-halogenoalkyl, C₁-C₄-halogenoalkoxy, C₁-C₄-alkylthio or optionally substituted phenyl or is optionally benzo-fused,
D represents oxygen or sulphur,
G represents hydrogen (a) or the groups 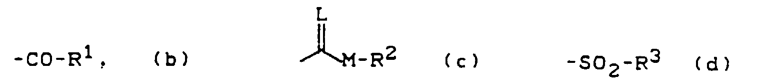 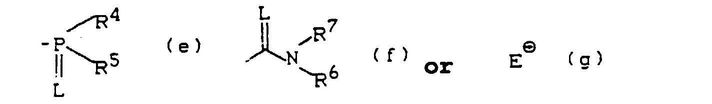
in which
E^{⊕} represents a metal ion equivalent or an ammonium ion,
L and M represent oxygen and/or sulphur,
R¹ represents optionally halogen-substituted C₁-C₂₀-alkyl, C₂-C₂₀-alkenyl, C₁-C₈-alkoxy-C₂-C₈-alkyl, C₁-C₈-alkylthio-C₂-C₈-alkyl, C₁-C₈-polyalkoxy-C₂-C₈-alkyl or cycloalkyl which has 3-8 ring atoms and which can be interrupted by oxygen and/or sulphur atoms,
or represents phenyl which is optionally substituted by halogen, nitro, C₁-C₆-alkyl, C₁-C₆-alkoxy, C₁-C₆-halogenoalkyl or C₁-C₆-halogenoalkoxy;
or represents phenyl-C₁-C₆-alkyl which is optionally substituted by halogen, C₁-C₆-alkyl, C₁-C₆-alkoxy, C₁-C₆-halogenoalkyl or C₁-C₆-halogenoalkoxy,
or represents pyridyl, pyrimidyl, thiazolyl and pyrazolyl, each of which is optionally substituted by halogen and/or C₁-C₆-alkyl,
or represents phenoxy-C₁-C₆-alkyl which is optionally substituted by halogen and/or C₁-C₆-alkyl,
R² represents C₁-C₂₀-alkyl, C₂-C₂₀-alkenyl, C₁-C₈-alkoxy-C₂-C₈-alkyl or C₁-C₈-polyalkoxy-C₂-C₈-alkyl, each of which is optionally substituted by halogen,
or represents phenyl or benzyl, each of which is optionally substituted by halogen, nitro, C₁-C₆-alkyl, C₁-C₆-alkoxy or C₁-C₆-halogenoalkyl,
R³, R⁴ and R⁵ independently of one another represent C₁-C₈-alkyl, C₁-C₈-alkoxy, C₁-C₈-alkylamino, di(C₁-C₈) -alkylamino, C₁-C₈-alkylthio, C₂-C₅-alkenylthio, C₂-C₅-alkinylthio or C₃-C₇-cycloalkylthio, each of which is optionally substituted by halogen, or represent phenyl, phenoxy or phenylthio, each of which is optionally substituted by halogen, nitro, cyano, C₁-C₄-alkoxy, C₁-C₄-halogenoalkoxy, C₁-C₄-alkylthio, C₁-C₄-halogenoalkylthio) C₁-C₄-alkyl or C₁-C₄-halogenoalkyl,
R⁶ and R⁷ independently of one another represent C₁-C₂₀-alkyl, C₁-C₂₀-alkoxy, C₂-C₈-alkenyl or C₁-C₂₀-alkoxy-C₁-C₂₀-alkyl, each of which is optionally substituted by halogen, or represent phenyl which is optionally substituted by halogen, C₁-C₂₀-halogenoalkyl, C₁-C₂₀-alkyl or C₁-C₂₀-alkoxy, or represent benzyl which is optionally substituted by halogen, C₁-C₂₀-alkyl, C₁-C₂₀-halogenoalkyl or C₁-C₂₀-alkoxy, or together represent a C₂-C₆-alkylene ring which is optionally interrupted by oxygen,
and the enantiomerically pure forms of compounds of the formula (I),
with the exception of the following compounds:
3-(2-methoxyphenyl)-4-hydroxy-Δ³-dihydrofuran-2-one,
3-(2-chlorophenyl)-4-hydroxy-Δ³-dihydrofuran-2-one,
3-(2-fluorophenyl)-4-hydroxy-Δ³-dihydrofuran-2-one,
and with the proviso that X does not represent halogen or halogenomethyl and 1 to 3 identical or different halogen atoms and simultaneously Y does not represent hydrogen and simultaneously n does not represent the number 0 when D represents oxygen and simultaneously R¹ represents alkylcarbonyl or halogenoalkylcarbonyl and simultaneously A and B independently of one another represent hydrogen, alkyl, or represent phenyl or phenylalkyl, each of which is unsubstituted or mono- or polysubstituted by identical or different substituents, where the following are selected as substituents in the phenyl moiety: nitro, halogen, alkyl, alkoxy, halogenoalkyl or halogenoalkoxy.

8. A 3 Aryl-4-hydroxy-Δ³-dihydrofuranone and 3-aryl-4-hydroxy-Δ³-dihydrothiophenone derivative of the formula (I) according to claim 7, characterized in that it has one of the following structures (Ia) to (Ig): 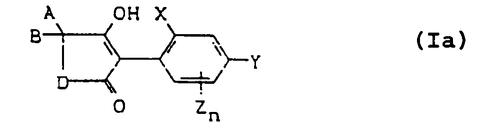 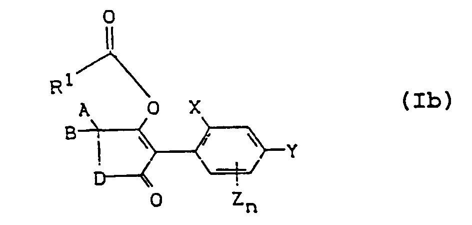 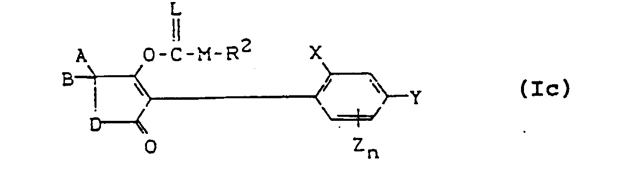 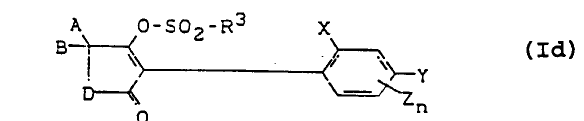 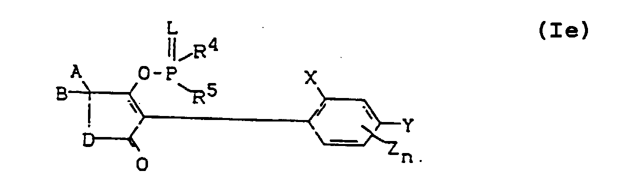 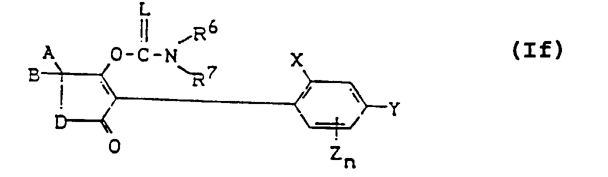 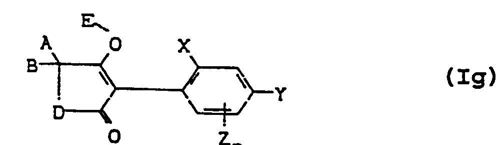 in which
A, B, D, E, L, M, X, Y, Zₙ, R¹, R², R³, R⁴, R⁵, R⁶ and R⁷ have the meanings given in Claim 7.

9. 3-Aryl-4-hydroxy-Δ³-dihydrofuranone and 3-aryl-4-hydroxy-Δ³-dihydrothiophenone derivatives of the formula (I) according to Claim 7,
in which
X represents C₁-C₄-alkyl, halogen, C₁-C₄-alkoxy or C₁-C₂-halogenoalkyl,
Y represents hydrogen, C₁-C₆-alkyl, halogen, C₁-C₄-alkoxy or C₁-C₂-halogenoalkyl,
Z represents C₁-C₄-alkyl, halogen or C₁-C₄-alkoxy,
n represents a number from 0 to 3,
or the radicals X and Z together with the phenyl radical to which they are bonded form the naphthalene radical of the formula 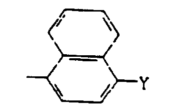 in which Y has the abovementioned meaning,
A and B are identical or different and represent hydrogen, or represent optionally halogen-substituted straight-chain or branched C₁-C₁₀-alkyl, C₃-C₆-alkenyl, C₃-C₆-alkinyl, C₁-C₈-alkoxy-C₂-C₆-alkyl, C₁-C₆-polyalkoxy-C₂-C₆-alkyl, C₁-C₈-alkylthio-C₂-C₆-alkyl or cycloalkyl which has 3 to 7 ring atoms and which can be interrupted by 1-2 oxygen and/or sulphur atoms, or represent phenyl or phenyl-C₁-C₄-alkyl, each of which is optionally substituted by halogen, C₁-C₄-alkyl, C₁-C₄-halogenoalkyl, C₁-C₄-alkoxy or nitro,
or
A and B together with the carbon atom to which they are bonded form a saturated or unsaturated 3-to 8-membered ring which is optionally interrupted by oxygen and/or sulphur and optionally substituted by halogen, C₁-C₅-alkyl, C₁-C₅-alkoxy, C₁-C₃-halogenoalkyl, C₁-C₄-halogenoalkoxy, C₁-C₃-alkylthio or optionally halogen-, alkyl- or alkoxy-substituted phenyl or is optionally benzo-fused,
D represents oxygen or sulphur,
G represents hydrogen (a) or the groups 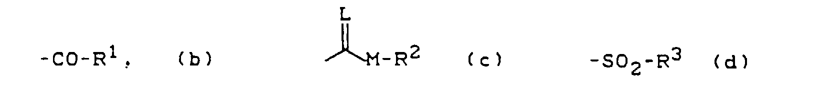 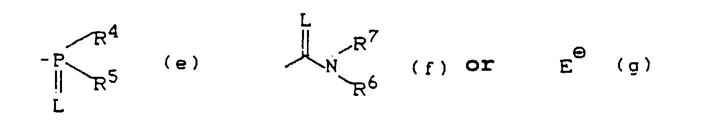
in which
E^{⊕} represents a metal ion equivalent or an ammonium ion,
L and M represent oxygen and/or sulphur,
R¹ represents optionally halogen-substituted C₁-C₁₆-alkyl, C₂-C₁₆-alkenyl, C₁-C₆-alkoxy-C₂-C₆-alkyl, C₁-C₁₆-alkylthio-C₂-C₆-alkyl, C₁-C₆-polyalkoxy-C₂-C₆-alkyl and cycloalkyl which has 3-7 ring atoms and which can be interrupted by 1-2 oxygen and/or sulphur atoms,
or represents phenyl which is optionally substituted by halogen, nitro, C₁-C₄-alkyl, C₁-C₄-alkoxy, C₁-C₃-halogenoalkyl or C₁-C₃-halogenoalkoxy,
or represents phenyl-C₁-C₄-alkyl which is optionally substituted by halogen, C₁-C₄-alkyl, C₁-C₄-alkoxy, C₁-C₃-halogenoalkyl or C₁-C₃-halogenoalkoxy,
or represents pyridyl, pyrimidyl, thiazolyl and pyrazolyl, each of which is optionally substituted by halogen and C₁-C₆-alkyl,
or represents phenoxy-C₁-C₅-alkyl which is optionally substituted by halogen and C₁-C₄-alkyl,
R² represents optionally halogen-substituted C₁-C₁₆-alkyl, C₂-C₁₆-alkenyl, C₁-C₁₆-alkoxy-C₂-C₆-alkyl or C₁-C₆-polyalkoxy-C₂-C₆-alkyl,
or represents phenyl or benzyl, each of which is optionally substituted by halogen, nitro, C₁-C₄-alkyl, C₁-C₃-alkoxy or C₁-C₃-halogenoalkyl,
R³, R⁴ and R⁵ independently of one another represent C₁-C₆-alkyl, C₁-C₆-alkoxy, C₁-C₆-alkylamino, di(C₁-C₆)-alkylamino, C₁-C₆-alkylthio, C₃-C₄-alkenylthio, C₂-C₄-alkinylthio or C₃-C₆-cycloalkylthio, each of which is optionally substituted by halogen, or represent phenyl, phenoxy or phenylthio, each of which is optionally substituted by fluorine, chlorine, bromine, nitro, cyano, C₁-C₃-alkoxy, C₁-C₃-halogenoalkoxy, C₁-C₃-alkylthio, C₁-C₃-halogenoalkylthio, C₁-C₃-alkyl or C₁-C₃-halogenoalkyl,
R⁶ and R⁷ independently of one another represent C₁-C₂₀-alkyl, C₁-C₂₀-alkoxy, C₂-C₈-alkenyl or C₁-C₂₀-alkoxy-C₁-C₂₀-alkyl, each of which is optionally substituted by halogen, or represent phenyl which is optionally substituted by halogen, C₁-C₅-halogenoalkyl, C₁-C₅-alkyl or C₁-C₅-alkoxy, or represent benzyl which is optionally substituted by halogen, C₁-C₅-alkyl, C₁-C₅-halogenoalkyl or C₁-C₅-alkoxy,
and the enantiomerically pure forms of compounds of the formula (I),
with the exception of the following compounds:
3-(2-methoxyphenyl)-4-hydroxy-Δ³-dihydrofuran-2-one,
3-(2-chlorophenyl)-4-hydroxy-Δ³-dihydrofuran-2-one,
3-(2-fluorophenyl)-4-hydroxy-Δ³-dihydrofuran-2-one,
and with the proviso that X does not represent halogen or halogenomethyl and 1 to 3 identical or different halogen atoms and simultaneously Y does not represent hydrogen and simultaneously n does not represent the number 0 when D represents oxygen and simultaneously R¹ represents alkylcarbonyl or halogenoalkylcarbonyl and simultaneously A and B independently of one another represent hydrogen, alkyl, or represent phenyl or phenylalkyl, each of which is unsubstituted or mono- or polysubstituted by identical or different substituents, where the following are selected as substituents in the phenyl moiety: nitro, halogen, alkyl, alkoxy, halogenoalkyl or halogenoalkoxy.

10. 3-Aryl-4-hydroxy-Δ³-dihydrofuranone and 3-aryl-4-hydroxy-Δ³-dihydrothiophenone derivatives of the formula (I) according to Claim 7,
in which
X represents methyl, ethyl, propyl, i-propyl, fluorine, chlorine, bromine, methoxy, ethoxy and trifluoromethyl,
Y represents hydrogen, methyl, ethyl, propyl, i-propyl, butyl, i-butyl, tert-butyl, fluorine, chlorine, bromine, methoxy, ethoxy and trifluoromethyl,
Z represents methyl, ethyl, i-propyl, butyl, i-butyl, tert-butyl, fluorine, chlorine, bromine, methoxy and ethoxy,
n represents a number from 0 to 3,
or the radicals X and Z together with the phenyl radical to which they are bonded form the napthalene radical of the formula 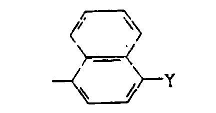 in which Y has the abovementioned meaning,
A and B are identical or different and represent hydrogen or represent optionally halogen-substituted straight-chain or branched C₁-C₈-alkyl, C₃-C₄-alkenyl, C₃-C₄-alkinyl, C₁-C₆-alkoxy-C₂-C₄-alkyl, C₁-C₄-polyalkoxy-C₂-C₄-alkyl, C₁-C₆-alkylthio-C₂-C₄-alkyl or cycloalkyl which has 3 to 6 ring atoms and which can be interrupted by 1-2 oxygen and/or sulphur atoms, or represent phenyl or phenyl-C₁-C₃-alkyl, each of which is optionally substituted by fluorine, chlorine, bromine, methyl, ethyl, propyl, iso-propyl, methoxy, ethoxy, trifluoromethyl or nitro,
or
A and B together with the carbon atom to which they are bonded form a saturated or unsaturated 3-to 8-membered ring which is optionally interrupted by oxygen and/or sulphur and optionally substituted by fluorine, chlorine, C₁-C₄-alkyl, C₁-C₄-alkoxy, trifluoromethyl, C₁-C₂-alkylthio or optionally fluorine-, chlorine-, methyl- or methoxy-substituted phenyl or is optionally benzo-fused,
D represents oxygen or sulphur,
G represents hydrogen (a) or the groups 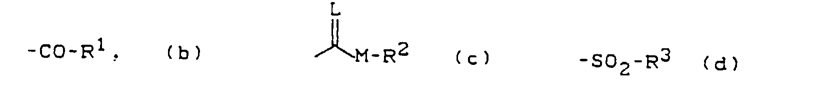 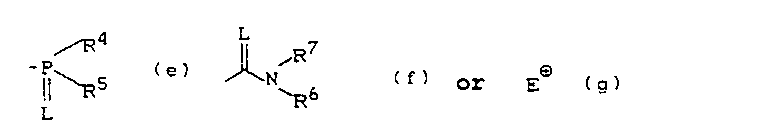
in which
E^{⊕} represents a metal ion equivalent or an ammonium ion,
L and M represent oxygen and/or sulphur,
R¹ represents optionally fluorine- or chlorine-substituted C₁-C₁₄-alkyl, C₂-C₁₄-alkenyl, C₁-C₄-alkoxy-C₂-C₆-alkyl, C₁-C₄-alkylthio-C₂-C₆-alkyl, C₁-C₄-polyalkoxy-C₂-C₄-alkyl and cycloalkyl which has 3-6 ring atoms and which can be interrupted by 1-2 oxygen and/or sulphur atoms,
or represents phenyl which is optionally substituted by fluorine, chlorine, bromine, methyl, ethyl, propyl, i-propyl, methoxy, ethoxy, trifluoromethyl, trifluoromethoxy or nitro,
or represents phenyl-C₁-C₃-alkyl which is optionally substituted by fluorine, chlorine, bromine, methyl, ethyl, propyl, i-propyl, methoxy, ethoxy, trifluoromethyl or trifluoromethoxy,
or represents pyridyl, pyrimidyl, thiazolyl and pyrazolyl, each of which is optionally substituted by fluorine, chlorine, bromine, methyl or ethyl,
or represents phenoxy-C₁-C₄-alkyl which is optionally substituted by fluorine, chlorine, methyl or ethyl,
R² represents C₁-C₁₄-alkyl, C₂-C₁₄-alkenyl, C₁-C₄-alkoxy-C₂-C₆-alkyl or C₁-C₄-polyalkoxy-C₂-C₆-alkyl, each of which is optionally substituted by fluorine or chlorine,
or represents phenyl or benzyl, each of which is optionally substituted by fluorine, chlorine, nitro, methyl, ethyl, propyl, i-propyl, methoxy, ethoxy or trifluoromethyl,
R³, R⁴ and R⁵ independently of one another represent C₁-C₄-alkyl, C₁-C₄-alkoxy, C₁-C₄-alkylamino, di(C₁-C₄-alkyl)-amino or C₁-C₄-alkylthio, each of which is optionally substituted by fluorine or chlorine, or represent phenyl, phenoxy or phenylthio, each of which is optionally substituted by fluorine, chlorine, bromine, nitro, cyano, C₁-C₂-alkoxy, C₁-C₄-fluoroalkoxy, C₁-C₂-chloroalkoxy, C₁-C₂-alkylthio, C₁-C₂-fluoroalkylthio, C₁-C₂-chloroalkylthio or C₁-C₃-alkyl,
R⁶ and R⁷ independently of one another represent C₁-C₁₀-alkyl, C₁-C₁₀-alkoxy or C₁-C₁₀-alkoxy-(C₁-C₁₀)-alkyl, each of which is optionally substituted by fluorine, chlorine or bromine, or represent phenyl which is optionally substituted by fluorine, chlorine, bromine, C₁-C₂₀-halogenoalkyl, C₁-C₂₀-alkyl or C₁-C₄-alkoxy, or represent benzyl which is optionally substituted by fluorine, chlorine, bromine, C₁-C₄-alkyl, C₁-C₄-halogenoalkyl or C₁-C₄-alkoxy,
and the enantiomerically pure forms of compounds of the formula (I),
with the exception of the following compounds:
3-(2-methoxyphenyl)-4-hydroxy-Δ³-dihydrofuran-2-one,
3-(2-chlorophenyl)-4-hydroxy-Δ³-dihydrofuran-2-one,
3-(2-fluorophenyl)-4-hydroxy-Δ³-dihydrofuran-2-one,
and with the proviso that X does not represent halogen or halogenomethyl and 1 to 3 identical or different halogen atoms and simultaneously Y does not represent hydrogen and simultaneously n does not represent the number 0 when D represents oxygen and simultaneously R¹ represents alkylcarbonyl or halogenoalkylcarbonyl and simultaneously A and B independently of one another represent hydrogen, alkyl, or represent phenyl or phenylalkyl, each of which is unsubstituted or mono- or polysubstituted by identical or different substituents, where the following are selected as substituents in the phenyl moiety: nitro, halogen, alkyl, alkoxy, halogenoalkyl or halogenoalkoxy.

11. Process for the preparation of 3-aryl-4-hydroxy-Δ³-dihydrofuranone and 3-aryl-4-hydroxy-Δ³-dihydrothiophenone derivatives of the general formula (I) according to Claim 7 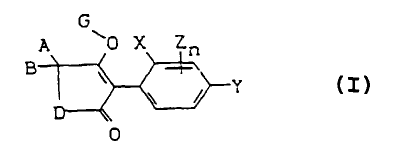 in which
A, B, D, G, X, Y, Z and n have the meaning mentioned in Claim 7,
characterized in that
to obtain 3-aryl-4-hydroxy-Δ³-dihydrofuranone and 3-aryl-4-hydroxy-Δ³-dihydrothiophenone derivatives of the formula (Ia) 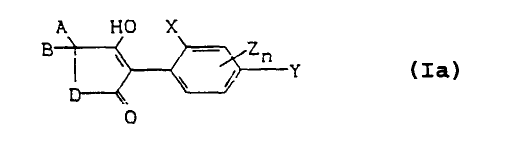 in which
A, B, D, X, Y, Z and n have the abovementioned meaning,
either
(A) carboxylic acid esters of the formula (II) 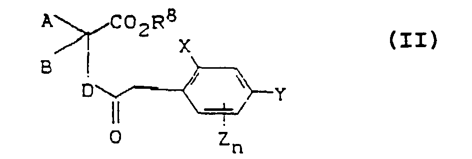 in which
A, B, D, X, Y, Z and n have the abovementioned meaning
and
R⁸ represents alkyl
are subjected to intramolecular condensation in the presence of a diluent and in the presence of a base,
or
(B) to obtain compounds of the formula (Ib) 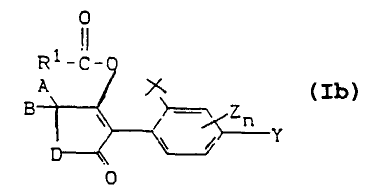 in which
A, B, D, X, Y, Z, R¹ and n have the abovementioned meaning,
compounds of the formula (Ia) 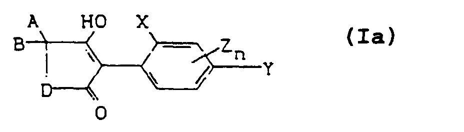 in which
A, B, D, X, Y, Z and n have the abovementioned meaning,
α) are reacted with acid halides of the general formula (III) 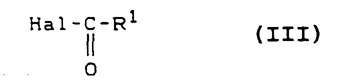 in which
R¹ has the abovementioned meaning
and
Hal represents halogen, in particular chlorine and bromine,
if appropriate in the presence of a diluent and if appropriate in the presence of an acid-binding agent
or
β) are reacted with carboxylic anhydrides of the general formula (IV)
R¹-CO-O-CO-R¹ (IV)
in which
R¹ has the abovementioned meaning,
if appropriate in the presence of a diluent and if appropriate in the presence of an acid-binding agent,
or in that
(C) to obtain compounds of the formula (Ic) 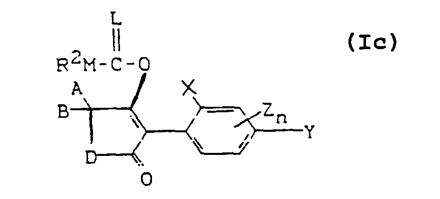 in which
A, B, D, X, Y, Z, R² and n have the abovementioned meaning,
L represents oxygen
and
M represents oxygen or sulphur,
compounds of the formula (Ia) 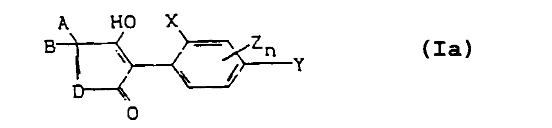 in which
A, B, D, X, Y, Z and n have the abovementioned meaning,
are reacted with chloroformic acid esters or chloroformic acid thioesters of the general formula (V)
R²-M-CO-Cl (V)
in which
R² and M have the abovementioned meaning,
if appropriate in the presence of a diluent and if appropriate in the presence of an acid-binding agent, or in that
D) to obtain compounds of the formula (Ic) 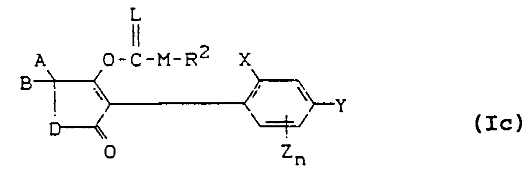 in which
A, B, D, R², X, Y, Z and n have the abovementioned meaning,
L represents sulphur
and
M represents oxygen or sulphur,
compounds of the formula (Ia) 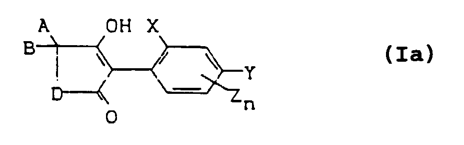 in which
A, B, D, X, Y, Z and n have the abovementioned meaning,
α) are reacted with chloromonothioformic acid esters or chlorodithioformic acid esters of the general formula (VI) 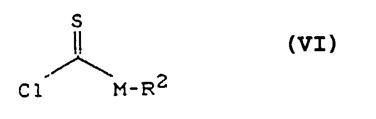 in which
M and R² have the abovementioned meaning,
if appropriate in the presence of a diluent and if appropriate in the presence of an acid-binding agent,
or
β) are reacted with carbon disulphide and subsequently with alkyl halides of the general formula (VII)
R²-Hal (VII)
in which
R² has the abovementioned meaning
and
Hal represents chlorine, bromine or iodine,
or in that
E) to obtain compounds of the formula (Id) 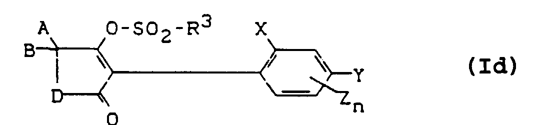 in which
A, B, D, X, Y, Z, R³ and n have the abovementioned meaning,
compounds of the formula (Ia) 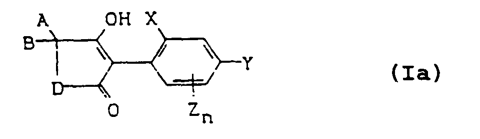 in which
A, B, D, X, Y, Z and n have the abovementioned meaning,
are reacted with sulphonyl chlorides of the general formula (VIII)
R³-SO₂-Cl (VIII)
in which
R³ has the abovementioned meaning,
if appropriate in the presence of a diluent and if appropriate in the presence of an acid-binding agent,
or in that
(F) to obtain compounds of the formula (Ie) 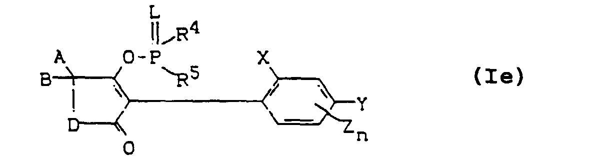 in which
A, B, D, L, X, Y, Z, R⁴, R⁵ and n have the abovementioned meaning,
compounds of the formula (Ia) 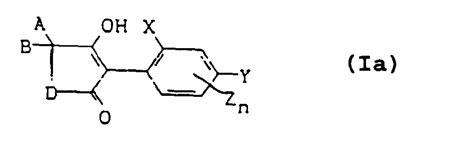 in which
A, B, D, X, Y, Z and n have the abovementioned meaning,
are reacted with phosphorus compounds of the general formula (IX) 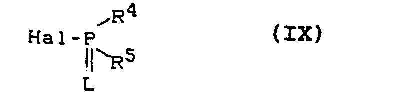 in which
L, R⁴ and R⁵ have the abovementioned meaning
and
Hal represents halogen,
if appropriate in the presence of a diluent and if appropriate in the presence of an acid-binding agent, or in that
(G) to obtain compounds of the formula (If) 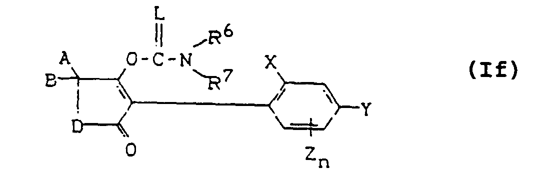 in which
A, B, D, L, X, Y, Z, R⁶, R⁷ and n have the abovementioned meaning,
compounds of the formula (Ia) 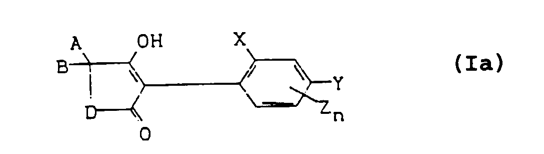 in which
A, B, D, X, Y, Z and n have the abovementioned meaning,
α) are reacted with isocyanates of the general formula (X)
R⁶-N=C=O (X)
in which
R⁶ has the abovementioned meaning,
if appropriate in the presence of a diluent and if appropriate in the presence of a catalyst,
or
β) are reacted with carbamoyl chlorides or thiocarbamoyl chlorides of the general formula (XI) 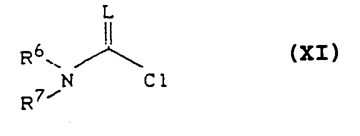 in which
L, R⁶ and R⁷ have the abovementioned meaning,
if appropriate in the presence of a diluent and if appropriate in the presence of an acid-binding agent,
or in that
(H) to obtain compounds of the formula (Ig) 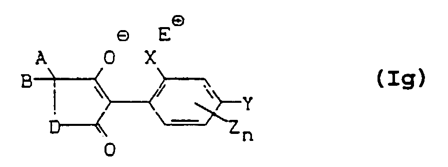 in which
X, Y, Z, A, B, D and n have the abovementioned meaning,
and E^{⊕} represents a metal ion equivalent or an ammonium ion,
compounds of the formula (Ia) 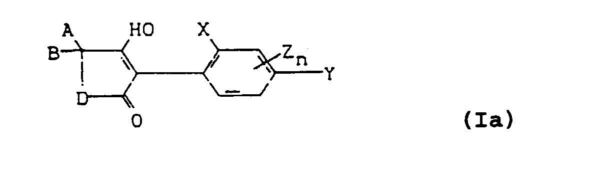 in which
X, Y, Z, A, B, D and n have the abovementioned meaning,
are reacted with metal hydroxides or amines of the general formulae (XII) and (XIII) 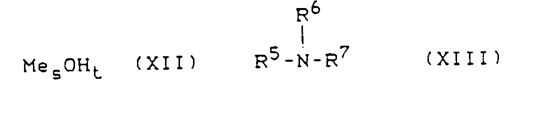 in which
Me represents mono- or divalent metal ions,
s and t represent the number 1 or 2 and
R⁵ , R⁶ and R⁷ independently of one another represent hydrogen and alkyl,
if appropriate in the presence of a diluent.

12. Herbicidal compositions, characterised in that they comprise at least one 3-aryl-4-hydroxy-Δ³-dihydrofuranone or 3-aryl-4-hydroxy-Δ³-dihydrothiophenone derivative of the formula (I) according to Claim 7.

13. Method of combating weeds, characterised in that 3-aryl-4-hydroxy-Δ³-dihydrofuranone or 3-aryl-4-hydroxy-Δ³-dihydrothiophenone derivatives of the formula (I) according to Claim 7 are allowed to act on weeds and/or their environment.

14. Use of 3-aryl-4-hydroxy-Δ³-dihydrofuranone or 3-aryl-4-hydroxy-Δ³-dihydrothiophenone derivatives of the formula (I) according to Claim 7 for combating weeds,

15. Process for the preparation of herbicidal agents, characterised in that 3-aryl-4-hydroxy-Δ³-dihydrofuranone or 3-aryl-4-hydroxy-Δ³-dihydrothiophenone derivatives of the formula (I) according to Claim 7 are mixed with extenders and/or surface-active agents.

## Revendications

1. Compositions insecticides et acaricides, caractérisées par une teneur en au moins un dérivé de 3-aryl-4-hydroxy-Δ³-dihydrofurannone ou de 3-aryl-4-hydroxy-Δ³-dihydrothiophénone de formule générale (I) 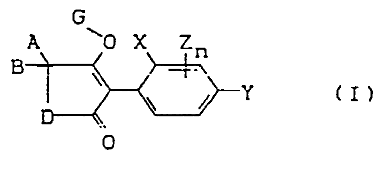 dans laquelle
X est un groupe alkyle en C₁ à C₆, un halogène, un groupe alkoxy en C₁ à C₆ ou un groupe halogénalkyle en C₁ à C₃,
Y est de l'hydrogène, un groupe alkyle en C₁ à C₆, un halogène, un groupe alkoxy en C₁ à C₆, halogénalkyle en C₁ à C₃,
Z est un groupe alkyle en C₁ à C₆, un halogène, un groupe alkoxy en C₁ à C₆,
n est un nombre de 0 à 3,
ou dans laquelle les restes X et Z forment, conjointement avec le reste phényle auquel ils sont liés, le reste naphtalène de formule 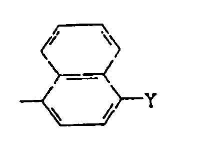 dans lequel Y a la définition indiquée ci-dessus,
A et B sont identiques ou différents et représentent de l'hydrogène ou un groupe, éventuellement substitué par un halogène, alkyle en C₁ à C₁₂ linéaire ou ramifié éventuellement substitué par un halogène, un groupe alcényle en C₃ à C₈, alcynyle en C₃ à C₈, (alkoxy en C₁ à C₁₀)(alkyle en C₂ à C₈), poly(alkoxy en C₁ à C₈)(alkyle en C₂ à C₈), (alkyltho en C₁ à C₁₀)(alkyle en C₂ à C₈), linéaire ou ramifié, cycloalkyle à noyau de 3 à 8 atomes, qui peut être interrompu par de l'oxygène et/ou par du soufre, et un groupe phényle ou phényl-(alkyle en C₁ à C₆) éventuellement substitué par un halogène, un radical alkyle en C₁ à C₆, halogénalkyle en C₁ à C₆, alkoxy en C₁ à C₆, halogénalkoxy en C₁ à C₆, nitro,
ou dans laquelle
A et B forment, conjointement avec l'atome de carbone auquel ils sont liés, un noyau de à 3 à 8 chaînons saturé ou non saturé, éventuellement interrompu par de l'oxygène et/ou du soufre et éventuellement substitué par un halogène, un radical alkyle en C₁ à C₆, alkoxy en C₁ à C₆, halogénalkyle en C₁ à C₄, halogénalkoxy en C₁ à C₄, alkylthio en C₁ à C₄ ou phényle éventuellement substitué, ou condensé, le cas échéant, à du benzène,
D est de l'oxygène ou du soufre,
G représente de l'hydrogène (a) ou les groupes 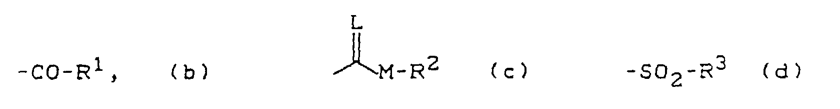 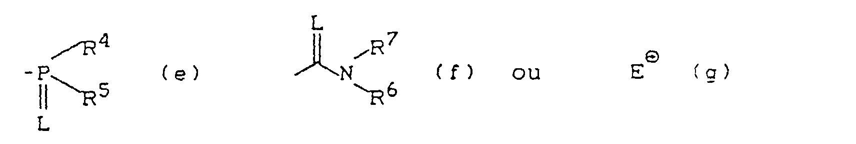
dans lesquels,
E^{⊕} représente un équivalent d'ion métallique ou un ion ammonium,
L et M représentent de l'oxygène et/ou du soufre,
R¹ est un groupe, éventuellement substitué par un halogène : alkyle en C₁ à C₂₀, alcényle en C₂ à C₂₀, (alkoxy en C₁ à C₈)-(alkyle en C₂ à C₈), (alkylthio en C₁ à C₈)-(alkyle en C₂ à C₈), poly(alkoxy en C₁ à C₈)-(alkyle en C₂ à C₈) ou cycloalkyle à noyau de 3 à 8 atomes, qui peut être interrompu par des atomes d'oxygène et/ou de soufre,
un groupe phényle éventuellement substitué par un radical halogéno, nitro, alkyle en C₁ à C₆, alkoxy en C₁ à C₆, halogénalkyle en C₁ à C₆, halogénalkoxy en C₁ à C₆,
un groupe phényl-(alkyle en C₁ à C₆) éventuellement substitué par un radical halogéno, alkyle en C₁ à C₆, alkoxy en C₁ à C₆, halogénalkyle en C₁ à C₆, halogénalkoxy en C₁ à C₆,
un groupe pyridyle, pyrimidyle, thiazolyle et pyrazolyle éventuellement substitué par un halogène et/ou un radical alkyle en C₁ à C₆,
un groupe phénoxy-(alkyle en C₁ à C₆) éventuellement substitué par un halogène et/ou un radical alkyle en C₁ à C₆,
R² est un groupe, éventuellement substitué par un halogène : alkyle en C₁ à C₂₀, alcényle en C₂ à C₂₀, (alkoxy en C₁ à C₈)-(alkyle en C₂ à C₈), poly(alkoxy en C₁ à C₈)-(alkyle en C₂ à C₈),
un groupe phényle ou benzyle éventuellement substitué par un radical halogéno, nitro, alkyle en C₁ à C₆, alkoxy en C₁ à C₆, halogénalkyle en C₁ à C₆,
R³, R⁴ et R⁵ représentent, indépendamment les uns des autres, un groupe, éventuellement substitué par un halogène, alkyle en C₁ à C₈, alkoxy en C₁ à C₈, alkylamino en C₁ à C₈, di-(alkyle en C₁ à C₈)-amino, alkylthio en C₁ à C₈, alcénylthio en C₂ à C₅, alcynylthio en C₂ à C₅, cycloalkylthio en C₃ à C₇, un groupe phényle, phénoxy ou phénylthio éventuellement substitué par un radical halogéno, nitro, cyano, alkoxy en C₁ à C₄, halogénalkoxy en C₁ à C₄, alkylthio en C₁ à C₄, halogénalkylthio en C₁ à C₄, alkyle en C₁ à C₄, halogénalkyle en C₁ à C₄,
R⁶ et R⁷ représentent, indépendamment l'un de l'autre, un groupe, éventuellement substitué par un halogène, alkyle en C₁ à C₂₀, alkoxy en C₁ à C₂₀, alcényle en C₂ à C₈, (alkoxy en C₁ à C₂₀)-(alkyle en C₁ à C₂₀), un groupe phényle éventuellement substitué par un radical halogéno, halogénalkyle en C₁ à C₂₀, alkyle en C₁ à C₂₀ ou alkoxy en C₁ à C₂₀, un groupe benzyle éventuellement substitué par un radical halogéno, alkyle en C₁ à C₂₀, halogénalkyle en C₁ à C₂₀ ou alkoxy en C₁ à C₂₀ ou forment ensemble un noyau alkylène en C₂ à C₆ éventuellement interrompu par de l'oxygène,
à l'exception des composés suivants :
3-(2-méthoxyphényl)-4-hydroxy-Δ³-dihydrofurannone-2,
3-(2-chlorophényl)-4-hydroxy-Δ³-dihydrofurannone-2,
3-(2-fluorophényl)-4-hydroxy-Δ³-dihydrofurannone-2,
ainsi que les formes énantiomères pures de composés de formule (I).

2. Compositions insecticides et acaricides, caractérisées par une teneur en au moins un dérivé de 3-aryl-4-hydroxy-Δ³-dihyrofurannone ou de 3-aryl-4-hydroxy-Δ³-dihydrothiophénone de formule générale (I) suivant la revendication 1,
dans laquelle
X est un groupe alkyle en C₁ à C₄, un halogène, un groupe alkoxy en C₁ à C₄ ou halogénalkyle en C₁ ou C₂,
Y représente de l'hydrogène, un groupe alkyle en C₁ à C₆, un halogène, un groupe alkoxy en C₁ à C₄, halogénalkyle en C₁ ou C₂,
Z est un groupe alkyle en C₁ à C₄, un halogène, un groupe alkoxY en C₁ à C₄,
n est un nombre de 0 à 3,
ou dans laquelle les restes X et Z forment, conjointement avec le reste phényle auquel ils sont liés, le reste naphtalène de formule 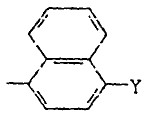 dans laquelle Y a la définition indiquée ci-dessus,
A et B sont identiques ou différents et représentent de l'hydrogène ou un groupe, éventuellement substitué par un halogène, alkyle en C₁ à C₁₀, alcényle en C₃ à C₆, alcynyle en C₃ à C₆, (alkoxy en C₁ à C₈)-(alkyle en C₂ à C₆), poly-(alkoxy en C₂ à C₆)-(alkyle en C₂ à C₆), (alkylthio en C₁ à C₈)-(alkyle en C₂ à C₆), linéaire ou ramifié, cycloalkyle à noyau de 3 à 7 atomes, qui peut être interrompu par 1 ou 2 atomes d'oxygène et/ou de soufre et un groupe phényle ou phényl-(alkyle en C₁ à C₄) éventuellement substitué par un halogène, un radical alkyle en C₁ à C₄, halogénalkyle en C₁ à C₄, alkoxy en C₁ à C₄), nitro,
ou dans laquelle
A et B forment, conjointement avec l'atome de carbone auquel ils sont liés, un noyau de 3 à 8 chaînons saturé ou non saturé, éventuellement interrompu par de l'oxygène et/ou du soufre et éventuellement substitué par un radical halogéno, alkyle en C₁ à C₅, alkoxy en C₁ à C₅, halogénalkyle en C₁ à C₃, halogénalkoxy en C₁ à C₄, alkylthio en C₁ à C₃ ou phényle éventuellement substitué par un radical halogéno, alkyle, alkoxy, ou condensé, le cas échéant, à du benzène,
D est de l'oxygène ou du soufre,
G est de l'hydrogène (a) ou représente les groupes 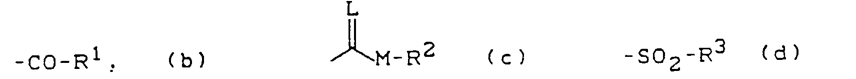 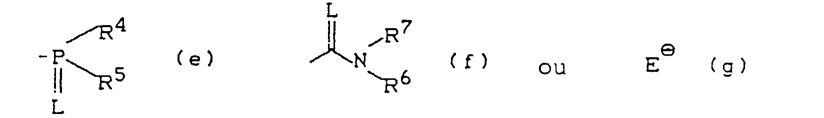
dans lesquels,
E^{⊕} est un équivalent d'ion métallique ou un ion ammonium,
L et M représentent de l'oxygène et/ou du soufre,
R¹ est un groupe, éventuellement substitué par un halogène : alkyle en C₁ à C₁₆, alcényle en C₁ à C₁₆, (alkoxy en C₁ à C₆)-(alkyle en C₂ à C₆), (alkylthio en C₁ à C₁₆)-(alkyle en C₂ à C₆), poly(alkoxy en C₁ à C₆)-(alkyle en C₂ à C₆) et cycloalkyle à noyau de 3 à 7 atomes, qui peut être interrompu par 1-2 atomes d'oxygène et/ou de soufre,
un groupe phényle éventuellement substitué par un radical halogéno, nitro, alkyle en C₁ à C₄, alkoxy en C₁ à C₄, halogénalkyle en C₁ à C₃, halogénalkoxy en C₁ à C₃ ;
un groupe phényl-(alkyle en C₁ à C₄) éventuellement substitué par un radical halogéno, alkyle en C₁ à C₄, alkoxy en C₁ à C₄, halogénalkyle en C₁ à C₃, halogénalkoxy en C₁ à C₃,
des groupes pyridyle, pyrimidyle, thiazolyle et pyrazolyle éventuellement substitués par un halogène et un radical alkyle en C₁ à C₆,
un groupe phénoxy-(alkyle en C₁ à C₅) éventuellement substitué par un halogène et un radical alkyle en C₁ à C₄,
R² est un groupe, éventuellement substitué par un halogène : alkyle en C₁ à C₁₆, alcényle en C₂ à C₁₆, (alkoxy en C₁ à C₁₆)-(alkyle en C₂ à C₆), poly(alkoxy en C₁ à C₆)-(alkyle en C₂ à C₆),
un groupe phényle ou benzyle éventuellement substitué par un radical halogéno, nitro, alkyle en C₁ à C₄, alkoxy en C₁ à C₃, halogénalkyle en C₁ à C₃,
R³, R⁴ et R⁵ représentent, indépendamment les uns des autres, un groupe, éventuellement substitué par un halogène, alkyle en C₁ à C₆, alkoxy en C₁ à C₆, alkylamino en C₁ à C₆, di-(alkyle en C₁ à C₆)-amino, alkylthio en C₁ à C₆, alcénylthio en C₃ ou C₄, alcynylthio en C₂ à C₄, cycloalkylthio en C₃ à C₆, un groupe phényle, phénoxy ou phénylthio éventuellement substitué par un radical fluoro, chloro, bromo, nitro, cyano, alkoxy en C₁ à C₃, halogénalkoxy en C₁ à C₃, alkylthio en C₁ à C₃, halogénalkylthio en C₁ à C₃, alkyle en C₁ à C₃, halogénalkyle en C₁ à C₃,
R⁶ et R⁷ représentent, indépendamment l'un de l'autre, un groupe, éventuellement substitué par un halogène, alkyle en C₁ à C₂₀, alkoxy en C₁ à C₂₀, alcényle en C₂ à C₈, (alkoxy en C₁ à C₂₀)-(alkyle en C₁ à C₂₀), un groupe phényle éventuellement substitué par un radical halogéno, halogénalkyle en C₁ à C₅, alkyle en C₁ à C₅ ou alkoxy en C₁ à C₅, un groupe benzyle éventuellement substitué par un radical halogène, un radical alkyle en C₁ à C₅, halogénalkyle en C₁ à C₅ ou alkoxy en C₁ à C₅,
à l'exception des composés suivants :
3-(2-méthoxyphényl)-4-hydroxy-Δ³-dihydrofurannone-2,
3-(3-chlorophényl)-4-hydroxy-Δ³-dihydrofurannone-2,
3-(2-fluorophényl)-4-hydroxy-Δ³-dihydrofurannone-2,
ainsi que les formes énantiomères pures de composés de formule (I).

3. Compositions insecticides et acaricides, caractérisées par une teneur en au moins un dérivé de 3-aryl-4-hydroxy-Δ³-dihyrofurannone ou de 3-aryl-4-hydroxy-Δ³-dihydrothiophénone de formule générale (I) suivant la revendication 1, dans laquelle
X représente les groupes méthyle, éthyle, propyle, isopropyle, le fluor, le chlore, le brome, les groupes méthoxy, éthoxy et trifluorométhyle,
Y représente de l'hydrogène, les groupes méthyle, éthyle, propyle, isopropyle, butyle, isobutyle, tertio-butyle, le fluor, le chlore, le brome, les groupes méthoxy, éthoxy et trifluorométhyle,
Z représente les groupes méthyle, éthyle, isopropyle, butyle, isobutyle, tertio-butyle, le fluor, le chlore, le brome, les groupes méthoxy et éthoxy,
n est un nombre de 0 à 3,
ou dans laquelle les restes X et Z forment, conjointement avec le reste phényle auquel ils sont liés, le reste naphtalène de formule 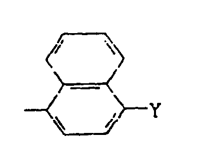
dans laquelle Y a la définition indiquée ci-dessus,
A et B sont identiques ou différents et représentent de l'hydrogène, un groupe, éventuellement substitué par un halogène, alkyle en C₁ à C₈, alcényle en C₃ ou C₄, alcynyle en C₃ ou C₄, (alkoxy en C₁ à C₆)-(alkyle en C₂ à C₄), poly(alkoxy en C₁ à C₄)-(alkyle en C₂ à C₄), (alkylthio en C₁ à C₆)-(alkyle en C₂ à C₄), linéaire ou ramifié, cycloalkyle à noyau de 3 à 6 atomes, qui peut être interrompu par 1-2 atomes d'oxygène et/ou de soufre ou un groupe phényle ou phényl-(alkyle en C₁ à C₃) éventuellement substitué par du fluor, du chlore, du brome, un radical méthyle, éthyle, propyle, isopropyle, méthoxy, éthoxy, trifluoro-méthyle, nitro,
ou dans laquelle
A et B forment, conjointement avec l'atome de carbone auquel ils sont liés, un noyau de 3 à 8 chaînons saturé ou non saturé, éventuellement interrompu par de l'oxygène et/ou du soufre et substitué, le cas échéant, par du fluor, du chlore, un radical alkyle en C₁ à C₄, alkoxy en C₁ à C₄, trifluorométhyle, alkylthio en C₁ ou C₂ ou par un radical phényle éventuellement substitué par du fluor, du chlore, un radical méthyle, méthoxy, ou condensé, le cas échéant, à du benzène,
D représente de l'oxygène ou du soufre,
G représente de l'hydrogène (a) ou les groupes 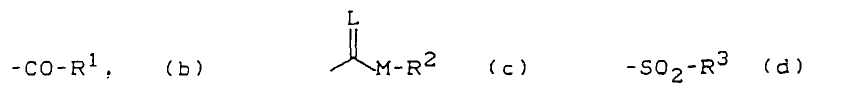 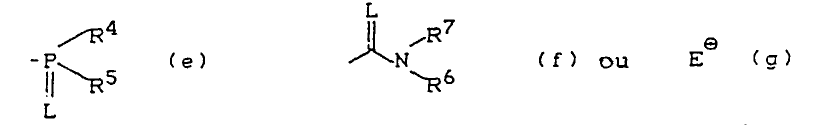
dans lesquels,
E^{⊕} est un équivalent d'un ion métallique ou un ion ammonium,
L et M représentent de l'oxygène et/ou du soufre,
R¹ est un groupe, éventuellement substitué par du fluor ou du chlore, alkyle en C₁ à C₁₄, alcényle en C₂ à C₁₄, (alkoxy en C₁ à C₄)- (alkyle en C₂ à C₆), (alkylthio en C₁ à C₄)-(alkyle en C₂ à C₆), poly(alkoxy en C₁ à C₄)-(alkyle en C₂ à C₄) et cycloalkyle à noyau de 3 à 6 atomes, qui peut être interrompu par 1-2 atomes d'oxygène et/ou de soufre,
un groupe phényle éventuellement substitué par du fluor, du chlore, du brome, un radical méthyle, éthyle, propyle, isopropyle, méthoxy, éthoxy, trifluorométhyle, trifluorométhoxy, nitro,
un groupe phényl-(alkyle en C₁ à C₃) éventuellement substitué par du fluor, du chlore, du brome, un radical méthyle, éthyle, propyle, isopropyle, méthoxy, éthoxy, trifluorométhyle, trifluorométhoxy,
un groupe pyridyle, pyrimidyle, thiazolyle et pyrazolyle, éventuellement substitué par du fluor, du chlore, du brome, un radical méthyle, éthyle,
un groupe phénoxy-(alkyle en C₁ à C₄) éventuellement substitué par du fluor, du chlore, un radical méthyle, éthyle,
R² est un groupe alkyle en C₁ à C₁₄ alcényle en C₂ à C₁₄, (alkoxy en C₁ à C₄)-(alkyle en C₂ à C₆), poly(alkoxy en C₁ à C₄)-(alkyle en C₂ à C₆), éventuellement substitué par du fluor ou du chlore,
ou un groupe phényle ou benzyle éventuellement substitué par du fluor, du chlore, un radical nitro, méthyle, éthyle, propyle, isopropyle, méthoxy, éthoxy, trifluorométhyle,
R³, R⁴ et R⁵ représentent, indépendamment les uns des autres, un groupe, éventuellement substitué par du fluor ou du chlore, alkyle en C₁ à C₄, alkoxy en C₁ à C₄, alkylamino en C₁ à C₄, di-(alkyle en C₁ à C₄)amino, alkylthio en C₁ à C₄, phényle, phénoxy ou phénylthio éventuellement substitué par du fluor, du chlore, du brome, un radical nitro, cyano, alkoxy en C₁ à C₂, fluoralkoxy en C₁ à C₄, chloralkoxy en C₁ ou C₂, alkylthio en C₁ ou C₂, fluoralkylthio en C₁ ou C₂, chloralkylthhio en C₁ ou C₂, alkyle en C₁ à C₃,
R⁶ et R⁷ représentent, indépendamment l'un de l'autre, un groupe, éventuellement substitué par du fluor, du chlore ou du brome, alkyle en C₁ à C₁₀, alkoxy en C₁ à C₁₀, (alkoxy en C₁ à C₁₀)-(alkyle en C₁ à C₁₀), un groupe phényle éventuellement substitué par du fluor, du chlore ou du brome, un radical halogénalkyle en C₁ à C₂₀, alkyle en C₁ à C₂₀ ou alkoxy en C₁ à C₄, un groupe benzyle éventuellement substitué par du fluor, du chlore, du brome, un radical alkyle en C₁ à C₄, halogénalkyle en C₁ à C₄ ou alkoxy en C₁ à C₄,
à l'exception des composés suivants :
3-(2-méthoxyphényl)-4-hydroxy-Δ³-dihydrofurannone-2,
3-(2-chlorophényl)-4-hydroxy-Δ³-dihydrofurannone-2,
3-(2-fluorophényl)-4-hydroxy-Δ³-dihydrofurannone-2,
ainsi que les formes énantiomères pures de composés de formule (I).

4. Procédé pour combattre des insectes et/ou des acariens, caractérisé en ce qu'on fait agir des dérivés de 3-aryl-4-hydroxy-Δ³-dihydrofurannone ou de 3-aryl-4-hydroxy-Δ³-dihydrothiophénone de formule (I) suivant la revendication 1 sur les insectes et/ou les acariens et/ou sur leur milieu.

5. Utilisation de dérivés de 3-aryl-4-hydroxy-Δ³-dihydrofurannone ou de 3-aryl-4-hydroxy-Δ³-dihydrothiophénone de formule (I) suivant la revendication 1 pour combattre des insectes et/ou des acariens.

6. Procédé de préparation de compositions insecticides et/ou acaricides, caractérisé en ce qu'on mélange des dérivés de 3-aryl-4-hydroxy-Δ³-dihydrofurannone ou de 3-aryl-4-hydroxy-Δ³-dihydrothiophénone de formule (I) suivant la revendication 1 avec des diluants et/ou des agents tensio-actifs.

7. Dérivés de 3-aryl-4-hydroxy-Δ³-dihydrofurannone et de 3-aryl-4-hydroxy-Δ³-dihydrothiophénone de formule générale (I) 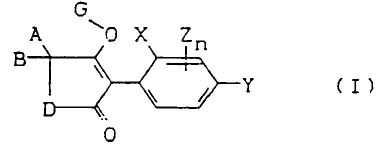 dans laquelle
X représente un groupe alkyle en C₁ à C₆, un halogène, un groupe alkoxy en C₁ à C₆ ou halogénalkyle en C₁ à C₃,
Y représente de l'hydrogène, un groupe alkyle en C₁ à C₆, un halogène, un groupe alkoxy en C₁ à C₆, halogénalkyle en C₁ à C₃,
z est un groupe alkyle en C₁ à C₆, un halogène, un groupe alkoxy en C₁ à C₆.
n est un nombre de 0 à 3,
ou dans laquelle les restes X et Z forment conjointement avec le reste phényle auquel ils sont liés, le reste naphtalène de formule 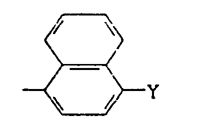 dans laquelle Y a la définition indiquée ci-dessus,
A et B sont identiques ou différents et représentent de l'hydrogène ou un groupe, éventuellement substitué par un halogène, alkyle en C₁ à C₁₂, un groupe alcényle en C₃ à C₈, alcynyle en C₃ à C₈, (alkoxy en C₁ à C₁₀)-(alkyle en C₂ à C₈), poly-(alkoxy en C₁ à C₈)-(alkyle en C₂ à C₈), (alkylthio en C₁ à C₁₀)-(alkyle en C₂ à C₈), linéaire ou ramifié, cycloalkyle à noyau de 3 à 8 atomes, qui peut être interrompu par de l'oxygène et/ou du soufre, et phényle ou phényl-(alkyle en C₁ à C₆) éventuellement substitué par un halogène, un radical alkyle en C₁ à C₆, halogénalkyle en C₁ à C₆, alkoxy en C₁ à C₆, halogénalkoxy en C₁ à C₆, nitro,
ou dans laquelle
A et B forment, conjointement avec l'atome de carbone auquel ils sont liés, un noyau de 3 à 8 chaînons saturé ou non saturé, éventuellement interrompu par de l'oxygène et/ou du soufre et substitué, le cas échéant par un halogène, un radical alkyle en C₁ à C₆, alkoxy en C₁ à C₆, halogénalkyle en C₁ à C₄, halogénalkoxy en C₁ à C₄, alkylthio en C₁ à C₄ ou phényle éventuellement substitué, ou, le cas échéant, condensé à du benzène,
D représente de l'oxygène ou du soufre,
G est de l'hydrogène (a) ou représente les groupes 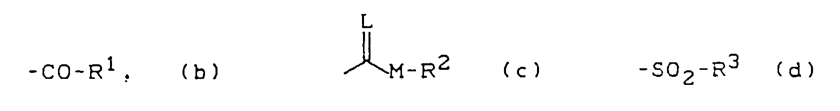 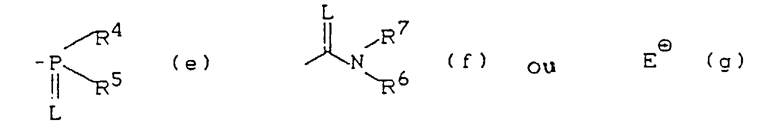
dans lesquels,
E^{⊕} est un équivalent d'ion métallique ou un ion ammonium,
L et M représentent de l'oxygène et/ou du soufre,
R¹ est un groupe, éventuellement substitué par un halogène : alkyle en C₁ à C₂₀, alcényle en C₂ à C₂₀, (alkoxy en C₁ à C₈)-(alkyle en C₂ à C₈), (alkylthio en C₁ à C₈)-(alkyle en C₂ à C₈), poly(alkoxy en C₁ à C₈)-(alkyle en C₂ à C₈) ou cycloalkyle à noyau de 3 à 8 atomes, qui peut être interrompu par des atomes d'oxygène et/ou de soufre,
un groupe phényle éventuellement substitué par un radical halogéno, nitro, alkyle en C₁ à C₆, alkoxy en C₁ à C₆, halogénalkyle en C₁ à C₆, halogénalkoxy en C₁ à C₆ ;
un groupe phényl-(alkyle en C₁ à C₆) éventuellement substitué par un halogène, un radical alkyle en C₁ à C₆, alkoxy en C₁ à C₆, halogénalkyle en C₁ à C₆, halogénalkoxy en C₁ à C₆,
des groupes pyridyle, pyrimidyle, thiazolyle et pyrazolyle portant éventuellement un substituant halogéno et/ou alkyle en C₁ à C₆,
un groupe phénoxy-(alkyle en C₁ à C₆) éventuellement substitué par un halogène et/ou un radical alkyle en C₁ à C₆,
R² est un groupe, éventuellement substitué par un halogène : alkyle en C₁ à C₂₀, alcényle en C₂ à C₂₀, (alkoxy en C₁ à C₈)-(alkyle en C₂ à C₈), poly(alkoxy en C₁ à C₈)-(alkyle en C₂ à C₈),
un groupe phényle ou benzyle éventuellement substitué par un radical halogéno, nitro, alkyle en C₁ à C₆, alkoxy en C₁ à C₆, halogénalkyle en C₁ à C₆,
R³, R⁴ et R⁵ représentent, indépendamment les uns des autres, un groupe, éventuellement substitué par un halogène, alkyle en C₁ à C₈, alkoxy en C₁ à C₈, alkylamino en C₁ à C₈, di-(alkyle en C₁ à C₈)-amino, alkylthio en C₁ à C₈, alcénylthio en C₃ ou C₅, alcynylthio en C₂ à C₅, cycloalkylthio en C₃ à C₇, un groupe phényle, phénoxy ou phénylthio éventuellement substitué par un radical halogéno, nitro, cyano, alkoxy en C₁ à C₄, halogénalkoxy en C₁ à C₄, alkylthio en C₁ à C₄, halogénalkylthio en C₁ à C₄, alkyle en C₁ à C₄, halogénalkyle en C₁ à C₄,
R⁶ et R⁷ représentent, indépendamment l'un de l'autre, un groupe, éventuellement substitué par un halogène, alkyle en C₁ à C₂₀, alkoxy en C₁ à C₂₀, alcényle en C₂ à C₈, (alkoxy en C₁ à C₂₀)-(alkyle en C₁ à C₂₀), un groupe phényle éventuellement substitué par un radical halogéno, halogénalkyle en C₁ à C₂₀, alkyle en C₁ à C₂₀ ou alkoxy en C₁ à C₂₀, un groupe benzyle éventuellement substitué par un radical halogéno, alkyle en C₁ à C₂₀, halogénalkyle en C₁ à C₂₀ ou alkoxy en C₁ à C₂₀, ou forment ensemble un noyau alkylène en C₂ à C₆ éventuellement interrompu par de l'oxygène,
ainsi que les formes énantiomères pures de composés de formule (I),
à l'exception des composés suivants :
3-(2-méthoxyphényl)-4-hydroxy-Δ³-dihydrofurannone-2,
3-(2-chlorophényl)-4-hydroxy-Δ³-dihydrofurannone-2,
3-(2-fluorophényl)-4-hydroxy-Δ³-dihydrofurannone-2,
et sous réserve que X ne représente pas un halogène ou un groupe halogénométhyle ayant 1 à 3 atomes d'halogènes identiques ou différents en même temps que Y est de l'hydrogène et que n est égal à 0 lorsque D représente de l'oxygène en même temps que R¹ est un groupe alkylcarbonyle ou un groupe halogénalkylcarbonyle et en même temps que A et B représentent, indépendamment l'un de l'autre, de l'hydrogène, un groupe alkyle ou bien un groupe phényle ou un groupe phénylalkyle dont chacun est non substitué ou porte un ou plusieurs substituants identiques ou différents, en choisissant comme substituants dans la partie phényle, les substituants : nitro, halogéno, alkyle, alkoxy, halogénalkyle ou halogénalkoxy.

8. Dérivés de 3-aryl-4-hydroxy-Δ³-dihydrofurannone et de 3-aryl-4-hydroxy-Δ³-dihydrothiophénone de formule (I) suivant la revendication 7, caractérisés en ce qu'il s'agit de l'une des structures (Ia) à (Ig) suivantes : 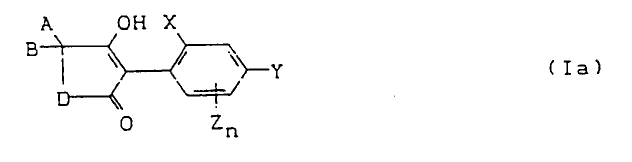 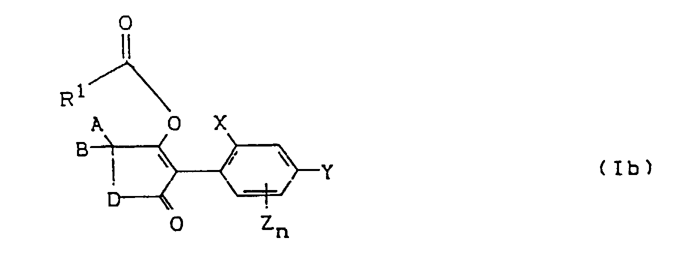 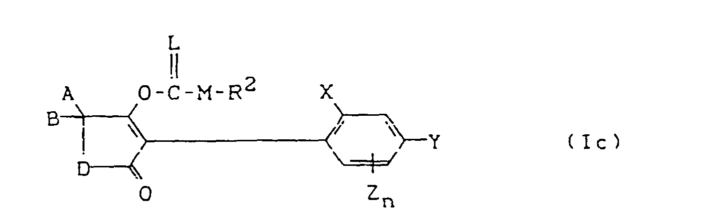 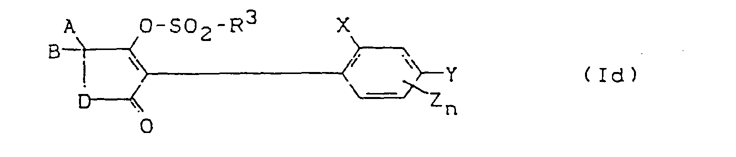 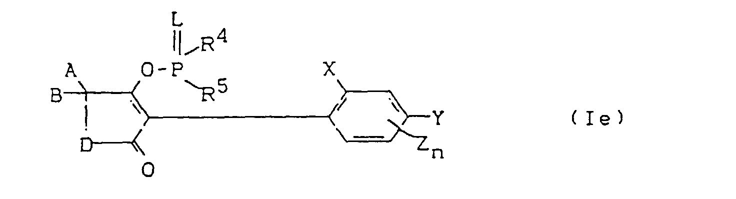 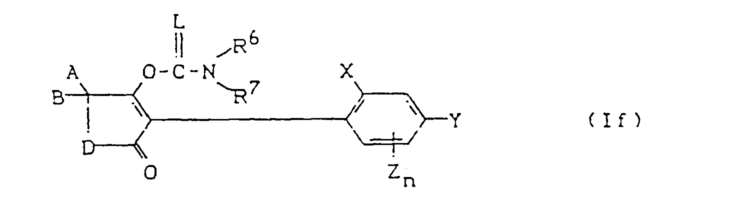 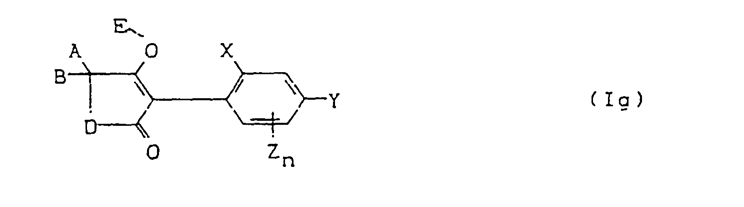 où
A, B, D, E, L, M, X, Y, Zₙ, R¹, R², R³, R⁴, R⁵, R⁶ et R⁷ ont les définitions indiquées dans la revendication 7.

9. Dérivés de 3-aryl-4-hydroxy-Δ³-dihydrofurannone et de 3-aryl-4-hydroxy-Δ³-dihydrothiophénone de formule (I) suivant la revendication 7, dans laquelle
X représente un groupe alkyle en C₁ à C₄, un halogène, un groupe alkoxy en C₁ à C₄ ou halogénalkyle en C₁ ou C₂,
Y est de l'hydrogène, un groupe alkyle en C₁ à C₆, un halogène, un groupe alkoxy en C₁ à C₄, halogénalkyle en C₁ ou C₂,
Z est un groupe alkyle en C₁ à C₄, un halogène, un groupe alkoxy en C₁ à C₄,
n est un nombre de 0 à 3,
ou dans laquelle les restes X et Z forment, conjointement avec le reste phényle auquel ils sont liés, le reste naphtalène de formule 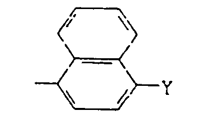 dans laquelle Y a la définition indiquée ci-dessus,
A et B sont identiques ou différents et représentent de l'hydrogène ou un groupe, éventuellement substitué par un halogène, alkyle en C₁ à C₁₀, alcényle en C₃ à C₆, alcynyle en C₃ à C₆, (alkoxy en C₁ à C₈)-(alkyle en C₂ à C₆), poly-(alkoxy en C₁ à C₆)-(alkyle en C₂ à C₆), (alkylthio en C₁ à C₈)-(alkyle en C₂ à C₆), linéaire ou ramifié, cycloalkyle à noyau de 3 à 7 atomes, qui peut être interrompu par 1 ou 2 atomes d'oxygène et/ou de soufre, et un groupe phényle ou phényl-(alkyle en C₁ à C₄) éventuellement substitué par un radical halogéno, alkyle en C₁ à C₄, halogénalkyle en C₁ à C₄, alkoxy en C₁ à C₄, nitro,
ou dans laquelle
A et B forment, conjointement avec l'atome de carbone auquel ils sont liés, un noyau de 3 à 8 atomes saturé ou non saturé, éventuellement interrompu par de l'oxygène et/ou du soufre et éventuellement substitué par un halogène, un radical alkyle en C₁ à C₅, alkoxy en C₁ à C₅, halogénalkyle en C₁ à C₃, halogénalkoxy en C₁ à C₄, alkylthio en C₁ à C₃ ou phényle éventuellement substitué par un radical halogéno, alkyle, alkoxy, ou, le cas échéant, condensé à du benzène,
D est de l'oxygène ou du soufre,
G représente de l'hydrogène (a) ou les groupes 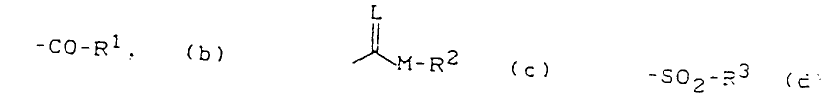 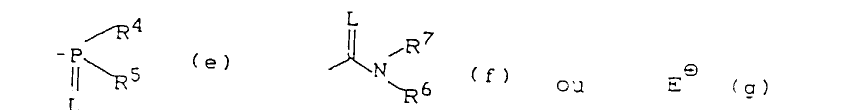
dans lesquels,
E^{⊕} représente un équivalent d'ion métallique ou un ion ammonium,
L et M représentent de l'oxygène et/ou du soufre,
R¹ est un groupe, éventuellement substitué par un halogène : alkyle en C₁ à C₁₆, alcényle en C₂ à C₁₆, (alkoxy en C₁ à C₆)-(alkyle en C₂ à C₆), (alkylthio en C₁ à C₁₆)-(alkyle en C₂ à C₆), poly(alkoxy en C₁ à C₆)-(alkyle en C₂ à C₆) et cycloalkyle à noyau de 3 à 7 atomes, qui peut être interrompu par 1 ou 2 atomes d'oxygène et/ou de soufre,
un groupe phényle éventuellement substitué par un radical halogéno, nitro, alkyle en C₁ à C₄, alkoxy en C₁ à C₄, halogénalkyle en C₁ à C₃, halogénalkoxy en C₁ à C₃ ;
un groupe phényl-(alkyle en C₁ à C₄) éventuellement substitué par un radical halogéno, alkyle en C₁ à C₄, alkoxy en C₁ à C₄, halogénalkyle en C₁ à C₃, halogénalkoxy en C₁ à C₃,
des groupes pyridyle, pyrimidyle, thiazolyle et pyrazolyle éventuellement substitués par un halogène et un radical alkyle en C₁ à C₆,
un groupe phénoxy-(alkyle en C₁ à C₅) éventuellement substitué par un halogène et un radical alkyle en C₁ à C₄,
R² est un groupe, éventuellement substitué par un halogène : alkyle en C₁ à C₁₆, alcényle en C₂ à C₁₆, (alkoxy en C₁ à C₁₆)-(alkyle en C₂ à C₆), poly(alkoxy en C₁ à C₆)-(alkyle en C₂ à C₆),
un groupe phényle ou benzyle éventuellement substitué par un radical halogéno, nitro, alkyle en C₁ à C₄, alkoxy en C₁ à C₃, halogénalkyle en C₁ à C₃,
R³, R⁴ et R⁵ représentent, indépendamment les uns des autres, un groupe, éventuellement substitué par un halogène, alkyle en C₁ à C₆, alkoxy en C₁ à C₆, alkylamino en C₁ à C₆, di-(alkyle en C₁ à C₆)-amino, alkylthio en C₁ à C₆, alcénylthio en C₃ ou C₄, alcynylthio en C₂ à C₄, cycloalkylthio en C₃ à C₆, un groupe phényle, phénoxy ou phénylthio éventuellement substitué par du fluor, du chlore, du brome, un radical nitro, cyano, alkoxy en C₁ à C₃, halogénalkoxy en C₁ à C₃, alkylthio en C₁ à C₃, halogénalkylthio en C₁ à C₃, alkyle en C₁ à C₃, halogénalkyle en C₁ à C₃,
R⁶ et R⁷ représentent, indépendamment l'un de l'autre, un groupe, éventuellement substitué par un halogène, alkyle en C₁ à C₂₀, alkoxy en C₁ à C₂₀, alcényle en C₂ à C₈, (alkoxy en C₁ à C₂₀)-(alkyle en C₁ à C₂₀), un groupe phényle éventuellement substitué par un radical halogéno, halogénalkyle en C₁ à C₅, alkyle en C₁ à C₅ ou alkoxy en C₁ à C₅, un groupe benzyle éventuellement substitué par un radical halogéno, alkyle en C₁ à C₅, halogénalkyle en C₁ à C₅ ou alkoxy en C₁ à C₅,
ainsi que les formes énantiomères pures de composés de formule (I),
à l'exception des composés suivants :
3-(2-méthoxyphényl)-4-hydroxy-Δ³-dihydrofurannone-2,
3-(3-chlorophényl)-4-hydroxy-Δ³-dihydrofurannone-2,
3-(2-fluorophényl)-4-hydroxy-Δ³-dihydrofurannone-2,
et sous réserve que X ne représente pas un halogène ou un groupe halogénométhyle ayant 1 à 3 atomes d'halogènes identiques ou différents en même temps que Y représente de l'hydrogène et que n est le nombre 0 lorsque D représente de l'oxygène en même temps que R¹ est un groupe alkylcarbonyle ou un groupe halogénalkylcarbonyle et en même temps que A et B représentent, indépendamment l'un de l'autre, un groupe phényle ou phénylalkyle dont chacun est non substitué ou porte un ou plusieurs substituants, identiques ou différents, en choisissant comme substituants de la partie phényle, les substituants : nitro, halogéno, alkyle, alkoxy, halogénalkyle ou halogénalkoxy.

10. Dérivés de 3-aryl-4-hydroxy-Δ³-dihydrofurannone et de 3-aryl-4-hydroxy-Δ³-dihydrothiophénone de formule (I) suivant la revendication 7,
dans laquelle
X représente les groupes méthyle, éthyle, propyle, isopropyle, le fluor, le chlore, le brome, les groupes méthoxy, éthoxy et trifluorométhyle,
Y représente de l'hydrogène, les groupes méthyle, éthyle, propyle, isopropyle, butyle, isobutyle, tertio-butyle, le fluor, le chlore, le brome, les groupes méthoxy, éthoxy et trifluorométhyle,
Z représente les groupes méthyle, éthyle, isopropyle, butyle, isobutyle, tertio-butyle, le fluor, le chlore, le brome, les groupes méthoxy et éthoxy,
n représente un nombre de 0 à 3,
ou dans laquelle les restes X et Z forment, conjointement avec le reste phényle auquel ils sont liés, le reste naphtalène de formule 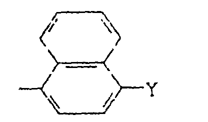 dans laquelle Y a la définition indiquée ci-dessus,
A et B sont identiques ou différents et représentent de l'hydrogène, un groupe, éventuellement substitué par un halogène, alkyle en C₁ à C₈, alcényle en C₃ ou C₄, alcynyle en C₃ ou C₄, (alkoxy en C₁ à C₆)-(alkyle en C₂ à C₄), poly(alkoxy en C₁ à C₄)-(alkyle en C₂ à C₄), (alkylthio en C₁ à C₆)-(alkyle en C₂ à C₄), linéaire ou ramifié, cycloalkyle à noyau de 3 à 6 atomes, qui peut être interrompu par 1 ou 2 atomes d'oxygène et/ou de soufre, ou un groupe phényle ou phényl-(alkyle en C₁ à C₃) éventuellement substitué par du fluor, du chlore, du brome, un radical méthyle, éthyle, propyle, isopropyle, méthoxy, éthoxy, trifluorométhyle, nitro,
ou dans laquelle
A et B forment, conjointement avec l'atome de carbone auquel ils sont liés, un noyau de 3 à 8 chaînons saturé ou non saturé, éventuellement interrompu par de l'oxygène et/ou du soufre et substitué, le cas échéant, par du fluor, du chlore, un radical alkyle en C₁ à C₄, alkoxy en C₁ à C₄, trifluorométhyle, alkylthio en C₁ ou C₂ ou phényle éventuellement substitué par un radical fluoro, chloro, méthyle, méthoxy, ou éventuellement condensé à du benzène,
D représente de l'oxygène ou du soufre,
G est de l'hydrogène (a) ou représente les groupes 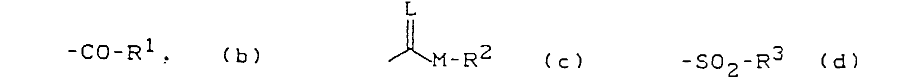 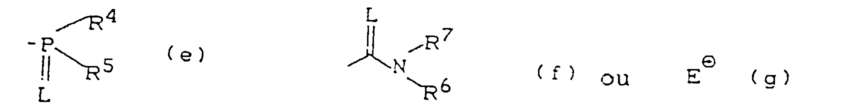
dans lesquels,
E^{⊕} est un équivalent d'un ion métallique ou un ion ammonium,
L et M représentent de l'oxygène et/ou du soufre,
R¹ est un groupe, éventuellement substitué par du fluor ou du chlore, alkyle en C₁ à C₁₄, alcényle en C₂ à C₁₄, (alkoxy en C₁ à C₄) -(alkyle en C₂ à C₆), (alkylthio en C₁ à C₄)-(alkyle en C₂ à C₆), poly(alkoxy en C₁ à C₄)-(alkyle en C₂ à C₄) et cycloalkyle à noyau de 3 à 6 atomes, qui peut être interrompu par 1 ou 2 atomes d'oxygène et/ou de soufre,
un groupe phényle éventuellement substitué par du fluor, du chlore, du brome, un radical méthyle, éthyle, propyle, isopropyle, méthoxy, éthoxy, trifluorométhyle, trifluorométhoxy, nitro,
un groupe phényl-(alkyle en C₁ à C₃) éventuellement substitué par du fluor, du chlore, du brome, un radical méthyle, éthyle, propyle, isopropyle, méthoxy, éthoxy, trifluorométhyle, trifluorométhoxy,
des groupes pyridyle, pyrimidyle, thiazolyle et pyrazolyle éventuellement substitués par du fluor, du chlore, du brome, un radical méthyle, éthyle,
un groupe phénoxy-(alkyle en C₁ à C₄) éventuellement substitué par un radical fluoro, chloro, méthyle, éthyle,
R² est un groupe, éventuellement substitué par du fluor ou du chlore, alkyle en C₁ à C₁₄, alcényle en C₂ à C₁₄, (alkoxy en C₁ à C₄)-(alkyle en C₂ à C₆), poly(alkoxy en C₁ à C₄)-(alkyle en C₂ à C₆),
ou un groupe phényle ou un groupe benzyle éventuellement substitué par du fluor, du chlore, un radical nitro, méthyle, éthyle, propyle, isopropyle, méthoxy, éthoxy, trifluorométhyle,
R³, R⁴ et R⁵ représentent, indépendamment les uns des autres, un groupe, éventuellement substitué par du fluor ou du chlore, alkyle en C₁ à C₄, alkoxy en C₁ à C₄, alkylamino en C₁ à C₄, di-(alkyle en C₁ à C₄) amino, alkylthio en C₁ à C₄, un groupe phényle, phénoxy ou phénylthio éventuellement substitué par du fluor, du chlore, du brome, un radical nitro, cyano, alkoxy en C₁ ou C₂, fluoralkoxy en C₁ à C₄, chloralkoxy en C₁ ou C₂, alkylthio en C₁ ou C₂, fluoralkylthio en C₁ ou C₂, chloralkylthio en C₁ ou C₂, alkyle en C₁ à C₃,
R⁶ et R⁷ représentent, indépendamment l'un de l'autre, un groupe, éventuellement substitué par du fluor, du chlore ou du brome, alkyle en C₁ à C₁₀, alkoxy en C₁ à C₁₀, (alkoxy en C₁ à C₁₀)-(alkyle en C₁ à C₁₀), un groupe phényle éventuellement substitué par du fluor, du chlore, du brome, un radical halogénalkyle en C₁ à C₂₀, alkyle en C₁ à C₂₀ ou alkoxy en C₁ à C₄, un groupe benzyle éventuellement substitué par du fluor, du chlore, du brome, un radical alkyle en C₁ à C₄, halogénalkyle en C₁ à C₄ ou alkoxy en C₁ à C₄,
ainsi que les formes énantiomères pures de composés de formule (I),
à l'exception des composés suivants :
3 -(2-méthoxyphényl)-4-hydroxy-Δ³-dihydrofurannone-2,
3-(2-chlorophényl)-4-hydroxy-Δ³-dihydrofurannone-2,
3-(2-fluorophényl)-4-hydroxy-Δ³-dihydrofurannone-2,
et sous réserve que X ne représente pas un halogène ou un groupe halogénométhyle ayant 1 à 3 atomes d'halogènes identiques ou différents, en même temps que Y est de l'hydrogène et que n est le nombre 0 lorsque D est de l'oxygène en même temps que R¹ est un groupe alkylcarbonyle ou un groupe halogénalkylcarbonyle et en même temps que A et B représentent, indépendamment l'un de l'autre, de l'hydrogène, un groupe alkyle ou un groupe phényle ou phénylalkyle dont chacun est non substitué ou porte un ou plusieurs substituants identiques ou différents, en choisissant comme substituants de la partie phényle, les substituants : nitro, halogéno, alkyle, alkoxy, halogénalkyle ou halogénalkoxy.

11. Procédé de production de dérivés de 3-aryl-4hydroxy-Δ³-dihydrofurannone et de 3-aryl-4-hydroxy-Δ³-dihydrothiophénone de formule générale (I) suivant la revendication 7 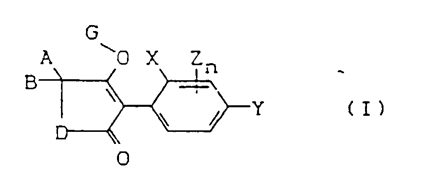 dans laquelle
A, B, D, G, X, Y, Z et n ont la définition indiquée dans la revendication 7,
caractérisé en ce que pour obtenir des dérivés de 3-aryl-4-hydroxy-Δ³-dihydrofurannone et de 3-aryl-4-hydroxy-Δ³-dihydrothiophénone de formule (Ia) 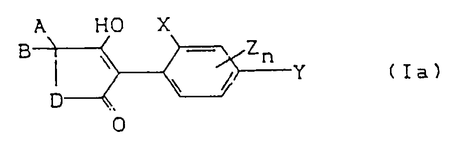 dans laquelle
A, B, D, X, Y, Z et n ont la définition indiquée ci-dessus,
(A) on effectue la condensation intramoléculaire d'esters d'acide carboxylique de formule (II) 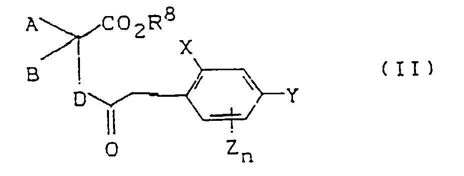 dans laquelle
A, B, D, X, Y, Z et n ont la définition indiquée ci-dessus
et
R⁸ est un groupe alkyle,
en présence d'un diluant et en présence d'une base,
ou bien
(B) pour l'obtention de composés de formule (Ib) 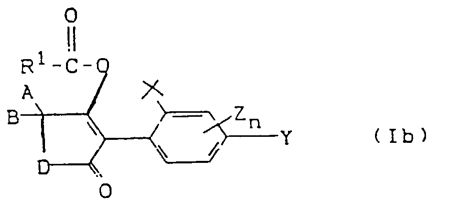 dans laquelle
A, B, D, X, Y, Z, R¹ et n ont la définition indiquée ci-dessus,
on fait réagir des composés de formule (Ia) 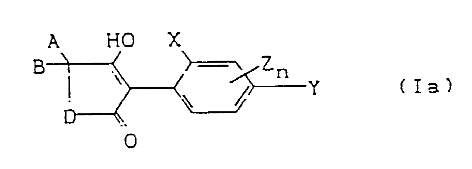 dans laquelle
A, B, D, X, Y, Z et n ont la définition indiquée ci-dessus,
α) avec des halogénures d'acides de formule générale (III) 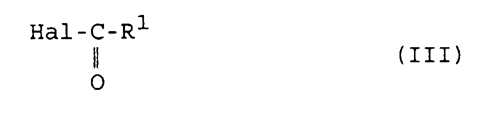 dans laquelle
R¹ a la définition indiquée ci-dessus et
Hal représente un halogène, notamment le chlore et le brome,
éventuellement en présence d'un diluant et en la présence éventuelle d'un accepteur d'acide,
ou bien
β) avec des anhydrides d'acides carboxyliques de formule générale (IV)
R¹-CO-O-CO-R¹ (IV)
dans laquelle
R¹ a la définition indiquée ci-dessus,
le cas échéant, en présence d'un diluant et en la présence éventuelle d'un accepteur d'acide,
ou bien
(C) pour obtenir des composés de formule (Ic) 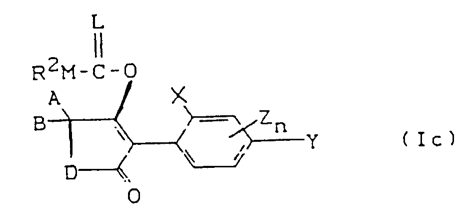 dans laquelle
A, B, D, X, Y, Z, R² et n ont la définition indiquée ci-dessus,
L est de l'oxygène
et
M est de l'oxygène ou du soufre,
on fait réagir des composés de formule (Ia) 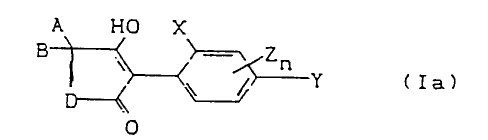
dans laquelle
A, B, D, X, Y, Z et n ont la définition indiquée ci-dessus, avec un ester d'acide chloroformique ou un thioester d'acide
chloroformique de formule générale (V)
R²-M-CO-Cl (V)
dans laquelle
R² et M ont la définition indiquée ci-dessus,
le cas échéant en présence d'un diluant et en la présence éventuelle d'un accepteur d'acide, ou bien
(D) pour obtenir des composés de formule (Ic) 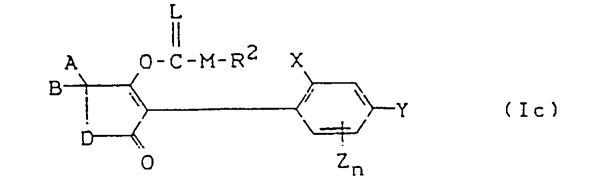 dans laquelle
A, B, D, R², X, Y, Z et n ont la définition indiquée ci-dessus,
L représente du soufre
et
M est de l'oxygène ou du soufre,
on fait réagir des composés de formule (Ia) 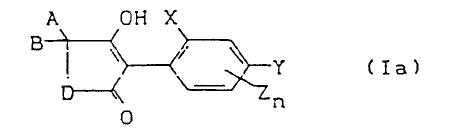
dans laquelle
A, B, D, X, Y, Z et n ont la définition indiquée ci-dessus
α) avec des esters d'acide chloromonothioformique ou des esters d'acide chlorodithioformique de formule générale (VI) 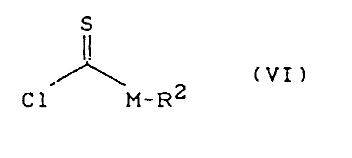 dans laquelle
M et R² ont la définition indiquée ci-dessus,
le cas échéant en présence d'un diluant et en la présence éventuelle d'un accepteur d'acide,
ou bien
β) avec le sulfure de carbone puis avec des halogénures d'alkyle de formule générale (VII)
R²-Hal (VII)
dans laquelle
_{R}2 a la définition indiquée ci-dessus
et
Hal représente du chlore, du brome, de l'iode,
ou bien
(E) pour obtenir des composés de formule (Id) 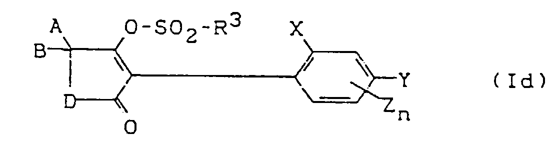 dans laquelle
A, B, D, X, Y, Z, R³ et n ont la définition indiquée ci-dessus,
on fait réagir des composés de formule (Ia) 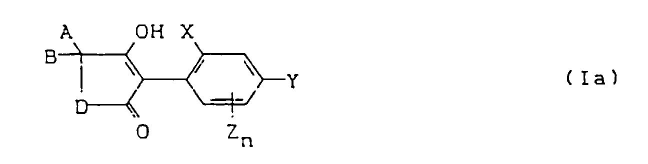 dans laquelle
A, B, D, X, Y, Z et n ont la définition indiquée ci-dessus, avec des chlorures d'acide sulfonique de formule générale (VIII)
R³-SO₂-Cl (VIII)
dans laquelle
R³ a la définition indiquée ci-dessus,
le cas échéant en présence d'un diluant et en la présence éventuelle d'un accepteur d'acide,
ou bien
(F) pour obtenir des composés de formule (le) 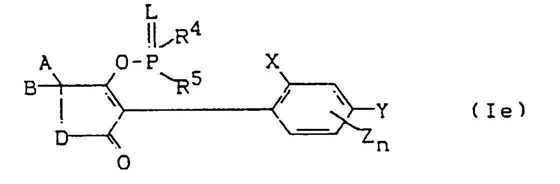 dans laquelle
A, B, D, L, X, Y, Z, R⁴, R⁵ et n ont la définition indiquée ci-dessus,
on fait réagir des composés de formule (Ia) 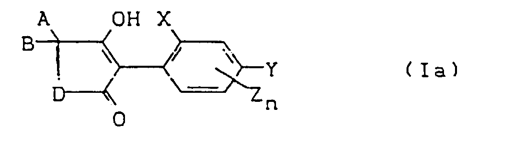 dans laquelle
A, B, D, X, Y, Z et n ont la définition indiquée ci-dessus, avec des composés de phosphore de formule générale (IX) 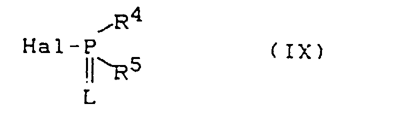 dans laquelle
L, R⁴ et R⁵ ont la définition indiquée ci-dessus,
et
Hal représente un halogène,
le cas échéant en présence d'un diluant et en la présence éventuelle d'un accepteur d'acide, ou bien
(G) pour obtenir des composés de formule (If) 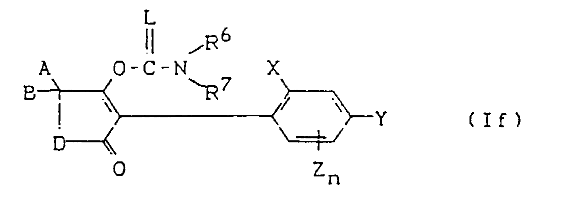 dans laquelle
A, B, D, L, X, Y, Z, R⁶, R⁷ et n ont la définition indiquée ci-dessus,
on fait réagir des composés de formule (Ia) 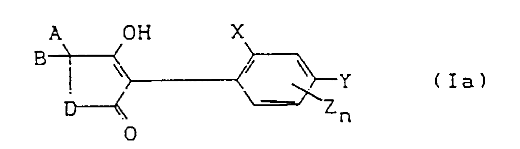 dans laquelle
A, B, D, X, Y, Z et n ont la définition indiquée ci-dessus
α) avec des isocyanates de formule générale (X)
R⁶-N=C=O (X)
dans laquelle
R⁶ a la définition indiquée ci-dessus
le cas échéant en présence d'un diluant et en la présence éventuelle d'un catalyseur,
ou bien
β avec des chlorures d'acides carbamiques ou des chlorures d'acides thiocarbamiques de formule générale (XI) 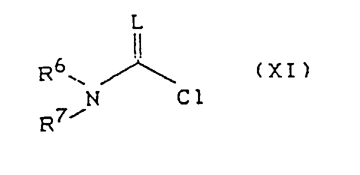 dans laquelle
L, R⁶ et R⁷ ont la définition indiquée ci-dessus,
le cas échéant en présence d'un diluant et en la présence éventuelle d'un accepteur d'acide,
ou bien
(H) pour obtenir des composés de formule (Ig) 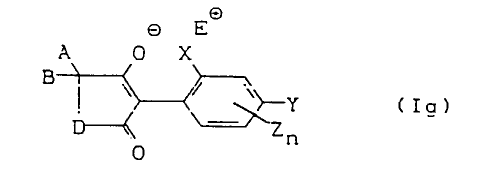 dans laquelle
X, Y, Z, A, B, D et n ont la définition indiquée ci-dessus et E^{⊕} représente un équivalent d'ion métallique ou un ion ammonium,
on fait réagir des composés de formule (Ia) 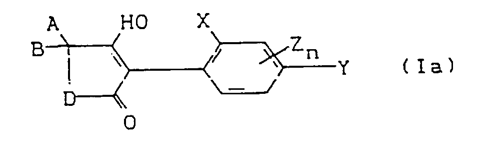 dans laquelle
X, Y, Z, A, B, D et n ont la définition indiquée ci-dessus, avec des hydroxydes métalliques ou des amines de formules générales (XII) et (XIII) 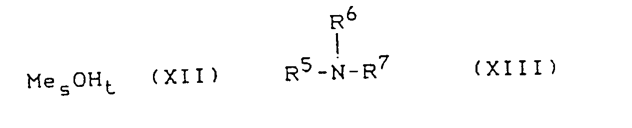 dans lesquelles
Me représente des ions métalliques monovalents ou divalents
s et t représentent le nombre 1 ou 2 et
R⁵, R⁶ et R⁷ représentent, indépendamment les uns des autres,
de l'hydrogène et un groupe alkyle,
le cas échéant en présence d'un diluant.

12. Compositions herbicides, caractérisées par une teneur en au moins un dérivé de 3-aryl-4-hydroxy-Δ³-dihydrofurannone ou de 3-aryl-4-hydroxy-Δ³-dihydrothiophénone de formule (I) suivant la revendication 7.

13. Procédé pour combattre des mauvaises herbes, caractérisé en ce qu'on fait agir des dérivés de 3-aryl-4-hydroxy-Δ³-dihydrofurannone ou de 3-aryl-4-hydroxy-Δ³-dihydrothiophénone de formule (I) suivant la revendication 7 sur des mauvaises herbes et/ou sur leur milieu.

14. Utilisation de dérivés de 3-aryl-4-hydroxy-Δ³-dihydrofurannone ou de 3-aryl-4-hydroxy-Δ³-dihydrothiophénone de formule (I) suivant la revendication 7 pour combattre des mauvaises herbes.

15. Procédé de préparation de compositions herbicides, caractérisé en ce qu'on mélange des dérivés de 3-aryl-4-hydroxy-Δ³-dihydrofurannone ou de 3-aryl-4 -hydroxy-Δ³-dihydrothiophénone de formule (I) suivant la revendication 7 avec des diluants et/ou des agents tensio-actifs.
